# EUROPEAN PATENT APPLICATION

(11) **EP 3 575 287 A1**
(43) Date of publication of application: **04.12.2019**
(21) Application number: 18175436.7
(22) Date of filing: 31.05.2018
(51) Int. Cl.: C07D 239/42, C07D 239/48, C07D 471/04, C07D 487/04, C07D 491/04, C07D 495/04, C07D 498/04, C07D 513/04, A61K 31/505, A61K 31/506, A61K 31/519, A61P 25/28, A61P 35/00

(54) **INHIBITORS OF N-ACYLPHOSPHATIDYLETHANOLAMINE PHOSPHOLIPASE D (NAPE-PLD)**

(71) Applicant: Universiteit Leiden, 2311 RA Leiden (NL)
(72) Inventor: The designation of the inventor has not yet been filed
(74) Representative: Arth, Hans-Lothar

(57) **Abstract**

The invention relates to a compound of the formula (I) as novel inhibitor of N-acylphosphatidylethanolamine phospholipase D (NAPE-PLD), and to use thereof for the prophylaxis or treatment of diseases associated with NAPE-PLD. 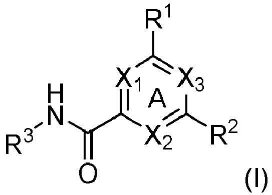 wherein
in a ring **A, X₁** is N, or C**R⁴**; **X₂** is N or C**R⁵**; **X**₃ is N or CH;
with the proviso that at least one of **X₁** and **X₃** is N.

## Description

The invention relates to novel inhibitors of N-acylphosphatidylethanolamine phospholipase D (NAPE-PLD), and to their use for the prophylaxis or treatment of diseases associated with NAPE-PLD.

### Background of the invention

*N*-acylphosphatidylethanolamine phospholipase D (NAPE-PLD) is an enzyme that synthesizes a family of bioactive signaling lipids, called *N*-acylethanolamines (NAEs), from *N*-acylphosphatidylethanolamines (NAPEs). The enzyme has a metallo-β-lactamase fold, making it distinct from the PLD enzyme family (Okamoto et al.; Journal of Biological Chemistry 2004, 279, 5298). A crystal structure of NAPE-PLD revealed that the active site contains two divalent zinc cations with one hydrolytic water molecule in between, all kept in place by several histidine and aspartic acid residues (Magotti et al.; Structure 2015, 23, 598). Furthermore, it was shown that NAPE-PLD forms a homo-dimer that can bind four bile acid molecules in an allosteric site which increase the enzymatic activity. NAPE-PLD associates with membranes and extracts its substrates from the lipid bilayer, converting them to NAEs and phosphatidic acid. Purified NAPE-PLD displays increased activity upon incubation with phosphatidylethanolamine, therefore it is proposed that the enzyme is constitutively active (Wang et al.; Neuropharmacology 2008, 54, 8).

NAEs may exert their biological effects through several protein classes, including G protein-coupled receptors (e.g. cannabinoid receptors type 1 and type 2, GPR110), ion channels (e.g. transient potential receptor vanilloid 1, N-Methyl-D-aspartate (NMDA)-receptor, GABA-receptors), nuclear receptors (e.g peroxisome proliferator-activated receptor (PPAR) α, β/δ and γ) that are involved in a multitude of biological processes, such as, but not limited to, neurotransmission, immunomodulation, energy balance, mood, motor coordination, addiction, pain sensation, appetite, inflammation, neurodegeneration and anxiety (Hussain et al.; Biochimica et Biophysica Acta (BBA) - Molecular and Cell Biology of Lipids 2017, 1862, 1546). For example, oleoylethanolamide (OEA) has been identified as a satiety factor by activating PPAR-α (Fu et al.; Nature 2003, 425, 90). Arachidonoylethanolamide (AEA) or anandamide is classified as an endocannabinoid and endovanilloid, activating cannabinoid receptors and TRPV1, respectively (Maccarrone, M.; Frontiers in Molecular Neuroscience 2017, 10*;* Van der Stelt et al.; European Journal of Biochemistry 2004, 271, 1827).

NAPE-PLD null mice display decreased levels of saturated, mono- and polyunsaturated NAEs, although AEA levels did not show a reduction in all NAPE-PLD^{-/-} models (Leishman et al.; Biochimica et Biophysica Acta (BBA) - Molecular and Cell Biology of Lipids 2016, 1861, 491; Leung, D.,.et al. Biochemistry 2006, 45, 4720; Tsuboi, K. et al. Biochim Biophys Acta 2011 1811, 565). This has prompted the discovery of alternative NAE biosynthetic pathways that can act as a compensatory mechanism (Blankman et al.; Pharmacological Reviews 2013, 65, 849). Since AEA is regarded to be generated "on demand", acute and temporal inhibition of its biosynthetic enzyme NAPE-PLD could shed more light on the relevance of this pathway. To date, no potent and *in vivo* active inhibitors have been reported for NAPE-PLD. Only compounds with micromolar activity have been disclosed so far (Castellani et al., Chem. Commun., 2017, 53, 12814; Petersen et al. Chem Phys Lipids , 2009, 162, 53; Scott, S. A. et al. ACS Chem Biol, 2015, 10, 421). NAPE-PLD inhibitors have the potential to modulate NAE signaling via various biological pathways, including CB1, CB2, TRPV1 or PPAR-α receptors that are involved in pathological processes such as neurodegenerative diseases, inflammatory or neuropathic pain, obesity and metabolic syndrome, mental health disorders and cancer (Pacher et al.; Pharmacological Reviews 2006, 58, 389; Chiurchiu et al.; Progress in Neurobiology 2018, 160, 82; Hansen et al.; British Journal of Pharmacology 2018). In addition, NAPE-PLD inhibition allows elevation of NAPE or plasmalogen NAPE lipids, which were shown to be neuroprotective and reduce food intake (Wellner et al.; Biochimica et Biophysica Acta (BBA) - Molecular and Cell Biology of Lipids 2013, 1831, 652).

The objective of the present invention is to provide novel N-acylphosphatidylethanolamine phospholipase D (NAPE-PLD) inhibitors and methods for the synthesis therof as well as their use for the prophylaxis and treatment of diseases associated with NAPE-PLD.

Said objective is solved by the technical teachings of the independent claims. Further advantageous embodiments, aspects and details of the invention are evident from the dependent claims, the description and the examples.

Surprisingly, it was found that the compounds of the present invention are potent inhibitors for the enzyme NAPE-PLD.

Thus, the present invention relates to a compound of the general formula (I): 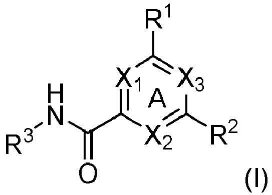 wherein
in a ring **A**, **X₁** is N, or C**R⁴**; **X₂** is N or C**R⁵**; **X₃** is N or CH; with the proviso that at least one of **X₁** and **X₃** is N;
**R¹** represents -**R⁶**, -O**R⁷**, -S**R⁷**, -N**R⁸R⁹**, or -CON**R⁸R⁹**, or **R¹** and **R⁴** form 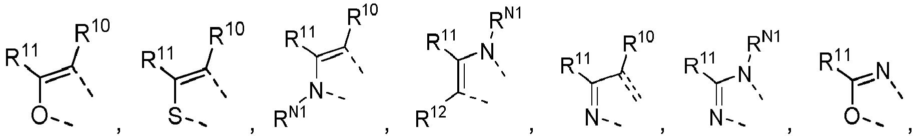 or 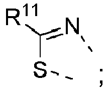
**R²** represents -**R¹⁶**, -O**R¹⁷**, -S**R¹⁷**, -N**R¹⁸R¹⁹**, or -CON**R¹⁸R¹⁹**, -N**R¹⁸**CH**R**CO₂**R¹⁹**,
or **R²** and **R⁵** form 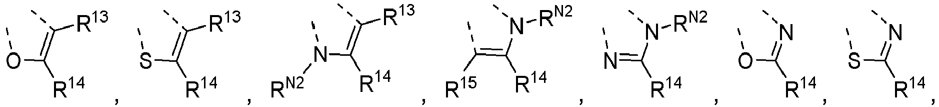 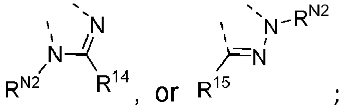
**R³** represents C₁-C₆ alkyl, C₁-C₆ alkyl-OH, C₁-C₆ alkyl-CO₂**R²⁰**, C₁-C₆ alkyl-CONH**R²⁰**, C₂-C₆ alkenyl, C₂-C₆ alkynyl, 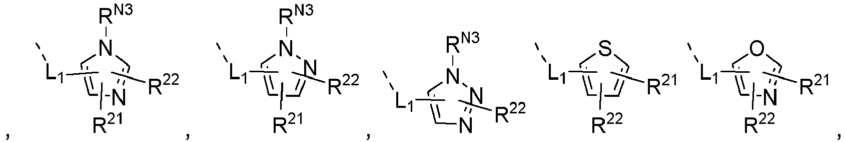 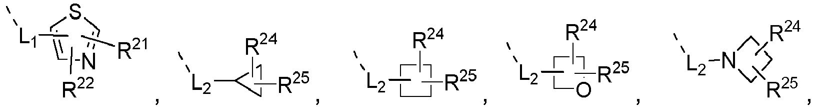 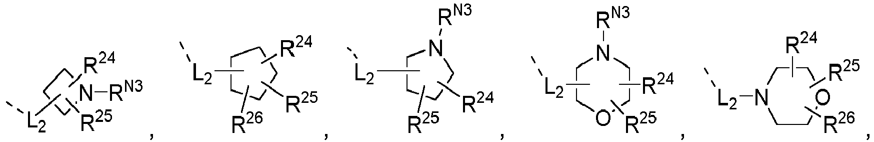 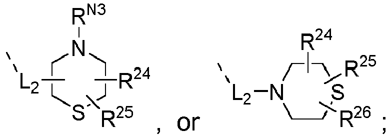
**R⁴** and **R⁵** represents independently of each other -H, -F, -CI, -Br, -I, C₁-C₃ alkyl, C₁-C₃ alkoxy, -CF₃, -OCF₂H or -OCF₃;
**R⁶**, **R⁷** and **R⁸** represent independently of each other, C₁- C₆ alkyl, C₃-C₆ cycloalkyl, -L₃-**R^{A}**, -L4-**R^{B}**;
, **R^{8'}**, **R⁹**, **R^{9'}**, **R¹⁰**, and **R¹¹** represent independently of each other, -H, C₁- C₆ alkyl, C₃-C₆ cycloalkyl, -L₃-**R^{A}**, -L₄-**R^{B}**;
**R^{A}** and **R^{B}** represent independently of each other 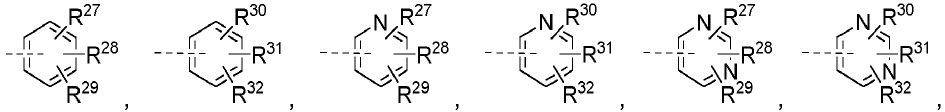 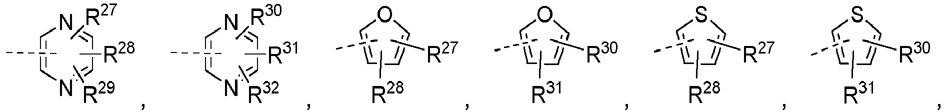 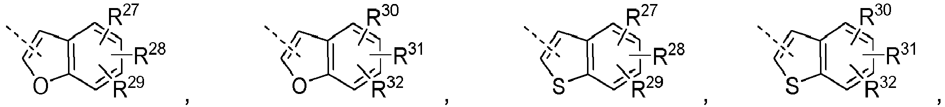 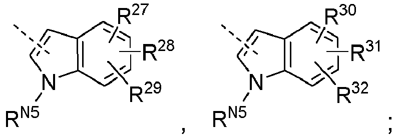 or -N**R⁸R⁹** represents 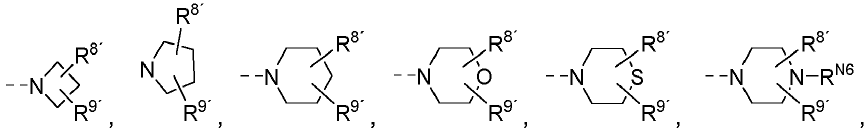 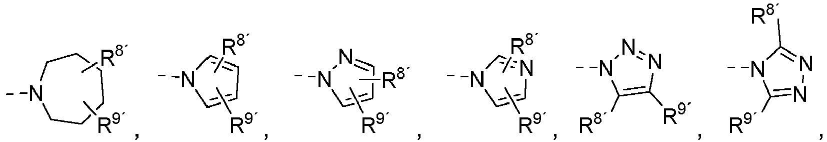 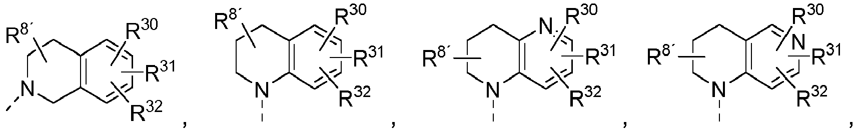 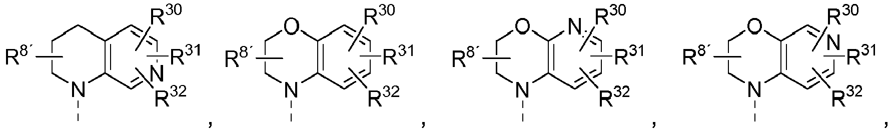 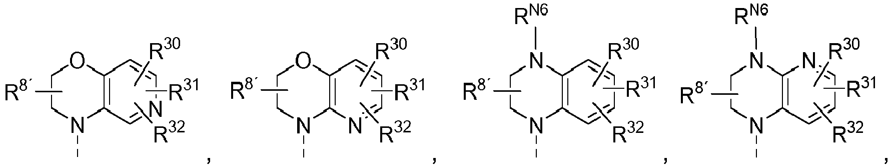 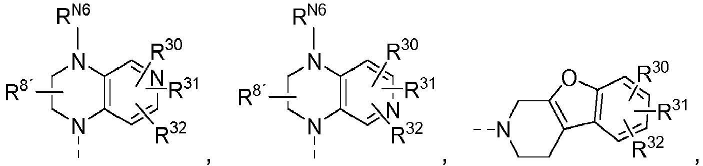 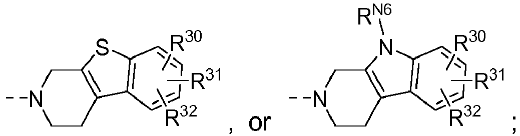
**R¹²**, **R¹³**, **R¹⁴** and **R¹⁵** represent independently of each other -H, -Cl, -F, -CF₃, C₁-C₃ alkyl, or C₁-C₃ alkoxy;
**R¹⁶**, **R¹⁷**, **R¹⁸** and **R¹⁹** represent independently of each other C₁-C₆ alkyl, C₃-C₆ cycloalkyl, C₁-C₆ alkylOH, C₁-C₆ alkylCO₂**R^{20'}**, 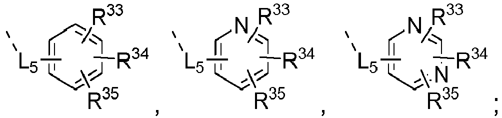 or -N**R¹⁸R¹⁹** represents 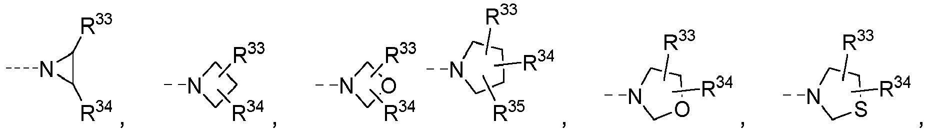 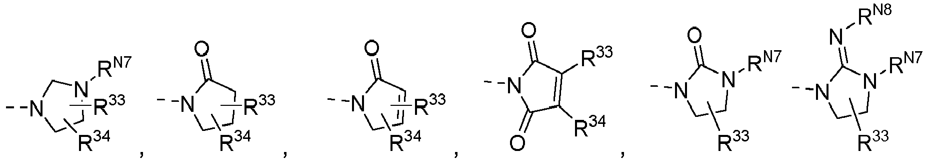 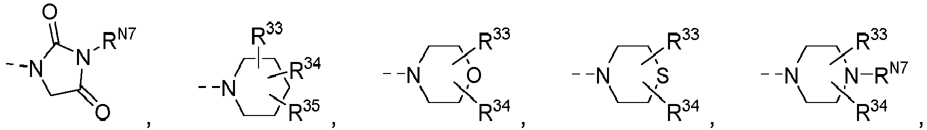 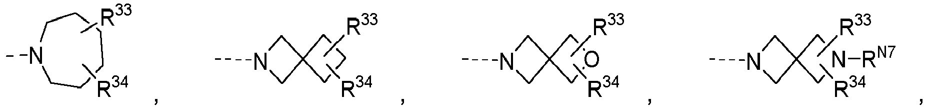 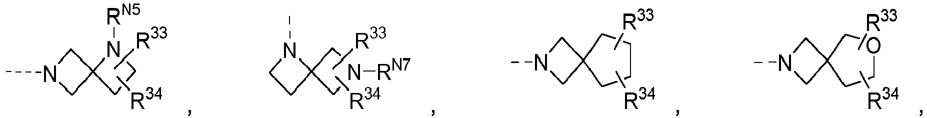 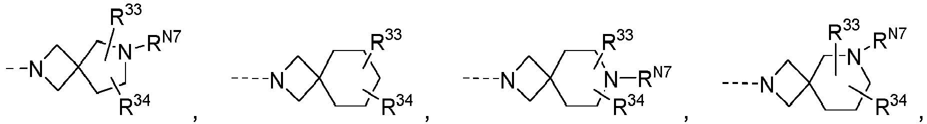 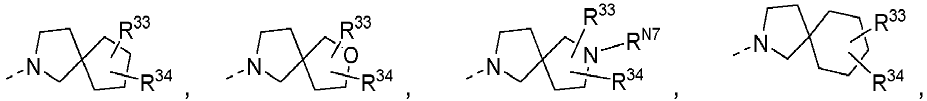 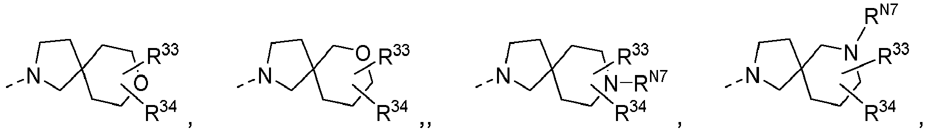 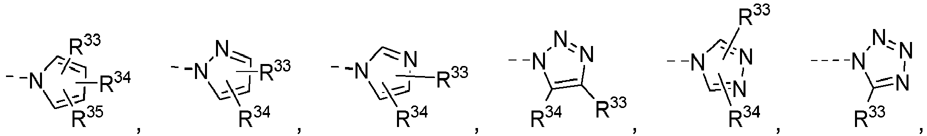 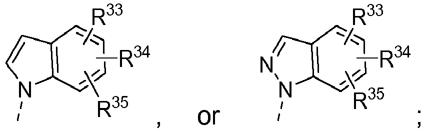
**R** represents -H, -CH₃, -CH₂OH, -CH₂SH, -CH₂CO₂H, -CH₂CONH₂, -CH(OH)CH₃, -CH(CH₃)₂, -CH₂CH(CH₃)₂, -CH(CH₃)CH₂CH₃, -CH₂CH₂SCH₃, -CH₂CH₂CO₂H, -CH₂CH₂CONH₂, -CH₂Ph, -CH₂CH₂CH₂NHC(=NH)NH₂, -CH₂CH₂CH₂CH₂NH₂, 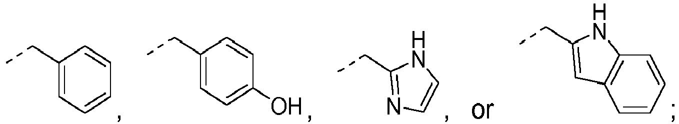
**L¹**, **L²**, **L³**, **L⁴** and **L⁵** represent independently of each other a covalent bond, C₁-C₆ alkyl group, linear C₂-C₆ alkenyl group, wherein one or more of -CH₂- groups present in the C₁-C₆ alkyl, or C₂-C₆ alkenyl group are optionally and independently of each other replaced by -O-, -S-, -N(R^{N9})-, -CO-, -COO-, -OOC-, -CON(R^{N9})-, -N(R^{N9})CO-, -NHCON(R^{N9})-, -N(R^{N9})CONH-, -SO-, -SO₂- , -SO₂N(R^{N9})-, or -N(R^{N9})SO₂-;
**R^{N1}**, **R^{N2}**, **R^{N3}**, **R^{N4}**, **R^{N5}**, **R^{N6}**, **R^{N7}**, **R^{N8}**, and **R^{N9}** represent independently of each other -H, -CH₃, -C₂H₅, -C₃H₇, -CH(CH₃)₂, -C₄H₉, -CH₂-CH(CH₃)₂, -CH(CH₃)-C₂H₅, -C(CH₃)₃, -cyclo-C₃H₅, -CH₂-cyclo-C₃H₅, -Ph, -CH₂Ph, -CH₂F, -CHF₂, -CF₃, -CH₂Cl, -CH₂Br, -CH₂I, -CH₂-CH₂F, -CH₂-CHF₂, -CH₂-CF₃, -CH₂-CH₂Cl, -CH₂-CH₂Br, -CH₂-CH=CH₂, -CH₂-C≡CH, -OH, -OCH₃, -CHO, -COCH₃, -COC₂H₅, -COC₃H₇, -COCH(CH₃)₂, -COC(CH₃)₃, -COOCH₃, - COOC₂H₅, -COOC₃H₇, -COOCH(CH₃)₂, -COOC(CH₃)₃, 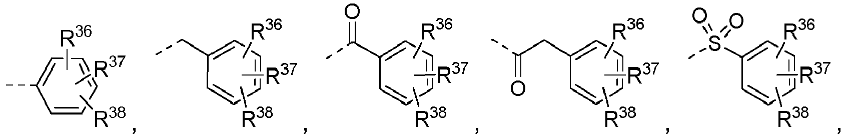 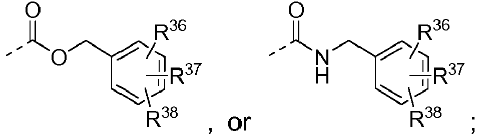
**R²⁰** and **R^{20'}** represent independently of each other -H, -CH₃, -C₂H₅, -C₃H₇, -CH(CH₃)₂, -C₄H₉, -CH₂-CH(CH₃)₂, -CH(CH₃)-C₂H₅, -C(CH₃)₃, -cyclo-C₃H₅, or-CH₂-cyclo-C₃H₅;
**R²¹, R²², R²³, R²⁴, R²⁵, R²⁶, R²⁷, R²⁸, R²⁹, R³⁰, R³¹, R³², R³³, R³⁴, R³⁵, R³⁶, R³⁷,** and **R³⁸** represent independently of each other -H, -OH, -OCH₃, -OC₂H₅, -OC₃H₇, -O-cyclo-C₃H₅, -OCH(CH₃)₂, -OC(CH₃)₃, -OC₄H₉, -OPh, -OCH₂-Ph, -OCPh₃, -CH₂-OCH₃, -C₂H₄-OCH₃, -C₃H₆-OCH₃, -CH₂-OC₂H₅, -C₂H₄-OC₂H₅, -C₃H₆-OC₂H₅, -CH₂-OC₃H₇, -C₂H₄-OC₃H₇, -C₃H₆-OC₃H₇, -CH₂-O-cyclo-C₃H₅, -C₂H₄-O-cyclo-C₃H₅, -C₃H₆--O-cyclo-C₃H₅, -CH₂-OCH(CH₃)₂, -C₂H₄-OCH(CH₃)₂, -C₃H₆-OCH(CH₃)₂, -CH₂-OC(CH₃)₃, -C₂H₄-OC(CH₃)₃, -c₃H₆-OC(CH₃)₃, -CH₂-OC₄H₉, -C₂H₄-OC₄H₉, -C₃H₆-OC₄H₉, -CH₂-OPh, -C₂H₄-OPh, -C₃H₆-OPh, -CH₂-OCH₂-Ph, -C₂H₄-OCH₂-Ph, -C₃H₆-OCH₂-Ph, -SH, -SCH₃, -SC₂H₅, -SC₃H₇, -S-cyclo-C₃H₅, -SCH(CH₃)₂, -SC(CH₃)₃, -F, -CI, -Br, -I, -CN, -COCH₃, -COC₂H₅, -COC₃H₇, -CO-cyclo-C₃H₅, -COCH(CH₃)₂, -COC(CH₃)₃, -COOH, -COOCH₃, -COOC₂H₅, -COOC₃H₇, -COO-cyclo-C₃H₅, -COOCH(CH₃)₂, -COOC(CH₃)₃, -OOC-CH₃, -OOC-C₂H₅, -OOC-C₃H₇, -OOC-cyclo-C₃H₅, -OOC-CH(CH₃)₂, -OOC-C(CH₃)₃, -CONH₂, -CONHCH₃, -CONHC₂H₅, -CONHC₃H₇, -CONH-cyclo-C₃H₅, -CONH[CH(CH₃)₂], -CONH[C(CH₃)₃], -CON(CH₃)₂, -CON(C₂H₅)₂, -CON(C₃H₇)₂, -CON(cyclo-C₃H₅)₂, -CON[CH(CH₃)₂]₂, -CON[C(CH₃)₃]₂, -NHCOCH₃, -NHCOC₂H₅, -NHCOC₃H₇, -NHCO-cyclo-C₃H₅, -NHCO-CH(CH₃)₂, -NHCO-C(CH₃)₃, -NHCO-OCH₃, -NHCO-OC₂H₅, -NHCO-OC₃H₇, -NHCO-O-cyclo-C₃H₅, -NHCO-OCH(CH₃)₂, -NHCO-OC(CH₃)₃, -NH₂, -NHCH₃, -NHC₂H₅, -NHC₃H₇, -NH-cyclo-C₃H₅, -NHCH(CH₃)₂, -NHC(CH₃)₃, -N(CH₃)₂, -N(C₂H₅)₂, -N(C₃H₇)₂, -N(cyclo-C₃H₅)₂, -N[CH(CH₃)₂]₂, -N[C(CH₃)₃]₂, -SOCH₃, -SOC₂H₅, -SOC₃H₇, -SO-cyclo-C₃H₅, -SOCH(CH₃)₂, -SOC(CH₃)₃, -SO₂CH₃, -SO₂C₂H₅, -SO₂C₃H₇, -SO₂-cyclo-C₃H₅, -SO₂CH(CH₃)₂, -SO₂C(CH₃)₃, -SO₃H, -SO₃CH₃, -SO₃C₂H₅, -SO₃C₃H₇, -SO₃-cyclo-C₃H₅, -SO₃CH(CH₃)₂, -SO₃C(CH₃)₃, -SO₂NH₂, -SO₂NHCH₃, -SO₂NHC₂H₅, -SO₂NHC₃H₇, -SO₂NH-cyclo-C₃H₅, -SO₂NHCH(CH₃)₂, -SO₂NHC(CH₃)₃, -SO₂N(CH₃)₂, -SO₂N(C₂H₅)₂, -SO₂N(C₃H₇)₂, -SO₂N(cyclo-C₃H₅)₂, -SO₂N[CH(CH₃)₂]₂, -SO₂N[C(CH₃)₃]₂, -O-S(=O)CH₃, -O-S(=O)C₂H₅, -O-S(=O)C₃H₇, -O-S(=O)-cyclo-C₃H₅, -O-S(=O)CH(CH₃)₂, -O-S(=O)C(CH₃)₃, -S(=O)(=NH)CH₃, -S(=O)(=NH)C₂H₅, -S(=O)(=NH)C₃H₇, -S(=O)(=NH)-cyclo-C₃H₅, -S(=O)(=NH)CH(CH₃)₂, -S(=O)(=NH)C(CH₃)₃, -NH-SO₂-CH₃, -NH-SO₂-C₂H₅, -NH-SO₂-C₃H₇, -NH-SO₂-cyclo-C₃H₅, -NH-SO₂-CH(CH₃)₂, -NH-SO₂-C(CH₃)₃, -O-SO₂-CH₃, -O-SO₂-C₂H₅, -O-SO₂-C₃H₇, -O-SO₂-cyclo-C₃H₅, -O-SO₂-CH(CH₃)₂, -O-SO₂-C(CH₃)₃, -OCH₂F, -OCHF₂ -OCF₃, -CH₂-OCF₃, -C₂H₄-OCF₃, -C₃H₆-OCF₃, -OC₂F₅, -CH₂-OC₂F₅, -C₂H₄-OC₂F₅, -C₃H₆-OC₂F₅, -O-COOCH₃, -O-COOC₂H₅, -O-COOC₃H₇, -O-COO-cyclo-C₃H₅, -O-COOCH(CH₃)₂, -O-COOC(CH₃)₃, -NH-CO-NH₂, -NH-CO-NHCH₃, -NH-CO-NHC₂H₅, -NH-CO-NHC₃H₇, -NH-C(=NH)-NH₂, -NH-CO-N(C₃H₇)₂, -NH-CO-NH[CH(CH₃)₂], -NH-CO-NH[C(CH₃)₃], -NH-CO-N(CH₃)₂, -NH-CO-N(C₂H₅)₂, -NH-CO-NH-cyclo-C₃H₅, -NH-CO-N(cyclo-C₃H₅)₂, -NH-CO-N[CH(CH₃)₂]₂, -NH-C(=NH)-NHCH₃, -NH-C(=NH)-NHC₂H₅, -NH-C(=NH)-NHC₃H₇, -O-CO-NH-cyclo-C₃H₅, -NH-C(=NH)-NH-cyclo-C₃H₅, -NH-C(=NH)-NH[CH(CH₃)₂], -O-CO-NH[CH(CH₃)₂], -NH-C(=NH)-NH[C(CH₃)₃], -NH-C(=NH)-N(CH₃)₂, -NH-C(=NH)-N(C₂H₅)₂, -NH-C(=NH)-N(C₃H₇)₂, -NH-C(=NH)-N(cyclo-C₃H₅)₂, -O-CO-NHC₃H₇, -NH-C(=NH)-N[CH(CH₃)₂]₂, -NH-C(=NH)-N[C(CH₃)₃]₂, -O-CO-NH₂, -O-CO-NHCH₃, -O-CO-NHC₂H₅, -O-CO-NH[C(CH₃)₃], -O-CO-N(CH₃)₂, -O-CO-N(C₂H₅)₂, -O-CO-N(C₃H₇)₂, -O-CO-N(cyclo-C₃H₅)₂, -O-CO-N[CH(CH₃)₂]₂, -O-CO-N[C(CH₃)₃]₂, -O-CO-OCH₃, -O-CO-OC₂H₅, -O-CO-OC₃H₇, -O-CO-O-cyclo-C₃H₅, -O-CO-OCH(CH₃)₂, -O-CO-OC(CH₃)₃, -CH₂F, -CHF₂, -CF₃, -CH₂-CH₂F, -CH₂-CHF₂, -CH₂-CF₃, cyclo-C₈H₁₅, -Ph, -CH₂-Ph, -CH₂-CH₂-Ph, -CH=CH-Ph, -CPh₃, -CH₃, -C₂H₅, -C₃H₇, -CH(CH₃)₂, -C₄H₉, -CH₂-CH(CH₃)₂, -CH(CH₃)-C₂H₅, -C(CH₃)₃, -C₅H₁₁, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, -C₂H₄-CH(CH₃)₂, -C₆H₁₃, -C₇H₁₅, -C₈H₁₇, -C₃H₆-CH(CH₃)₂, -C₂H₄-CH(CH₃)-C₂H₅, -CH(CH₃)-C₄H₉, -CH₂-CH(CH₃)-C₃H₇, -CH(CH₃)-CH₂-CH(CH₃)₂, -CH(CH₃)-CH(CH₃)-C₂H₅, -CH₂-CH(CH₃)-CH(CH₃)₂, -CH₂-C(CH₃)₂-C₂H₅, -C(CH₃)₂-C₃H₇, -C(CH₃)₂-CH(CH₃)₂, -C₂H₄-C(CH₃)₃, -CH(CH₃)-C(CH₃)₃, -CH=CH₂, -CH₂-CH=CH₂, -C(CH₃)=CH₂, -CH=CH-CH₃, -C₂H₄-CH=CH₂, -CH₂-CH=CH-CH₃, -CH=CH-C₂H₅, -CH₂-C(CH₃)=CH₂, -CH(CH₃)-CH=CH, -CH=C(CH₃)₂, -C(CH₃)=CH-CH₃, -CH=CH-CH=CH₂, -C₃H₆-CH=CH₂, -C₂H₄-CH=CH-CH₃, -CH₂-CH=CH-C₂H₅, -CH=CH-C₃H₇, -CH=CH-CH=CH-CH₃, -C₂H₄-C(CH₃)=CH₂, -CH₂-CH(CH₃)-CH=CH₂, -CH(CH₃)-CH₂-CH=CH₂, -CH₂-CH=C(CH₃)₂, -CH₂-C(CH₃)=CH-CH₃, -CH(CH₃)-CH=CH-CH₃, -CH=CH-CH(CH₃)₂, -CH=C(CH₃)-C₂H₅, -C(CH₃)=CH-C₂H₅, -C(CH₃)=C(CH₃)₂, -C(CH₃)₂-CH=CH₂, -CH(CH₃)-C(CH₃)=CH₂, -C₄H₈-CH=CH₂, -C₃H₆-CH=CH-CH₃, -C₂H₄-CH=CH-C₂H₅, -CH₂-CH=CH-C₃H₇, -CH=CH-C₄H₉, -C₃H₆-C(CH₃)=CH₂, -C₂H₄-CH(CH₃)-CH=CH₂, -CH₂-CH(CH₃)-CH₂-CH=CH₂, -C₂H₄-CH=C(CH₃)₂, -CH(CH₃)-C₂H₄-CH=CH₂, -C₂H₄-C(CH₃)=CH-CH₃, -CH₂-CH(CH₃)-CH=CH-CH₃, -CH(CH₃)-CH₂-CH=CH-CH₃, -CH₂-CH=CH-CH(CH₃)₂, -CH₂-CH=C(CH₃)-C₂H₅, -CH₂-C(CH₃)=CH-C₂H₅, -CH(CH₃)-CH=CH-C₂H₅, -CH=CH-CH₂-CH(CH₃)₂, -CH=CH-CH(CH₃)-C₂H₅, -CH=C(CH₃)-C₃H₇, -C(CH₃)=CH-C₃H₇, -CH₂-CH(CH₃)-C(CH₃)=CH₂, -C[C(CH₃)₃]=CH₂, -CH(CH₃)-CH₂-C(CH₃)=CH₂, -CH(CH₃)-CH(CH₃)-CH=CH₂, -CH=CH-C₂H₄-CH=CH₂, -C(CH₃)₂-CH₂-CH=CH₂, -CH₂-C(CH₃)=C(CH₃)₂, -CH(CH₃)-CH=C(CH₃)₂, -C(CH₃)₂-CH=CH-CH₃, -CH=CH-CH₂-CH=CH-CH₃, -CH(CH₃)-C(CH₃)=CH-CH₃, -CH=C(CH₃)-CH(CH₃)₂, -C(CH₃)=CH-CH(CH₃)₂, -C(CH₃)=C(CH₃)-C₂H₅, -CH=CH-C(CH₃)₃, -C(CH₃)₂-C(CH₃)=CH₂, -CH(C₂H₅)-C(CH₃)=CH₂, -C(CH₃)(C₂H₅)-CH=CH₂, -CH(CH₃)-C(C₂H₅)=CH₂, -CH₂-C(C₃H₇)=CH₂, -CH₂-C(C₂H₅)=CH-CH₃, -CH(C₂H₅)-CH=CH-CH₃, -C(C₄Hg)=CH₂, -C(C₃H₇)=CH-CH₃, -C(C₂H₅)=CH-C₂H₅, -C(C₂H₅)=C(CH₃)₂, -C[CH(CH₃)(C₂H₅)]=CH₂, -C[CH₂-CH(CH₃)₂]=CH₂, -C₂H₄-CH=CH-CH=CH₂, -CH₂-CH=CH-CH₂-CH=CH₂, -C₃H₆-C≡C-CH₃, -CH₂-CH=CH-CH=CH-CH₃, -CH=CH-CH=CH-C₂H₅, -CH(CH₃)-CH₂-C≡CH, -CH(CH₃)-C≡C-CH₃, -C₂H₄-CH(CH₃)-C≡CH, -CH=CH-CH=C(CH₃)₂, -CH₂-CH(CH₃)-CH₂-C≡CH, -CH=CH-C(CH₃)=CH-CH₃, -CH=C(CH₃)-CH=CH-CH₃, -CH₂-CH(CH₃)-C≡CH, -C(CH₃)=CH-CH=CH-CH₃, -C≡CH, -C≡C-CH₃, -CH₂-C≡CH, -C₂H₄-C≡CH, -CH₂-C≡C-CH₃, -C≡C-C₂H₅, -C₃H₆-C≡CH, -C₂H₄-C≡C-CH₃, -CH₂-C≡C-C₂H₅, -C≡C-C₃H₇, -CH(CH₃)-C≡CH, -C₄H₈-C≡CH, -C₂H₄-C≡C-C₂H₅, -CH₂-C≡C-C₃H₇, -C≡C-C₄H₉, -C≡C-CH₂-CH(CH₃)₂, -CH(CH₃)-C₂H₄-C≡CH, -CH₂-CH(CH₃)-C≡C-CH₃, -C(CH₃)(C₂H₅)-C≡CH, -CH(CH₃)-CH₂-C≡C-CH₃, -CH(CH₃)-C≡C-C₂H₅, -CH₂-C≡C-CH(CH₃)₂, -C≡C-CH(CH₃)-C₂H₅, -CH₂-C≡C-C≡C-CH₃, -CH(C₂H₅)-C≡C-CH₃, -C(CH₃)₂-C≡C-CH₃, -CH(C₂H₅)-CH₂-C≡CH, -CH₂-CH(C₂H₅)-C≡CH, -C(CH₃)₂-CH₂-C≡CH, -CH₂-C(CH₃)₂-C≡CH, -CH(CH₃)-CH(CH₃)-C≡CH, -CH(C₃H₇)-C≡CH, -CH₂-CH(C≡CH)₂, -C≡C-C≡CH, -CH₂-C≡C-C≡CH, -C≡C-C≡C-CH₃, -CH(C≡CH)₂, -C₂H₄-C≡C-C≡CH, -CH₂-C≡C-CH₂-C≡CH, -C≡C-C₂H₄-C≡CH, -C≡C-C(CH₃)₃, -C≡C-CH₂-C≡C-CH₃, -C≡C-C≡C-C₂H₅, 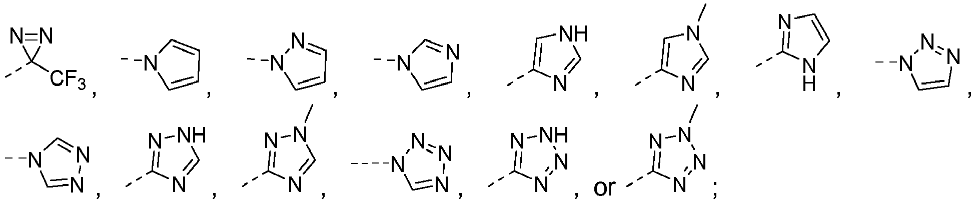
or tautomer, enantiomer, diastereomer, a mixture of tautomers, a mixture of enantiomers, a mixture of diastereomers, or a pharmaceutically acceptable salt thereof.

Preferred is the compound of the fomula (I), wherein the ring **A** represents 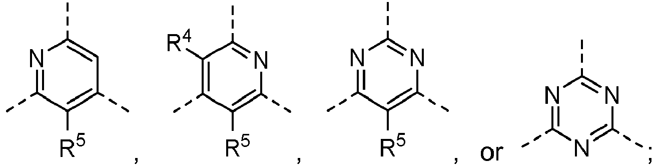 and **R⁴** and **R⁵** have the same meanings as defined in formula (I) or as defined in any preferred selection of substituents disclosed herein.

Preferred are the compounds of any one of the formulae (**II-1**) - (**II-3**): 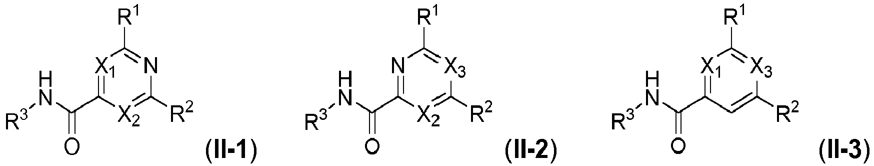 wherein **R¹**, **R²**, **R³**, **X₁**, **X₂** and **X₃** have the same meanings as defined in formula (**I**) or as defined in any preferred selection of substituents disclosed herein.

Preferred are the compounds of any one of the formulae (**III-1**) - (**III-4**): 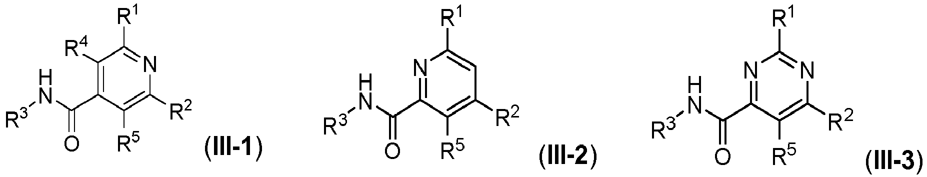 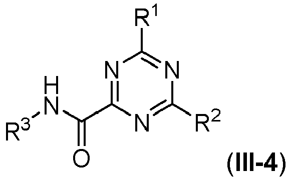 wherein **R¹**, **R²**, **R³**, **R⁴**, and **R⁵** have the same meanings as defined in formula (I) or as defined in any preferred selection of substituents disclosed herein.

Also preffered are the compounds of any one of the formulae (**IV-1**) -(**IV-8**): 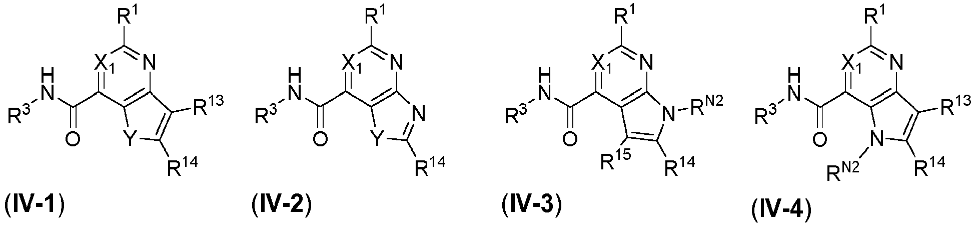 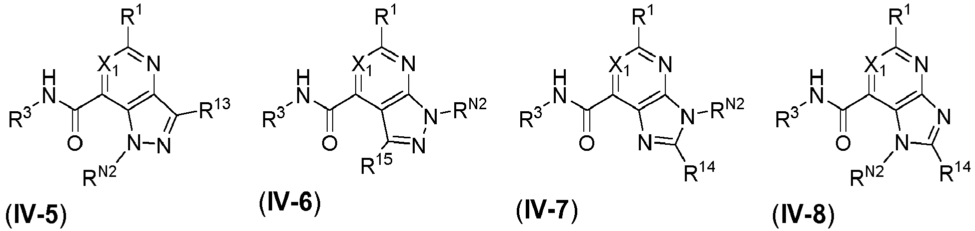 wherein **R²**, **R³**, **R¹⁰**, **R¹¹**, **R¹²**, and **R^{N1}** have the same meanings as defined in formula (I) or as defined in any preferred selection of substituents disclosed herein.

Still preffered are the compounds of any one of the formulae (**V-1**) - (**V-8**): 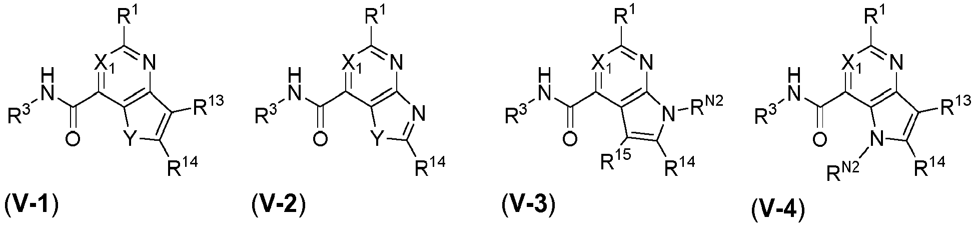 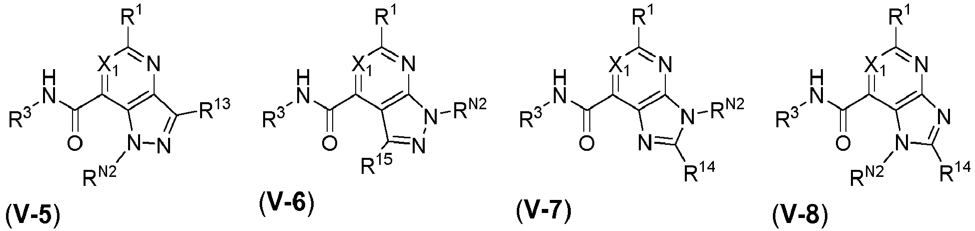
wherein **X₁** represent N or CH;
Y represents O or S; and
**R¹**, **R³**, **R¹³**, **R¹⁴**, **R¹⁵** and **R^{N2}** have the same meanings as defined in formula (**I**) or as defined in any preferred selection of substituents disclosed herein.

More preferred are the compounds of any one of the formulae (**VI-1**) - (**VI-9**):

| | | |
|---|---|---|
| 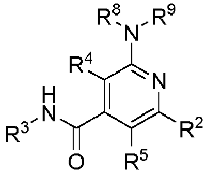 | 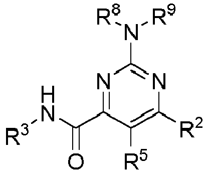 | 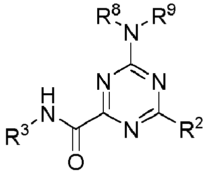 |
| (**VI-1**) | (**VI-2**) | (**VI-3**) |
| 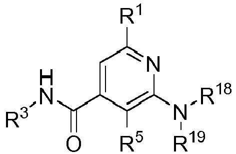 | 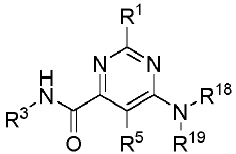 | 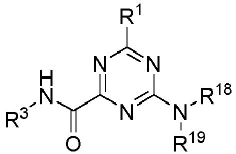 |
| (**VI-4**) | (**VI-5**) | (**VI-6**) |
| 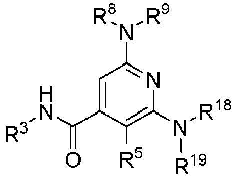 | 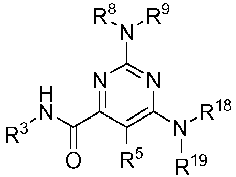 | 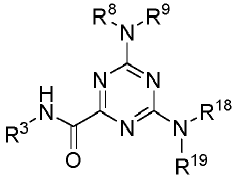 |
| (**VI-7**) | (**VI-8**) | (**VI-9**) |

wherein **R¹**, **R²**, **R³**, **R⁴**, **R⁵**, **R⁸**, **R⁹**, **R¹⁸** and **R¹⁹** have the same meanings as defined in formula (**I**) or as defined in any preferred selection of substituents disclosed herein.

Prererably, in the formulae (**I**), **(II-1)** - (**II-3**), (**III-1**) - (**III-3**), (**IV**-**1**) - (**IV**-**8**), (**V-1**) - (**V-8**), and (**VI-4**) - (**VI-9**),
**L³** and **L⁴** represent independently of each other a covalent bond, -CH₂-, 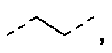 , 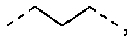 , or 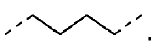 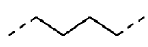 .

More prererably, in the formulae (**I**), (**II-1**) - (**II-3**), (**III-1**) - (**III-3**), (**IV-1**) - (**IV-8**), (**V-1**) - (**V-8**), and (**VI-4**) - (**VI-9**), **R¹** represents 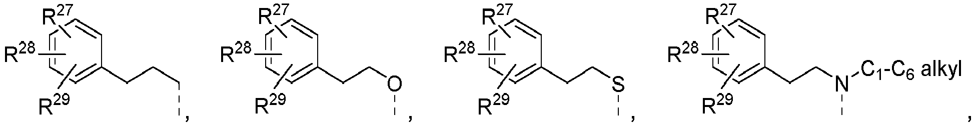 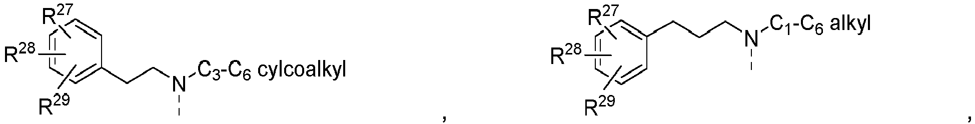 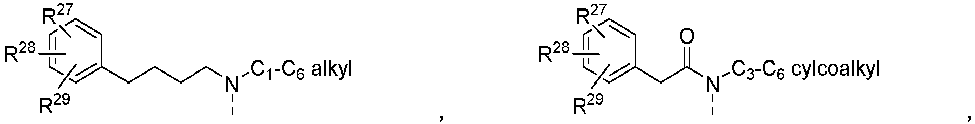 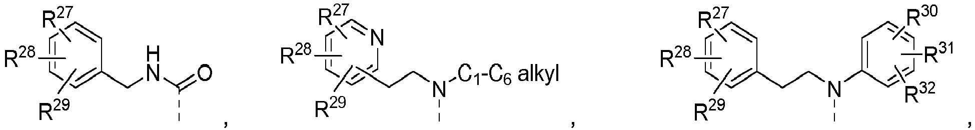 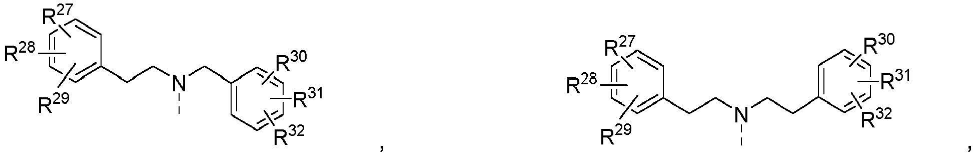 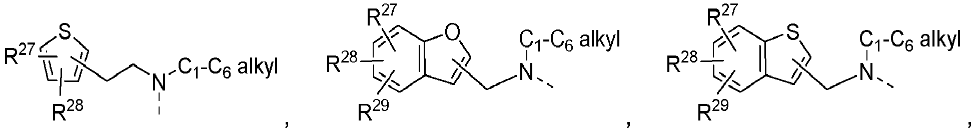 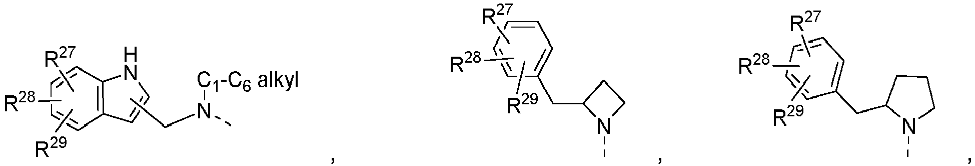 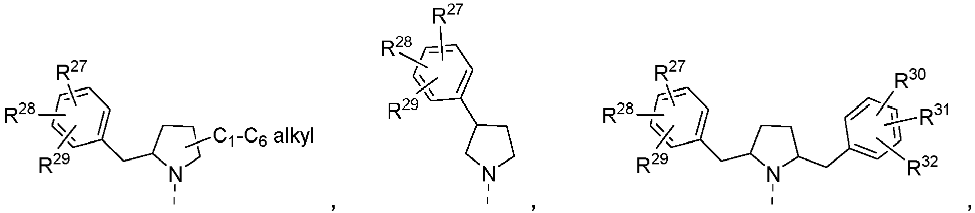 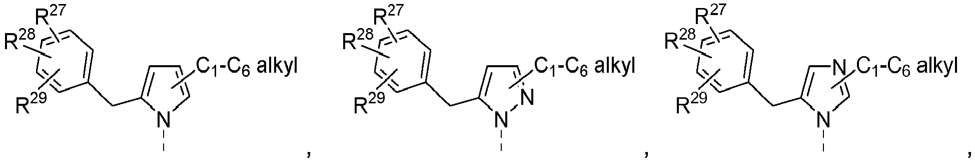 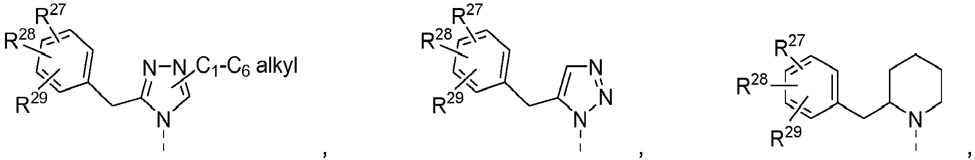 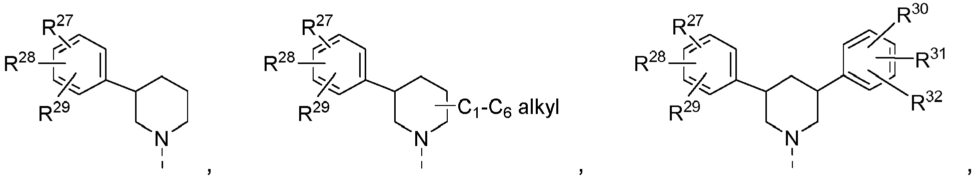 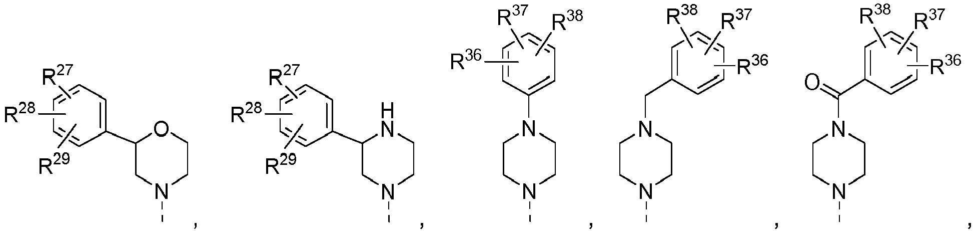 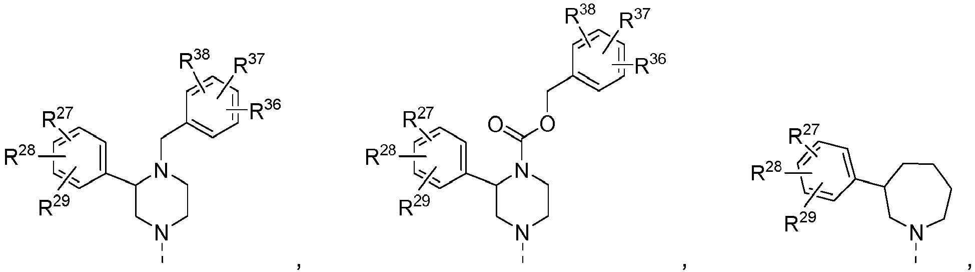 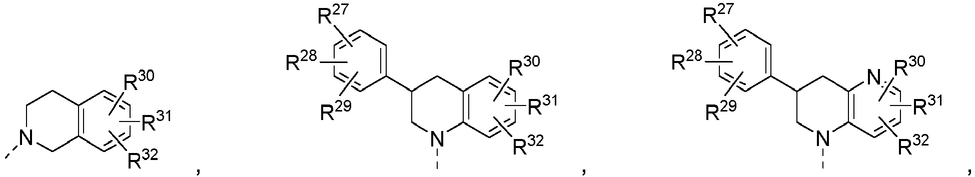 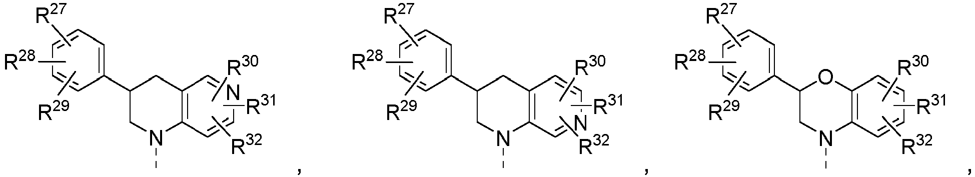 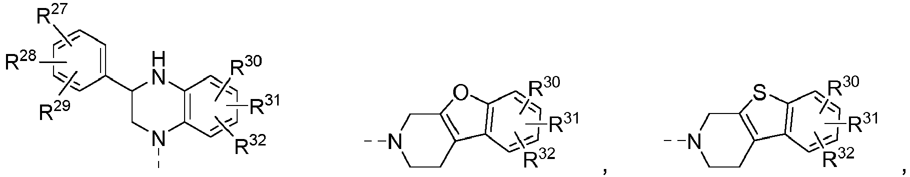 or 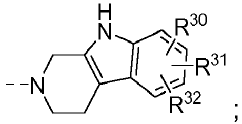 and **R**^{**2**7}, **R²⁸, R²⁹**, **R³⁰**, **R³¹**, and **R³²** have the same meanings as defined in formula (**I**) or as defined in any preferred selection of substituents disclosed herein.

More preferably, **R¹** represents

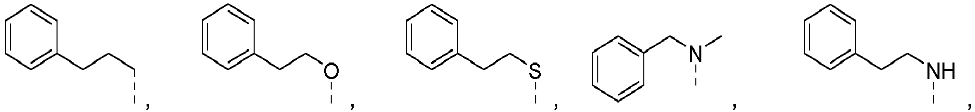

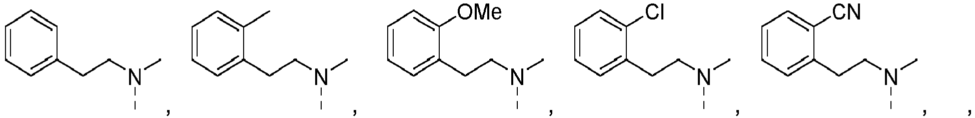

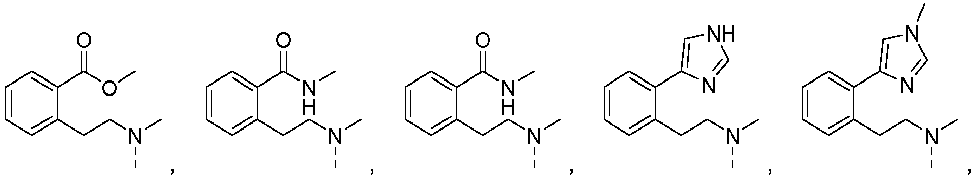

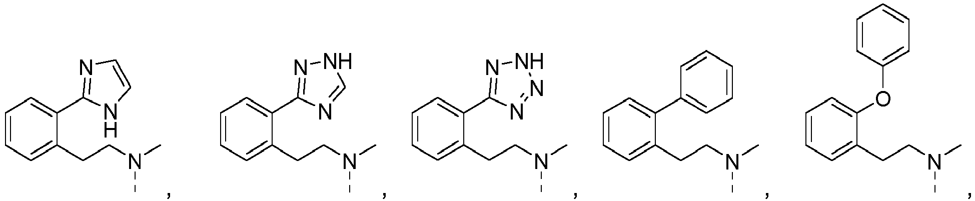

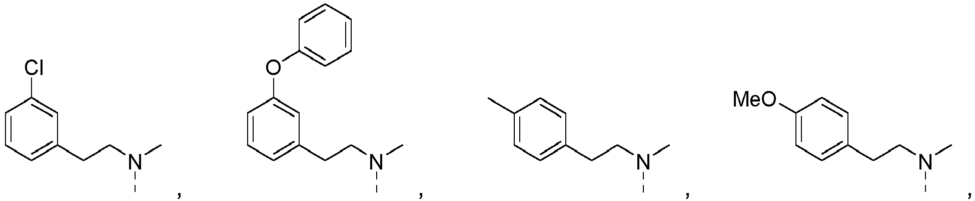

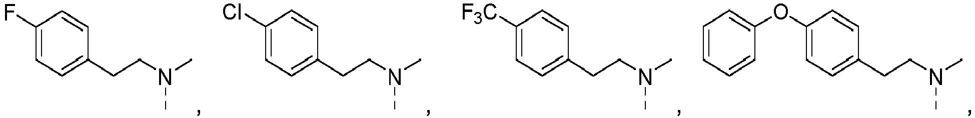

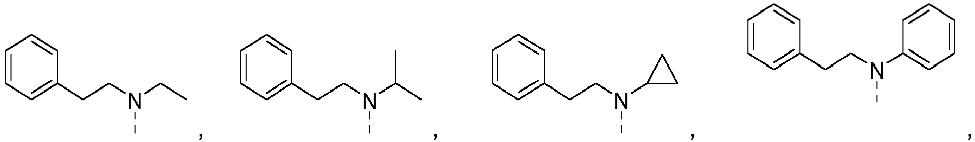

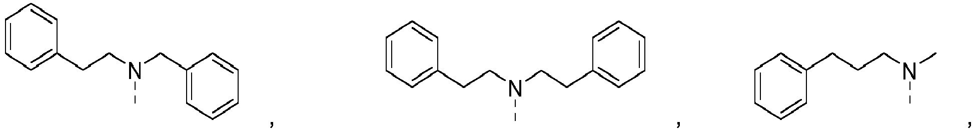

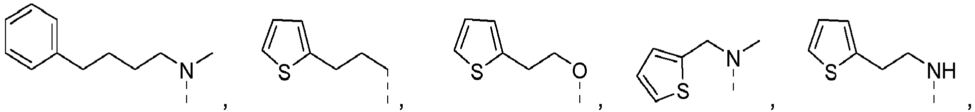

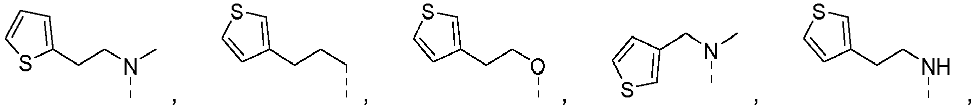

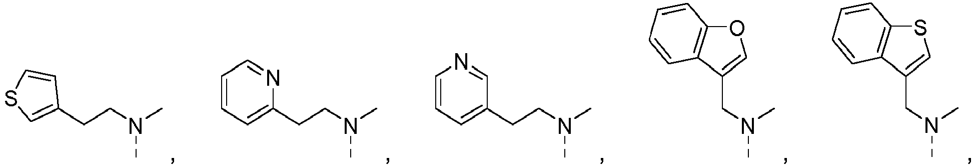

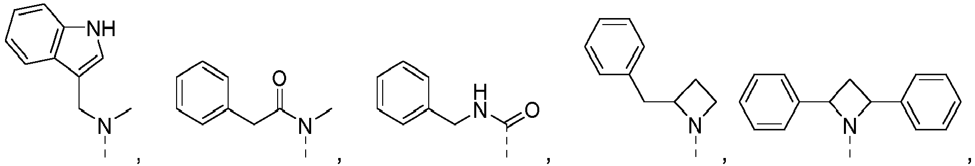

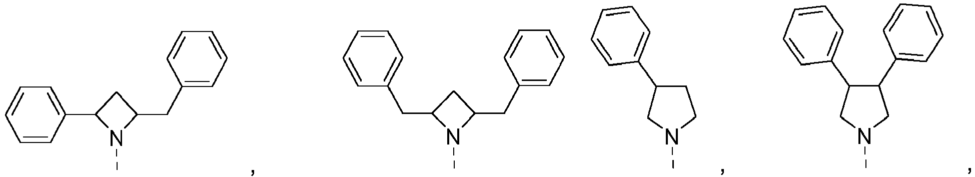

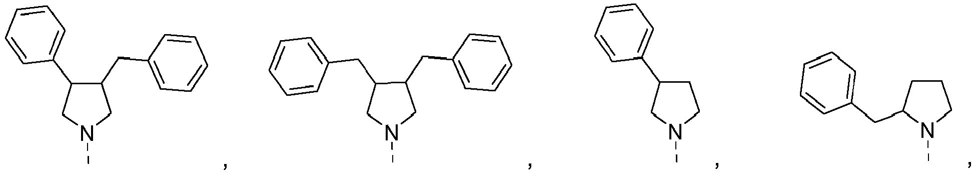

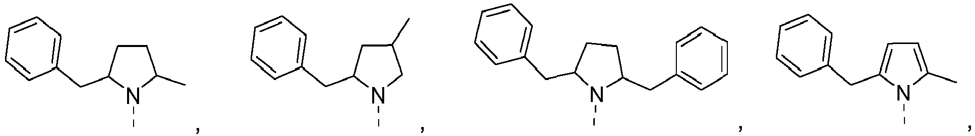

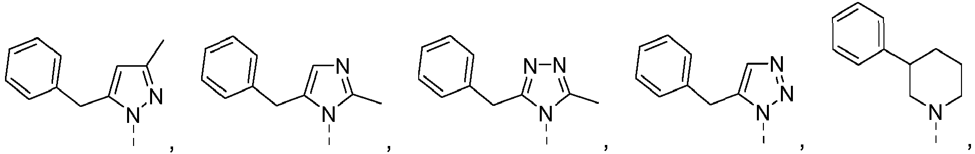

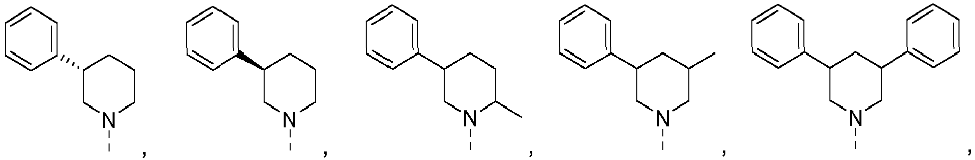

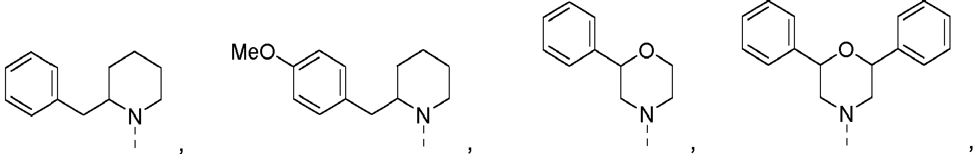

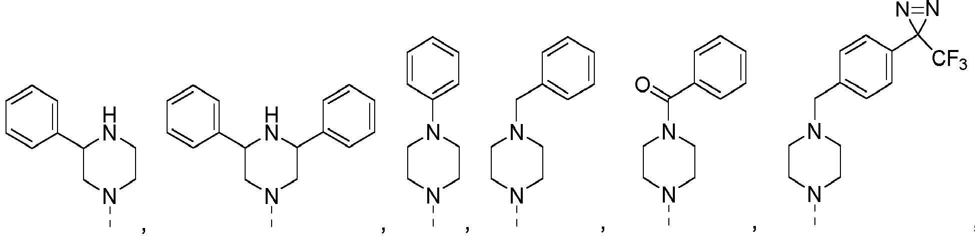

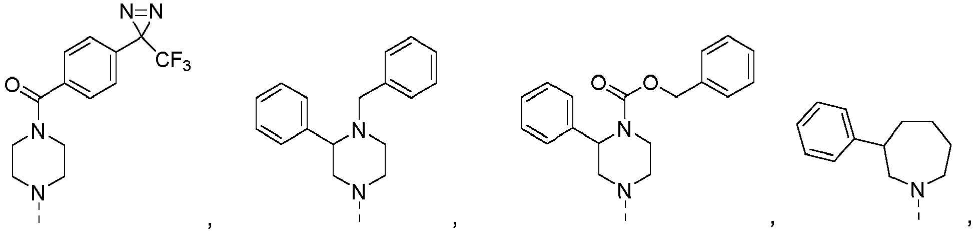

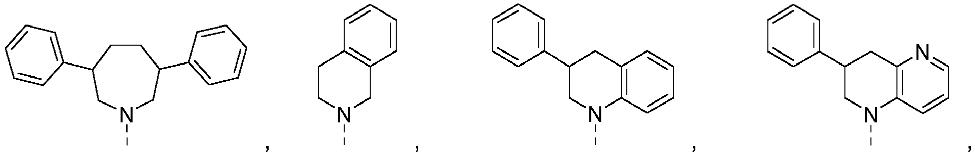

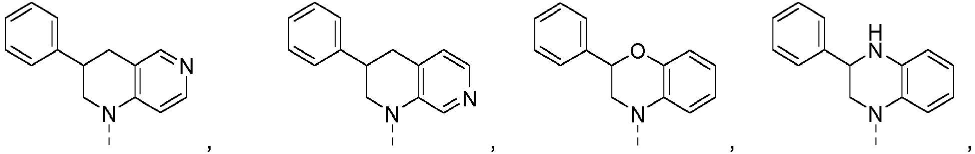

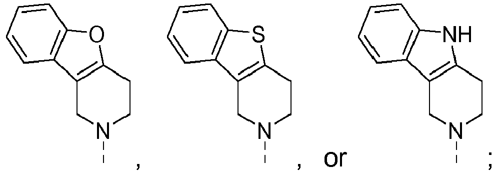

Preferably, **R²** represents -OC₁-C₆ alkyl, -SC₁-C₆ alkyl, -N(C₁-C₆ alkyl)₂, -OC₃-C₆ cycloalkyl, -N(C₁-C₆ alkyl)(C₁-C₆ alkylOH), C₁-C₆ alkylCO₂**R^{20'}**,

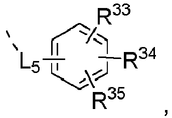

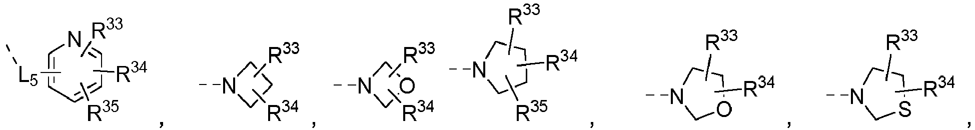

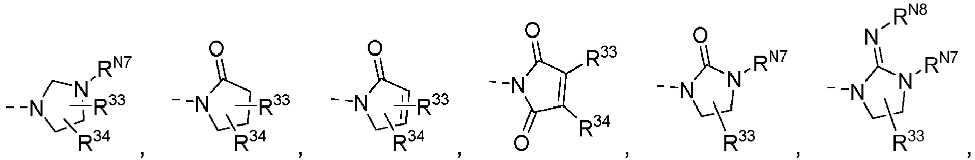

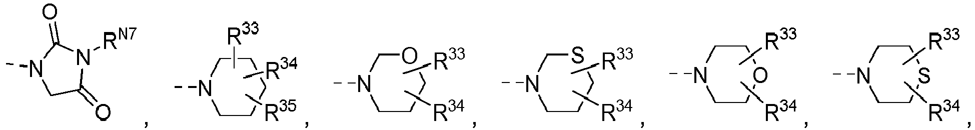

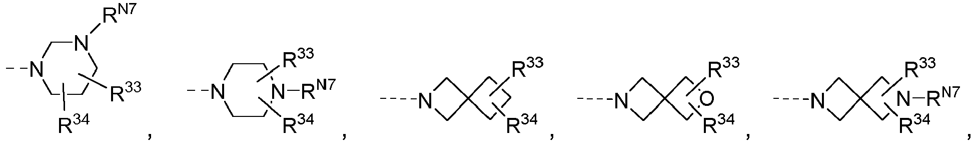

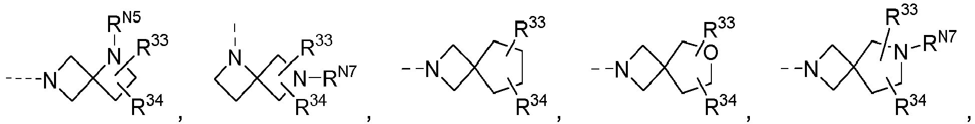

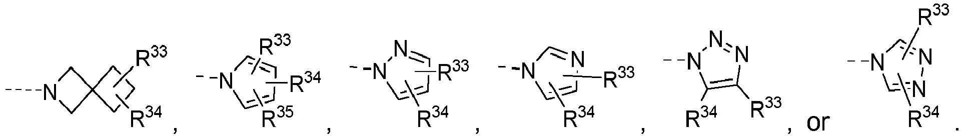

More preferably, **R²** represents

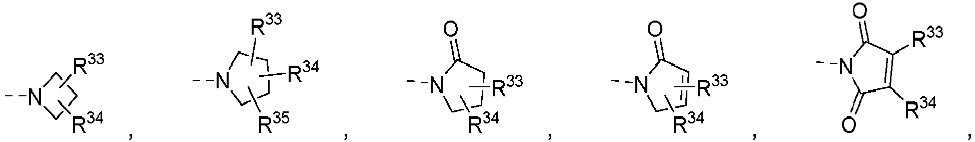

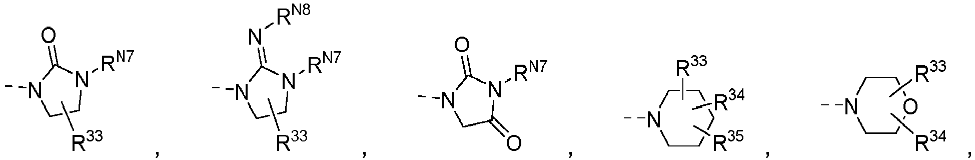

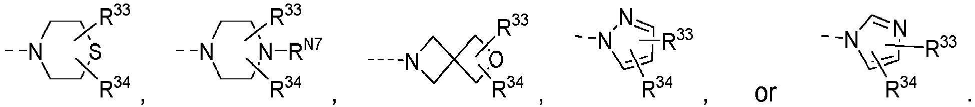

Most preferably, **R²** represents -CH₃, -Ph, -OCH₃, -SCH₃, -NHCH₃, -N(CH₃)₂, -N(CH₂CH₃)₂, -N(CH₃)(CH₂CH₂OH), -CONHCH₃, -N(CH₃)COCH₃, -N(CH₃)COCH₂OH, -N(CH₃)COCH(CH₃)CO₂CH₃, -N(CH₃)(CH₂Ph), -N(CH₃)(CH₂CH₂Ph), -NHCH(CH₃)CO₂CH₃, -NHCH(CH₂Ph)CO₂CH₃, -NHCH(CH₂SH)CO₂CH₃, -N(CH₃)CH(CH₃)CO₂CH₃, -N(CH₃)CH(CH₂Ph)CO₂CH₃, -N(CH₃)CH(CH₂SH)CO₂CH₃,

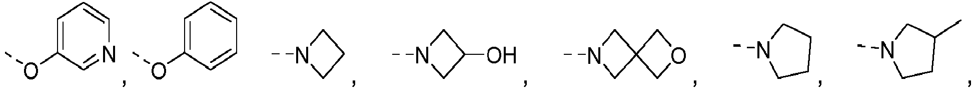

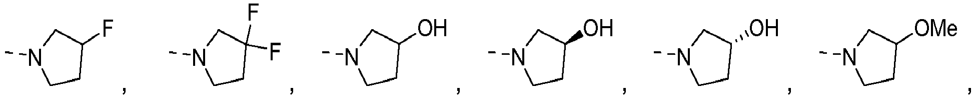

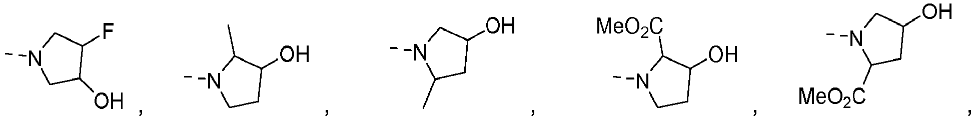

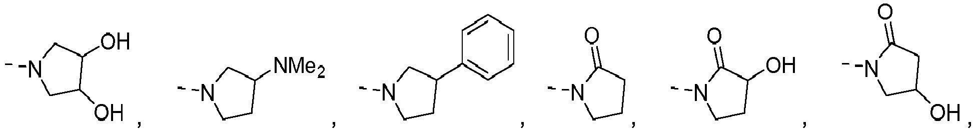

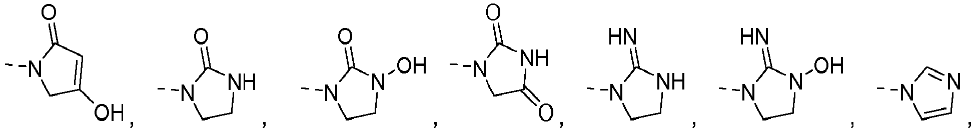

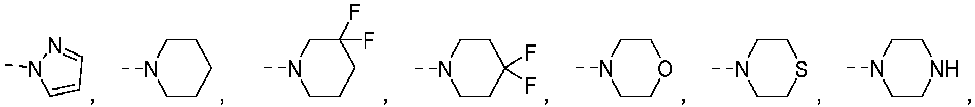

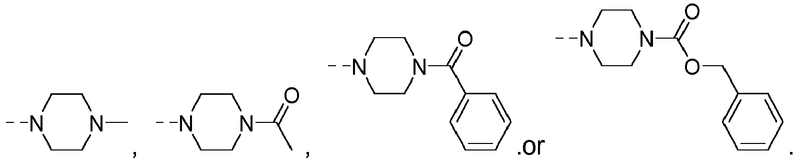

Preferred, **R³** represents C₁-C₆ alkyl, C₁-C₆ alkyl-OH, C₁-C₆ alkyl-CO₂**R²⁰**, C₁-C₆ alkyl-CONH**R²⁰**, C₂-C₆ alkenyl, C₂-C₆ alkynyl,

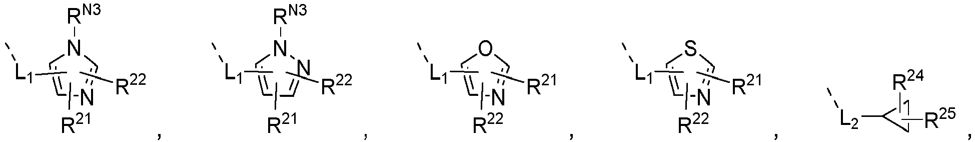

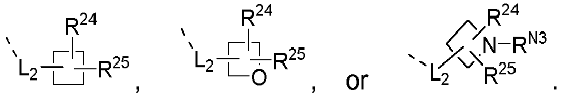

Most preferably, **R³** represents -CH₃, -CH₂CH₃, -CH₂CH₂CH₃, -CH₂CH₂CH₂CH₃, -CH₂CH(CH₃)₂, -CH₂CH₂OH, -CH₂CO₂CH₃, -CH₂C≡CH,

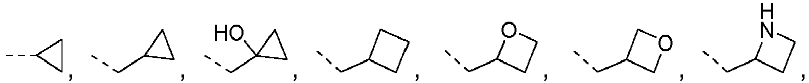

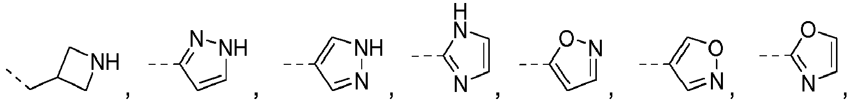

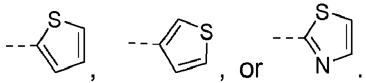

Preferred, -N**R⁸R⁹** represents

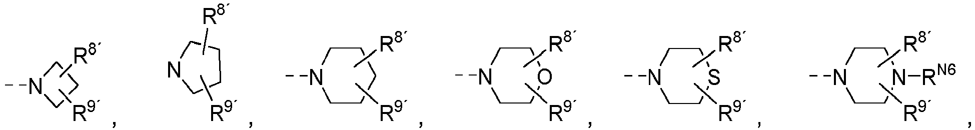

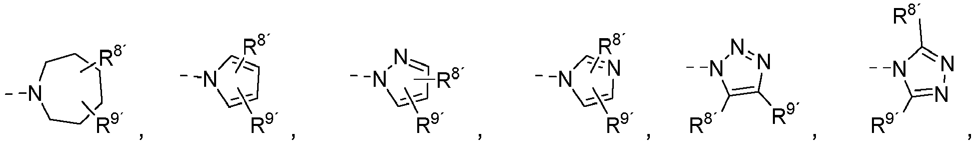

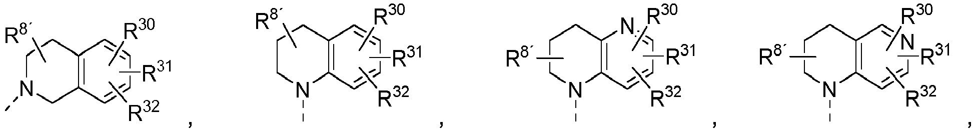

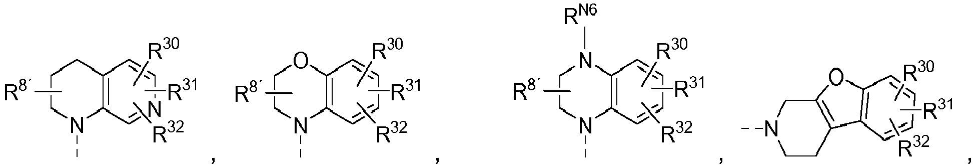

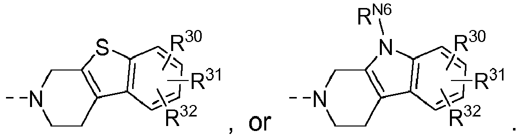

Preferred, -N**R¹⁸R¹⁹** represents

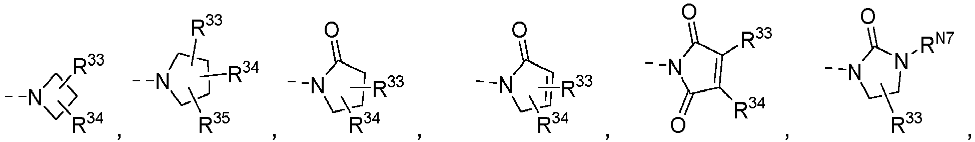

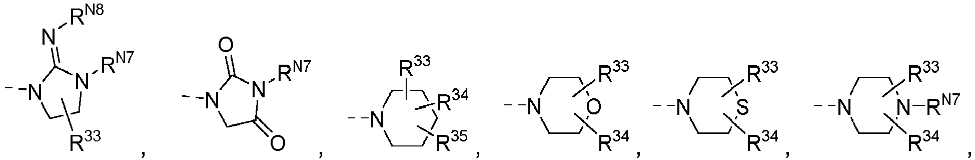

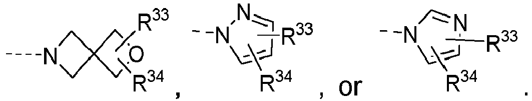

Therefore, the compunds of any one of the formulae (**I**), (**II-1**) - (**II-3**), (**III-1**) - (**III-3**), (**IV-1**) - (**IV-8**), (**V-1**) - (**V-8**), and (**VI-4**) - (**VI-9**) are still more preferred, wherein **R¹** represents 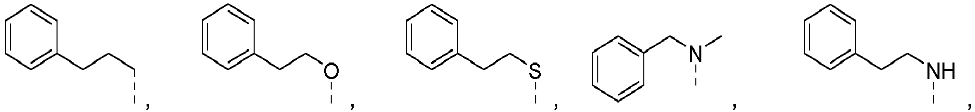 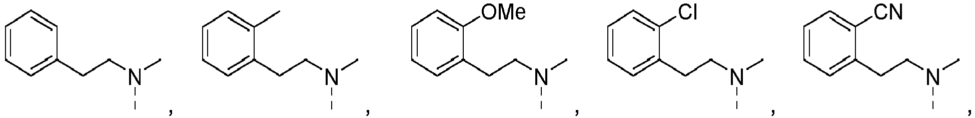 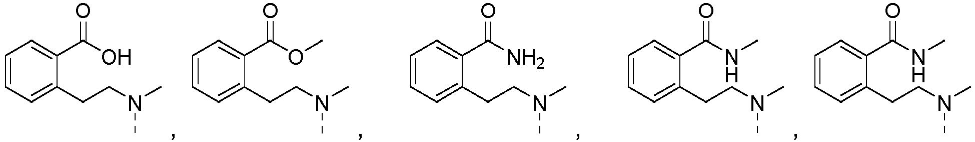 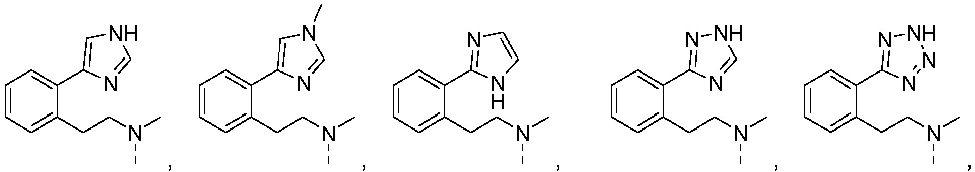 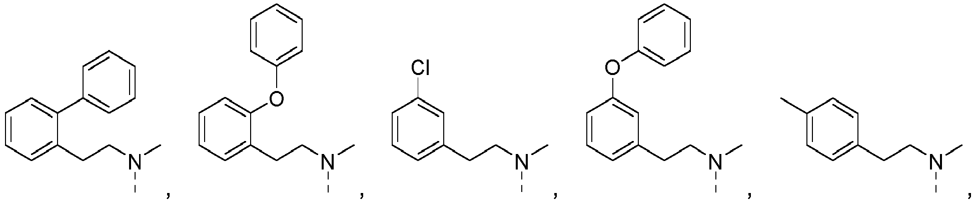 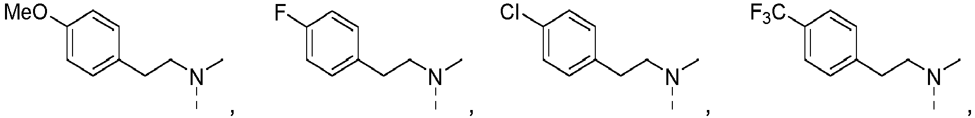 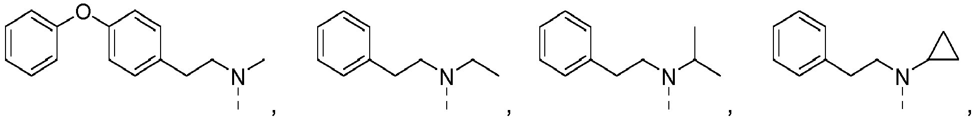 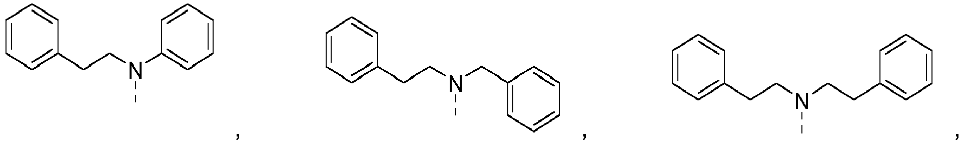 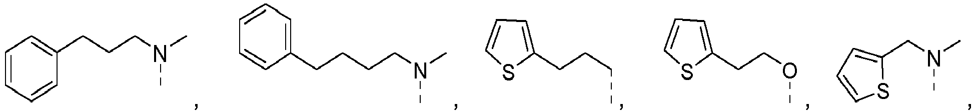 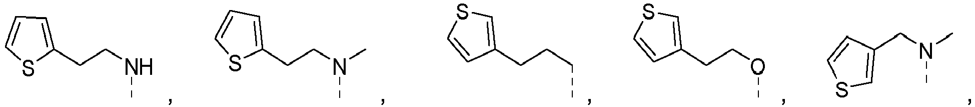 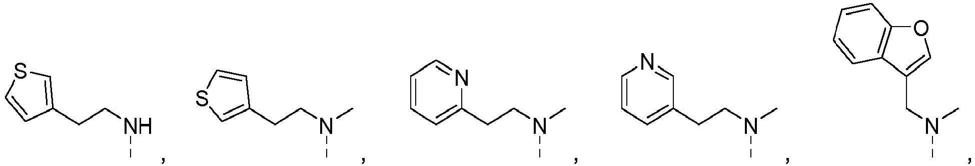 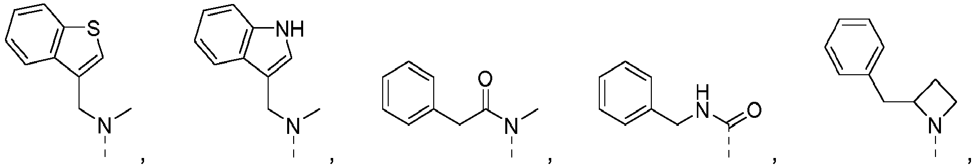 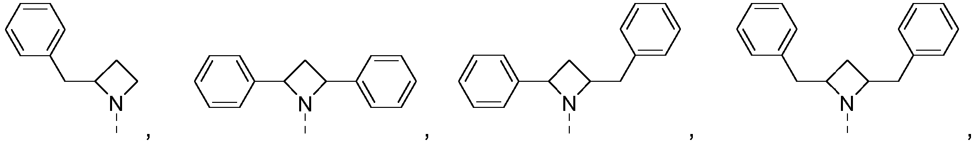 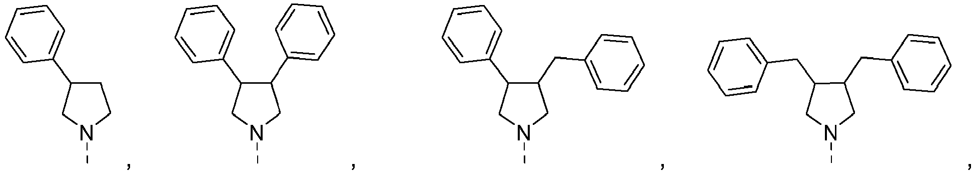 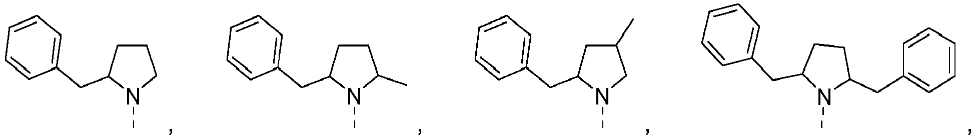 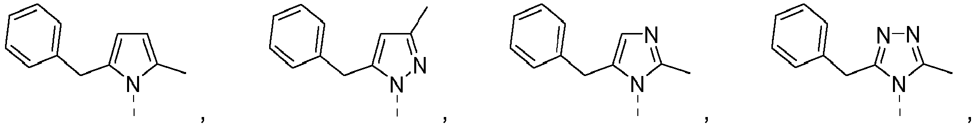 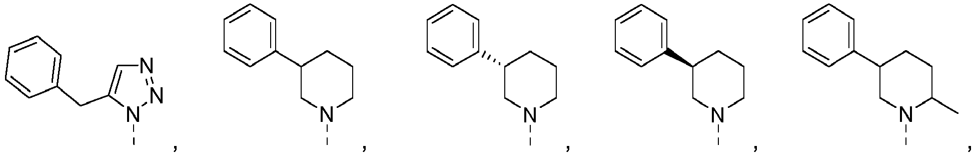 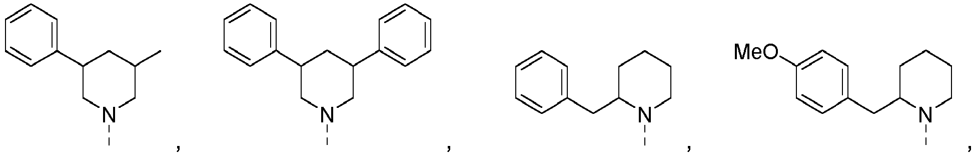 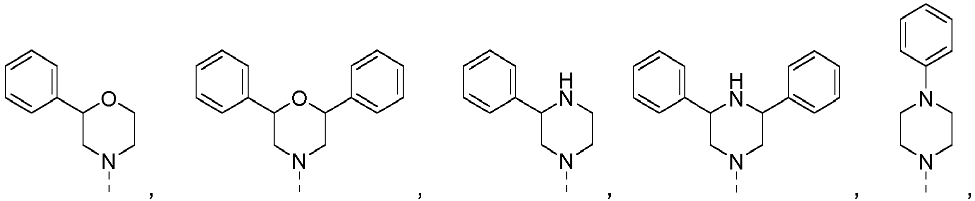 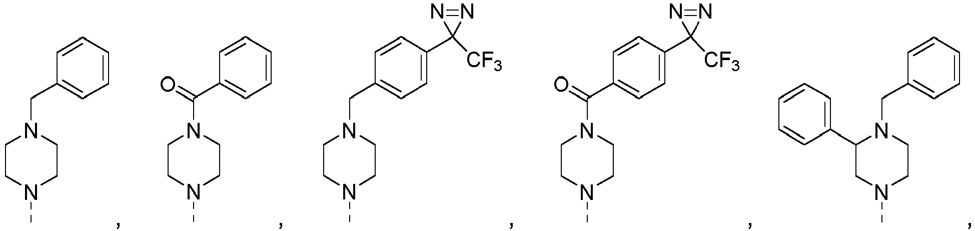 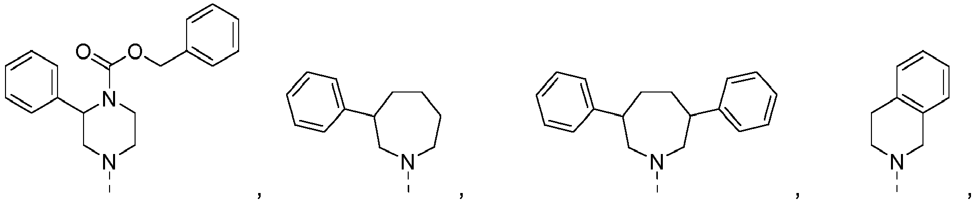 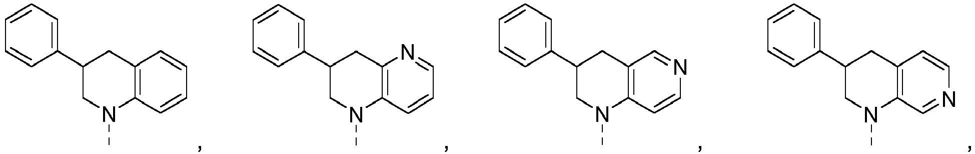 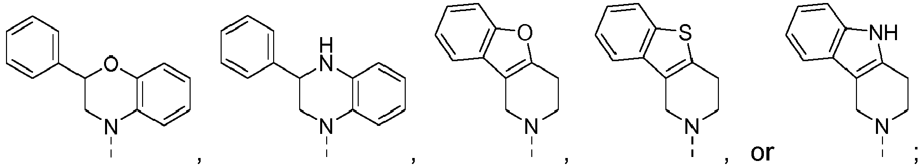 **R²** represents -CH₃, -Ph, -OCH₃, -SCH₃, -NHCH₃, -N(CH₃)₂, -N(CH₂CH₃)₂, -N(CH₃)(CH₂CH₂OH), -CONHCH₃, -N(CH₃)COCH₃, -N(CH₃)COCH₂OH, -N(CH₃)COCH(CH₃)CO₂CH₃, -N(CH₃)(CH₂Ph), -N(CH₃)(CH₂CH₂Ph), -NHCH(CH₃)CO₂CH₃, -NHCH(CH₂Ph)CO₂CH₃, -NHCH(CH₂SH)CO₂CH₃, -N(CH₃)CH(CH₃)CO₂CH₃, -N(CH₃)CH(CH₂Ph)CO₂CH₃, -N(CH₃)CH(CH₂SH)CO₂CH₃, 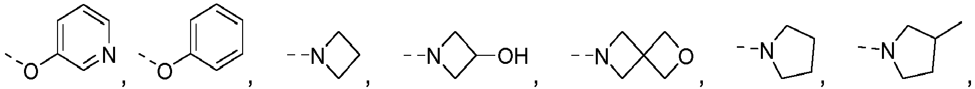 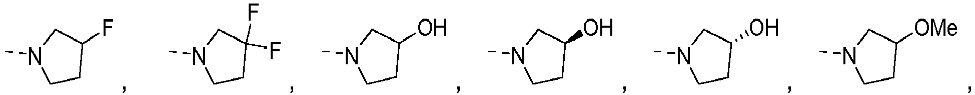 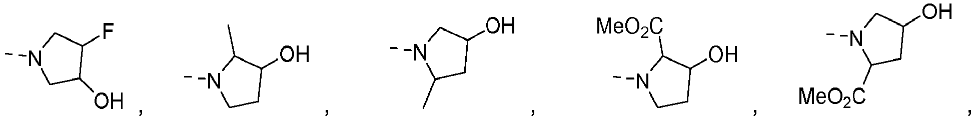 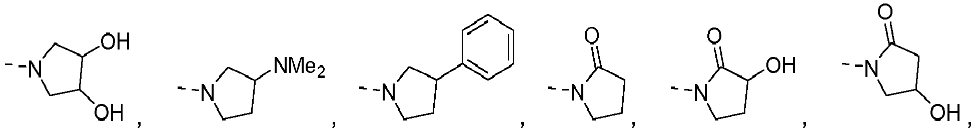 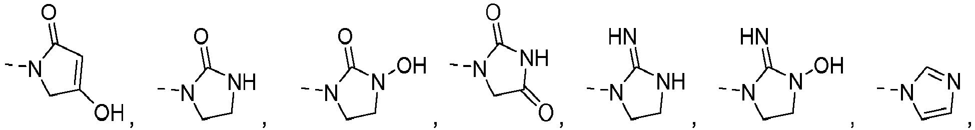 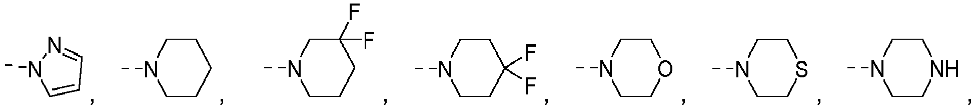 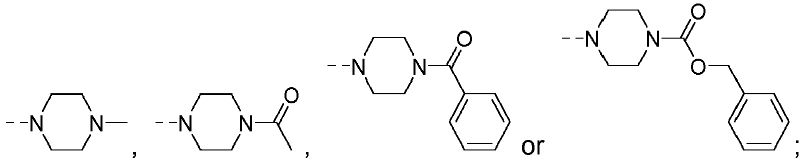 and **R³** represents -CH₃, -CH₂CH₃, -CH₂CH₂CH₃, -CH₂CH₂CH₂CH₃, -CH₂CH(CH₃)₂, -CH₂CH₂OH, -CH₂CO₂CH₃, -CH₂C≡CH, 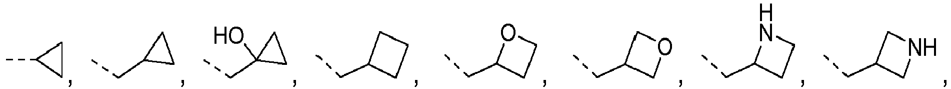 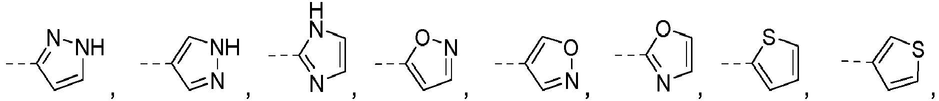 or

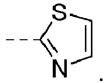

Preferred are the compound of any one of the formulae (**I**), (**II-1**) - (**II-3**), and (**IV-1**) - (**IV-8**), wherein **R¹¹** represents 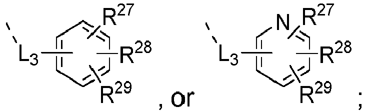 and **L₃**, **R**^{**2**7}, **R²⁸,** and **R²⁹** have the same meanings as defined herein.

**L¹** - **L⁴** represent independently of each other a covalent bond, -CH₂-, -CH(CH₃)-, -CH(CH₃)₂-, -CO-, -SO-, -SO₂-,

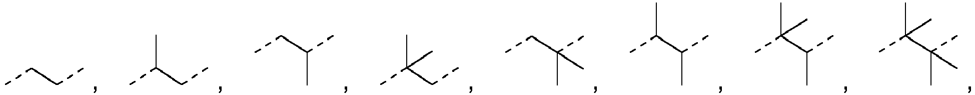

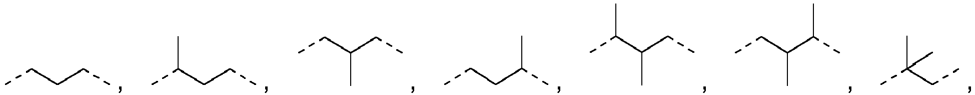

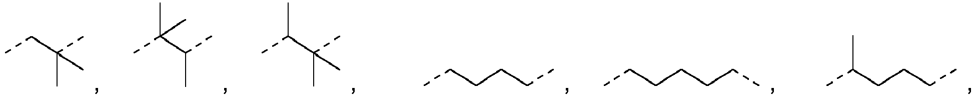

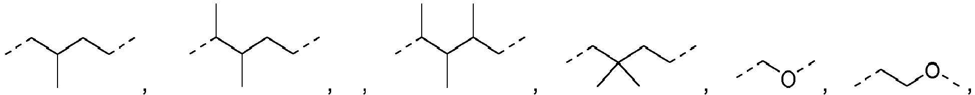

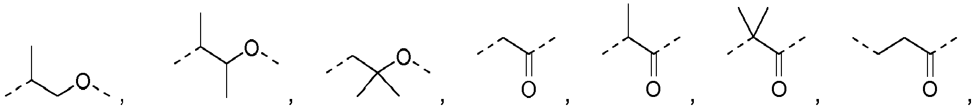

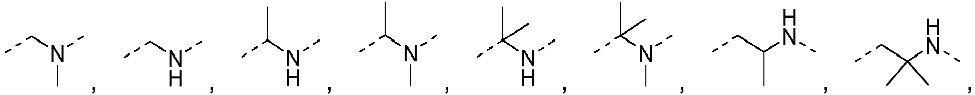

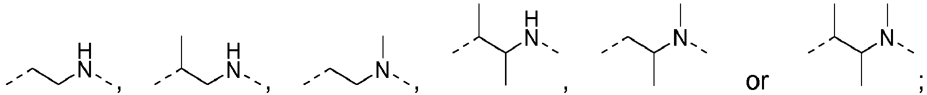

Most preferred, the present invention is directed to the compound selected from the group consisiting of: compounds **1 - 205.**

| **Compound** | **Structure** | **Nomenclature** |
|---|---|---|
| **1** | 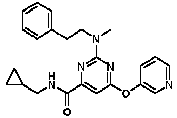 | *N*-(cyclopropylmethyl)-2-(methyl(phenethyl)amino)-6-(pyridin-3-yloxy)pyrimidine-4-carboxamide |
| **2** | 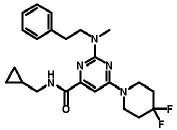 | *N*-(cyclopropylmethyl)-6-(4,4-difluoropiperidin-1-yl)-2-(methyl(phenethyl)amino)pyrimidine-4-carboxamide |
| **3** | 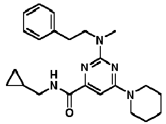 | *N*-(cyclopropylmethyl)-2-(methyl(phenethyl)amino)-6-(piperidin-1-yl)pyrimidine-4-carboxamide |
| **4** | 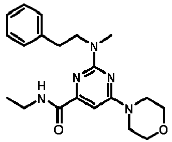 | *N*-ethyl-2-(methyl (phenethyl)amino)-6-morpholinopyrimidine-4-carboxamide |
| **5** | 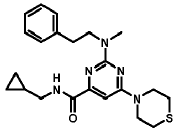 | *N*-(cyclopropylmethyl)-2-(methyl(phenethyl)amino)-6-thiomorpholinopyrimidine-4-carboxamide |
| **6** | 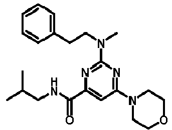 | *N*-isobutyl-2-(methyl(phenethyl)amino)-6-morpholinopyrimidine-4-carboxamide |
| **7** | 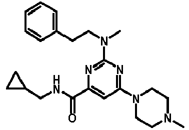 | *N*-(cyclopropyl methyl)-2-(methyl(phenethyl)am ino)-6-(4-methylpiperazin-1-yl)pyrimidine-4-carboxamide |
| **8** | 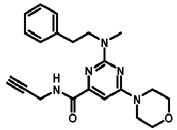 | 2-(methyl(phenethyl)amino)-6-morpholino-*N*-(prop-2-yn-1-yl)pyrimidine-4-carboxamide |
| **9** | 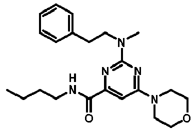 | *N*-butyl-2-(methyl (phenethyl)am ino)-6-morpholinopyrimidine-4-carboxamide |
| **10** | 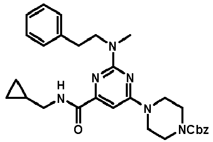 | benzyl 4-(6-((cyclopropylmethyl)carbamoyl)-2-(methyl(phenethyl)amino)pyrimidin-4-yl)piperazine-1-carboxylate |
| **11** | 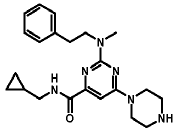 | *N*-(cyclopropyl methyl)-2-(methyl(phenethyl)amino)-6-(piperazin-1-yl)pyrimidine-4-carboxamide |
| **12** | 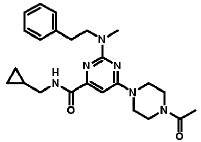 | 6-(4-acetylpiperazin-1-yl)-*N*-(cyclopropylmethyl)-2-(methyl(phenethyl)amino)pyrimidine-4-carboxamide |
| **13** | 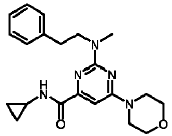 | *N*-cyclopropyl-2-(methyl (phenethyl)amino)-6-morpholinopyrimidine-4-carboxamide |
| **14** | 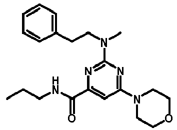 | *N*-propyl-2-(methyl (phenethyl)amino)-6-morpholino-pyrimidine-4-carboxamide |
| **15** | 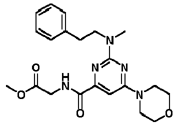 | methyl(2-(methyl(phenethyl)amino)-6-morpholinopyrimidine-4-carbonyl)glycinate |
| **16** | 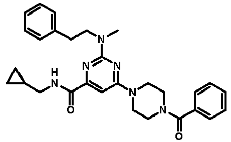 | 6-(4-benzoylpiperazin-1-yl)-*N*-(cyclopropylmethyl)-2-(methyl(phenethyl)amino)pyrimidine-4-carboxamide |
| **17** | 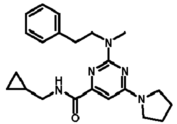 | *N*-(cyclopropylmethyl)-2-(methyl(phenethyl)amino)-6-(pyrrolidin-1-yl)pyrimidine-4-carboxamide |
| **18** | 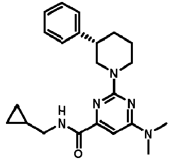 | (*S*)-*N*-(cyclopropylmethyl)-6-(dimethylamino)-2-(3-phenylpiperidin-1-yl)pyrimidine-4-carboxamide |
| **19** | 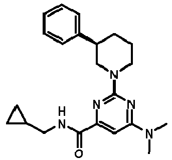 | (*R*)-*N*-(cyclopropylmethyl)-6-(dimethylamino)-2-(3-phenylpiperidin-1-yl)pyrimidine-4-carboxamide |
| **20** | 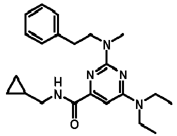 | *N*-(cyclopropylmethyl)-6-(diethylamino)-2-(methyl(phenethyl)amino)pyrimidine-4-carboxamide |
| **21** | 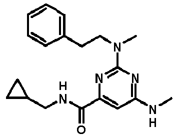 | *N*-(cyclopropylmethyl)-2-(methyl(phenethyl)amino)-6-(methylamino)pyrimidine-4-carboxamide |
| **22** | 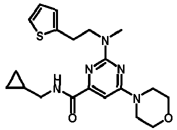 | *N*-(cyclopropylmethyl)-2-(methyl(2-(thiophen-2-yl)ethyl)amino)-6-morpholinopyrimidine-4-carboxamide |
| **23** | 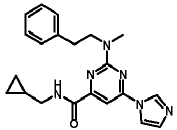 | *N*-(cyclopropylmethyl)-6-(1H-imidazol-1-yl)-2-(methyl(phenethyl)amino)pyrimidine-4-carboxamide |
| **24** | 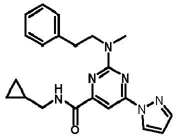 | *N*-(cyclopropylmethyl)-2-(methyl(phenethyl)amino)-6-(1H-pyrazol-1-yl)pyrimidine-4-carboxamide |
| **25** | 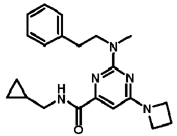 | *N*-(cyclopropylmethyl)-2-(methyl(phenethyl)amino)-6-(azetidin-1-yl)pyrimidine-4-carboxamide |
| **26** | 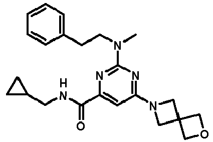 | *N*-(cyclopropylmethyl)-2-(methyl(phenethyl)amino)-6-(2-oxa-6-azaspiro[3.3]heptan-6-yl)pyrimidine-4-carboxamide |
| **27** | 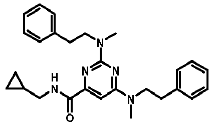 | *N*-(cyclopropylmethyl)-2,6-bis(methyl(phenethyl)amino)pyrimidine-4-carboxamide |
| **28** | 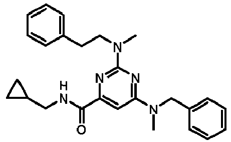 | *N*-(cyclopropylmethyl)-2-(methyl(phenethyl)amino)-6-(benzyl(methyl)amino)pyrimidine-4-carboxamide |
| **29** | 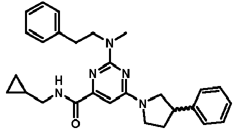 | *N*-(cyclopropyl methyl)-2-(methyl(phenethyl)am ino)-6-(3-phenylpyrrolidin-1-yl)pyrimidine-4-carboxamide |
| **30** | 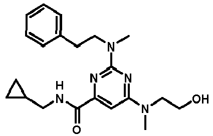 | *N*-(cyclopropylmethyl)-6-((2-hydroxyethyl)(methyl)amino)-2-(methyl(phenethyl)amino)pyrimidine-4-carboxamide |
| **31** | 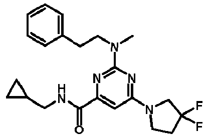 | *N*-(cyclopropylmethyl)-6-(3,3-difluoropyrrolidin-1-yl)-2-(methyl(phenethyl)amino)pyrimidine-4-carboxamide |
| **32** | 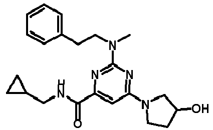 | *N*-(cyclopropylmethyl)-6-(3-hydroxypyrrolidin-1-yl)-2-(methyl(phenethyl)amino)pyrimidine-4-carboxamide |
| **33** | 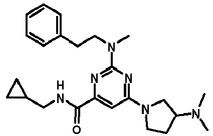 | *N*-(cyclopropylmethyl)-6-(3-(dimethylamino)pyrrolidin-1-yl)-2-(methyl(phenethyl)amino)pyrimidine-4-carboxamide |
| **34** | 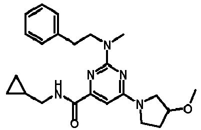 | *N*-(cyclopropylmethyl)-6-(3-methoxypyrrolidin-1-yl)-2-(methyl(phenethyl)amino)pyrimidine-4-carboxamide |
| **35** | 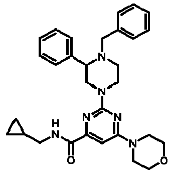 | 2-(4-benzyl-3-phenylpiperazin-1-yl)-*N-*(cyclopropylmethyl)-6-morpholinopyrimidine-4-carboxamide |
| **36** | 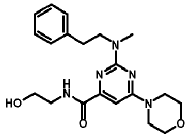 | *N*-(2-hydroxyethyl)-2-(methyl(phenethyl)amino)-6-morpholinopyrimidine-4-carboxamide |
| **37** | 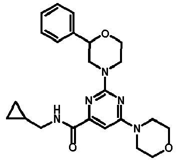 | *N*-(cyclopropylmethyl)-6-morpholino-2-(2-phenylmorpholino)pyrimidine-4-carboxamide |
| **38** | 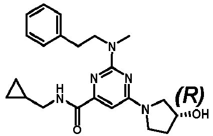 | (*R*)-*N*-(cyclopropylmethyl)-6-(3-hydroxypyrrolidin-1-yl)-2-(methyl(phenethyl)amino)pyrimidine-4-carboxamide |
| **39** | 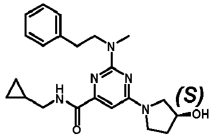 | (*S*)-*N*-(cyclopropylmethyl)-6-(3-hydroxypyrrolidin-1-yl)-2-(methyl(phenethyl)amino)pyrimidine-4-carboxamide |
| **40** | 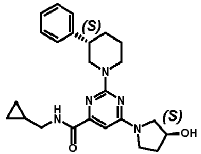 | *N*-(cyclopropylmethyl)-6-((*S*)-3-hydroxypyrrolidin-1-yl)-2-((*S*)-3-phenylpiperidin-1-yl)pyrimidine-4-carboxamide |
| **41** | 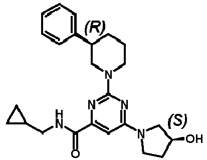 | *N*-(cyclopropylmethyl)-6-((*S*)-3-hydroxypyrrolidin-1-yl)-2-((*R*)-3-phenylpiperidin-1-yl)pyrimidine-4-carboxamide |
| **42** | 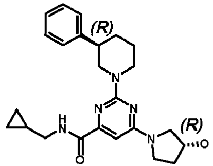 | *N*-(cyclopropylmethyl)-6-((*R*)-3-hydroxypyrrolidin-1-yl)-2-((*R*)-3-phenylpiperidin-1-yl)pyrimidine-4-carboxamide |
| **43** | 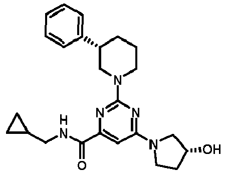 | *N*-(cyclopropylmethyl)-6-((*R*)-3-hydroxypyrrolidin-1-yl)-2-((*S*)-3-phenylpiperidin-1-yl)pyrimidine-4-carboxamide |
| **44** | 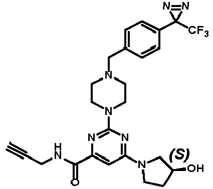 | (*S*)-6-(3-hydroxypyrrolidin-1-yl)-*N*-(prop-2-yn-1-yl)-2-(4-(4-(3-(trifluoromethyl)-3H-diazirin-3-yl)benzyl)piperazin-1-yl)pyrimidine-4-carboxamide |
| **45** | 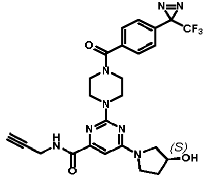 | (*S*)-6-(3-hydroxypyrrolidin-1-yl)-*N*-(prop-2-yn-1-yl)-2-(4-(4-(3-(trifluoromethyl)-3H-diazirin-3-yl)benzoyl)piperazin-1-yl)pyrimidine-4-carboxamide |
| **46** | 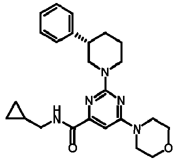 | (*S*)-*N*-(cyclopropylmethyl)-6-morpholino-2-(3-phenylpiperidin-1-yl)pyrimidine-4-carboxamide |
| **47** | 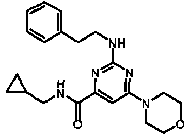 | *N*-(cyclopropylmethyl)-6-morpholino-2-(phenethylamino)pyrimidine-4-carboxamide |
| **48** | 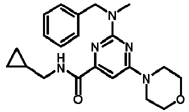 | 2-(benzyl(methyl)amino)-*N*-(cyclopropylmethyl)-6-morpholinopyrimidine-4-carboxamide |
| **49** | 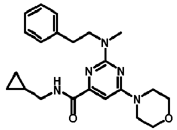 | *N*-(cyclopropylmethyl)-2-(methyl(phenethyl)amino)-6-morpholinopyrimidine-4-carboxamide |
| **50** | 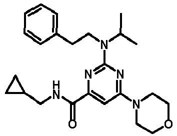 | *N*-(cyclopropylmethyl)-2-(isopropyl(phenethyl)amino)-6-morpholinopyrimidine-4-carboxamide |
| **51** | 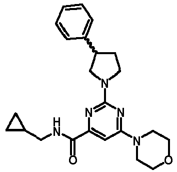 | *N*-(cyclopropylmethyl)-6-morphol ino-2-(3-phenylpyrrol idin-1-yl)pyrimidine-4-carboxamide |
| **52** | 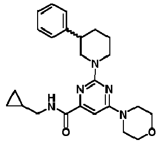 | *N*-(cyclopropylmethyl)-6-morphol ino-2-(3-phenylpiperidin-1-yl)pyrimidine-4-carboxamide |
| **53** | 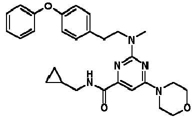 | *N*-(cyclopropylmethyl)-2-(methyl(4-phenoxyphenethyl)amino)-6-morpholinopyrimidine-4-carboxamide |
| **54** | 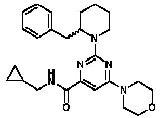 | 2-(2-benzylpiperidin-1-yl)-*N-*(cyclopropylmethyl)-6-morpholinopyrimidine-4-carboxamide |
| **55** | 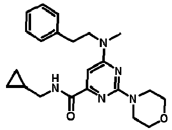 | *N*-(cyclopropylmethyl)-6-(methyl(phenethyl)amino)-2-morpholinopyrimidine-4-carboxamide |
| **56** | 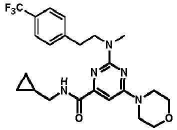 | *N*-(cyclopropylmethyl)-2-(methyl(4-(trifluoromethyl)phenethyl)amino)-6-morpholino-pyrimidine-4-carboxamide |
| **57** | 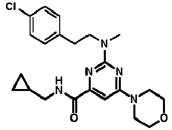 | 2-((4-chlorophenethyl)(methyl)amino)-*N-*(cyclopropylmethyl)-6-morphol inopyrimidine-4-carboxamide |
| **58** | 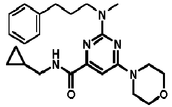 | *N*-(cyclopropylmethyl)-2-(methyl(3-phenylpropyl)amino)-6-morpholinopyrimidine-4-carboxamide |
| **59** | 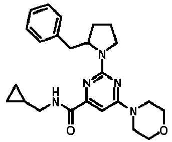 | 2-(2-benzylpyrrolidin-1-yl)-*N-*(cyclopropylmethyl)-6-morpholinopyrimidine-4-carboxamide |
| **60** | 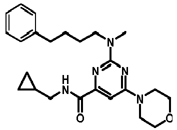 | *N*-(cyclopropylmethyl)-2-(methyl(4-phenylbutyl)amino)-6-morpholinopyrimidine-4-carboxamide |
| **61** | 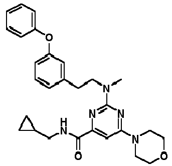 | *N*-(cyclopropylmethyl)-2-(methyl(3-phenoxyphenethyl)amino)-6-morpholinopyrimidine-4-carboxamide |
| **62** | 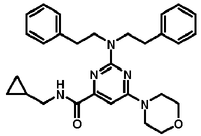 | *N*-(cyclopropylmethyl)-2-(diphenethylamino)-6-morpholinopyrimidine-4-carboxamide |
| **63** | 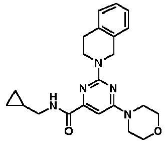 | *N*-(cyclopropylmethyl)-2-(3,4-dihydroisoquinolin-2(1H)-yl)-6-morphol inopyrimidine-4-carboxamide |
| **64** | 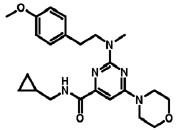 | *N*-(cyclopropylmethyl)-2-((4-methoxyphenethyl)(methyl)amino)-6-morpholinopyrimidine-4-carboxamide |
| **65** | 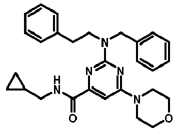 | 2-(benzyl(phenethyl)amino)-*N-*(cyclopropylmethyl)-6-morpholinopyrimidine-4-carboxamide |
| **66** | 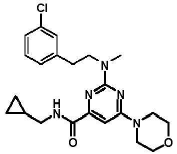 | 2-((3-chlorophenethyl)(methyl)amino)-*N-*(cyclopropylmethyl)-6-morpholinopyrimidine-4-carboxamide |
| **67** | 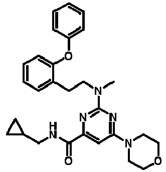 | *N*-(cyclopropylmethyl)-2-(methyl(2-phenoxyphenethyl)amino)-6-morpholinopyrimidine-4-carboxamide |
| **68** | 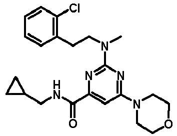 | 2-((2-chlorophenethyl)(methyl)amino)-*N-*(cyclopropylmethyl)-6-morpholinopyrimidine-4-carboxamide |
| **69** | 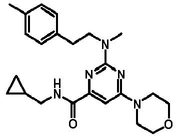 | *N*-(cyclopropylmethyl)-2-(methyl(4-methylphenethyl)amino)-6-morpholinopyrimidine-4-carboxamide |
| **70** | 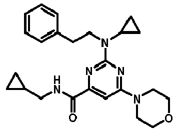 | 2-(cyclopropyl(phenethyl)amino)-*N-*(cyclopropylmethyl)-6-morpholinopyrimidine-4-carboxamide |
| **71** | 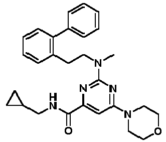 | 2-((2-([1,1'-biphenyl]-2-yl)ethyl)(methyl)amino)-*N*-(cyclopropylmethyl)-6-morpholino-pyrimidine-4-carboxamide |
| **72** | 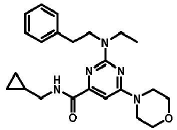 | *N*-(cyclopropylmethyl)-2-(ethyl(phenethyl)amino)-6-morpholinopyrimidine-4-carboxamide |
| **73** | 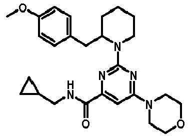 | *N*-(cyclopropylmethyl)-2-(2-(4-methoxybenzyl)piperidin-1-yl)-6-morpholinopyrimidine-4-carboxamide |
| **74** | 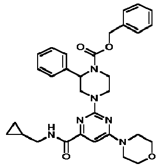 | benzyl 4-(4-((cyclopropylmethyl)carbamoyl)-6-morpholinopyrimidin-2-yl)-2-phenylpiperazine-1-carboxylate |
| **75** | 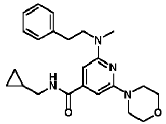 | *N*-(cyclopropylmethyl)-2-(methyl(phenethyl)amino)-6-morpholino isonicotinamide |
| **76** | 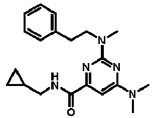 | *N*-(cyclopropylmethyl)-6-(dimethylamino)-2-(methyl(phenethyl)amino)pyrimidine-4-carboxamide |
| **77** | 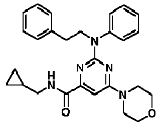 | *N*-(cyclopropylmethyl)-6-morpholino-2-(phenethyl(phenyl)amino)pyrimidine-4-carboxamide |
| **78** | 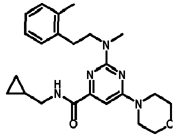 | *N*-(cyclopropylmethyl)-2-(methyl(2-methylphenethyl)amino)-6-morpholinopyrimidine-4-carboxamide |
| **79** | 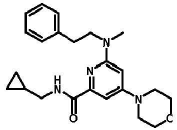 | *N*-(cyclopropylmethyl)-6-(methyl(phenethyl)amino)-4-morpholinopicolinamide |
| **80** | 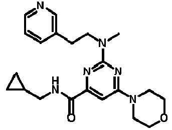 | *N*-(cyclopropylmethyl)-2-(methyl(2-(pyridin-3-yl)ethyl)amino)-6-morpholinopyrimidine-4-carboxamide |
| **81** | 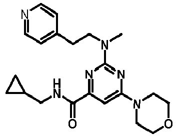 | *N*-(cyclopropylmethyl)-2-(methyl(2-(pyridin-4-yl)ethyl)amino)-6-morpholinopyrimidine-4-carboxamide |
| **82** | 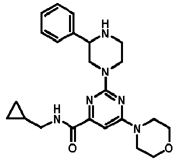 | *N*-(cyclopropylmethyl)-6-morpholino-2-(3-phenylpiperazin-1-yl)pyrimidine-4-carboxamide |
| **83** | 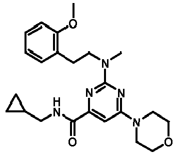 | *N*-(cyclopropylmethyl)-2-((2-methoxyphenethyl)(methyl)amino)-6-morpholinopyrimidine-4-carboxamide |
| **84** | 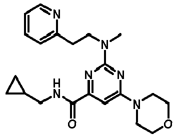 | *N*-(cyclopropylmethyl)-2-(methyl(2-(pyridin-2-yl)ethyl)amino)-6-morpholinopyrimidine-4-carboxamide |
| **85** | 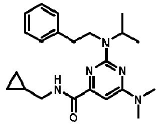 | *N*-(cyclopropylmethyl)-6-(dimethylamino)-2-(isopropyl(phenethyl)amino)pyrimidine-4-carboxamide |
| **86** | 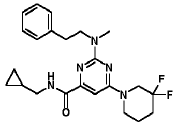 | *N*-(cyclopropylmethyl)-6-(3,3-difluoropiperidin-1-yl)-2-(methyl(phenethyl)amino)pyrimidine-4-carboxamide |
| **87** | 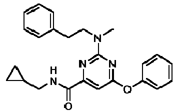 | *N*-(cyclopropylmethyl)-2-(methyl(phenethyl)amino)-6-phenoxypyrimidine-4-carboxamide |
| **88** | 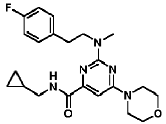 | *N*-(cyclopropylmethyl)-2-((4-fluorophenethyl)(methyl)amino)-6-morpholinopyrimidine-4-carboxamide |
| **89** | 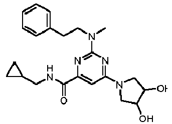 | *N*-(cyclopropylmethyl)-6-(3,4-dihydroxypyrrolidin-1-yl)-2-(methyl(phenethyl)amino)pyrimidine-4-carboxamide |
| **90** | 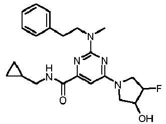 | *N*-(cyclopropylmethyl)-6-(3-fluoro-4-hydroxypyrrolidin-1-yl)-2-(methyl(phenethyl)amino)pyrimidine-4-carboxamide |
| **91** | 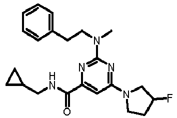 | *N*-(cyclopropylmethyl)-6-(3-fluoropyrrolidin-1-yl)-2-(methyl(phenethyl)amino)pyrimidine-4-carboxamide |
| **92** | 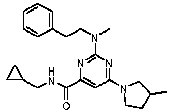 | *N*-(cyclopropylmethyl)-2-(methyl(phenethyl)amino)-6-(3-methylpyrrolidin-1-yl)pyrimidine-4-carboxamide |
| **93** | 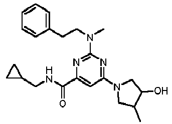 | *N*-(cyclopropylmethyl)-6-(3-hydroxy-4-methylpyrrolidin-1-yl)-2-(methyl(phenethyl)amino)pyrimidine-4-carboxamide |
| **94** | 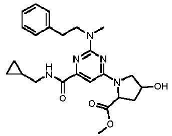 | methyl 1-(6-((cyclopropylmethyl)carbamoyl)-2-(methyl(phenethyl)amino)pyrimidin-4-yl)-4-hydroxypyrrolidine-2-carboxylate |
| **95** | 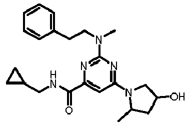 | *N*-(cyclopropylmethyl)-6-(4-hydroxy-2-methylpyrrolidin-1-yl)-2-(methyl(phenethyl)amino)pyrimidine-4-carboxamide |
| **96** | 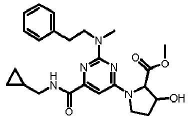 | methyl 1-(6-((cyclopropylmethyl)carbamoyl)-2-(methyl(phenethyl)amino)pyrimidin-4-yl)-3-hydroxypyrrolidine-2-carboxylate |
| **97** | 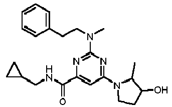 | *N*-(cyclopropylmethyl)-6-(3-hydroxy-2-methylpyrrolidin-1-yl)-2-(methyl(phenethyl)amino)pyrimidine-4-carboxamide |
| **98** | 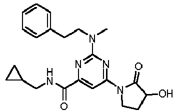 | *N*-(cyclopropylmethyl)-6-(3-hydroxy-2-oxopyrrolidin-1-yl)-2-(methyl(phenethyl)amino)pyrimidine-4-carboxamide |
| **99** | 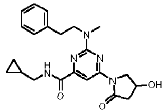 | *N*-(cyclopropylmethyl)-6-(4-hydroxy-2-oxopyrrolidin-1-yl)-2-(methyl(phenethyl)amino)pyrimidine-4-carboxamide |
| **100** | 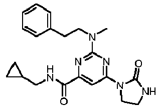 | *N*-(cyclopropylmethyl)-2-(methyl(phenethyl)amino)-6-(2-oxoimidazolidin-1-yl)pyrimidine-4-carboxamide |
| **101** | 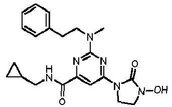 | *N*-(cyclopropylmethyl)-6-(3-hydroxy-2-oxoimidazolidin-1-yl)-2-(methyl(phenethyl)amino)pyrimidine-4-carboxamide |
| **102** | 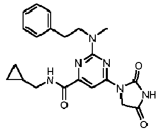 | *N*-(cyclopropylmethyl)-6-(2,4-dioxoimidazolidin-1-yl)-2-(methyl(phenethyl)amino)pyrimidine-4-carboxamide |
| **103** | 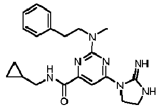 | *N*-(cyclopropylmethyl)-6-(2-iminoimidazolidin-1-yl)-2-(methyl(phenethyl)amino)pyrimidine-4-carboxamide |
| **104** | 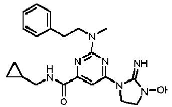 | *N*-(cyclopropylmethyl)-6-(3-hydroxy-2-iminoimidazolidin-1-yl)-2-(methyl(phenethyl)amino)pyrimidine-4-carboxamide |
| **105** | 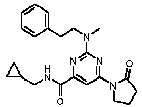 | *N*-(cyclopropylmethyl)-2-(methyl(phenethyl)amino)-6-(2-oxopyrrolidin-1-yl)pyrimidine-4-carboxamide |
| **106** | 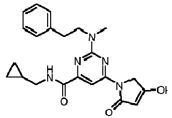 | *N*-(cyclopropylmethyl)-6-(4-hydroxy-2-oxo-2,5-dihydro-1 *H*-pyrrol-1 -yl)-2-(methyl(phenethyl)amino)pyrimidine-4-carboxamide |
| **107** | 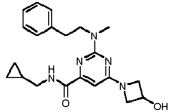 | *N*-(cyclopropylmethyl)-6-(3-hydroxyazetidin-1-yl)-2-(methyl(phenethyl)amino)pyrimidine-4-carboxamide |
| **108** | 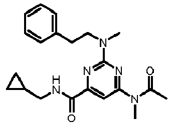 | *N*-(cyclopropylmethyl)-2-(methyl(phenethyl)amino)-6-(*N-*methylacetamido)pyrimidine-4-carboxamide |
| **109** | 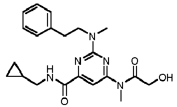 | *N*-(cyclopropylmethyl)-6-(2-hydroxy-*N-*methylacetamido)-2-(methyl(phenethyl)amino)pyrimidine-4-carboxamide |
| **110** | 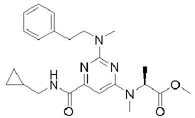 | methyl *N*-(6-((cyclopropylmethyl)carbamoyl)-2-(methyl(phenethyl)amino)pyrimidin-4-yl)-N-methyl-L-alaninate |
| **111** | 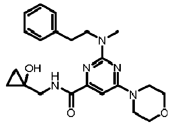 | *N*-((1-hydroxycyclopropyl)methyl)-2-(methyl(phenethyl)amino)-6-morpholinopyrimidine-4-carboxamide |
| **112** | 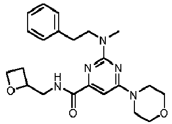 | 2-(methyl(phenethyl)amino)-6-morpholino-*N-*(oxetan-2-ylmethyl)pyrimidine-4-carboxamide |
| **113** | 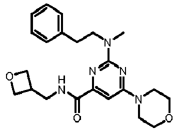 | 2-(methyl(phenethyl)amino)-6-morpholino-*N-*(oxetan-3-ylmethyl)pyrimidine-4-carboxamide |
| **114** | 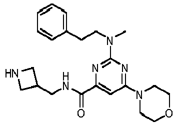 | *N*-(azetidin-3-ylmethyl)-2-(methyl(phenethyl)amino)-6-morpholinopyrimidine-4-carboxamide |
| **115** | 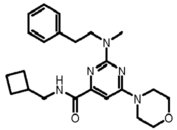 | *N*-(cyclobutylmethyl)-2-(methyl(phenethyl)amino)-6-morpholinopyrimidine-4-carboxamide |
| **116** | 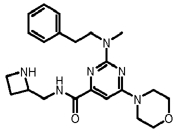 | *N*-(azetidin-2-ylmethyl)-2-(methyl(phenethyl)amino)-6-morpholinopyrimidine-4-carboxamide |
| **117** | 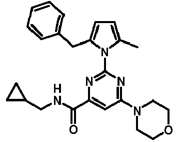 | 2-(2-benzyl-5-methyl-1 *H*-pyrrol-1 -yl)-*N-*(cyclopropylmethyl)-6-morpholinopyrimidine-4-carboxamide |
| **118** | 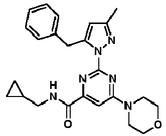 | 2-(5-benzyl-3-methyl-1 *H*-pyrazol-1 -yl)-*N-*(cyclopropylmethyl)-6-morpholinopyrimidine-4-carboxamide |
| **119** | 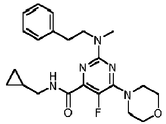 | *N*-(cyclopropylmethyl)-5-fluoro-2-(methyl(phenethyl)amino)-6-morpholinopyrimidine-4-carboxamide |
| **120** | 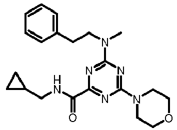 | *N*-(cyclopropylmethyl)-4-(methyl(phenethyl)amino)-6-morpholino-1,3,5-triazine-2-carboxamide |
| **121** | 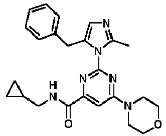 | 2-(5-benzyl-2-methyl-1 *H*-imidazol-1 -yl)-*N-*(cyclopropylmethyl)-6-morpholinopyrimidine-4-carboxamide |
| **122** | 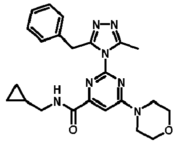 | 2-(3-benzyl-5-methyl-4H-1,2,4-triazol-4-yl)-*N-*(cyclopropylmethyl)-6-morpholinopyrimidine-4-carboxamide |
| **123** | 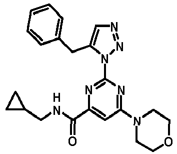 | 2-(5-benzyl-1 *H*-1,2,3-triazol-1 -yl)-*N-*(cyclopropylmethyl)-6-morpholinopyrimidine-4-carboxamide |
| **124** | 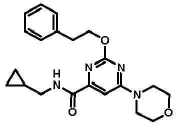 | *N*-(cyclopropylmethyl)-6-morpholino-2-phenethoxypyrimidine-4-carboxamide |
| **125** | 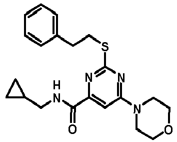 | *N*-(cyclopropylmethyl)-6-morpholino-2-(phenethylthio)pyrimidine-4-carboxamide |
| **126** | 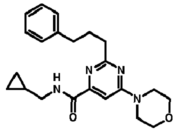 | *N*-(cyclopropylmethyl)-6-morpholino-2-(3-phenylpropyl)pyrimidine-4-carboxamide |
| **127** | 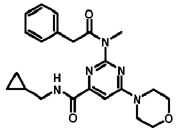 | *N*-(cyclopropylmethyl)-2-(*N*-methyl-2-phenylacetamido)-6-morpholinopyrimidine-4-carboxamide |
| **128** | 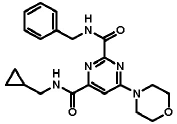 | *N*2-benzyl-*N*4-(cyclopropylmethyl)-6-morpholinopyrimidine-2,4-dicarboxamide |
| **129** | 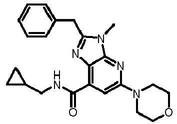 | 2-benzyl-*N*-(cyclopropylmethyl)-3-methyl-5-morpholino-3H-imidazo[4,5-*b*]pyridine-7-carboxamide |
| **130** | 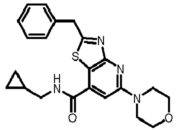 | 2-benzyl-*N*-(cyclopropylmethyl)-5-morpholinothiazolo[4,5-*b*]pyridine-7-carboxamide |
| **131** | 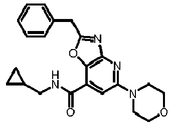 | 2-benzyl-*N*-(cyclopropylmethyl)-5-morpholinooxazolo[4,5-*b*]pyridine-7-carboxamide |
| **132** | 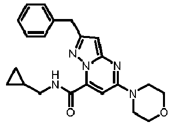 | 2-benzyl-*N*-(cyclopropylmethyl)-5-morpholinopyrazolo[1,5-*a*]pyrimidine-7-carboxamide |
| **133** | 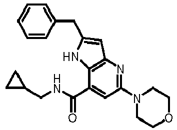 | 2-benzyl-*N*-(cyclopropylmethyl)-5-morpholino-1 *H*-pyrrolo[3,2-*b*]pyridine-7-carboxamide |
| **134** | 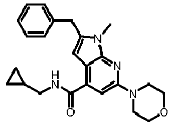 | 2-benzyl-*N*-(cyclopropylmethyl)-1-methyl-6-morpholino-1*H*-pyrrolo[2,3-*b*]pyridine-4-carboxamide |
| **135** | 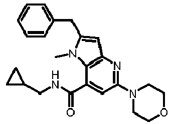 | 2-benzyl-*N*-(cyclopropylmethyl)-1-methyl-5-morpholino-1*H*-pyrrolo[3,2-*b*]pyridine-7-carboxamide |
| **136** | 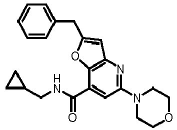 | 2-benzyl-*N*-(cyclopropylmethyl)-5-morpholinofuro[3,2-*b*]pyridine-7-carboxamide |
| **137** | 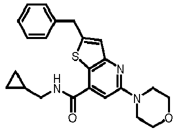 | 2-benzyl-*N*-(cyclopropylmethyl)-5-morpholinothieno[3,2-*b*]pyridine-7-carboxamide |
| **138** | 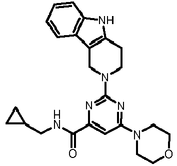 | *N*-(cyclopropylmethyl)-6-morpholino-2-(1,3,4,5-tetrahydro-2*H*-pyrido[4,3-*b*]indol-2-yl)pyrimidine-4-carboxamide |
| **139** | 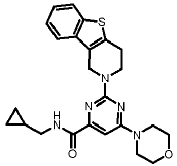 | *N*-(cyclopropylmethyl)-2-(3,4-dihydrobenzo[4,5]thieno[3,2-*c*]pyridin-2(1H)-yl)-6-morpholinopyrimidine-4-carboxamide |
| **140** | 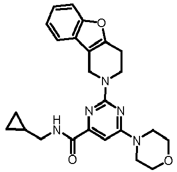 | *N*-(cyclopropylmethyl)-2-(3,4-dihydrobenzofuro[3,2-*c*]pyridin-2(1H)-yl)-6-morpholinopyrimidine-4-carboxamide |
| **141** | 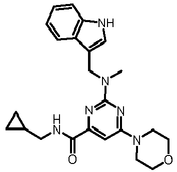 | 2-(((1 *H*-indol-3-yl)methyl)(methyl)amino)-*N-*(cyclopropylmethyl)-6-morpholinopyrimidine-4-carboxamide |
| **142** | 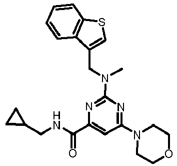 | 2-((benzo[*b*]thiophen-3-ylmethyl)(methyl)amino)-*N*-(cyclopropylmethyl)-6-morpholinopyrimidine-4-carboxamide |
| **143** | 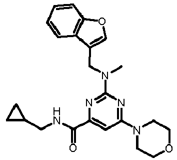 | 2-((benzofuran-3-ylmethyl)(methyl)amino)-*N-*(cyclopropylmethyl)-6-morpholinopyrimidine-4-carboxamide |
| **144** | 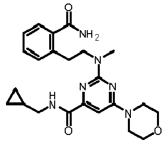 | 2-((2-carbamoylphenethyl)(methyl)amino)-*N-*(cyclopropylmethyl)-6-morpholinopyrimidine-4-carboxamide |
| **145** | 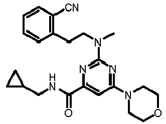 | 2-((2-cyanophenethyl)(methyl)amino)-*N-*(cyclopropylmethyl)-6-morpholinopyrimidine-4-carboxamide |
| **146** | 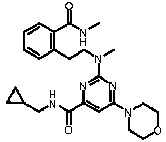 | *N-*(cyclopropylmethyl)-2-(methyl(2-(methylcarbamoyl)phenethyl)amino)-6-morpholinopyrimidine-4-carboxamide |
| **147** | 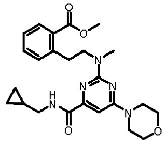 | methyl2-(2-((4-((cyclopropylmethyl)carbamoyl)-6-morpholinopyrimidin-2-yl) (methyl)amino)ethyl)benzoate |
| **148** | 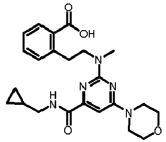 | 2-(2-((4-((cyclopropylmethyl)carbamoyl)-6-morpholinopyrimidin-2-yl)(methyl)amino)ethyl)benzoic acid |
| **149** | 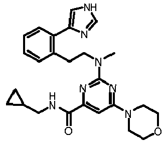 | 2-((2-(1*H*-imidazol-4-yl)phenethyl)(methyl)amino)-*N-*(cyclopropylmethyl)-6-morpholinopyrimidine-4-carboxamide |
| **150** | 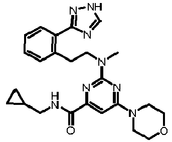 | 2-((2-(1 *H*-1,2,4-triazol-3-yl)phenethyl)(methyl)amino)-*N-*(cyclopropylmethyl)-6-morpholinopyrimidine-4-carboxamide |
| **151** | 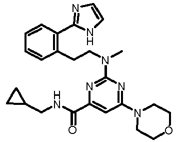 | 2-((2-(1 *H*-imidazol-2-yl)phenethyl)(methyl)amino)-*N-*(cyclopropylmethyl)-6-morpholinopyrimidine-4-carboxamide |
| **152** | 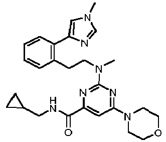 | *N*-(cyclopropylmethyl)-2-(methyl(2-(1-methyl-1 *H*-imidazol-4-yl)phenethyl)amino)-6-morpholinopyrimidine-4-carboxamide |
| **153** | 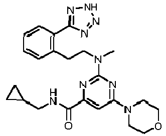 | 2-((2-(2H-tetrazol-5-yl)phenethyl)(methyl)amino)-*N-*(cyclopropylmethyl)-6-morpholinopyrimidine-4-carboxamide |
| **154** | 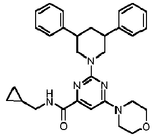 | *N*-(cyclopropylmethyl)-2-(3,5-diphenylpiperidin-1-yl)-6-morpholinopyrimidine-4-carboxamide |
| **155** | 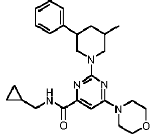 | *N*-(cyclopropylmethyl)-2-(3-methyl-5-phenylpiperidin-1-yl)-6-morpholinopyrimidine-4-carboxamide |
| **156** | 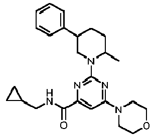 | *N*-(cyclopropylmethyl)-2-(2-methyl-5-phenylpiperidin-1-yl)-6-morpholinopyrimidine-4-carboxamide |
| **157** | 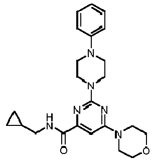 | *N*-(cyclopropylmethyl)-6-morpholino-2-(4-phenylpiperazin-1-yl)pyrimidine-4-carboxamide |
| **158** | 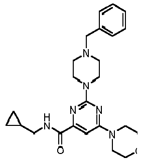 | 2-(4-benzylpiperazin-1-yl)-*N-*(cyclopropylmethyl)-6-morpholinopyrimidine-4-carboxamide |
| **159** | 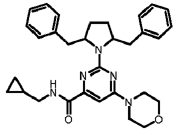 | *N*-(cyclopropylmethyl)-2-(2,5-dibenzylpyrrolidin-1-yl)-6-morpholinopyrimidine-4-carboxamide |
| **160** | 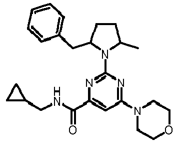 | 2-(2-benzyl-5-methylpyrrolidin-1-yl)-*N-*(cyclopropylmethyl)-6-morpholinopyrimidine-4-carboxamide |
| **161** | 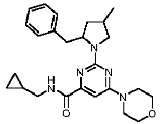 | 2-(2-benzyl-4-methylpyrrolidin-1-yl)-*N-*(cyclopropylmethyl)-6-morpholinopyrimidine-4-carboxamide |
| **162** | 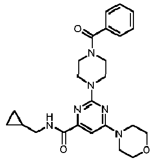 | 2-(4-benzoylpiperazin-1-yl)-*N-*(cyclopropylmethyl)-6-morpholinopyrimidine-4-carboxamide |
| **163** | 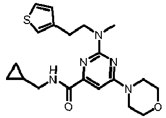 | *N*-(cyclopropylmethyl)-2-(methyl(2-(thiophen-3-yl)ethyl)amino)-6-morpholinopyrimidine-4-carboxamide |
| **164** | 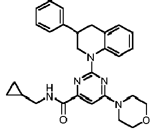 | *N*-(cyclopropylmethyl)-6-morpholino-2-(3-phenyl-3,4-dihydroquinolin-1(2*H*)-yl)pyrimidine-4-carboxamide |
| **165** | 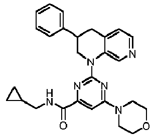 | *N*-(cyclopropylmethyl)-6-morpholino-2-(3-phenyl-3,4-dihydro-1,7-naphthyridin-1(2*H*)-yl)pyrimidine-4-carboxamide |
| **166** | 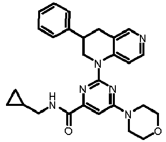 | *N*-(cyclopropylmethyl)-6-morpholino-2-(3-phenyl-3,4-dihydro-1,6-naphthyridin-1(2*H*)-yl)pyrimidine-4-carboxamide |
| **167** | 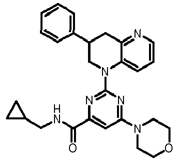 | *N*-(cyclopropylmethyl)-6-morpholino-2-(3-phenyl-3,4-dihydro-1,5-naphthyridin-1(2*H*)-yl)pyrimidine-4-carboxamide |
| **168** | 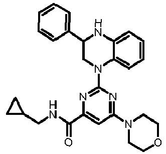 | *N*-(cyclopropylmethyl)-6-morpholino-2-(3-phenyl-3,4-dihydroquinoxalin-1(2*H*)-yl)pyrimidine-4-carboxamide |
| **169** | 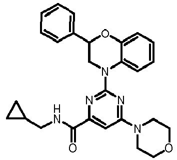 | *N*-(cyclopropylmethyl)-6-morpholino-2-(2-phenyl-2,3-dihydro-4H-benzo[*b*][1,4]oxazin-4-yl)pyrimidine-4-carboxamide |
| **170** | 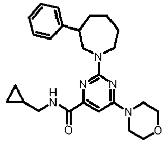 | *N*-(cyclopropylmethyl)-6-morpholino-2-(3-phenylazepan-1-yl)pyrimidine-4-carboxamide |
| **171** | 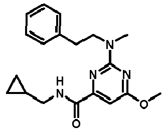 | *N*-(cyclopropylmethyl)-6-methoxy-2-(methyl(phenethyl)amino)pyrimidine-4-carboxamide |
| **172** | 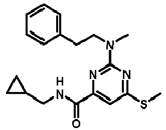 | *N*-(cyclopropylmethyl)-2-(methyl(phenethyl)amino)-6-(methylthio)pyrimidine-4-carboxamide |
| **173** | 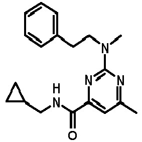 | *N*-(cyclopropylmethyl)-2-(methyl(phenethyl)amino)pyrimidine-4-carboxamide |
| **174** | 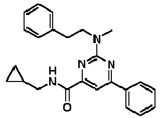 | *N*-(cyclopropylmethyl)-2-(methyl(phenethyl)amino)-6-phenylpyrimidine-4-carboxamide |
| **175** | 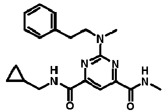 | *N*4-(cyclopropylmethyl)-*N*6-methyl-2-(methyl(phenethyl)amino)pyrimidine-4,6-dicarboxamide |
| **176** | 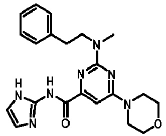 | *N*-(1*H*-imidazol-2-yl)-2-(methyl(phenethyl)amino)-6-morpholinopyrimidine-4-carboxamide |
| **177** | 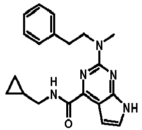 | *N*-(cyclopropylmethyl)-2-(methyl(phenethyl)amino)-7*H*-pyrrolo[2,3-*d*]pyrimidine-4-carboxamide |
| **178** | 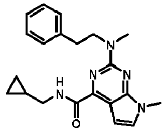 | *N*-(cyclopropylmethyl)-7-methyl-2-(methyl(phenethyl)amino)-7*H*-pyrrolo[2,3-*d*]pyrimidine-4-carboxamide |
| **179** | 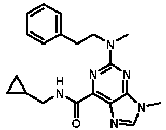 | *N*-(cyclopropylmethyl)-9-methyl-2-(methyl(phenethyl)amino)-9*H*-purine-6-carboxamide |
| **180** | 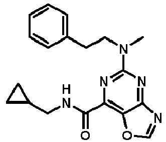 | *N*-(cyclopropylmethyl)-5-(methyl(phenethyl)amino)oxazolo[4,5-*d*]pyrimidine-7-carboxamide |
| **181** | 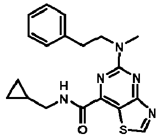 | *N*-(cyclopropylmethyl)-5-(methyl(phenethyl)amino)thiazolo[4,5-*d*]pyrimidine-7-carboxamide |
| **182** | 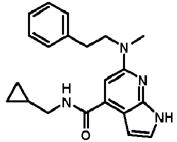 | *N*-(cyclopropylmethyl)-6-(methyl(phenethyl)amino)-1*H*-pyrrolo[2,3-*b*]pyridine-4-carboxamide |
| **183** | 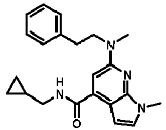 | *N*-(cyclopropylmethyl)-1-methyl-6-(methyl(phenethyl)amino)-1*H*-pyrrolo[2,3-*b*]pyridine-4-carboxamide |
| **184** | 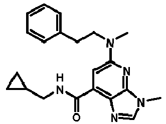 | *N*-(cyclopropylmethyl)-3-methyl-5-(methyl(phenethyl)amino)-3*H*-imidazo[4,5-*b*]pyridine-7-carboxamide |
| **185** | 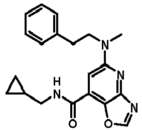 | *N*-(cyclopropylmethyl)-5-(methyl(phenethyl)amino)oxazolo[4,5-*b*]pyridine-7-carboxamide |
| **186** | 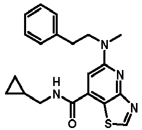 | *N*-(cyclopropylmethyl)-5-(methyl(phenethyl)amino)thiazolo[4,5-*b*]pyridine-7-carboxamide |
| **187** | 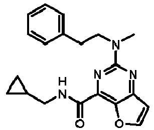 | *N*-(cyclopropylmethyl)-2-(methyl(phenethyl)amino)furo[3,2-*d*]pyrimidine-4-carboxamide |
| **188** | 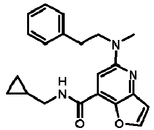 | *N*-(cyclopropylmethyl)-5-(methyl(phenethyl)amino)furo[3,2-*b*]pyridine-7-carboxamide |
| **189** | 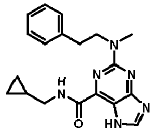 | *N*-(cyclopropylmethyl)-2-(methyl(phenethyl)amino)-7*H*-purine-6-carboxamide |
| **190** | 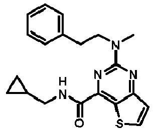 | *N*-(cyclopropylmethyl)-2-(methyl(phenethyl)amino)thieno[3,2-*d*]pyrimidine-4-carboxamide |
| **191** | 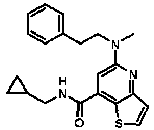 | *N*-(cyclopropylmethyl)-5-(methyl(phenethyl)amino)thieno[3,2-*b*]pyridine-7-carboxamide |
| **192** | 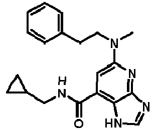 | *N*-(cyclopropylmethyl)-5-(methyl(phenethyl)amino)-1*H*-imidazo[4,5-*b*]pyridine-7-carboxamide |
| **193** | 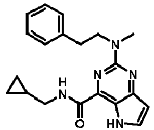 | *N*-(cyclopropylmethyl)-2-(methyl(phenethyl)amino)-5*H*-pyrrolo[3,2-*d*]pyrimidine-4-carboxamide |
| **194** | 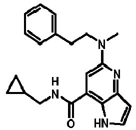 | *N*-(cyclopropylmethyl)-5-(methyl(phenethyl)amino)-1*H*-pyrrolo[3,2-*b*]pyridine-7-carboxamide |
| **195** | 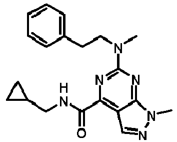 | *N*-(cyclopropylmethyl)-1-methyl-6-(methyl(phenethyl)amino)-1*H*-pyrazolo[3,4-*d*]pyrimidine-4-carboxamide |
| **196** | 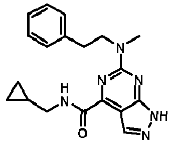 | *N*-(cyclopropylmethyl)-6-(methyl(phenethyl)amino)-1*H*-pyrazolo[3,4-*d*]pyrimidine-4-carboxamide |
| **197** | 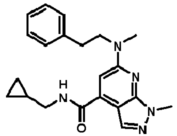 | *N*-(cyclopropylmethyl)-1-methyl-6-(methyl(phenethyl)amino)-1*H*-pyrazolo[3,4-*b*]pyridine-4-carboxamide |
| **198** | 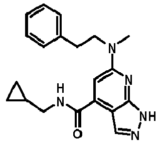 | *N*-(cyclopropylmethyl)-6-(methyl(phenethyl)amino)-1*H*-pyrazolo[3,4-*b*]pyridine-4-carboxamide |
| **199** | 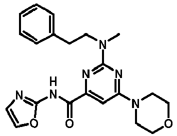 | 2-(methyl(phenethyl)amino)-6-morpholino-*N-*(oxazol-2-yl)pyrimidine-4-carboxamide |
| **200** | 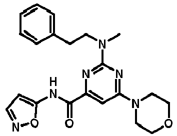 | *N*-(isoxazol-5-yl)-2-(methyl(phenethyl)amino)-6-morpholinopyrimidine-4-carboxamide |
| **201** | 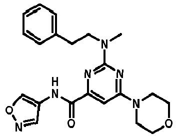 | *N*-(isoxazol-4-yl)-2-(methyl(phenethyl)amino)-6-morpholinopyrimidine-4-carboxamide |
| **202** | 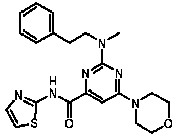 | 2-(methyl(phenethyl)amino)-6-morpholino-*N-*(thiazol-2-yl)pyrimidine-4-carboxamide |
| **203** | 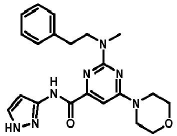 | 2-(methyl(phenethyl)amino)-6-morpholino-*N-*(thiazol-2-yl)pyrimidine-4-carboxamide |
| **204** | 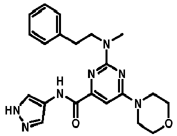 | 2-(methyl(phenethyl)amino)-6-morpholino-*N-*(1*H*-pyrazol-4-yl)pyrimidine-4-carboxamide |
| **205** | 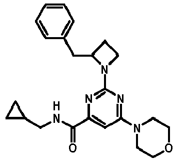 | 2-(2-benzylazetidin-1-yl)-*N*-(cyclopropylmethyl)-6-morpholinopyrimidine-4-carboxamide |

A further aspect of the present invention relates to the production of compound of the formula (I). As shown in Scheme 1, a method for producing the compound of the present invention comprises:
**Step (0A):** providing a compound **1A** having an activated carboxylic group 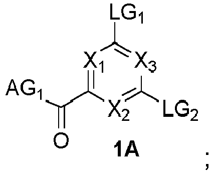
**Step 1A:** performing coupling reaction of the compound **1A** with an amine H₂N-R³ in the presence of a first base to obtain an intermediate compound **2A** 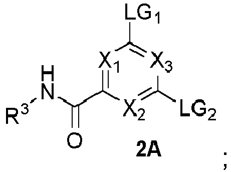
**Step 2A:** performing coupling reaction of the compound **2A with** H-R² in the presence of a second base to obtain an intermediate compound **3A** 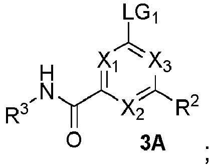
**Step 3A:** performing coupling reaction of the compound **3A** with H-R¹ in the presence of a third base and/or a catalyst to obtain a compound of the formula (I) 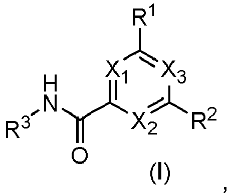 wherein LG₁ and LG₂ are leaving groups; AG₁ is an activating group; and X₁, X₂, X₃, R¹, R², R³ have the same meanings as defined in the formula (I).

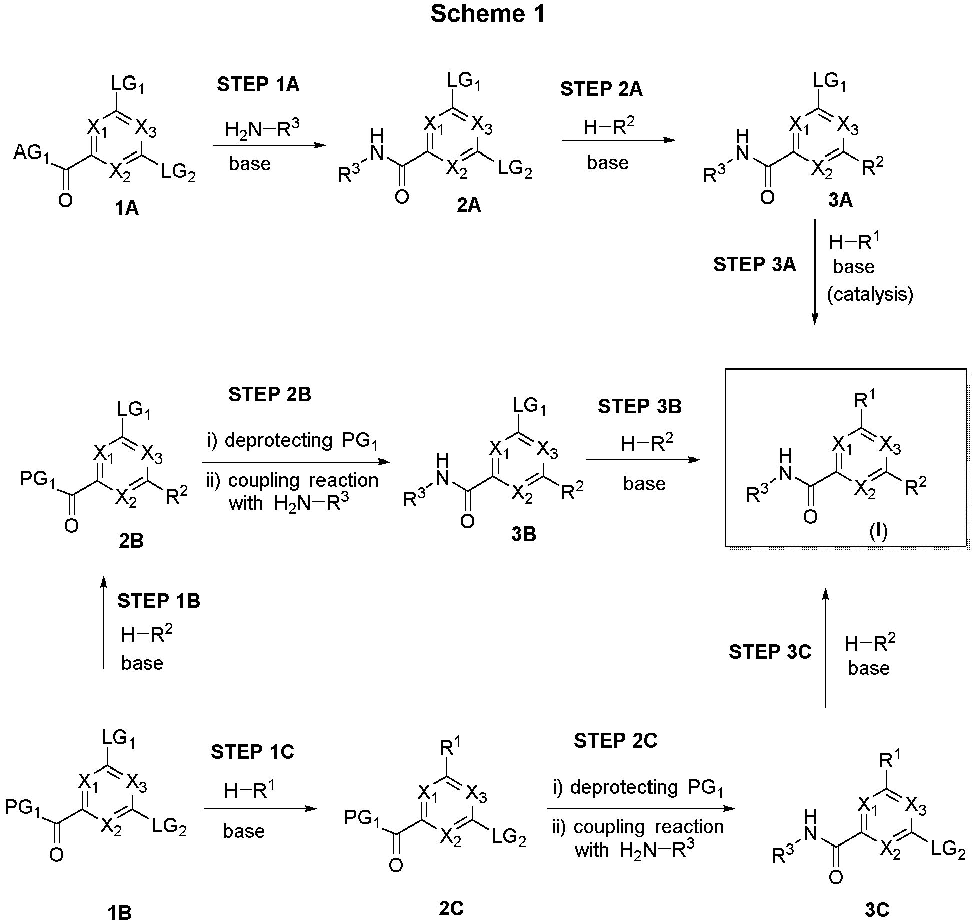

As shown in Scheme 1, an alternative method for producing the compound of the present invention comprises:
**Step (0B)**: providing a compound **1B** having a protected carboxylic group 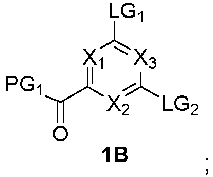
**Step 1B:** performing coupling reaction of the compound **1B** with H-R² in the presence of a first base to obtain an intermediate compound **2B** 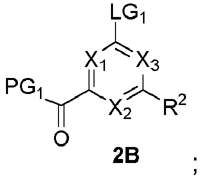
**Step 2B:**
   i) deprotecting the carboxylic protecting group PG₁,
   ii) performing coupling reaction of a resulting compound obtained by the step i) with an amine H₂N-R³ in the presence of a second base to obtain an intermediate compound **3B** 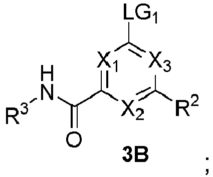
**Step 3C:** performing coupling reaction of the compound **3B** with H-R¹ in the presence of a third base and/or a catalyst to obtain a compound of the formula (I) 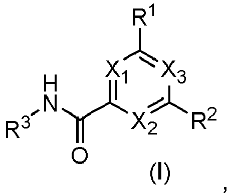 wherein LG₁ and LG₂ are leaving groups; PG₁ is a protecting group; and X₁, X₂, X₃, R¹, R², R³ have the same meanings as defined in the formula (I).

Further alternative method for producing the compound of the present invention comprises:
**Step (0B):** providing a compound **1B** having a protected carboxylic group 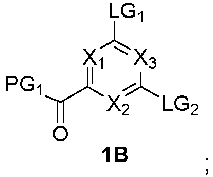
**Step 1C:** performing coupling reaction of the compound **1B** with H-R¹ in the presence of a first base and/or a catalyst to obtain an intermediate compound **2C** 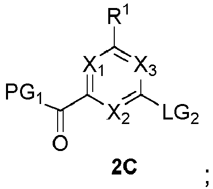
**Step 2C:**
   i) deprotecting the carboxylic protecting group PG₁,
   ii) performing coupling reaction of a resulting compound obtained by the step i) with an amine H₂N-R³ in the presence of a second base to obtain an intermediate compound **3C** 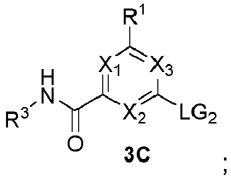
**Step 3C:** performing coupling reaction of the compound **3C** with H-R² in the presence of a third base to obtain a compound of the formula (I) 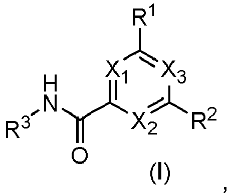 wherein LG₁ and LG₂ are leaving groups; PG₁ is a protecting group; and X₁, X₂, X₃, R¹, R², R³ have the same meanings as defined in the formula (I).

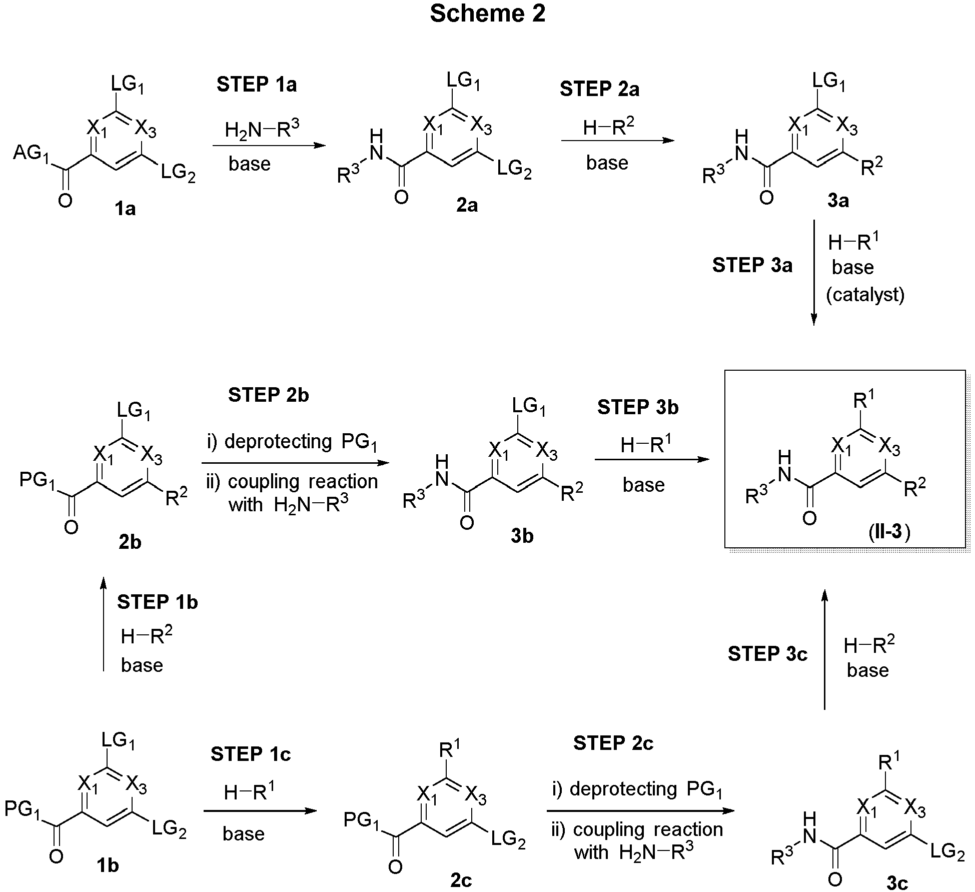

As shown in Scheme 2, a method for producing the preferred compound of the fomula **(II-3)** comprises:
**Step (0a):** providing a compound **1a** having an activated carboxylic group 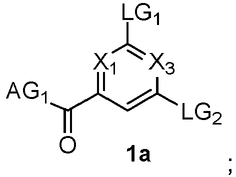
**Step 1a:** performing coupling reaction of the compound **1a** with an amine H₂N-R³ in the presence of a first base to obtain an intermediate compound **2a** 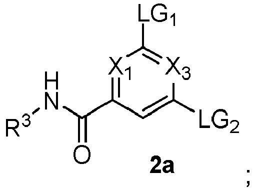
**Step 2a:** performing coupling reaction of the compound **2a** with H-R² in the presence of a second base to obtain an intermediate compound **3a** 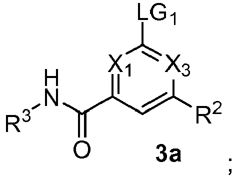
**Step** 3a: performing coupling reaction of the compound **3a** with H-R¹ in the presence of a third base and/or a catalyst to obtain a compound of the formula **(II-3)** 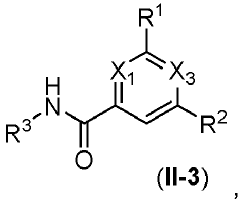 wherein LG₁ and LG₂ are leaving groups; AG₁ is an activating group; and X₁, X₃, R¹, R², R³ have the same meanings as defined in the formula (II-3).

As shown in Scheme 1, an alternative method for producing the preferred compound of the fomula **(II-3)** comprises:
**Step (0b)**: providing a compound **1b** having a protected carboxylic group 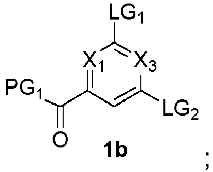
**Step 1b:** performing coupling reaction of the compound **1b** with H-R² in the presence of a first base to obtain an intermediate compound **2b** 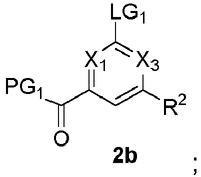
**Step 2b:**
   i) deprotecting the carboxylic protecting group PG₁,
   ii) performing coupling reaction of a resulting compound obtained by the step i) with an amine H₂N-R³ in the presence of a second base to obtain an intermediate compound **3b** 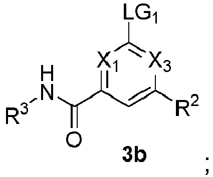
**Step 3c:** performing coupling reaction of the compound **3b** with H-R¹ in the presence of a third base and/or a catalyst to obtain a compound of the formula **(II-3)** 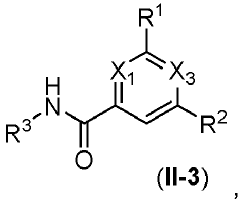 wherein LG₁ and LG₂ are leaving groups; PG₁ is a protecting group; and X₁, X₃, R¹, R², R³ have the same meanings as defined in the formula (II-3).

Further alternative method for producing the preferred compound of the fomula **(II-3)** comprises:
**Step (0b)**: providing a compound **1b** having a protected carboxylic group 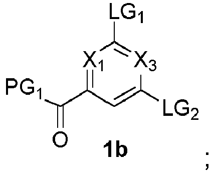
**Step 1b:** performing coupling reaction of the compound **1b** with H-R¹ in the presence of a first base and/or a catalyst to obtain an intermediate compound **2c** 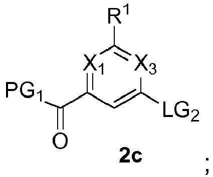
**Step 2c:**
   i) deprotecting the carboxylic protecting group PG₁,
   ii) performing coupling reaction of a resulting compound obtained by the step i) with an amine H₂N-R³ in the presence of a second base to obtain an intermediate compound **3c** 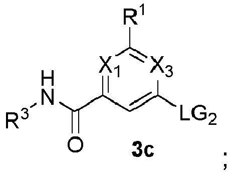
**Step 3c:** performing coupling reaction of the compound **3c** with H-R² in the presence of a third base to obtain a compound of the formula **(II-3)** 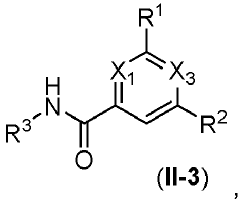 wherein LG₁ and LG₂ are leaving groups; PG₁ is a protecting group; and X₁, X₃, R¹, R², R³ have the same meanings as defined in the formula (II-3).

In the above-described synthetic method for producing the compound of the general formula **(I),** preferred the compound of the fomula **(II-3), AG₁** is an activating group, **LG₁** and **LG₂ are** leaving groups; and **R¹, R², R³,X₁, X₂,** and **X₃** have the same meanings as defined as above.

The term "protecting groups" as used herein refers to commonly used protection groups in organic synthesis, preferably for amino and carboxyl groups. PG¹ is suitable protecting group for a carboxyl group. Prerfably, PG¹ may be selected from the group consisting of or comprising: methoxy, ethoxy, isobutoxy, *tert*-butoxy, benzyloxy; preferably, methoxy group.

Leaving groups LG₁ and LG₂ may be selected from the group consisting of or comprising: halides such as -F, -Br, -Cl, -I, and perfluoroalkylsulfonates such as triflate (-OSO₂CF₃) and nonaflate (-OSO₂C₄F₉).

For the coupling reaction of H-R¹ performed in Steps 3A, 3a, 3B, 3b, 1C, 1c, the coupling reaction as used herein refers to commonly used in aromatic substitution reaction in the presence of a base selected from trialkylamine such as Et₃N, or DIPEA, a metal hyoxide such as NaOH, KOH and a metal carbonate such as Li₂CO₃, Na₂CO₃, K₂CO₃, in a polar protic solevent at a reactions temperature in a range of 80 to 250°C, preferred, 100 to 200°C, more preferred 100 to 200, and most preferred 120 to 170°C. This coupling reaction may be carried out in a oil bath or by means of a microwave reactor.

For the coupling reaction of H-R² performed in Steps 1B, 1b, 2A, 2a, 3C, and 3c, the coupling reaction as used herein refers to commonly used in aromatic substitution reaction in the presence of a base such as Et₃N, or DIPEA, or an C-N coupling reaction in the presence of a specific catalysis at a reactions temperature in a range of -20 to 30°C, preferred, -10 to 20°C, more preferred -10 to 10, and most preferred -5 to 5°C. The specific catalysis for C-N coupling reaction is selected from Pd, Cu, and Ni catalyses, preferred Pd catalyses with various phosphine ligands. More preferred, any of Buchwald type Pd catalyses, for examples, BrettPhosPd G1, BrettPhosPd G2, BrettPhosPd G3, BrettPhosPd G4, RuPhosPd G1, RuPhosPd G2, RuPhosPd G3, RuPhosPd G4, RuPhosPd(allyl)CI, RuphosPd(crotyl)cl, RuphosPd(cinamyl)CI, SPhosPd G1, SPhosPd G2, SPhosPd G3, SPhosPd G4, XPhos Pd G1, XPhos Pd G2, XPhos Pd G3, XPhos Pd G4, XPhosPd(crotyl)CI, tBuXPhos Pd G1, tBuXPhos Pd G2, tBuXPhos Pd G3, tBuXPhos Pd G4, is used.

A ratio of the specific catalysis to the starting meterial is in the range of 0.01 to 20 mol-%, preferred 0.1-10 mol-%, more preferred 0.1-5 mol-%, most preferred 0.5-1 mol-%. For perfoming the amide coupling reaction in Steps 1A, 2B and 2c, firstly carboxylic acid group is activated by introducing an activating group (AG₁) and promote the coupling reaction with amino group of H₂N-R³ at a reactions temperature in a range of - 100 to 20°C, preferred, -100 to 10°C, more preferred -80 to 0 °C. The activating group (AG₁) of carboxylic acid may be introduced by a separate reaction or *in situ* reaction. Prerfably, the activating group may be selected from the group consisting of or comprising: halides such as -F, -Br, -CI, -I, anhydride group such as -OCOCH₃, N-oxy-benzotriazol group and N-oxy-succinimide. Preferably, the activating group is introduced *in situ* and it is well-known as amide coupling reaction. Any of the following coupling reagent can be used to introduce activating group: BOP, PyBOP, AOP, PyAOP, TBTU, EEDQ, Polyphosphoric Acid (PPA), DPPA, HATU, HOBt, HOAt, DCC, EDCI, BOP-Cl, TFFH, Brop, PyBrop, and CIP.

The pharmaceutically acceptable salts of the compound of the present invention may be formed with organic or inorganic acids or bases. Examples of suitable acids for such acid addition salt formation are hydrochloric acid, hydrobromic acid, sulfuric acid, phosphoric acid, acetic acid, citric acid, oxalic acid, malonic acid, salicylic acid, p-aminosalicylic acid, malic acid, fumaric acid, succinic acid, ascorbic acid, maleic acid, sulfonic acid, phosphonic acid, perchloric acid, nitric acid, formic acid, propionic acid, gluconic acid, lactic acid, tartaric acid, hydroxymaleic acid, pyruvic acid, phenylacetic acid, benzoic acid, p-aminobenzoic acid, p-hydroxybenzoic acid, methanesulfonic acid, ethanesulfonic acid, nitrous acid, hydroxyethanesulfonic acid, ethylenesulfonic acid, p-toluenesulfonic acid, naphthylsulfonic acid, sulfanilic acid, camphorsulfonic acid, china acid, mandelic acid, o-methylmandelic acid, hydrogen-benzenesulfonic acid, picric acid, adipic acid, d-o-tolyltartaric acid, tartronic acid, (o, m, p)-toluic acid, naphthylamine sulfonic acid, trifluoroacetic acid, and other mineral or carboxylic acids well known to those skilled in the art. The salts are prepared by contacting the free base form with a sufficient amount of the desired acid to produce a salt in the conventional manner.

In the case the inventive compounds bear acidic groups, salts could also be formed with inorganic or organic bases. Examples for suitable inorganic or organic bases are, for example, NaOH, KOH, NH₄OH, tetraalkylammonium hydroxide, lysine or arginine and the like. Salts may be prepared in a conventional manner using methods well known in the art, for example by treatment of a solution of the compound of the general formula (I) with a solution of an acid, selected out of the group mentioned above.

Therefore another aspect of the present invention relates to compound according to the general formula (I) for use as medicament as well as use thereof in medicine. Especially preferred is the use in anticoagulation and as an inhibitor of enzyme NAPE-PLD.

The compounds according to general formula (I) described herein are especially suitable for the treatment or prophylaxis of diseases associated with and/or caused by enzyme NAPE-PLD: 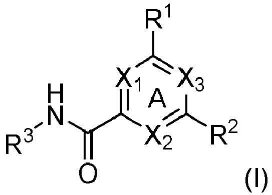 wherein
in a ring **A, X₁** is N, or C**R⁴; X₂** is N or C**R⁵; X₃** is N or CH; with the proviso that at least one of **X₁** and **X₃** is N;
**R¹** represents **-R⁶,** -O**R⁷,** -S**R⁷,** -N**R⁸R⁹,** or -CON**R⁸R⁹,** or **R¹** and **R⁴** form 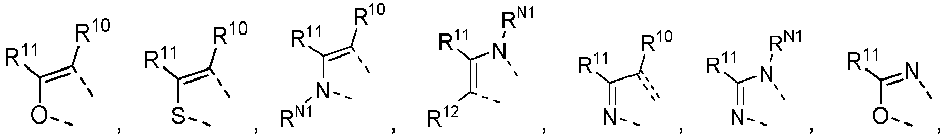 or 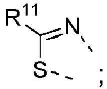
**R²** represents **-R¹⁶,** -O**R¹⁷,** -S**R¹⁷,** -N**R¹⁸R¹⁹,** -CON**R¹⁸R¹⁹,** -N**R¹⁸**CH**R^{'}**CO₂**R¹⁹,**
or **R²** and **R⁵** form 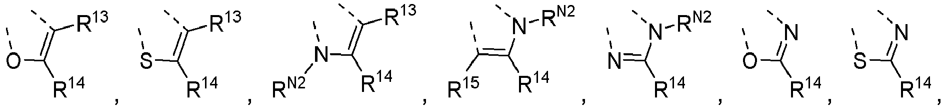 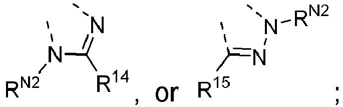
**R³** represents C₁₋C₆ alkyl, C₁₋C₆ alkyl-OH, C₁₋C₆ alkyl-CO₂**R²⁰**, C₁₋C₆ alkyl-CONH**R²⁰**, C₂-C₆ alkenyl, C₂-C₆ alkynyl, 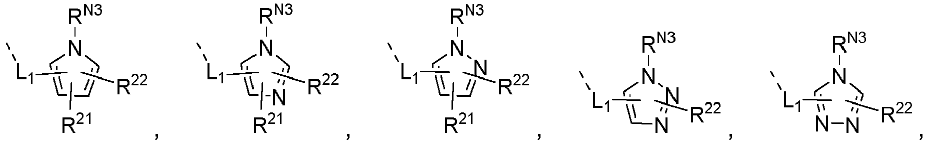 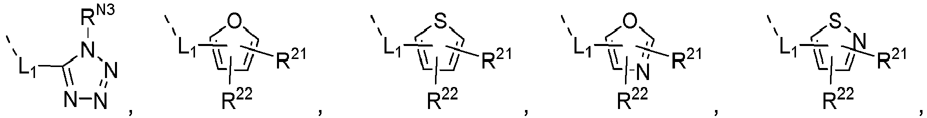 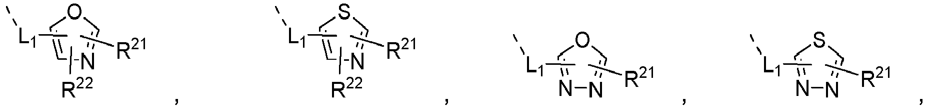 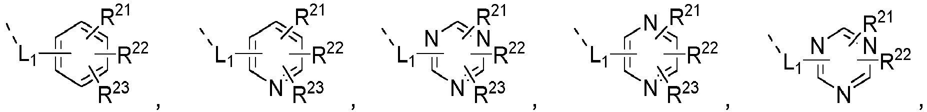 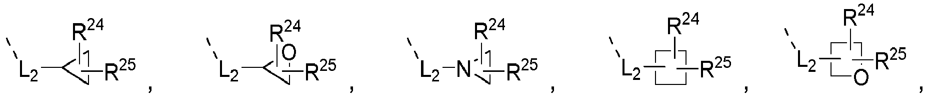 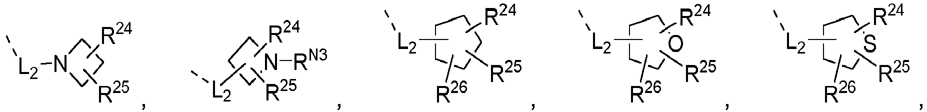 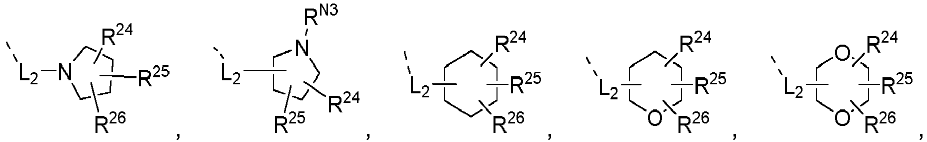 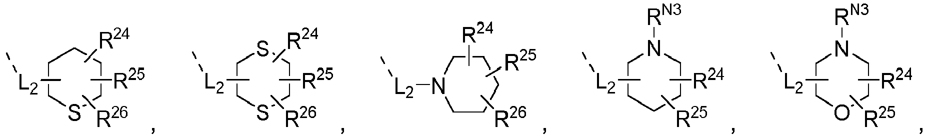 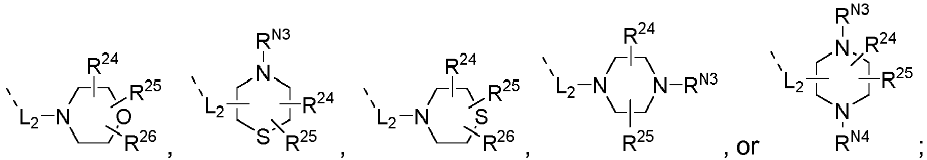
**R**⁴ and **R⁵** represents -H, -F, -CI, -Br, -I, C₁-C₃ alkyl, C₁-C₃ alkoxy, -CF₃ or -OCF₃;
**R⁶, R⁷** and **R⁸** represent independently of each other, C₁-C₆ alkyl, C₃-C₆ cycloalkyl, -L₃-**R^{A}**, -L₄-**R^{B}**;
**R^{8'}, R⁹, R^{9'} , R¹⁰,** and **R¹¹** represent independently of each other, -H, C₁-C₆ alkyl, C₃-C₆ cycloalkyl, -L₃-**R^{A}**, -L₄-**R^{B}**;
**R^{A}** and **R^{B}** represent independently of each other 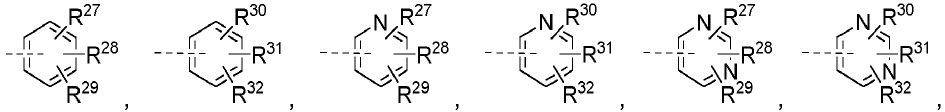 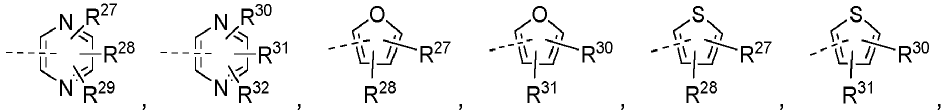 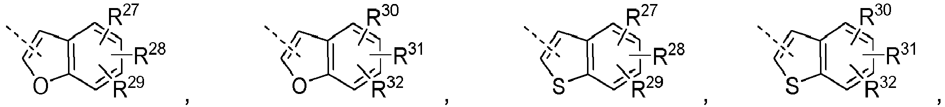 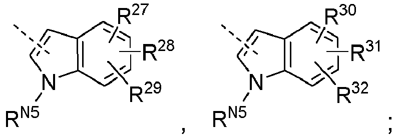
or -N**R⁸R⁹** represents 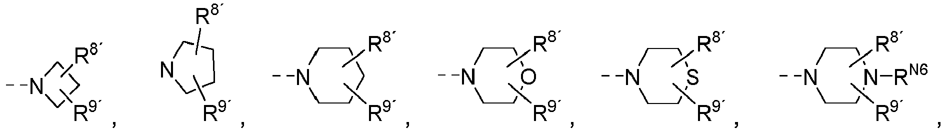 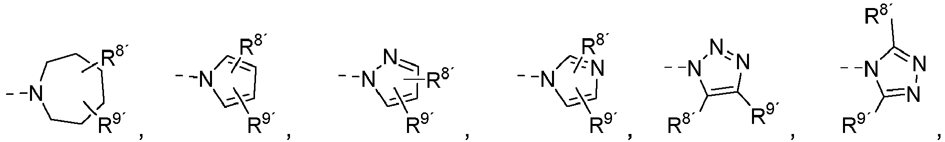 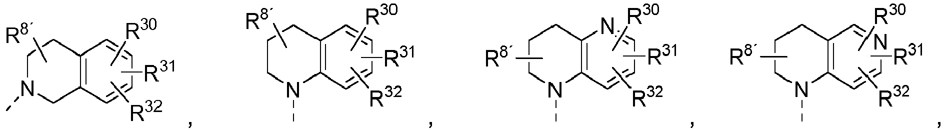 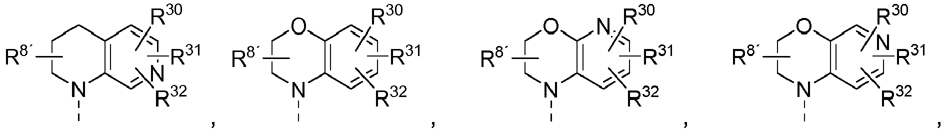 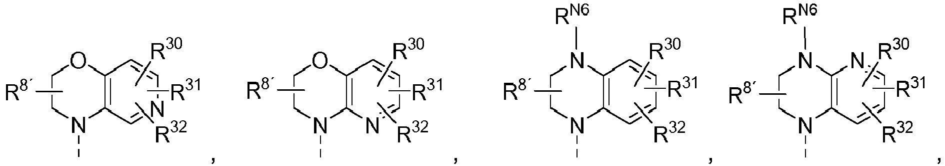 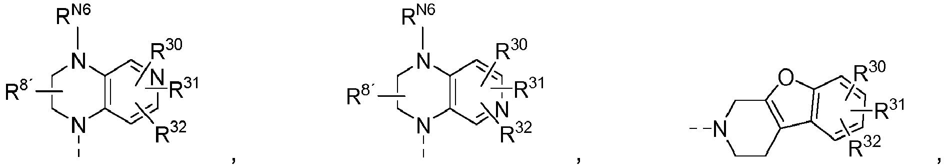 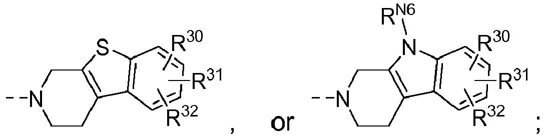
**R¹², R¹³, R¹⁴** and **R¹⁵** represent independently of each other -H, -CI, -F, -CF₃, C₁-C₃ alkyl, or C₁-C₃ alkoxy;
**R¹⁶, R¹⁷, R¹⁸** and **R¹⁹** represent independently of each other C₁₋C₆ alkyl, C₃-C₆ cycloalkyl, C₁₋C₆ alkylOH, C₁₋C₆ alkylCO₂**R^{20'},** 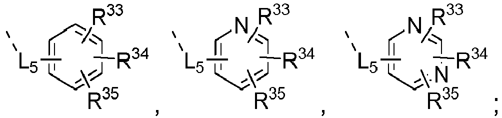 or -N**R¹⁸R¹⁹** represents 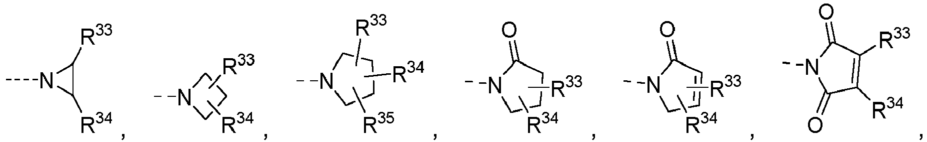 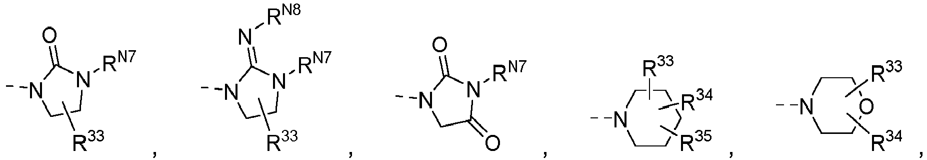 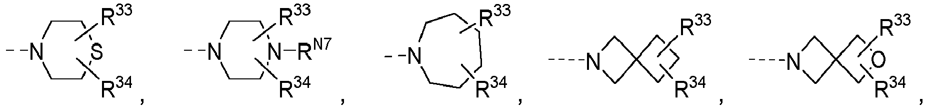 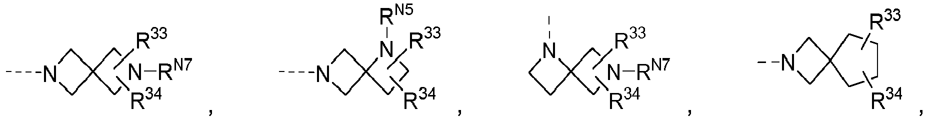 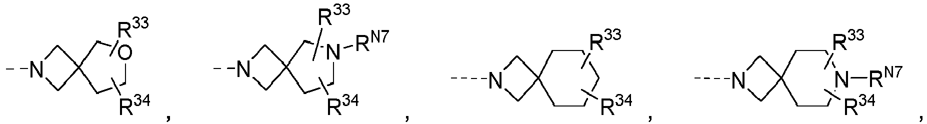 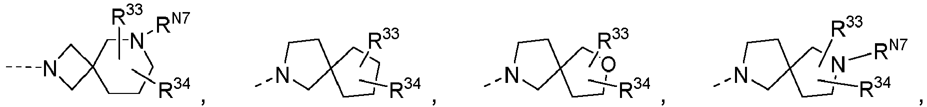 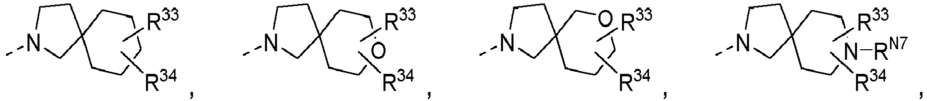 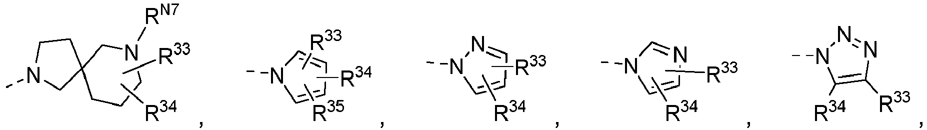 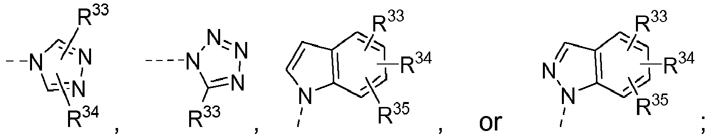
**R^{'}** represents -H, -CH₃, -CH₂OH, -CH₂SH, -CH₂CO₂H, -CH₂CONH₂, -CH(OH)CH₃, -CH(CH₃)₂, -CH₂CH(CH₃)₂, -CH(CH₃)CH₂CH₃, -CH₂CH₂SCH₃, -CH₂CH₂CO₂H, -CH₂CH₂CONH₂, -CH₂Ph, -CH₂CH₂CH₂NHC(=NH)NH₂, -CH₂CH₂CH₂CH₂N H₂, 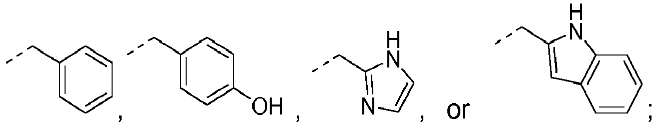
**L¹, L², L³, L⁴** and **L⁵** represent independently of each other a covalent bond, C₁₋C₆ alkyl group, linear C₂-C₆ alkenyl group, wherein one or more of -CH₂- groups present in the C₁₋C₆ alkyl, or C₂-C₆ alkenyl group are optionally and independently of each other replaced by -O-, -S-, -N(R^{N9})-, -CO-, -COO-, -OOC-, -CON(R^{N9})-, -N(R^{N9})CO-, -NHCON(R^{N9})-, -N(R^{N9})CONH-, -SO-, -SO₂- , -SO₂N(R^{N9})-, or -N(R^{N9})SO₂-;
**R^{N1}, R^{N2}, R^{N3}, R^{N4}, R^{N5}, R^{N6}, R^{N7}, R^{N8},** and **R^{N9}** represent independently of each other -H, -CH₃, -C₂H₅, -C₃H₇, -CH(CH₃)₂, -C₄H₉, -CH₂-CH(CH₃)₂, -CH(CH₃)-C₂H₅, -C(CH₃)₃, -cyclo-C₃H₅, -CH₂-cyclo-C₃H₅, -Ph, -CH₂Ph, -CH₂F, -CHF₂, -CF₃, -CH₂Cl, -CH₂Br, -CH₂l, -CH₂-CH₂F, -CH₂-CHF₂, -CH₂-CF₃, -CH₂-CH₂Cl, -CH₂-CH₂Br, -CH₂-CH₂l, -CH₂-CH=CH₂, -CH₂-C≡CH, -OH, -OCH₃, -CHO, -COCH₃, -COC₂H₅, -COC₃H₇, -COCH(CH₃)₂, -COC(CH₃)₃, -COOCH₃, -COOC₂H₅, -COOC₃H₇, -COOCH(CH₃)₂, -COOC(CH₃)₃, 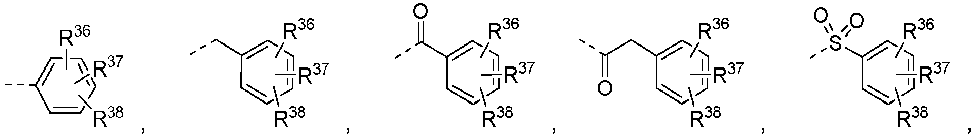 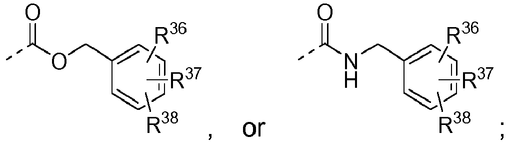
**R²⁰** and **R^{20'}** represent independently of each other -H, -CH₃, -C₂H₅, -C₃H₇, -CH(CH₃)₂, -C₄H₉, -CH₂-CH(CH₃)₂, -CH(CH₃)-C₂H₅, -C(CH₃)₃, -cyclo-C₃H₅, or - CH₂-cyclo-C₃H₅;
**R²¹, R²², R²³, R²⁴, R²⁵, R²⁶, R²⁷, R²⁸, R²⁹, R³⁰, R³¹, R³², R³³, R³⁴, R³⁵, R³⁶, R³⁷,** and **R³⁸** represent independently of each other -H, -OH, -OCH₃, -OC₂H₅, -OC₃H₇, -O-cyclo-C₃H₅, -OCH(CH₃)₂, -OC(CH₃)₃, -OC₄H₉, -OPh, -OCH₂-Ph, -OCPh₃, -CH₂-OCH₃, -C₂H₄-OCH₃, -C₃H₆-OCH₃, -CH₂-OC₂H₅, -C₂H₄-OC₂H₅, -C₃H₆-OC₂H₅, -CH₂-OC₃H₇, -C₂H₄-OC₃H₇, -C₃H₆-OC₃H₇, -CH₂-O-cyclo-C₃H₅, -C₂H₄-O-cyclo-C₃H₅, -C₃H₆-O-cyclo-C₃H₅, -CH₂-OCH(CH₃)₂, -C₂H₄-OCH(CH₃)₂, -C₃H₆-OCH(CH₃)₂, -CH₂-OC(CH₃)₃, -C₂H₄-OC(CH₃)₃, -C₃H₆-OC(CH₃)₃, -CH₂-OC₄H₉, -C₂H₄-OC₄H₉, -C₃H₆-OC₄H₉, -CH₂-OPh, -C₂H₄-OPh, -C₃H₆-OPh, -CH₂-OCH₂-Ph, -C₂H₄-OCH₂-Ph, -C₃H₆-OCH₂-Ph, -SH, -SCH₃, -SC₂H₅, -SC₃H₇, -S-cyclo-C₃H₅, -SCH(CH₃)₂, -SC(CH₃)₃, -F, -Cl, -Br, -I, -CN, -COCH₃, -COC₂H₅, -COC₃H₇, -CO-cyclo-C₃H₅, -COCH(CH₃)₂, -COC(CH₃)₃, -COOH, -COOCH₃, -COOC₂H₅, -COOC₃H₇, -COO-cyclo-C₃H₅, -COOCH(CH₃)₂, -COOC(CH₃)₃, -OOC-CH₃, -OOC-C₂H₅, -OOC-C₃H₇, -OOC-cyclo-C₃H₅, -OOC-CH(CH₃)₂, -OOC-C(CH₃)₃, -CONH₂, -CONHCH₃, -CONHC₂H₅, -CONHC₃H₇, -CONH-cyclo-C₃H₅, -CONH[CH(CH₃)₂], -CONH[C(CH₃)₃], -CON(CH₃)₂, -CON(C₂H₅)₂, -CON(C₃H₇)₂, -CON(cyclo-C₃H₅)₂, -CON[CH(CH₃)₂]₂, -CON[C(CH₃)₃]₂, -NHCOCH₃, -NHCOC₂H₅, -NHCOC₃H₇, -NHCO-cyclo-C₃H₅, -NHCO-CH(CH₃)₂, -NHCO-C(CH₃)₃, -NHCO-OCH₃, -NHCO-OC₂H₅, -NHCO-OC₃H₇, -NHCO-O-cyclo-C₃H₅, -NHCO-OCH(CH₃)₂, -NHCO-OC(CH₃)₃, -NH₂, -NHCH₃, -NHC₂H₅, -NHC₃H₇, -NH-cyclo-C₃H₅, -NHCH(CH₃)₂, -NHC(CH₃)₃, -N(CH₃)₂, -N(C₂H₅)₂, -N(C₃H₇)₂, -N(cyclo-C₃H₅)₂, -N[CH(CH₃)₂]₂, -N[C(CH₃)₃]₂, -SOCH₃, -SOC₂H₅, -SOC₃H₇, -SO-cyclo-C₃H₅, -SOCH(CH₃)₂, -SOC(CH₃)₃, -SO₂CH₃, -SO₂C₂H₅, -SO₂C₃H₇, -SO₂₋cyclo-C₃H₅, -SO₂CH(CH₃)₂, -SO₂C(CH₃)₃, -SO₃H, -SO₃CH₃, -SO₃C₂H₅, -SO₃C₃H₇, -SO₃-cyclo-C₃H₅, -SO₃CH(CH₃)₂, -SO₃C(CH₃)₃, -SO₂NH₂, -SO₂NHCH₃, -SO₂NHC₂H₅, -SO₂NHC₃H₇, -SO₂NH-cyclo-C₃H₅, -SO₂NHCH(CH₃)₂, -SO₂NHC(CH₃)₃, -SO₂N(CH₃)₂, -SO₂N(C₂H₅)₂, -SO₂N(C₃H₇)₂, -SO₂N(cyclo-C₃H₅)₂, -SO₂N[CH(CH₃)₂]₂, -SO₂N[C(CH₃)₃]₂, -O-S(=O)CH₃, -O-S(=O)C₂H₅, -O-S(=O)C₃H₇, -O-S(=O)-cyclo-C₃H₅, -O-S(=O)CH(CH₃)₂, -O-S(=O)C(CH₃)₃, -S(=O)(=NH)CH₃, -S(=O)(=NH)C₂H₅, -S(=O)(=NH)C₃H₇, -S(=O)(=NH)-cyclo-C₃H₅, -S(=O)(=NH)CH(CH₃)₂, -S(=O)(=NH)C(CH₃)₃, -NH-SO₂-CH₃, -NH-SO₂-C₂H₅, -NH-SO₂-C₃H₇, -NH-SO₂-cyclo-C₃H₅, -NH-SO₂-CH(CH₃)₂, -NH-SO₂-C(CH₃)₃, -O-SO₂-CH₃, -O-SO₂-C₂H₅, -O-SO₂-C₃H₇, -O-SO₂-cyclo-C₃H₅, -O-SO₂-CH(CH₃)₂, -O-SO₂-C(CH₃)₃, -OCH₂F, -OCHF₂ -OCF₃, -CH₂-OCF₃, -C₂H₄-OCF₃, -C₃H₆-OCF₃, -OC₂F₅, -CH₂-OC₂F₅, -C₂H₄-OC₂F₅, -C₃H₆-OC₂F₅, -O-COOCH₃, -O-COOC₂H₅, -O-COOC₃H₇, -O-COO-cyclo-C₃H₅, -O-COOCH(CH₃)₂, -O-COOC(CH₃)₃, -NH-CO-NH₂, -NH-CO-NHCH₃, -NH-CO-NHC₂H₅, -NH-CO-NHC₃H₇, -NH-C(=NH)-NH₂, -NH-CO-N(C₃H₇)₂, -NH-CO-NH[CH(CH₃)₂], -NH-CO-NH[C(CH₃)₃], -NH-CO-N(CH₃)₂] -NH-CO-N(C₂H₅)₂, -NH-CO-NH-cyclo-C₃H₅, -NH-CO-N(cyclo-C₃H₅)₂, -NH-CO-N[CH(CH₃)₂]₂, -NH-C(=NH)-NHCH₃, -NH-C(=NH)-NHC₂H₅, -NH-C(=NH)-NHC₃H₇, -O-CO-NH-cyclo-C₃H₅, -NH-C(=NH)-NH-cyclo-C₃H₅, -NH-C(=NH)-NH[CH(CH₃)₂], -O-CO-NH[CH(CH₃)₂], -NH-C(=NH)-NH[C(CH₃)₃], -NH-C(=NH)-N(CH₃)₂, -NH-C(=NH)-N(C₂H₅)₂, -NH-C(=NH)-N(C₃H₇)₂, -NH-C(=NH)-N(cyclo-C₃H₅)₂, -O-CO-NHC₃H₇, -NH-C(=NH)-N[CH(CH₃)₂]₂, -NH-C(=NH)-N[C(CH₃)₃]₂, -O-CO-NH₂, -O-CO-NHCH₃, -O-CO-NHC₂H₅, -O-CO-NH[C(CH₃)₃], -O-CO-N(CH₃)₂, -O-CO-N(C₂H₅)₂, -O-CO-N(C₃H₇)₂, -O-CO-N(cyclo-C₃H₅)₂, -O-CO-N[CH(CH₃)₂]₂, -O-CO-N[C(CH₃)₃]₂, -O-CO-OCH₃, -O-CO-OC₂H₅, -O-CO-OC₃H₇, -O-CO-O-cyclo-C₃H₅, -O-CO-OCH(CH₃)₂, -O-CO-OC(CH₃)₃, -CH₂F, -CHF₂, -CF₃, -CH₂-CH₂F, -CH₂-CHF₂, -CH₂-CF₃, cyclo-C₈H₁₅, -Ph, -CH₂-Ph, -CH₂-CH₂-Ph, -CH=CH-Ph, -CPh₃, -CH₃, -C₂H₅, -C₃H₇, -CH(CH₃)₂, -C₄H₉, -CH₂-CH(CH₃)₂, -CH(CH₃)-C₂H₅, -C(CH₃)₃, -C₅H₁₁, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, -C₂H₄-CH(CH₃)₂, -C₆H₁₃, -C₇H₁₅, -C₈H₁₇, -C₃H₆-CH(CH₃)₂, -C₂H₄-CH(CH₃)-C₂H₅, -CH(CH₃)-C₄H₉, -CH₂-CH(CH₃)-C₃H₇, -CH(CH₃)-CH₂-CH(CH₃)₂, -CH(CH₃)-CH(CH₃)-C₂H₅, -CH₂-CH(CH₃)-CH(CH₃)₂, -CH₂-C(CH₃)₂-C₂H₅, -C(CH₃)₂-C₃H₇, -C(CH₃)₂-CH(CH₃)₂, -C₂H₄-C(CH₃)₃, -CH(CH₃)-C(CH₃)₃, -CH=CH₂, -CH₂-CH=CH₂, -C(CH₃)=CH₂, -CH=CH-CH₃, -C₂H₄-CH=CH₂, -CH₂-CH=CH-CH₃, -CH=CH-C₂H₅, -CH₂-C(CH₃)=CH₂, -CH(CH₃)-CH=CH, -CH=C(CH₃)₂, -C(CH₃)=CH-CH₃, -CH=CH-CH=CH₂, -C₃H₆-CH=CH₂, -C₂H₄-CH=CH-CH₃, -CH₂-CH=CH-C₂H₅, -CH=CH-C₃H₇, -CH=CH-CH=CH-CH₃, -C₂H₄-C(CH₃)=CH₂, -CH₂-CH(CH₃)-CH=CH₂, -CH(CH₃)-CH₂-CH=CH₂, -CH₂-CH=C(CH₃)₂, -CH₂-C(CH₃)=CH-CH₃, -CH(CH₃)-CH=CH-CH₃, -CH=CH-CH(CH₃)₂, -CH=C(CH₃)-C₂H₅, -C(CH₃)=CH-C₂H₅, -C(CH₃)=C(CH₃)₂, -C(CH₃)₂-CH=CH₂, -CH(CH₃)-C(CH₃)=CH₂, -C₄H₃-CH=CH₂, -C₃H₆-CH=CH-CH₃, -C₂H₄-CH=CH-C₂H₅, -CH₂-CH=CH-C₃H₇, -CH=CH-C₄H₉, -C₃H₆-C(CH₃)=CH₂, -C₂H₄-CH(CH₃)-CH=CH₂, -CH₂-CH(CH₃)-CH₂-CH=CH₂, -C₂H₄-CH=C(CH₃)₂, -CH(CH₃)-C₂H₄-CH=CH₂, -C₂H₄-C(CH₃)=CH-CH₃, -CH₂-CH(CH₃)-CH=CH-CH₃, -CH(CH₃)-CH₂-CH=CH-CH₃, -CH₂-CH=CH-CH(CH₃)₂, -CH₂-CH=C(CH₃)-C₂H₅, -CH₂-C(CH₃)=CH-C₂H₅, -CH(CH₃)-CH=CH-C₂H₅, -CH=CH-CH₂-CH(CH₃)₂, -CH=CH-CH(CH₃)-C₂H₅, -CH=C(CH₃)-C₃H₇, -C(CH₃)=CH-C₃H₇, -CH₂-CH(CH₃)-C(CH₃)=CH₂, -C[C(CH₃)₃]=CH₂, -CH(CH₃)-CH₂-C(CH₃)=CH₂, -CH(CH₃)-CH(CH₃)-CH=CH₂, -CH=CH-C₂H₄-CH=CH₂, -C(CH₃)₂-CH₂-CH=CH₂, -CH₂-C(CH₃)=C(CH₃)₂, -CH(CH₃)-CH=C(CH₃)₂, -C(CH₃)₂-CH=CH-CH₃, -CH=CH-CH₂-CH=CH-CH₃, -CH(CH₃)-C(CH₃)=CH-CH₃, -CH=C(CH₃)-CH(CH₃)₂, -C(CH₃)=CH-CH(CH₃)₂, -C(CH₃)=C(CH₃)-C₂H₅, -CH=CH-C(CH₃)₃, -C(CH₃)₂-C(CH₃)=CH₂, -CH(C₂H₅)-C(CH₃)=CH₂, -C(CH₃)(C₂H₅)-CH=CH₂, -CH(CH₃)-C(C₂H₅)=CH₂, -CH₂-C(C₃H₇)=CH₂, -CH₂-C(C₂H₅)=CH-CH₃, -CH(C₂H₅)-CH=CH-CH₃, -C(C₄H₉)=CH₂, -C(C₃H₇)=CH-CH₃, -C(C₂H₅)=CH-C₂H₅, -C(C₂H₅)=C(CH₃)₂, -C[CH(CH₃)(C₂H₅)]=CH₂, -C[CH₂-CH(CH₃)₂]=CH₂, -C₂H₄-CH=CH-CH=CH₂, -CH₂-CH=CH-CH₂-CH=CH₂, -C₃H₆-C≡C-CH₃, -CH₂-CH=CH-CH=CH-CH₃, -CH=CH-CH=CH-C₂H₅, -CH(CH₃)-CH₂-C≡CH, -CH(CH₃)-C≡C-CH₃, -C₂H₄-CH(CH₃)-C≡CH, -CH=CH-CH=C(CH₃)₂, -CH₂-CH(CH₃)-CH₂-C≡CH, -CH=CH-C(CH₃)=CH-CH₃, -CH=C(CH₃)-CH=CH-CH₃, -CH₂-CH(CH₃)-C≡CH, -C(CH₃)=CH-CH=CH-CH₃, -C=CH, -C≡C-CH₃, -CH₂-C≡CH, -C₂H₄-C≡CH, -CH₂-C≡C-CH₃, -C≡C-C₂H₅, -C₃H₆-C≡CH, -C₂H₄-C≡C-CH₃, -CH₂-C≡C-C₂H₅, -C≡C-C₃H₇, -CH(CH₃)-C≡CH, -C₄H₈-C≡CH, -C₂H₄-C≡C-C₂H₅, -CH₂-C≡C-C₃H₇, -C≡C-C₄H₉, -C≡C-CH₂-CH(CH₃)₂, -CH(CH₃)-C₂H₄-C≡CH, -CH₂-CH(CH₃)-C≡C-CH₃, -C(CH₃)(C₂H₅)-C≡CH, -CH(CH₃)-CH₂-C≡C-CH₃, -CH(CH₃)-C≡C-C₂H₅, -CH₂-C≡C-CH(CH₃)₂, -C≡C-CH(CH₃)-C₂H₅, -CH₂-C≡C-C≡C-CH₃, -CH(C₂H₅)-C≡C-CH₃, -C(CH₃)₂-C≡C-CH₃, -CH(C₂H₅)-CH₂-C≡CH, -CH₂-CH(C₂H₅)-C≡CH, -C(CH₃)₂-CH₂-C≡CH, -CH₂-C(CH₃)₂-C≡CH, -CH(CH₃)-CH(CH₃)-C≡CH, -CH(C₃H₇)-C≡CH, -CH₂-CH(C≡CH)₂, -C=C-C=CH, -CH₂-C≡C-C≡CH, -C≡C-C≡C-CH₃, -CH(C≡CH)₂, -C₂H₄-C≡C-C≡CH, -CH₂-C≡C-CH₂-C≡CH, -C≡C-C₂H₄-C≡CH, -C≡C-C(CH₃)₃, -C≡C-CH₂-C≡C-CH₃, -C≡C-C≡C-C₂H₅,

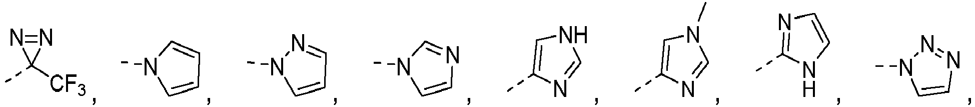

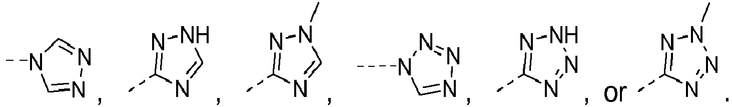

The disease or the disorder caused by or associated with the enzyme NAPE-PLD is selected from the group consisting of: neurodegenerative diseases, metabolic syndrome, insulin resistance, diabetes type II, obesity, liver steatosis, drug addiction, smoking cessation, mental health disorders, osteoporosis, traumatic brain injury, neuropathic and neuro-inflammatory pain, peripheral neuropathy in diabetes, endometriosis, eating disorders, and cancer.

Preferably, the neurodegenerative disease is selected from the group consisting of: Alzheimer's disease, Amyotrophic lateral sclerosis, Friedreich's ataxia, Huntington's disease, Lewy body disease, Parkinson's disease, Prion disease, Motor neuron diseases, Spinocerebellar ataxia, and Spinal muscular atrophy.

Preferably, the cancer is selected from the group consisting of:
adenocarcinoma, choroidal melanoma, acute leukemia, acoustic neurinoma, ampullary carcinoma, anal carcinoma, astrocytoma, basal cell carcinoma, pancreatic cancer, desmoid tumor, bladder cancer, bronchial carcinoma, estrogen dependent and independent breast cancer, Burkitt's lymphoma, corpus cancer, CUP-syndrome (carcinoma of unknown primary), colorectal cancer, small intestine cancer, small intestinal tumors, ovarian cancer, endometrial carcinoma, ependymoma, epithelial cancer types, Ewing's tumors, gastrointestinal tumors, gastric cancer, gallbladder cancer, gall bladder carcinomas, uterine cancer, cervical cancer, glioblastomas, gynecologic tumors, ear, nose and throat tumors, hematologic neoplasias, hairy cell leukemia, urethral cancer, skin cancer, skin testis cancer, brain tumors (gliomas), brain metastases, testicle cancer, hypophysis tumor, carcinoids, Kaposi's sarcoma, laryngeal cancer, germ cell tumor, bone cancer, colorectal carcinoma, head and neck tumors (tumors of the ear, nose and throat area), colon carcinoma, craniopharyngiomas, oral cancer (cancer in the mouth area and on lips), cancer of the central nervous system, liver cancer, liver metastases, leukemia, eyelid tumor, lung cancer, lymph node cancer (Hodgkin's/Non-Hodgkin's lymphomas), lymphomas, stomach cancer, malignant melanoma, malignant neoplasia, malignant tumors gastrointestinal tract, breast carcinoma, rectal cancer, medulloblastomas, melanoma, meningiomas, Hodgkin's disease, mycosis fungoides, nasal cancer, neurinoma, neuroblastoma, kidney cancer, renal cell carcinomas, non-Hodgkin's lymphomas, oligodendroglioma, esophageal carcinoma, osteolytic carcinomas and osteoplastic carcinomas, osteosarcomas, ovarial carcinoma, pancreatic carcinoma, penile cancer, plasmocytoma, prostate cancer, pharyngeal cancer, rectal carcinoma, retinoblastoma, vaginal cancer, thyroid carcinoma, Schneeberger disease, esophageal cancer, spinalioms, T-cell lymphoma (mycosis fungoides), thymoma, tube carcinoma, eye tumors, urethral cancer, urologic tumors, urothelial carcinoma, vulva cancer, wart appearance, soft tissue tumors, soft tissue sarcoma, Wilm's tumor, cervical carcinoma, tongue cancer, invasive ductal carcinoma, invasive lobular carcinoma, ductal carcinoma in situ, lobular carcinoma in situ, small-cell lung carcinoma, non-small-cell lung carcinoma, bronchial adenoma, pleuropulmonary blastoma, mesothelioma, brain stem glioma, hypophtalmic glioma, cerebellar astrocytoma, cerebral astrocytoma, neuroectodermal tumours, pineal tumors, sarcoma of the uterus, salivary gland cancers, anal gland adenocarcinomas, mast cell tumors, pelvis tumours, ureter tumours, hereditary papillary renal cancers, sporadic papillary renal cancers, intraocular melanoma, hepatocellular carcinoma (liver cell carcinomas with or without fibrolamellar variant), cholangiocarcinoma (intrahepatic bile duct carcinoma), mixed hepatocellular cholangiocarcinoma, squamous cell carcinoma, malignant melanoma, Merkel cell skin cancer, non-melanoma skin cancer, hypopharyngeal cancer, nasopharyngeal cancer, oropharyngeal cancer, oral cavity cancer, squamous cell cancer, oral melanoma, AIDS-related lymphoma, cutaneous T-cell lymphoma, lymphoma of the central nervous system, malignant fibrous histiocytoma, lymphosarcoma, rhabdomyosarcoma, malignant histiocytosis, fibrosarcoma, hemangiosarcoma, hemangiopericytoma, leiomyosarcoma, canine mammary carcinoma, and feline mammary carcinoma.

Preferred is the compound of the fomula (I), wherein the ring **A** represents 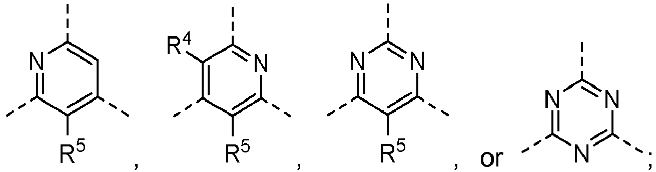 and **R⁴** and **R⁵** have the same meanings as defined in formula **(I)** or as defined in any preferred selection of substituents disclosed herein.

Preferred are the compounds of any one of the formulae **(II-1)** - **(II-3)**: 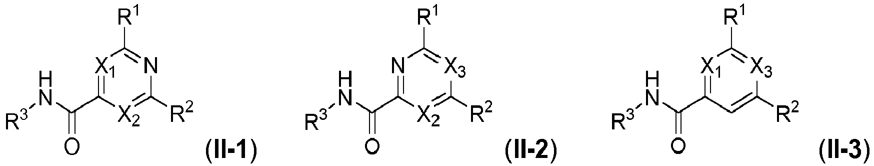 wherein **R¹**, **R²**, **R³**, **X₁**, **X₂** and **X₃** have the same meanings as defined in formula **(I)** or as defined in any preferred selection of substituents disclosed herein.

Preferred are the compounds of any one of the formulae **(III-1)** - **(III-4)**: 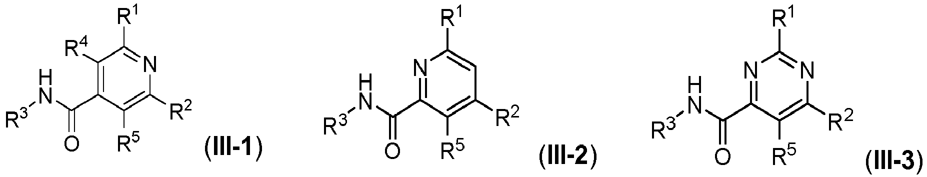 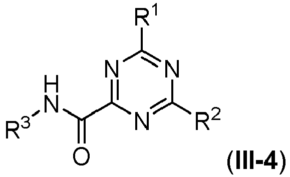 wherein **R¹**, **R²**, **R³**, R⁴, and **R⁵** have the same meanings as defined in formula **(I)** or as defined in any preferred selection of substituents disclosed herein.

Also preffered are the compounds of any one of the formulae **(IV-1) -(IV-8):** 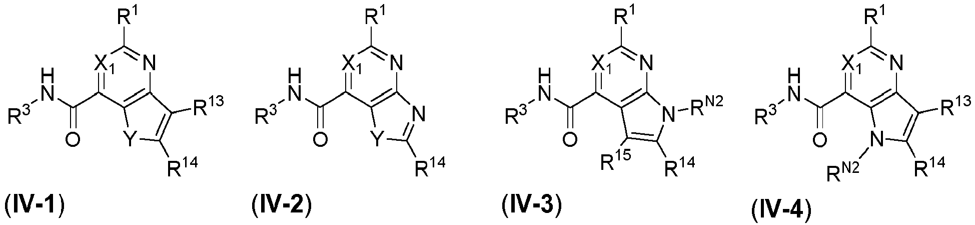 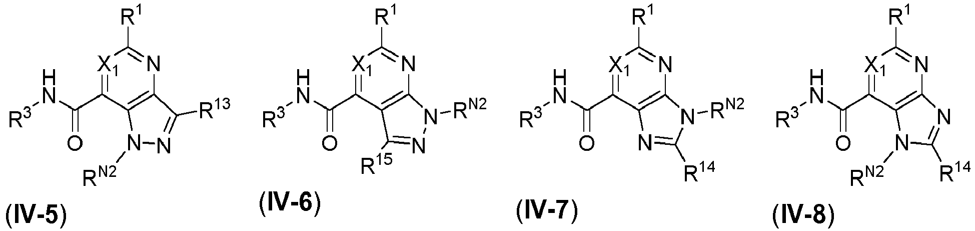 wherein **R²**, **R³**, **R¹⁰**, **R¹¹**, **R¹²**, and **R^{N1}** have the same meanings as defined in formula (I) or as defined in any preferred selection of substituents disclosed herein.

Still preffered are the compounds of any one of the formulae **(V-1) - (V-8):** 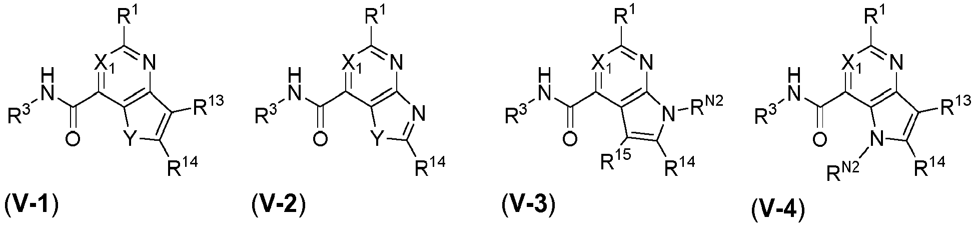 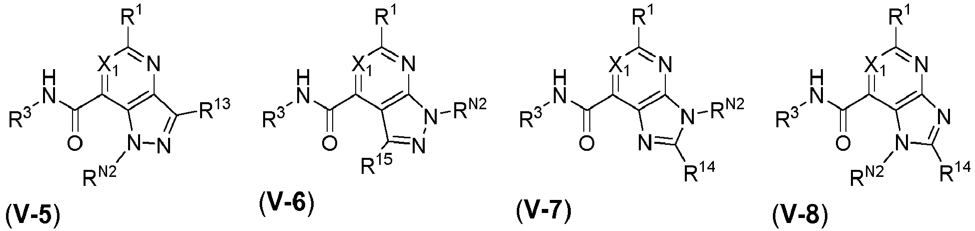
wherein **X₁** represent N or CH;
Y represents O or S; and
**R¹**, **R³**, **R¹³**, **R¹⁴**, **R¹⁵** and **R^{N2}** have the same meanings as defined in formaula **(I)** or as defined in any preferred selection of substituents disclosed herein.

More preferred are the compounds of any one of the formulae **(VI-1) - (VI-9):**

| | | |
|---|---|---|
| 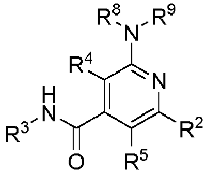 | 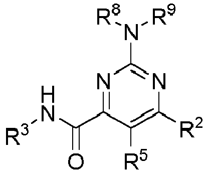 | 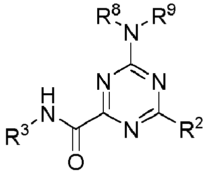 |
| **(VI-1)** | **(VI-2)** | **(VI-3)** |
| 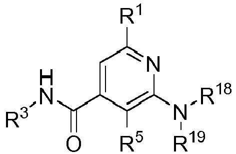 | 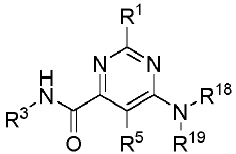 | 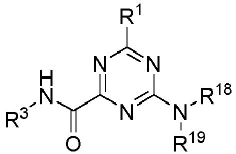 |
| **(VI-4)** | **(VI-5)** | **(VI-6)** |
| 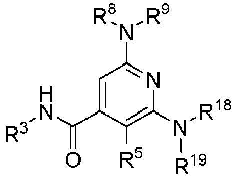 | 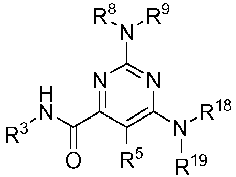 | 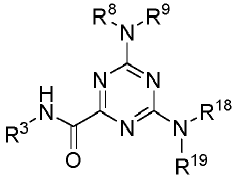 |
| **(VI-7)** | **(VI-8)** | **(VI-9)** |

wherein **R¹**, **R²**, **R³**, **R⁴**, **R⁵**, **R⁸**, **R⁹**, **R¹⁸** and **R¹⁹** have the same meanings as defined in formula (**I**) or as defined in any preferred selection of substituents disclosed herein.

Prererably, in the formulae **(I), (II-1)** - **(II-3)**, **(III-1)** - **(III-3)**, **(IV-1) - (IV-8), (V-1)** - **(V-8),** and **(VI-4) - (VI-9)**, **R¹** represents 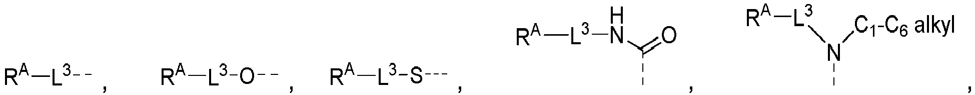 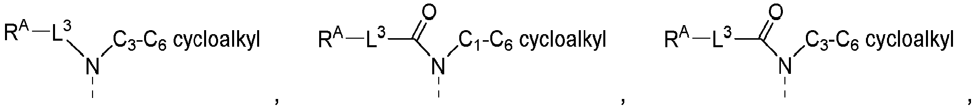 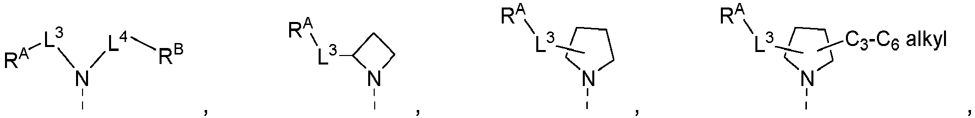 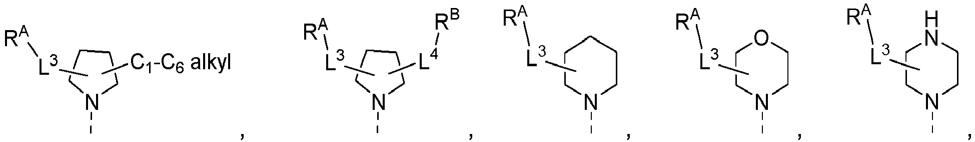 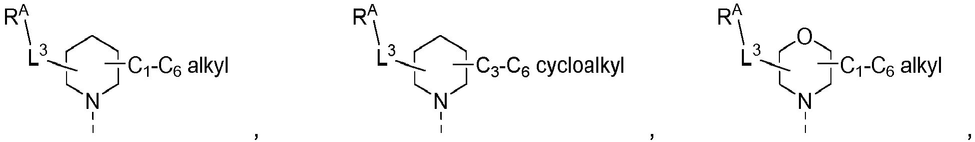 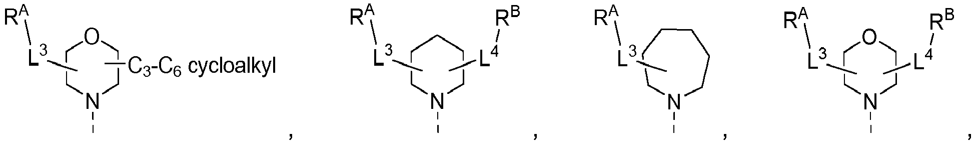 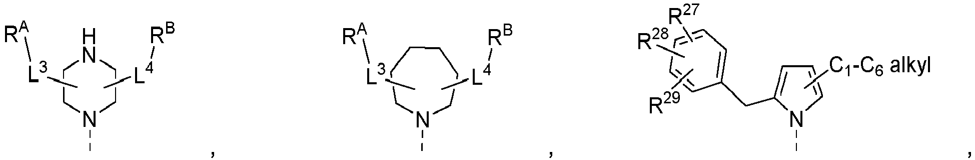 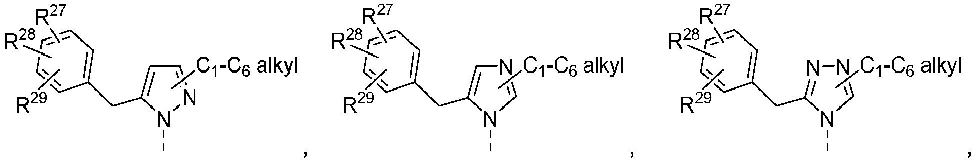 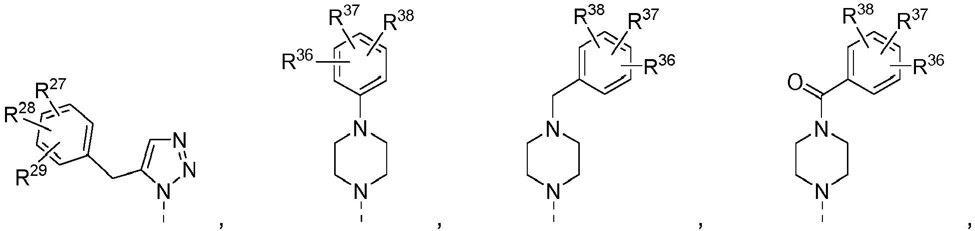 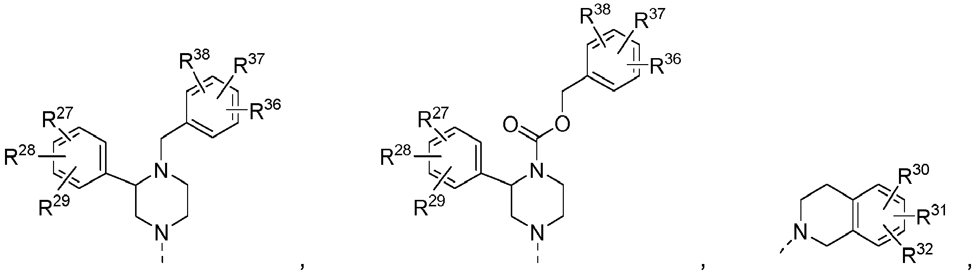 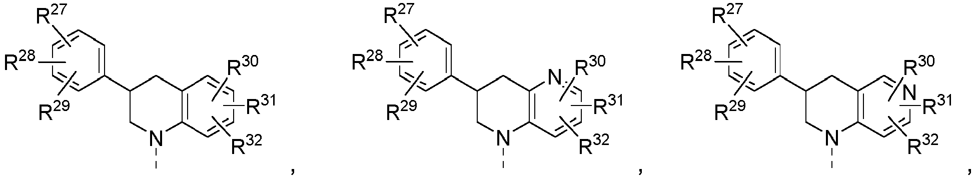 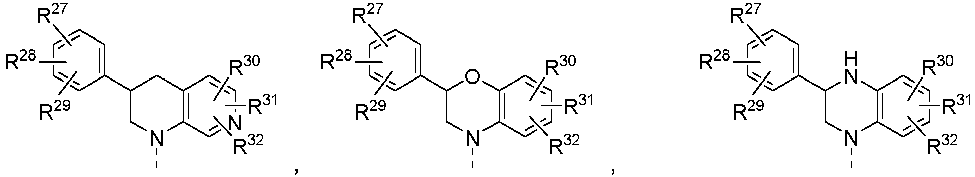 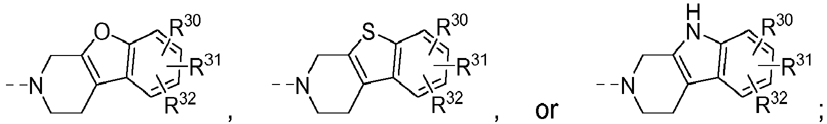 wherein R^{A}, represents

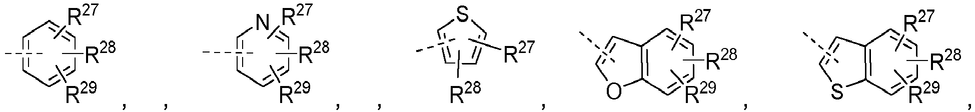

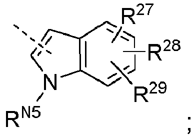

R^{B} represents 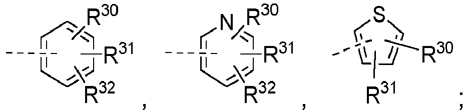 **L³**, and **L⁴** represent independently of each other a covalent bond, -CH₂-, 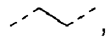 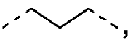 or 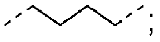 and **R²⁷**, **R²⁸**, **R²⁹**, **R³⁰**, **R³¹**, and **R³²** have the same meanings defined in the formula **(I).**

More prererably, in the formulae **(I), (II-1)** - **(II-3)**, **(III-1)** - **(III-3)**, **(IV-1) - (IV-8), (V-1)** - **(V-8),** and **(VI-4) - (VI-9), R¹** represents 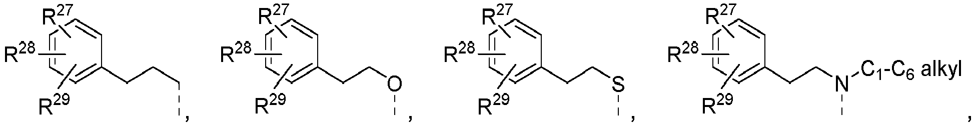 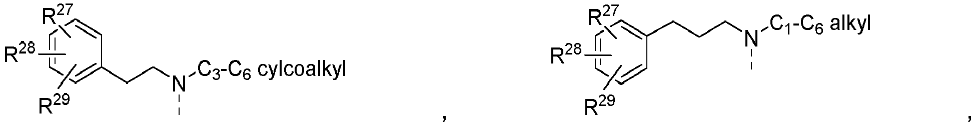 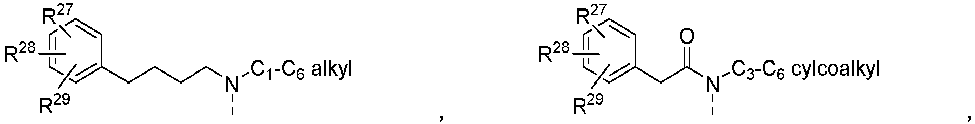 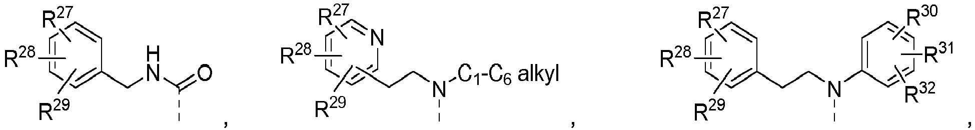 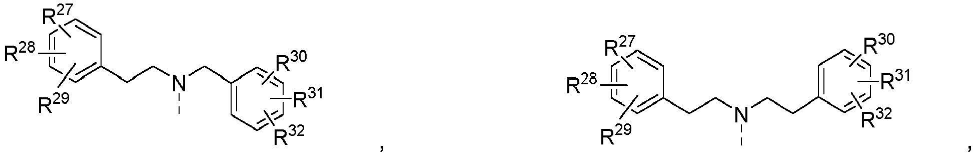 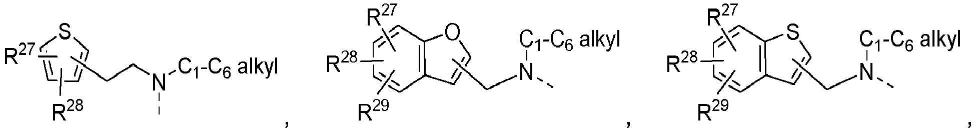 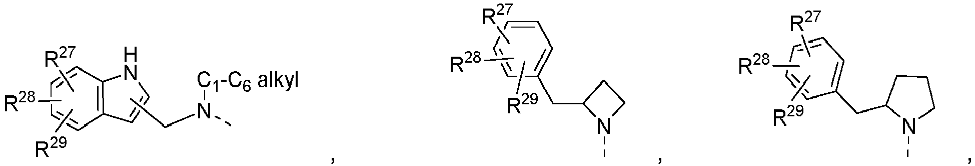 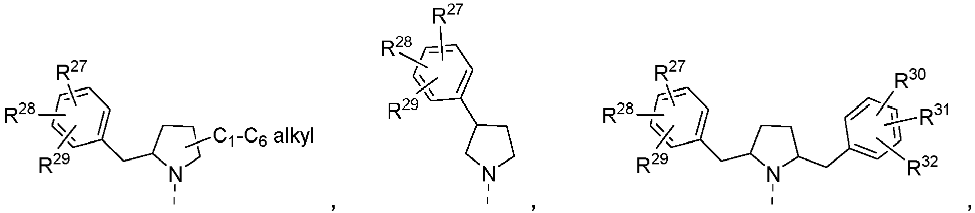 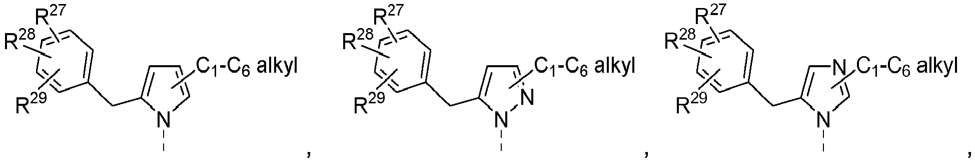 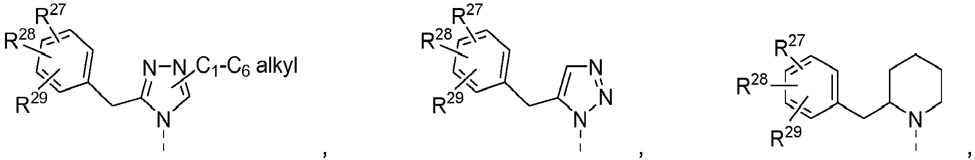 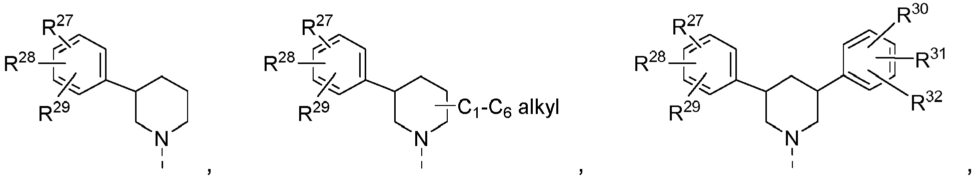 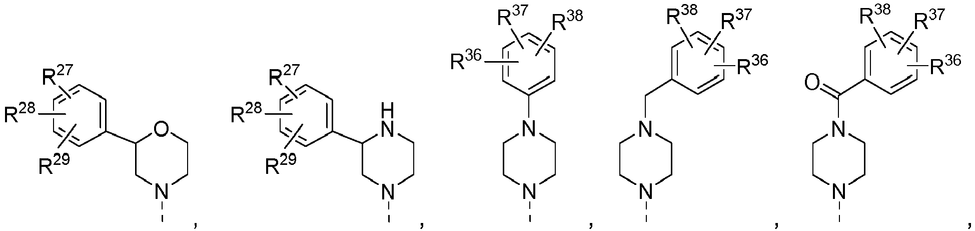 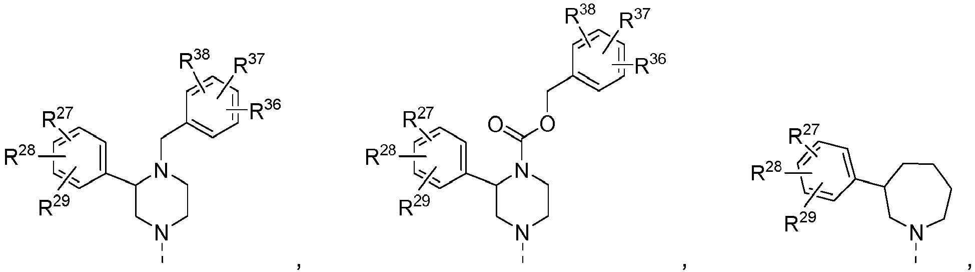 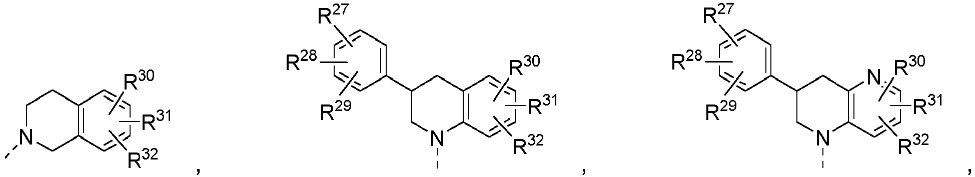 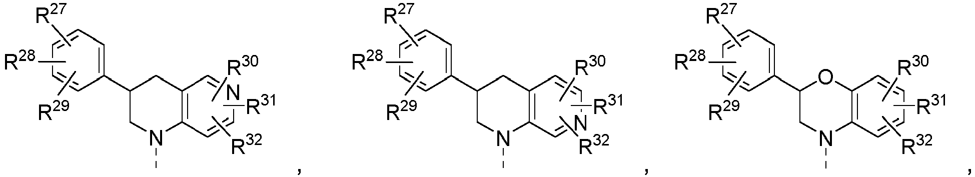 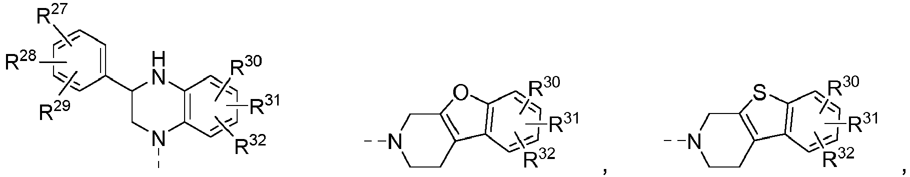 or 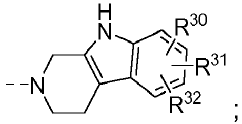 and **R²⁷**, **R²⁸**, **R²⁹**, **R³⁰**, **R³¹**, and **R³²** have the same meanings as defined in formula **(I)** or as defined in any preferred selection of substituents disclosed herein.

More preferably, **R¹** represents in the formulae **(I), (II-1)** - **(II-3)**, **(III-1)** - **(III-3)**, **(IV-1)** - **(IV-8), (V-1) - (V-8),** and **(VI-4) - (VI-9),**

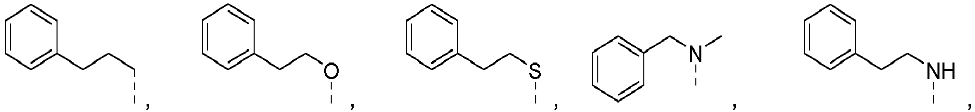

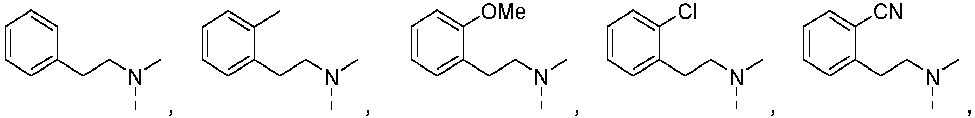

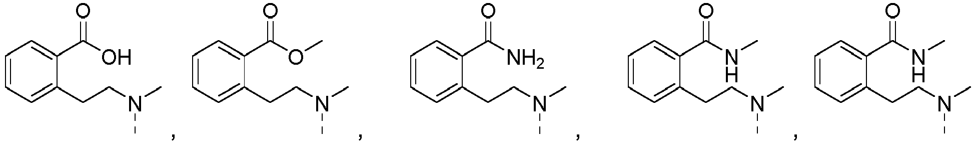

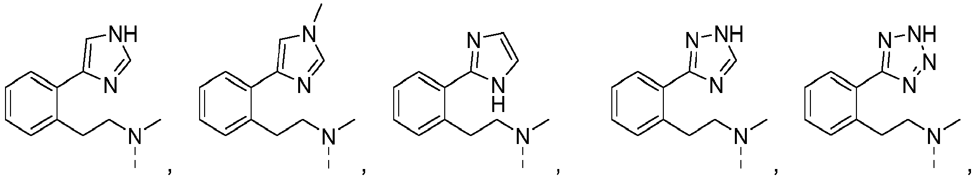

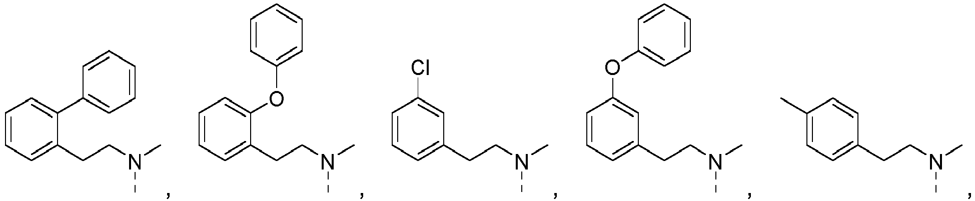

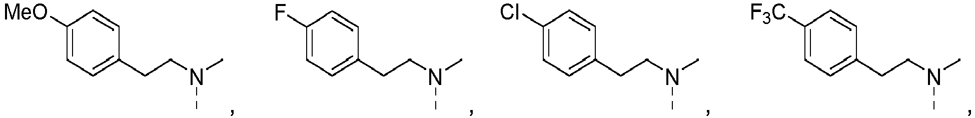

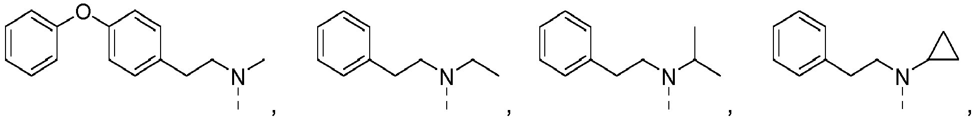

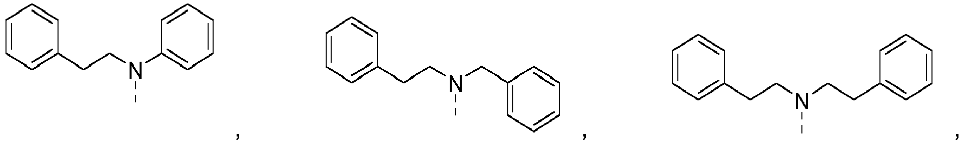

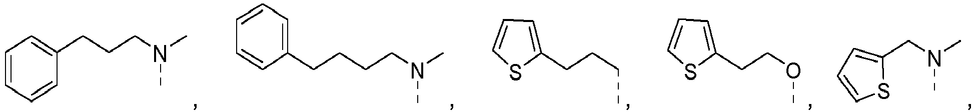

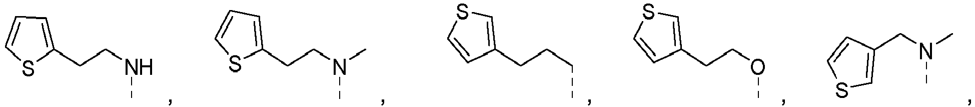

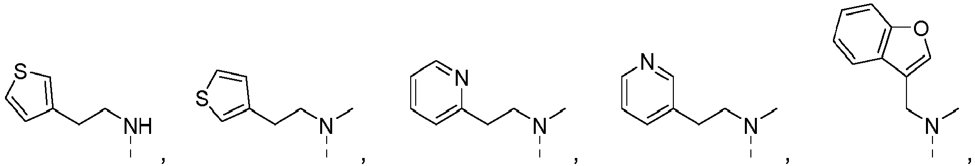

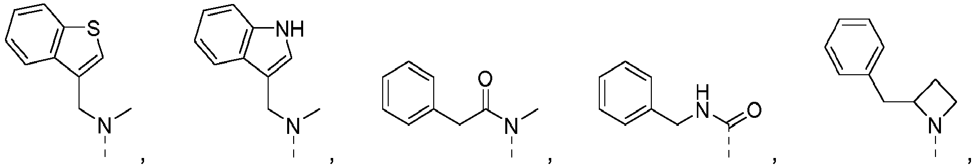

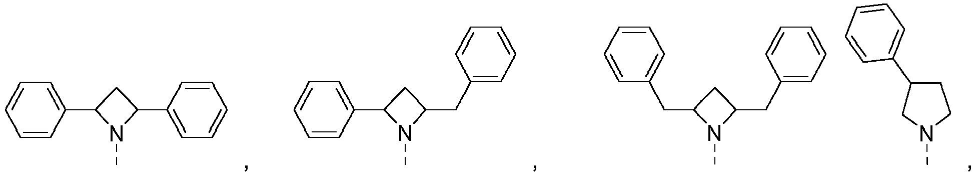

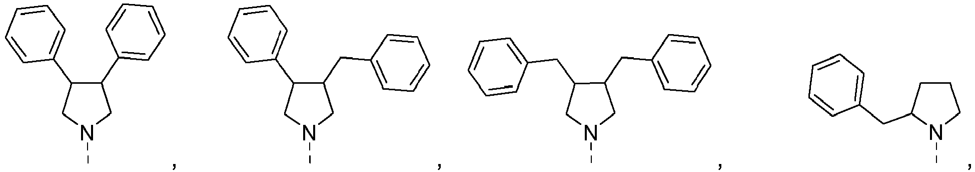

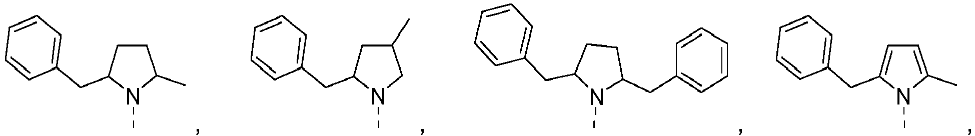

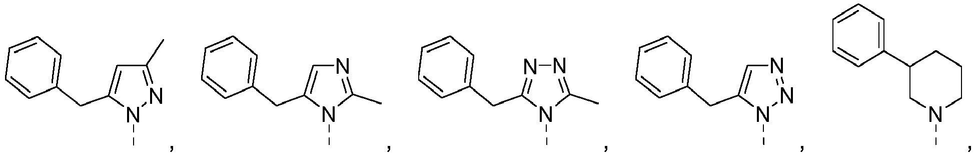

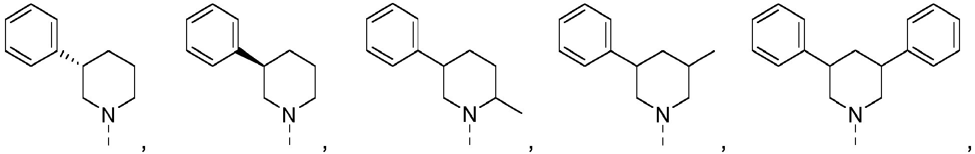

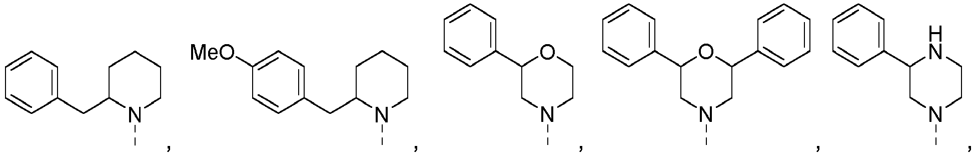

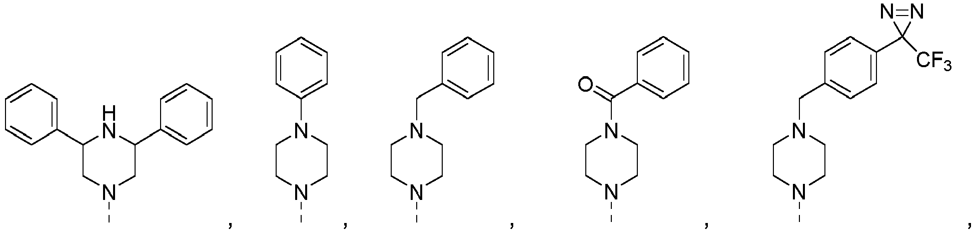

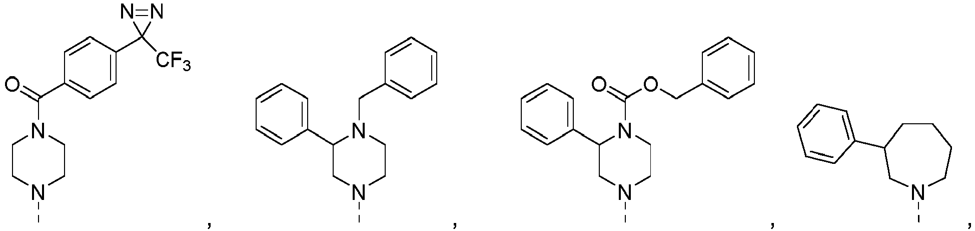

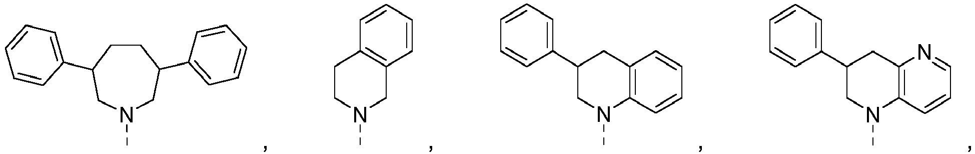

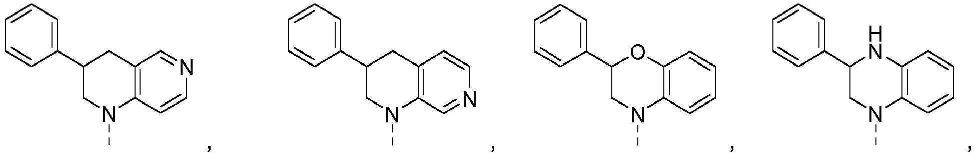

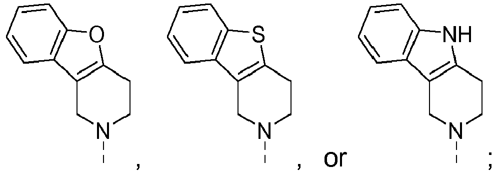

Preferably, **R²** represnts other C₁-C₆ alkyl, C₃-C₆ cycloalkyl;
or -N**R¹⁸R¹⁹** represents

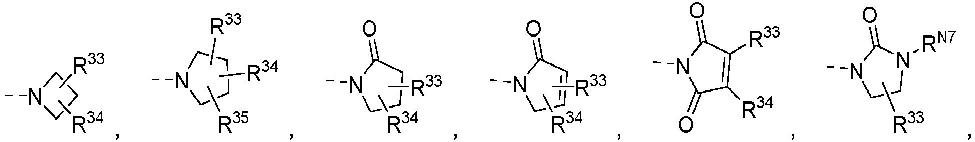

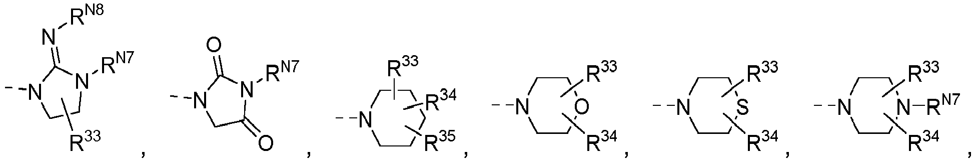

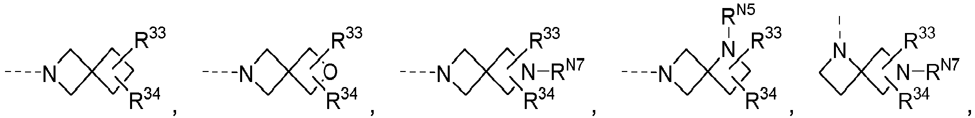

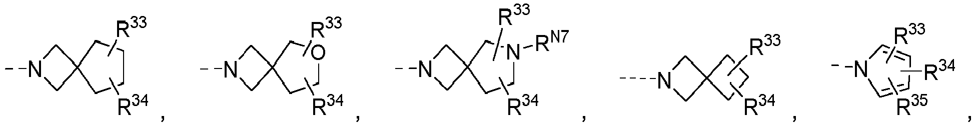

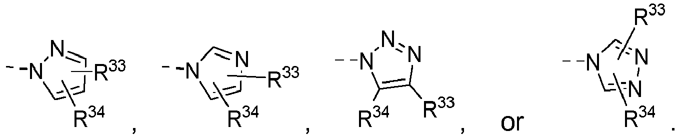

More preferably, **R²** represents other C₁-C₆ alkyl, C₃-C₆ cycloalkyl;

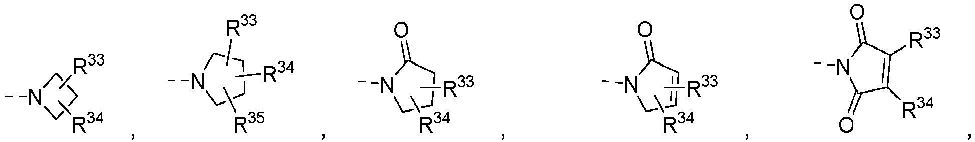

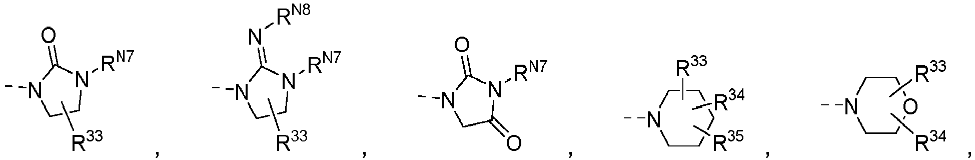

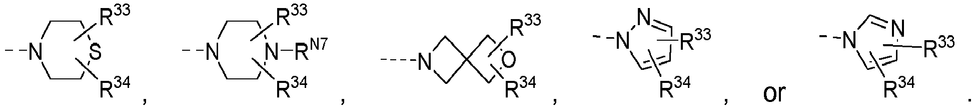

Most preferably, **R²** represents -CH₃, -Ph, -OCH₃, -SCH₃, -NHCH₃, -N(CH₃)₂, -N(CH₂CH₃)₂, -N(CH₃)(CH₂CH₂OH), -CONHCH₃, -N(CH₃)COCH₃, -N(CH₃)COCH₂OH, -N(CH₃)COCH(CH₃)CO₂CH₃, -N(CH₃)(CH₂Ph), -N(CH₃)(CH₂CH₂Ph), -NHCH(CH₃)CO₂CH₃, -NHCH(CH₂Ph)CO₂CH₃, -NHCH(CH₂SH)CO₂CH₃, -N(CH₃)CH(CH₃)CO₂CH₃, -N(CH₃)CH(CH₂Ph)CO₂CH₃, -N(CH₃)CH(CH₂SH)CO₂CH₃,

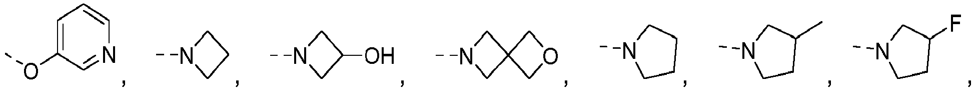

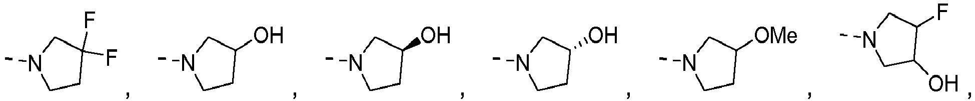

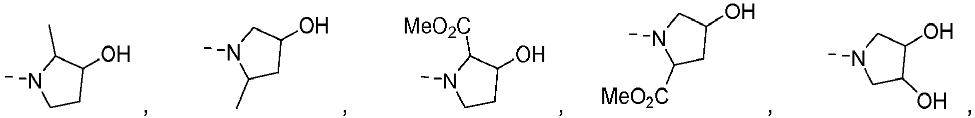

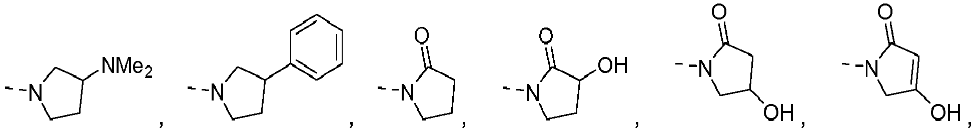

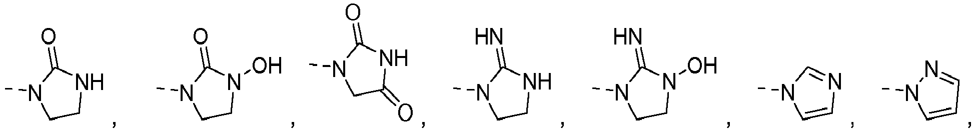

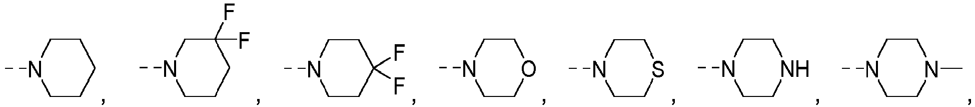

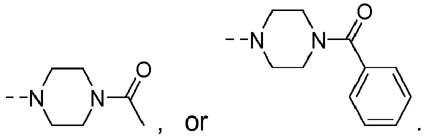

Preferred, **R³** represents C₁-C₆ alkyl, C₁-C₆ alkyl-OH, C₁-C₆ alkyl-CO₂**R²⁰**, C₁-C₆ alkyl-CONH**R²⁰**, C₂-C₆ alkenyl, C₂-C₆ alkynyl,

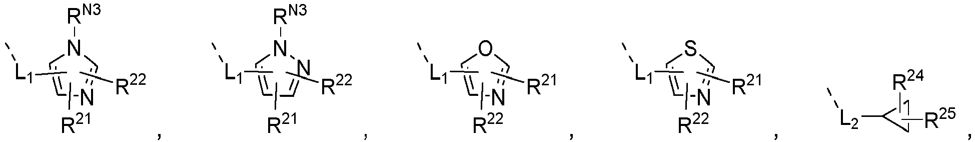

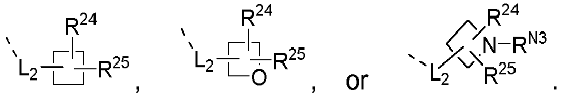

Most preferably, **R³** represents -CH₃, -CH₂CH₃, -CH₂CH₂CH₃, -CH₂CH₂CH₂CH₃, -CH₂CH(CH₃)₂, -CH₂CH₂OH, -CH₂CO₂CH₃, -CH₂C≡CH,

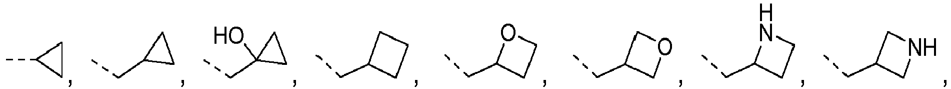

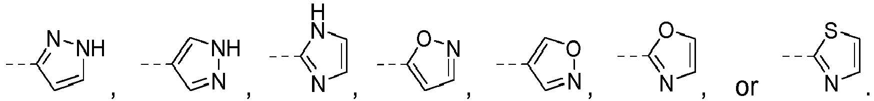

Preferred, -N**R⁸R⁹** represents

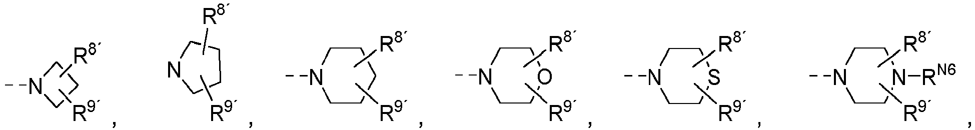

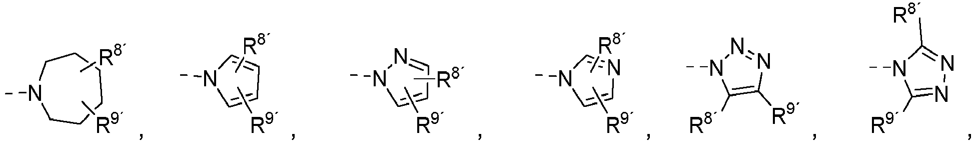

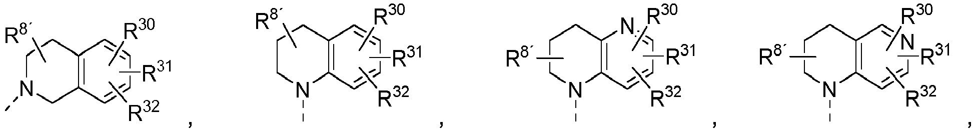

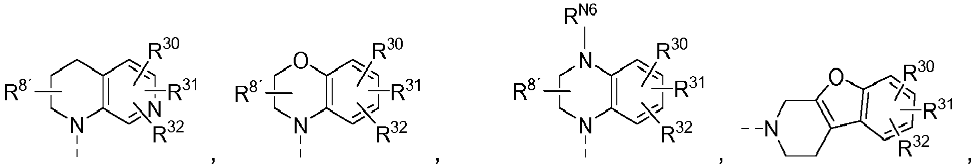

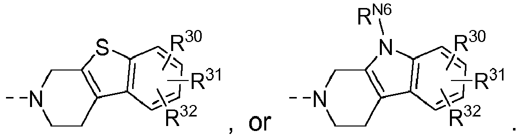

Preferred, -N**R¹⁸R¹⁹** represents

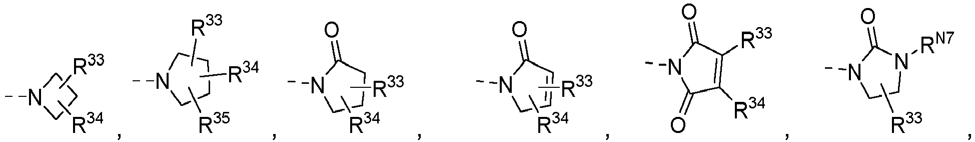

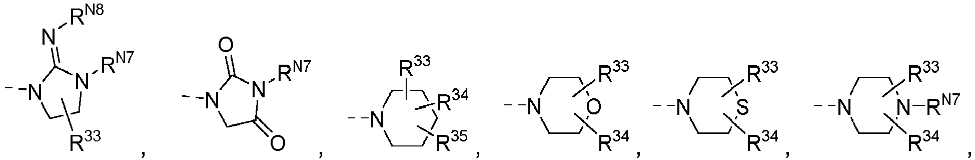

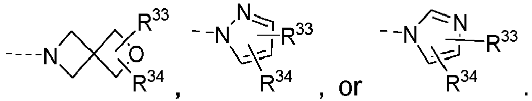

Therefore, the compunds of any one of the formulae **(I), (II-1)** - **(II-3)**, **(III-1)** - **(III-3)**, **(IV-1) - (IV-8), (V-1) - (V-8),** and **(VI-4) - (VI-9),** are still more preferred, wherein **R¹** is 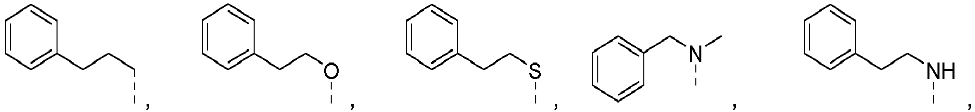 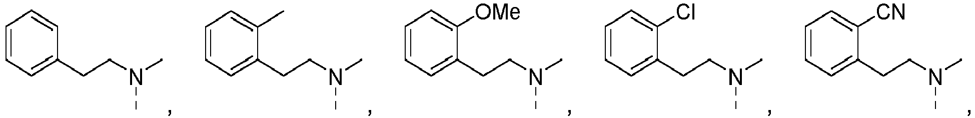 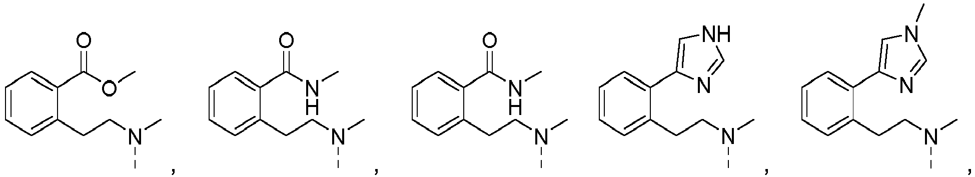 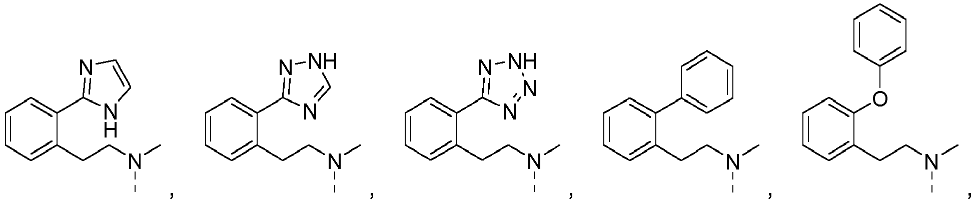 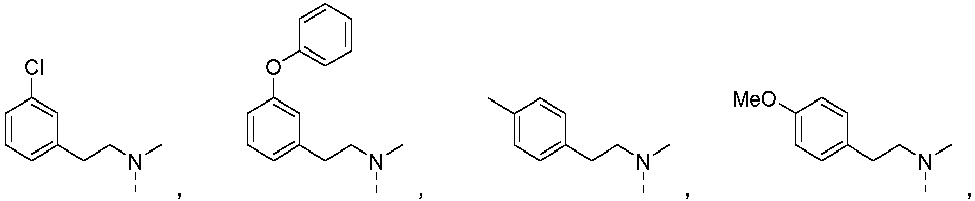 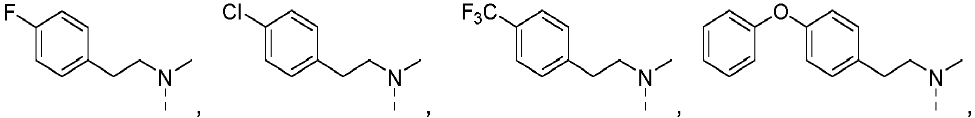 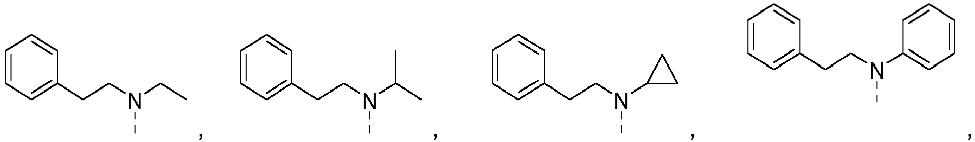 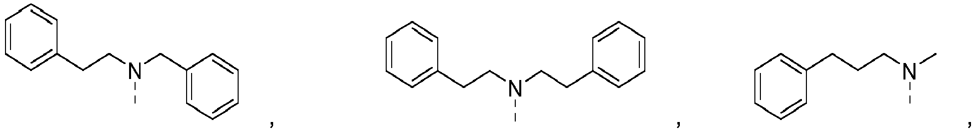 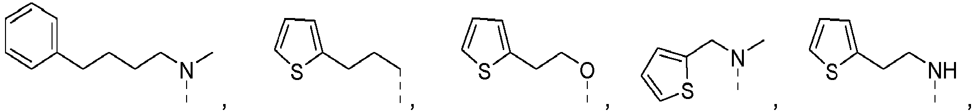 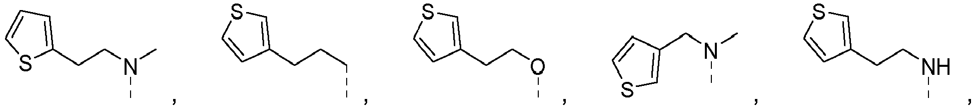 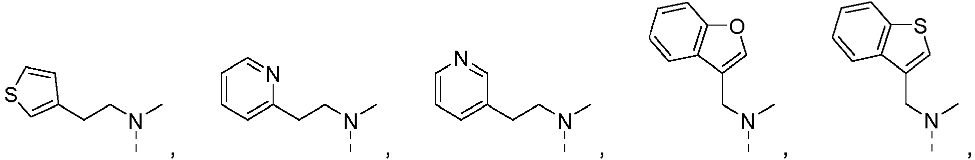 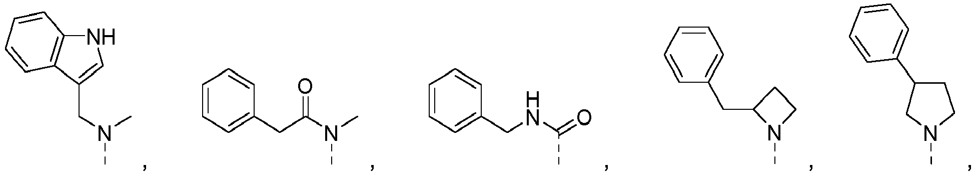 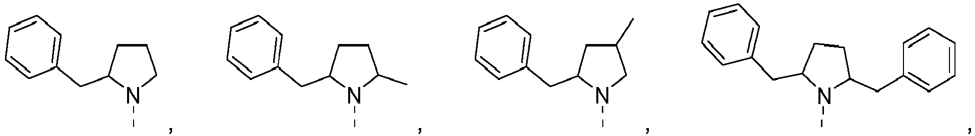 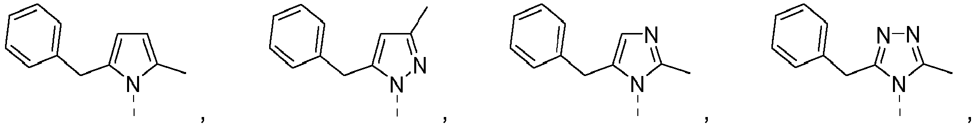 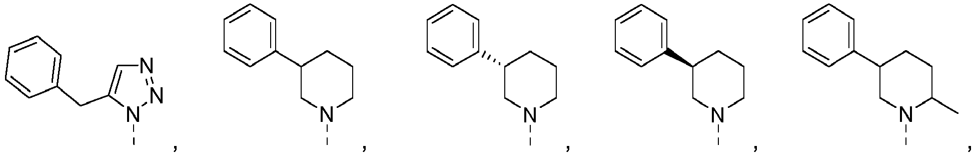 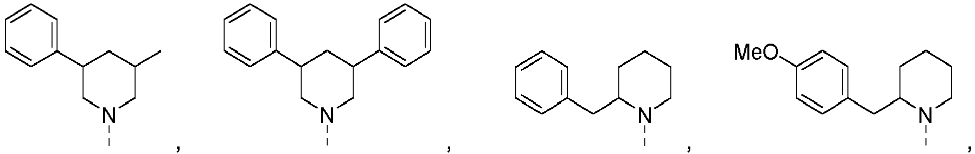 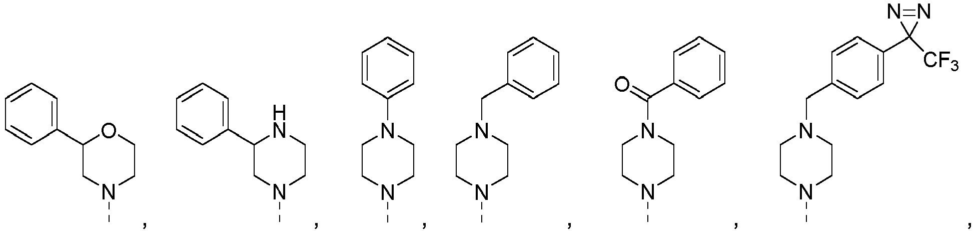 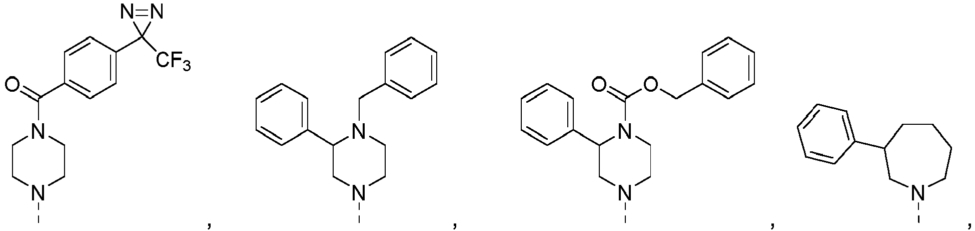 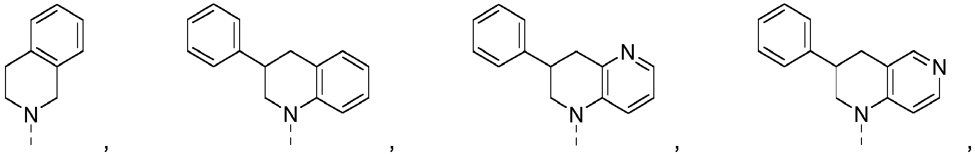 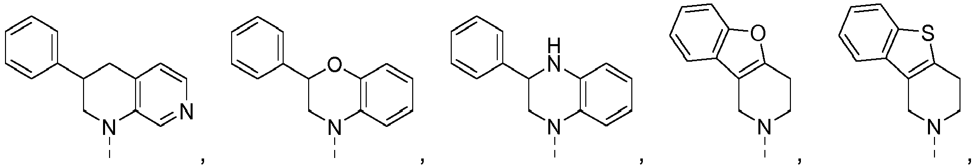 or 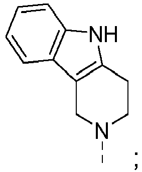 **R²** represents -CH₃, -Ph, -OCH₃, -SCH₃, -NHCH₃, -N(CH₃)₂, -N(CH₂CH₃)₂, -N(CH₃)(CH₂CH₂OH), -CONHCH₃, -N(CH₃)COCH₃, -N(CH₃)COCH₂OH, -N(CH₃)COCH(CH₃)CO₂CH₃, -N(CH₃)(CH₂Ph), -N(CH₃)(CH₂CH₂Ph), -NHCH(CH₃)CO₂CH₃, -NHCH(CH₂Ph)CO₂CH₃, -NHCH(CH₂SH)CO₂CH₃, -N(CH₃)CH(CH₃)CO₂CH₃, -N(CH₃)CH(CH₂Ph)CO₂CH₃, -N(CH₃)CH(CH₂SH)CO₂CH₃, 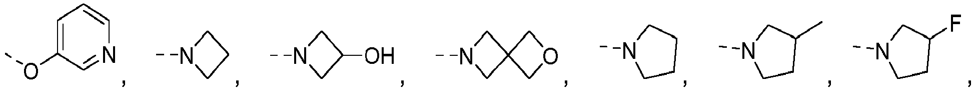 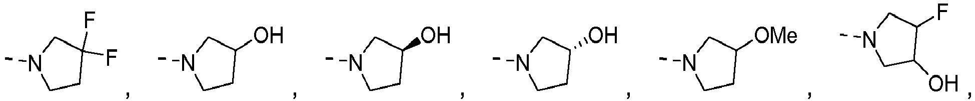 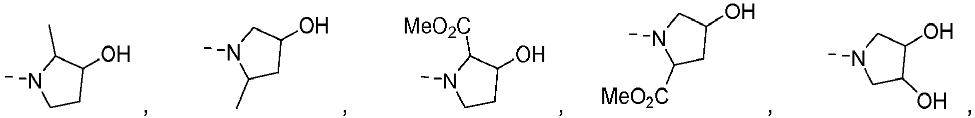 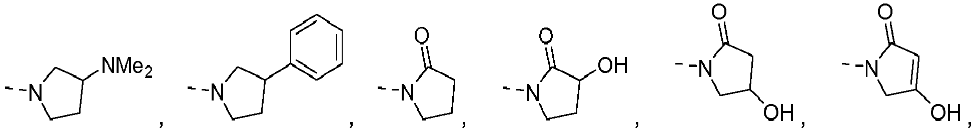 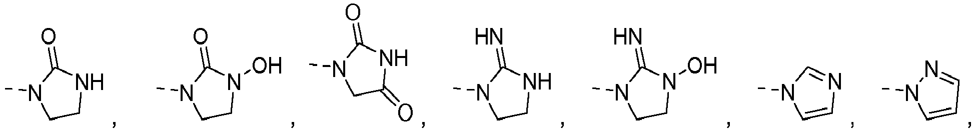 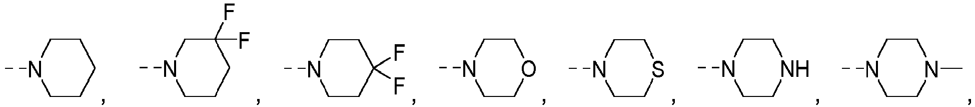 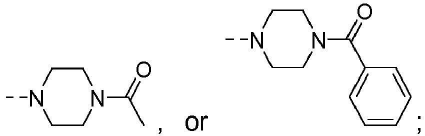 and
**R³** represents -CH₃, -CH₂CH₃, -CH₂CH₂CH₃, -CH₂CH₂CH₂CH₃, -CH₂CH(CH₃)₂, -CH₂CH₂OH, -CH₂CO₂CH₃, -CH₂C≡CH, 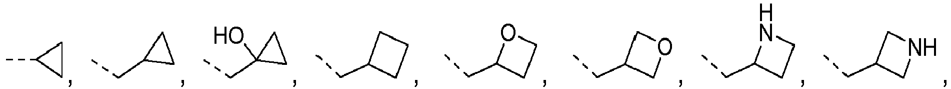 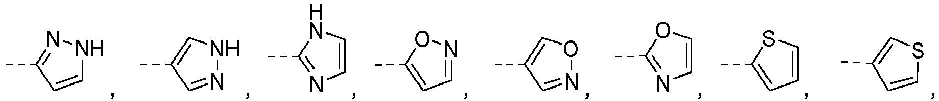 or

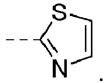

Preferred are the compounds of any one of the formulae **(I), (II-1)** - **(II-3)**, and **(IV-1)** - **(IV-8),** wherein **R¹¹** represents 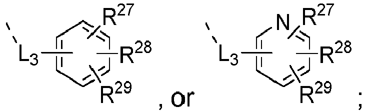 and **L₃**, **R²⁷**, **R²⁸**, and **R²⁹** have the same meanings as defined here or as defined in any preferred selection of substituents disclosed herein.

Preferred, **L¹** - **L⁵** represent independently of each other a covalent bond, -CH₂-, -CH(CH₃)-, -CH(CH₃)₂-, -CO-, -SO-, -SO₂-,

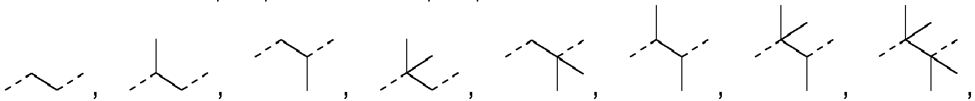

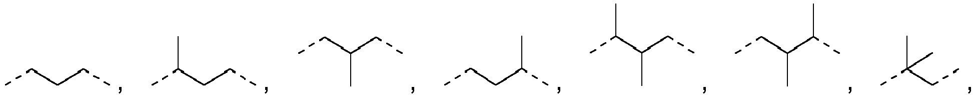

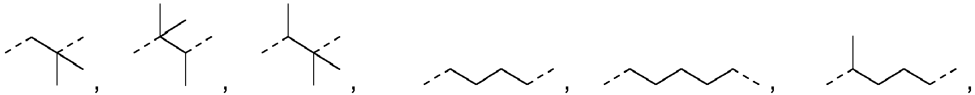

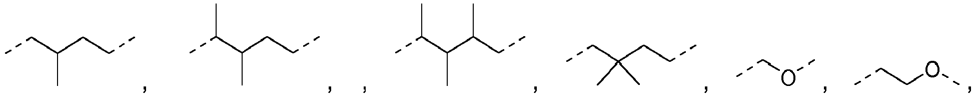

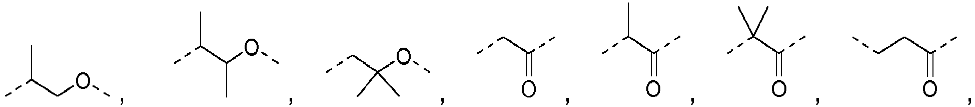

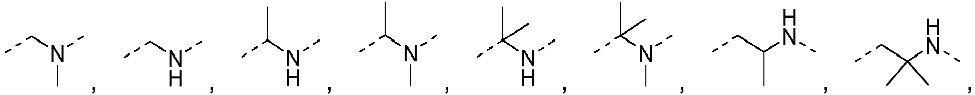

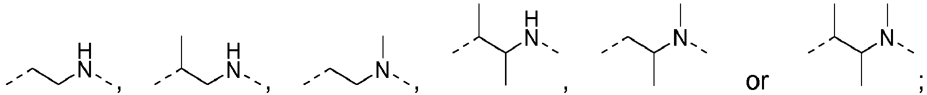

In particular, the compound of any one of the formulae **(I), (II-1)-(II-3), (III-1)-(III-3), (IV-1)-(IV-8)**, **(V-1)-(V-8)** and **(VI-1)-(VI-9)** is useful for the treatment or prophylaxis of neurodegenerative diseases, metabolic syndrome, insulin resistance, diabetes type II, obesity, liver steatosis, drug addiction, smoking cessation, mental health disorders, osteoporosis, traumatic brain injury, neuropathic and neuro-inflammatory pain, peripheral neuropathy in diabetes, endometriosis, eating disorders, and cancer.

Most preferred, the specific compound selected from the group consisiting of: compounds **1 - 205,** is useful for the treatment or prophylaxis of neurodegenerative diseases, metabolic syndrome, insulin resistance, diabetes type II, obesity, liver steatosis, drug addiction, smoking cessation, mental health disorders, osteoporosis, traumatic brain injury, neuropathic and neuro-inflammatory pain, peripheral neuropathy in diabetes, endometriosis, eating disorders, and cancer.

The pharmaceutical compositions according to the present invention comprise at least one compound according to the present invention. Preferably, the pharmaceutical compositions according to the present invention comprise at least one compound according to the present invention as an active ingredient together with at least one pharmaceutically acceptable (i.e. non-toxic) carrier, excipient and/or diluent.

The Pharmaceutical composition according to the present invention is useful for the treatment or prophylaxis of neurodegenerative diseases, metabolic syndrome, insulin resistance, diabetes type II, obesity, liver steatosis, drug addiction, smoking cessation, mental health disorders, osteoporosis, traumatic brain injury, neuropathic and neuro-inflammatory pain, peripheral neuropathy in diabetes, endometriosis, eating disorders, and cancer.

The pharmaceutical compositions of the present invention can be prepared in a conventional solid or liquid carrier or diluent and a conventional pharmaceutically made adjuvant at suitable dosage level in a known way. The preferred preparations are adapted for oral application. These administration forms include, for example, pills, tablets, film tablets, coated tablets, capsules, powders and deposits.

Furthermore, the present invention also includes pharmaceutical preparations for parenteral application, including dermal, intradermal, intragastral, intracutaneous, intravasal, intravenous, intramuscular, intraperitoneal, intranasal, intravaginal, intrabuccal, percutan, rectal, subcutaneous, sublingual, topical, or transdermal application, which preparations in addition to typical vehicles and/or diluents contain at least one compound according to the present invention and/or a pharmaceutical acceptable salt thereof as active ingredient.

The pharmaceutical compositions according to the present invention containing at least one compound according to the present invention and/or a pharmaceutical acceptable salt thereof as active ingredient will typically be administered together with suitable carrier materials selected with respect to the intended form of administration, i.e. for oral administration in the form of tablets, capsules (either solid filled, semi-solid filled or liquid filled), powders for constitution, gels, elixirs, dispersable granules, syrups, suspensions, and the like, and consistent with conventional pharmaceutical practices. For example, for oral administration in the form of tablets or capsules, the active drug component may be combined with any oral non-toxic pharmaceutically acceptable carrier, preferably with an inert carrier like lactose, starch, sucrose, cellulose, magnesium stearate, dicalcium phosphate, calcium sulfate, talc, mannitol, ethyl alcohol (liquid filled capsules) and the like. Moreover, suitable binders, lubricants, disintegrating agents and coloring agents may also be incorporated into the tablet or capsule. Powders and tablets may contain about 5 to about 95-weight % of the derivatives according to the general formula (I) or analogues compound thereof or the respective pharmaceutically active salt as active ingredient.

Suitable binders include starch, gelatin, natural sugars, corn sweeteners, natural and synthetic gums such as acacia, sodium alginate, carboxymethylcellulose, polyethylene glycol and waxes. Among suitable lubricants there may be mentioned boric acid, sodium benzoate, sodium acetate, sodium chloride, and the like. Suitable disintegrants include starch, methylcellulose, guar gum, and the like. Sweetening and flavoring agents as well as preservatives may also be included, where appropriate. The disintegrants, diluents, lubricants, binders etc. are discussed in more detail below.

Moreover, the pharmaceutical compositions of the present invention may be formulated in sustained release form to provide the rate controlled release of any one or more of the components or active ingredients to optimise the therapeutic effect(s), e.g. anticancer activity or activity against cancer metastases and the like. Suitable dosage forms for sustained release include tablets having layers of varying disintegration rates or controlled release, polymeric matrices impregnated with the active components and shaped in tablet form or capsules containing such impregnated or encapsulated porous polymeric matrices.

Liquid form preparations include solutions, suspensions, and emulsions. As an example, there may be mentioned water or water/propylene glycol solutions for parenteral injections or addition of sweeteners and opacifiers for oral solutions, suspensions, and emulsions. Liquid form preparations may also include solutions for intranasal administration.

Aerosol preparations suitable for inhalation may include solutions and solids in powder form, which may be present in combination with a pharmaceutically acceptable carrier such as an inert, compressed gas, e.g. nitrogen.

For preparing suppositories, a low melting wax, such as a mixture of fatty acid glycerides like cocoa butter is melted first, and the active ingredient is then dispersed homogeneously therein e.g. by stirring. The molten, homogeneous mixture is then poured into conveniently sized moulds, allowed to cool, and thereby solidified.

Also included are solid form preparations, which are intended to be converted, shortly before use, to liquid form preparations for either oral or parenteral administration. Such liquid forms include solutions, suspensions, and emulsions.

The compounds according to the present invention may also be delivered transdermally. The transdermal compositions may have the form of a cream, a lotion, an aerosol and/or an emulsion and may be included in a transdermal patch of the matrix or reservoir type as is known in the art for this purpose.

The term capsule as recited herein refers to a specific container or enclosure made e.g. of methylcellulose, polyvinyl alcohols, or denatured gelatins or starch for holding or containing compositions comprising the active ingredient(s). Capsules with hard shells are typically made of blended of relatively high gel strength gelatins from bones or pork skin. The capsule itself may contain small amounts of dyes, opaquing agents, plasticisers and/or preservatives.

Under tablet a compressed or moulded solid dosage form is understood which comprises the active ingredients with suitable diluents. The tablet may be prepared by compression of mixtures or granulations obtained by wet granulation, dry granulation, or by compaction well known to a person of ordinary skill in the art.

Oral gels refer to the active ingredients dispersed or solubilised in a hydrophilic semi-solid matrix.

Powders for constitution refers to powder blends containing the active ingredients and suitable diluents which can be suspended e.g. in water or in juice.

Suitable diluents are substances that usually make up the major portion of the composition or dosage form. Suitable diluents include sugars such as lactose, sucrose, mannitol, and sorbitol, starches derived from wheat, corn, rice, and potato, and celluloses such as microcrystalline cellulose. The amount of diluent in the composition can range from about 5 to about 95 % by weight of the total composition, preferably from about 25 to about 75 weight %, and more preferably from about 30 to about 60 weight %.

The term disintegrants refers to materials added to the composition to support break apart (disintegrate) and release the pharmaceutically active ingredients of a medicament. Suitable disintegrants include starches, "cold water soluble" modified starches such as sodium carboxymethyl starch, natural and synthetic gums such as locust bean, karaya, guar, tragacanth and agar, cellulose derivatives such as methylcellulose and sodium carboxymethylcellulose, microcrystalline celluloses, and cross-linked microcrystalline celluloses such as sodium croscaramellose, alginates such as alginic acid and sodium alginate, clays such as bentonites, and effervescent mixtures. The amount of disintegrant in the composition may range from about 2 to about 20 weight % of the composition, more preferably from about 5 to about 10 weight %.

Binders are substances which bind or "glue" together powder particles and make them cohesive by forming granules, thus serving as the "adhesive" in the formulation. Binders add cohesive strength already available in the diluent or bulking agent. Suitable binders include sugars such as sucrose, starches derived from wheat, corn, rice and potato, natural gums such as acacia, gelatin and tragacanth, derivatives of seaweed such as alginic acid, sodium alginate and ammonium calcium alginate, cellulose materials such as methylcellulose, sodium carboxymethylcellulose and hydroxypropylmethylcellulose, polyvinylpyrrolidone, and inorganic compounds such as magnesium aluminum silicate. The amount of binder in the composition may range from about 2 to about 20 weight % of the composition, preferably from about 3 to about 10 weight %, and more preferably from about 3 to about 6 weight %.

Lubricants refer to a class of substances which are added to the dosage form to enable the tablet granules etc. after being compressed to release from the mould by reducing friction or wear. Suitable lubricants include metallic stearates such as magnesium stearate, calcium stearate, or potassium stearate, stearic acid, high melting point waxes, and other water soluble lubricants such as sodium chloride, sodium benzoate, sodium acetate, sodium oleate, polyethylene glycols and D,L-leucine. Lubricants are usually added at the very last step before compression, since they must be present at the surface of the granules. The amount of lubricant in the composition may range from about 0.2 to about 5 weight % of the composition, preferably from about 0.5 to about 2 weight %, and more preferably from about 0.3 to about 1.5 weight % of the composition.

Glidents are materials that prevent caking of the components of the pharmaceutical composition and improve the flow characteristics of granulate so that flow is smooth and uniform. Suitable glidents include silicon dioxide and talc. The amount of glident in the composition may range from about 0.1 to about 5 weight % of the final composition, preferably from about 0.5 to about 2 weight %.

Coloring agents are excipients that provide coloration to the composition or the dosage form. Such excipients can include food grade dyes adsorbed onto a suitable adsorbent such as clay or aluminum oxide. The amount of the coloring agent may vary from about 0.1 to about 5 weight % of the composition, preferably from about 0.1 to about 1 weight %.

### Description of the Figures

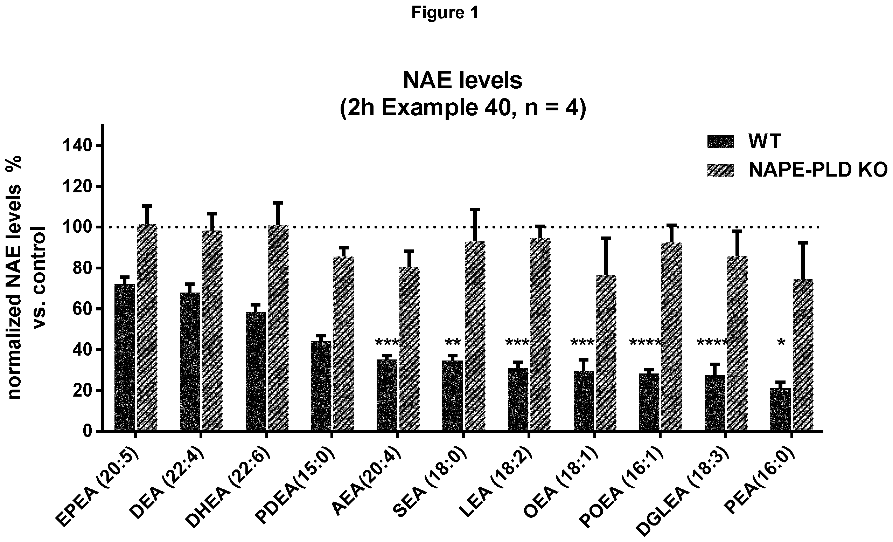

**Figure 1****.** NAPE-PLD inhibitor shows reduced NAE levels in wild-type Neuro2A cells but not in NAPE-PLD^{-/-} cells.

### Examples

Following abbreviations used in the examples have the following meaning.
- µW: Microwave
- Ac: Acetyl
- Ac₂O: Acetic anhydride
- AcOH: Acetic acid
- AEA: Arachidonoylethanolamide
- BnBr: Benzyl bromide
- Boc: *tert*-Butyloxycarbonyl
- Bz: Benzoyl
- Cbz: Carboxybenzoyl
- d: Day(s)
- DCE: Dichloroethane
- DCM: Dichloromethane
- DEA: Docosatetraenoylethanolamide
- DGLEA: Dihomo-γ-linoleoylethanolamide
- DHEA: Docosahexaenoylethanolamide
- DIPEA: *N*-Ethyldiisopropylamine
- DMAP: 4-(Dimethylamino)-pyridine
- DMEM: Dulbecco's Modified Eagle Medium
- DMF: Dimethylformamide
- DMSO: Dimethylsulfoxide
- DTT: Dithiothreitol
- EDC: *N*-(3-Dimethylaminopropyl)-*N*'-ethylcarbodiimide hydrochloride
- EPEA: Eicosapentaenoylethanolamide
- eq: Equivalent
- Et₂O: Diethylether
- EtOAc: Ethyl acetate
- EtOH: Ethanol
- h: Hour(s)
- HATU: 1-[Bis(dimethylamino)methylene]-1*H*-1,2,3-triazolo[4,5-b]pyridinium 3-oxid hexafluorophosphate
- HEK293: Human embryonic kidney cells 293
- HEPES: 4-(2-Hydroxyethyl)piperazine-1-ethanesulfonic acid
- HOBt: Hydroxybenzotriazole
- iPrOH: iso-Propanol
- IC₅₀: Half maximal inhibitory concentration
- LEA: Linoleoylethanolamide
- MeCN: Acetonitrile
- Mel: Methyl iodide
- MeOD: Deuterated methanol
- MeOH: Methanol
- min: Minute(s)
- MS: Molecular sieves
- NAE: *N*-acylethanolamine
- NaOtBu: Sodium *tert*-butoxide
- NAPE-PLD: *N*-acylphosphatidylethanolamide phospholipase D
- *n*-BuOH: *n*-Butanol
- OEA: Oleoylethanolamide
- Pd/C: Palladium on carbon
- PDEA: Pentadecanoylethanolamide
- PED6: *N*-((6-(2,4-Dinitrophenyl)amino)hexanoyl)-2-(4,4-difluoro-5,7-dimethyl-4-bora-3a,4a-diaza-*s*-indacene-3-pentanoyl)-1-hexadecanoyl-*sn*-glycero-3-phosphoethanolamine, triethylammonium Salt)
- PEA: Palmitoylethanolamide
- PEI: Polyethyleneimine
- POEA: Palmitoleoylethanolamide
- PyAOP: (7-Azabenzotriazol-1-yloxy)tripyrrolidinophosphonium hexafluorophosphate
- PyBOP: Benzotriazol-1-yl-oxytripyrrolidinophosphonium hexafluorophosphate
- SEA: Stearoylethanolamide
- sat. aq.: Saturated aqueous
- *t*-BuOH: *tert*-Butanol
- *t*-Bu: *tert*-Butyl
- TEA: Triethylamine
- TFA: Trifluoroacetic acid
- THF: Tetrahydrofuran
- Tol: Toluene
- Tris: Tris(hydroxymethyl)aminomethane
- HOBt: Hydroxybenzotriazole

The following examples are intended to illustrate the invention with selected compounds without limiting the protecting scope of the present intellectual property right on these concrete examples. It is clear for a person skilled in the art that analogous compounds and compounds produced according to analogous synthetic ways fall under the protecting scope of the present intellectual property right.

### Example 1-9, 14, 16-32, 36-42, 45-49, 51, 52, 54-80, 84, 86

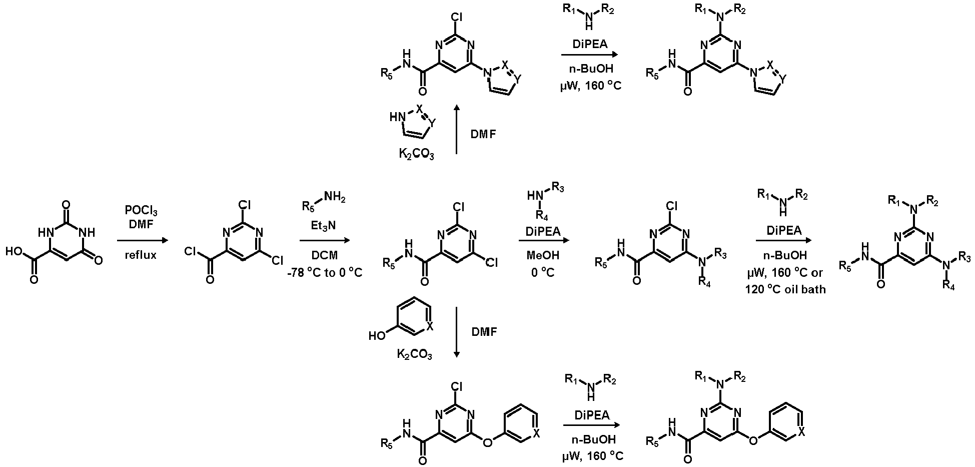

### Example 10, 16

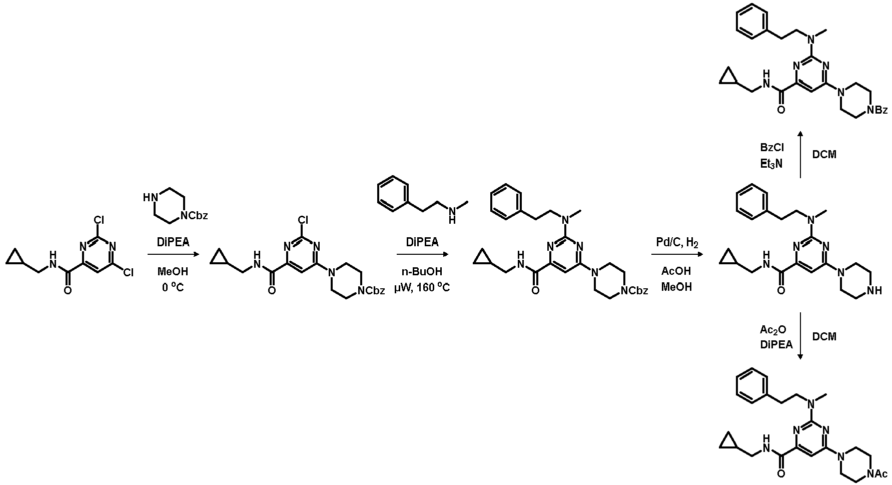

### Example 12, 13, 35

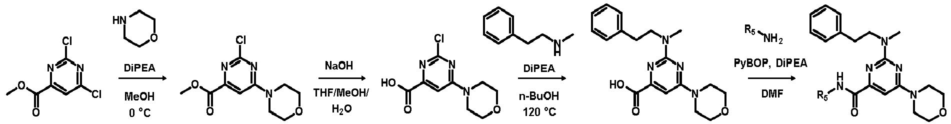

### Example 31, 33

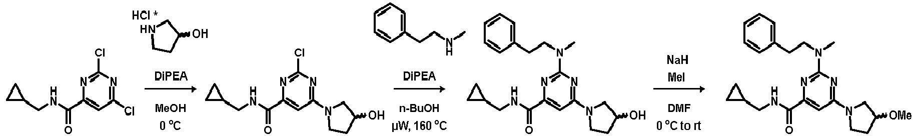

### Example intermediate amines

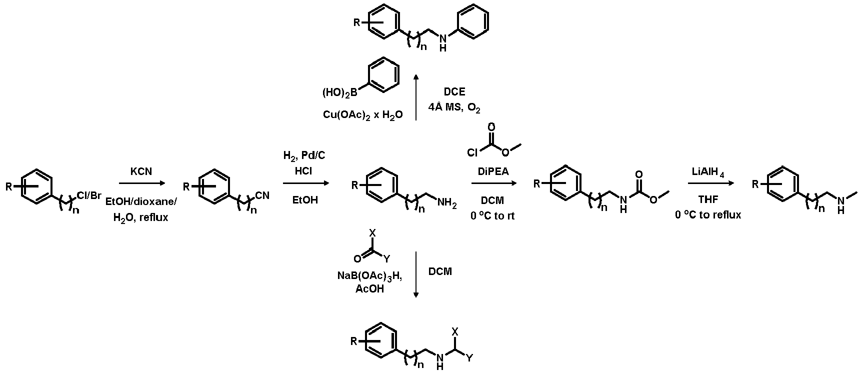

### Examples for 34, 53, 81

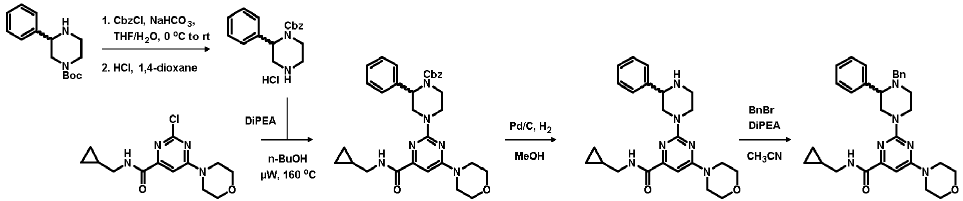

### Examples 43, 44

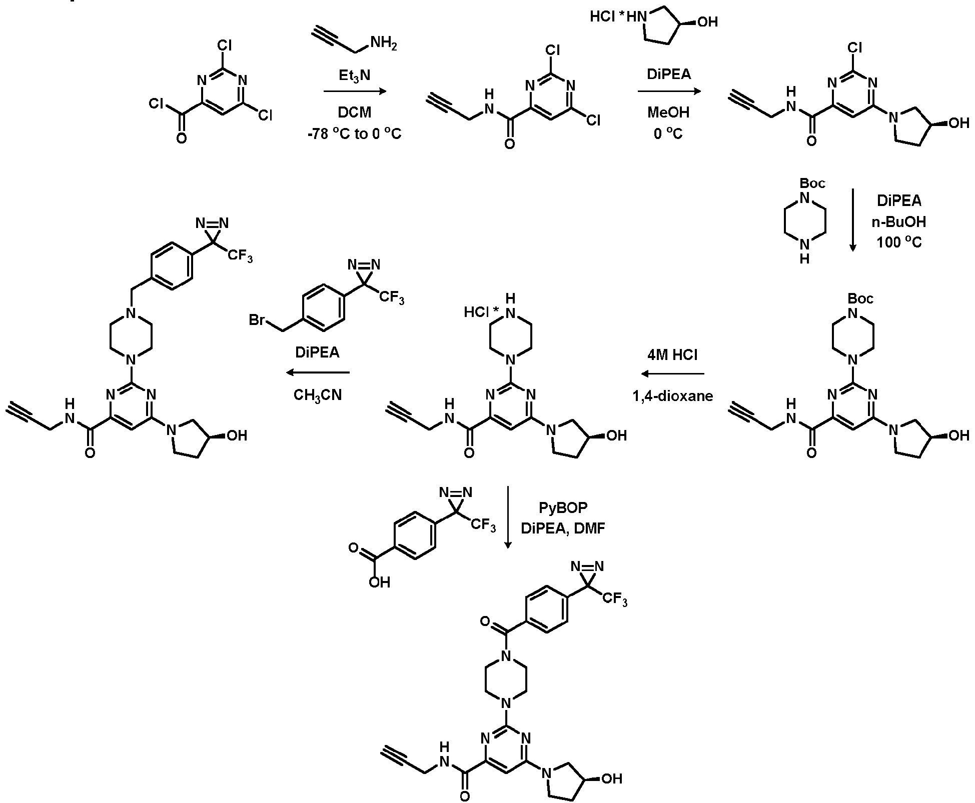

### Example 50

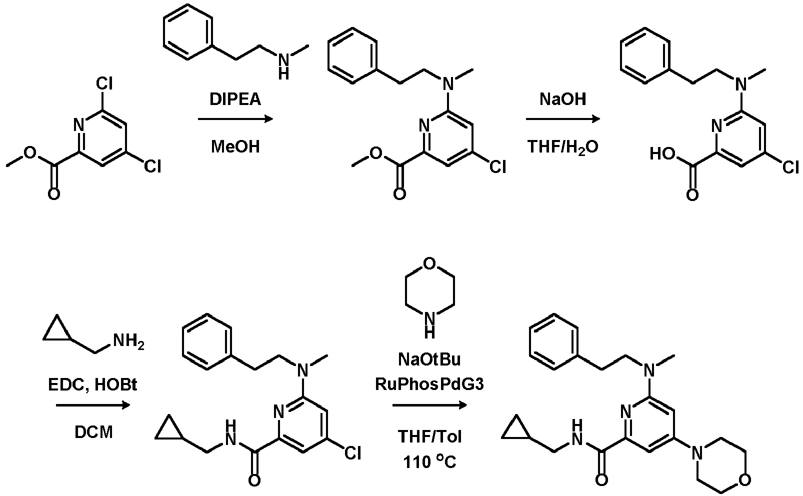

### Example 83

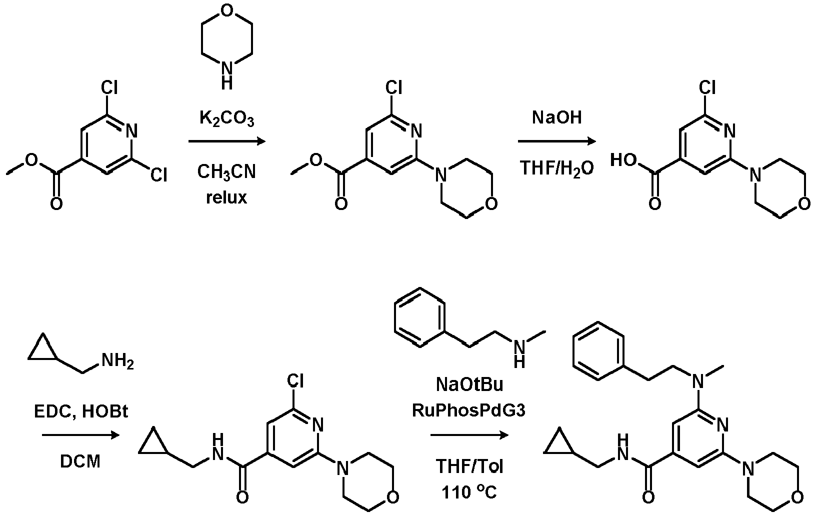

### Example 85

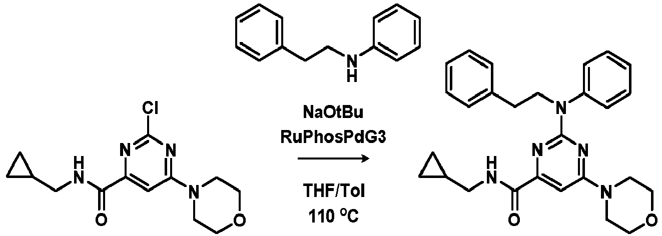

### General procedure A:

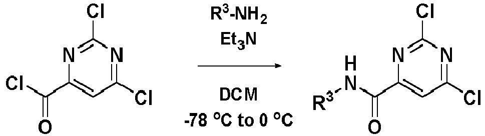

To a round bottom flask with dry DCM (0.1 M) was added via syringe 2,6-dichloropyrimidine-4-carbonyl chloride (1 eq) and cooled to -78 °C. Et₃N (1.3-2.3 eq) and the appropriate amine (1.025 eq) were added and the mixture was stirred, while letting the acetone bath warm up to 0 °C (3-4 h). The mixture was transferred to a seperatory funnel and the organic layer was washed with H₂O (2x) and brine (1x), dried (Na₂SO₄), filtered and concentrated under reduced pressure. Silica gel column chromatography afforded the pure amide.

### General procedure B:

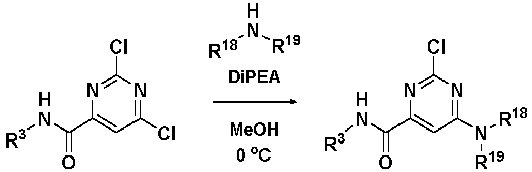

To a round bottom flask was added the dichloropyrimidine (1 eq) which was dissolved in dry MeOH (0.1 M) and brought to 0 °C. DiPEA (1.5-2.5 eq) and the appropriate amine (1.05 eq) were added and the mixture was stirred for 1-2 h at 0 °C. The solvents were evaporated under reduced pressure and the crude material was purified by silica gel column chromatography, affording the pure product.

### General procedure C:

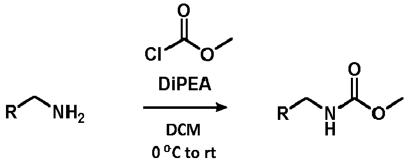

A round bottom flask was charged with the primary amine (1 eq) and dry DCM (0.2 M). The solution was cooled to 0 °C and DiPEA (2 eq) and methyl chloroformate (1.5 eq) were added. The reaction was stirred and allowed to warm up to room temperature over 1-2 h. Then the mixture was diluted with DCM and washed with sat. aq. NaHCO₃ (2x), brine (1x), dried (MgSO₄), filtered and concentrated under reduced pressure. The resulting crude material was purified by silica gel column chromatography affording the pure product.

### General procedure D:

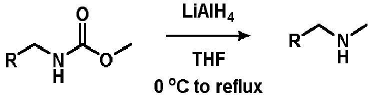

A round bottom flask was charged with the methyl carbamate (1 eq) and dry THF (0.15 M). The solution is cooled to 0 °C and LiAlH₄ (2 M in THF solution, 1.6 eq) is added dropwise. The reaction is then stirred at reflux for 1-2 h. Fieser workup involved dilution of the reaction mixture with Et₂O (3x) and cooling to 0 °C, followed by the sequential addition of water (1 µL for every 1 mg of LiAlH₄), NaOH (aq) 15% (1 µL for every 1 mg of LiAlH₄) and water (3 µL for every 1 mg of LiAlH₄). The mixture was allowed to warm to room temperature and stirred for 15 min. Then it was dried (MgSO₄), filtered and concentrated under reduced pressure to afford the product as a clear oil, which was used without further purification or purified by silica gel chromatography.

### General procedure E:

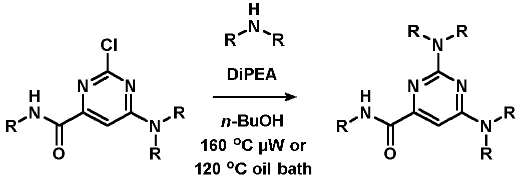

A microwave tube with a magnetic stir bar was charged with the appropriate 2-chloropyrimidine (1 eq), n-BuOH (0.2 M), the appropriate amine (1.5 eq) and DiPEA (3-4 eq). The tube was capped, flushed with N₂ and heated to 160 °C in a microwave reactor (75 W) for 4 h to 36 h or heated to 120 °C in an oil bath for 1 to 6 days. When the reaction was complete as judged by LC/MS, it was transferred to a round-bottom flask, concentrated under reduced pressure and coevaporated with toluene (2x). The residue was purified by silica gel column chromatography affording the pure product, or alternatively by HPLC-MS purification yielding the pure TFA salt. The free base was generated by dissolving the TFA salt in EtOAc and washing with sat. aq. NaHCO₃ (2x), drying (Na₂SO₄), filtering and concentration under reduced pressure.

### General procedure F:

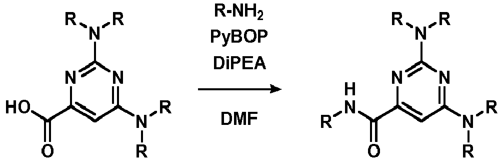

A round bottom flask was charged with carboxylic acid (1 eq) and dissolved in dry DMF (0.2 M). PyBOP (1.2-1.5 eq), DiPEA (3-5 eq) and the appropriate amine (1.2-5 eq) were added and the mixture was stirred overnight at rt. Work-up involved dilution with EtOAc, washing with H₂O (1x) and brine (2x), drying (Na₂SO₄), filtering and concentration under reduced pressure. The residue was purified by silica gel column chromatography affording the pure product.

### General procedure G:

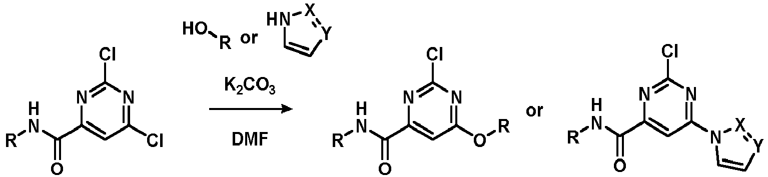

To a round bottom flask was added the dichloropyrimidine (1 eq) in dry DMF (0.1 M). K₂CO₃ (1.5 eq) and the appropriate phenol or heteroaryl (1.05 eq) were added and the mixture was stirred overnight at rt. H₂O was added and the mixture was extracted with EtOAc (3x). The organic layers were combined and washed with brine (2x), dried (Na₂SO₄) and concentrated under reduced pressure. The residue was purified by silica gel column chromatography, affording the pure product.

### General procedure H:

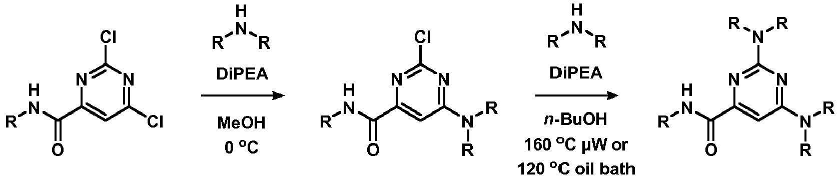

To a microwave vial was added the dichloropyrimidine (1 eq) which was dissolved in dry MeOH (0.1 M) and brought to 0 °C. DiPEA (1.5-2.5 eq) and the appropriate amine (1.05 eq) were added and the mixture was stirred for 1-2 h at 0 °C. The solvents were evaporated under reduced pressure. The vial was charged with n-BuOH (0.2 M), N-methylphenethylamine (1.5 eq) and DiPEA (3-4 eq). The tube was capped, flushed with N₂ and heated to 160 °C in a microwave reactor (75 W) for 4 h. When the reaction was complete as judged by LC/MS, it was transferred to a round-bottom flask, concentrated under reduced pressure and co-evaporated with toluene (2x). The residue is purified by silica gel column chromatography affording the pure product, or alternatively by HPLC-MS purification yielding the pure TFA salt. The free base was generated by dissolving the TFA salt in EtOAc and washing with sat. aq. NaHCO₃ (2x), drying (Na₂SO₄), filtering and concentration under reduced pressure.

### Example 1-1: Preparation of 2,6-dichloropyrimidine-4-carbonyl chloride (I-1)

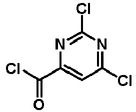

In a 500 mL round bottom flask orotic acid (15.6 g, 100 mmol, 1 equiv.) was dissolved in phosphorous oxychloride (46 mL, 500 mmol, 5 equiv.) and 10 drops of DMF were added. The mixture was heated to reflux and stirred for 19 h. n-Hexane (250 mL) was added and the mixture was stirred vigorously for 10 min and then transferred to a seperatory funnel containing 100 mL H₂O. The flask was washed with 50 mL hexane. After shaking, the aqueous layer was removed and the hexane was washed with brine (1 x 100 mL), dried with MgSO₄ and concentrated under reduced pressure to yield the product (12.8 g, 60.4 mmol, 60%). ¹H NMR (500 MHz, CDCl₃) δ 8.00 (s, 1H). ¹³C NMR (126 MHz, CDCl₃) δ 167.17, 165.27, 161.56, 158.19, 119.70.

### Example 1-1: 2,6-dichloro-N-ethylpyrimidine-4-carboxamide (I-2)

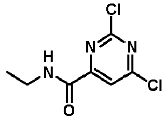

The title compound was prepared according to General Procedure A using 2,6-dichloropyrimidine-4-carbonyl chloride **I-1** (0.63 mL, 5 mmol, 1 eq), Et₃N (1.6 mL, 11.5 mmol, 2.3 eq) and ethylamine HCl salt (0.42 g, 5.13 mmol, 1.025 eq). Column chromatography (5% -> 20% EtOAc/pentane) afforded the product (0.88 g, 4.0 mmol, 80%). TLC: R_{f} = 0.6 (20% EtOAc/pentane). ¹H NMR (400 MHz, CDCl₃) δ 8.10 (s, 1H), 7.83 (br s, 1H), 3.65 - 3.38 (m, 2H), 1.30 (t, *J* = 7.3 Hz, 3H). ¹³C NMR (101 MHz, CDCl₃) δ 164.84, 160.47, 160.06, 159.83, 118.18, 34.86, 14.63. HRMS [C₇H₇Cl₂N₃O + H]⁺ : 220.0039 calculated, 220.0040 found (Δ = 0.45 ppm).

### 2-Chloro-N-ethyl-6-morpholinopyrimidine-4-carboxamide (I-3)

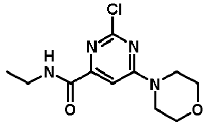

The title compound was prepared according to General Procedure B using dichloropyrimidine **I-2** (0.42 g, 1.89 mmol, 1 eq), DiPEA (0.49 mL, 2.84 mmol, 1.5 eq) and morpholine (0.17 mL, 1.98 mmol, 1.05 eq). Column chromatography (50% -> 70% EtOAc/pentane) afforded the product (0.48 g, 1.77 mmol, 94%). TLC: R_{f} = 0.2 (50% EtOAc/pentane). ¹H NMR (400 MHz, CDCl₃) δ 7.82 (br s, 1H), 7.27 (s, 1H), 3.99 - 3.55 (m, 8H), 3.54 - 3.37 (m, 2H), 1.25 (t, *J* = 7.3 Hz, 3H). ¹³C NMR (101 MHz, CDCl₃) δ 163.86, 162.17, 159.85, 157.93, 99.27, 66.39, 44.63, 34.53, 14.71. HRMS [C₁₁H₁₅ClN₄O₂ + H]⁺ : 271.0956 calculated, 271.0957 found (Δ = 0.17 ppm).

### N-Ethyl-2-(methyl(phenethyl)amino)-6-morpholinopyrimidine-4-carboxamide (Example 4)

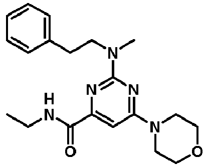

The title compound was prepared according to General Procedure E using 2-chloropyrimidine **I-3** (54 mg, 0.20 mmol, 1 eq), DiPEA (139 µL, 0.80 mmol, 4 eq) and N-methylphenethylamine HBr salt (65 mg, 0.30 mmol, 1.5 eq). Total heating time: 4 h at 160 °C with µW irradiation. Column chromatography (50% -> 70% EtOAc/pentane) afforded the product (64 mg, 0.17 mmol, 86%). TLC: R_{f} = 0.3 (50% EtOAc/pentane). ¹H NMR (500 MHz, CDCl₃) δ 7.88 (s, 1H), 7.32 - 7.26 (m, 2H), 7.24 - 7.16 (m, 3H), 6.71 (s, 1H), 3.84 - 3.77 (m, 2H), 3.77 - 3.73 (m, 4H), 3.69 - 3.62 (m, 4H), 3.50 - 3.42 (m, 2H), 3.11 (s, 3H), 2.94 - 2.84 (m, 2H), 1.25 (t, *J* = 7.3 Hz, 3H). ¹³C NMR (126 MHz, CDCl₃) δ 164.65, 163.98, 160.88, 156.82, 139.99, 128.83, 128.59, 126.29, 90.02, 66.74, 51.63, 44.51, 35.71, 34.26, 33.98, 14.94. HRMS [C₂₀H₂₇N₅O₂ + H]⁺ : 370.2238 calculated, 370.2236 found (Δ = -0.46 ppm).

### 2,6-Dichloro-N-(cyclopropylmethyl)pyrimidine-4-carboxamide (I-4)

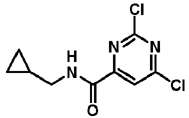

The title compound was prepared according to General Procedure A using 2,6-dichloropyrimidine-4-carbonyl chloride **I-1** (0.63 mL, 5 mmol, 1 eq), Et₃N (0.91 mL, 6.5 mmol, 1.3 eq) and cyclopropylmethanamine (444 µL, 5.13 mmol, 1.025 eq). Column chromatography (5% -> 20% EtOAc/pentane) afforded the product (0.99 g, 4.0 mmol, 81%). TLC: R_{f} = 0.8 (20% EtOAc/pentane). ¹H NMR (400 MHz, CDCl₃) δ 8.11 (s, 1H), 7.96 (br s, 1H), 3.44 - 3.27 (m, 2H), 1.20 - 1.03 (m, 1H), 0.68 - 0.52 (m, 2H), 0.32 (q, *J* = 4.8 Hz, 2H). ¹³C NMR (101 MHz, CDCl₃) δ 164.74, 160.44, 160.04, 159.77, 118.22, 44.72, 10.57, 3.67. HRMS [C₉H₁₀Cl₂N₃O + H]⁺ : 246.0195 calculated, 246.0196 found (Δ = 0.12 ppm).

### 2-Chloro-N-(cyclopropylmethyl)-6-(piperidin-1-yl)pyrimidine-4-carboxamide (I-5)

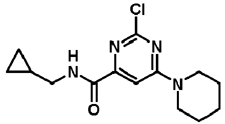

The title compound was prepared according to General Procedure B using dichloropyrimidine **I-4** (258 mg, 1.05 mmol, 1 eq), DiPEA (274 µL, 1.58 mmol, 1.5 eq) and piperidine (109 µL, 1.10 mmol, 1.05 eq). Column chromatography (10% -> 40% EtOAc/pentane) afforded the product (293 mg, 0.99 mmol, 95%). TLC: R_{f} = 0.2 (20% EtOAc/pentane). ¹H NMR (400 MHz, CDCl₃) δ 7.95 (br s, 1H), 7.27 (s, 1H), 3.71 (br s, 4H), 3.29 (t, *J* = 6.5 Hz, 2H), 1.85 - 1.52 (m, 6H), 1.15 - 0.98 (m, 1H), 0.55 (q, *J* = 5.4 Hz, 2H), 0.28 (q, *J =* 4.8 Hz, 2H). ¹³C NMR (101 MHz, CDCl₃) δ 163.12, 162.45, 159.77, 157.35, 99.29, 44.31, 25.55, 24.31, 10.61, 3.58. HRMS [C₁₄H₁₉ClN₄O₂ + H]⁺ : 295.1320 calculated, 295.1321 found (Δ = 0.20 ppm).

### N-(Cyclopropylmethyl)-2-(methyl(phenethyl)amino)-6-(piperidin-1-yl)pyrimidine-4-carboxamide (Example 3)

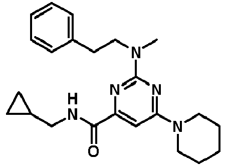

The title compound was prepared according to General Procedure E using 2-chloropyrimidine **I-5** (44 mg, 0.15 mmol, 1 eq), DiPEA (105 µL, 0.60 mmol, 4 eq) and N-methylphenethylamine HBr salt (49 mg, 0.225 mmol, 1.5 eq). Total heating time: 4 h at 160 °C with µW irradiation. Column chromatography (20% -> 50% EtOAc/pentane) afforded the product (37 mg, 94 µmol, 63%). TLC: R_{f} = 0.4 (20% EtOAc/pentane). ¹H NMR (400 MHz, CDCl₃) δ 8.07 (t, *J* = 4.8 Hz, 1H), 7.35 - 7.16 (m, 5H), 6.76 (s, 1H), 3.84 - 3.75 (m, 2H), 3.67 (br s, 4H), 3.34 - 3.25 (m, 2H), 3.13 (s, 3H), 2.95 - 2.87 (m, 2H), 1.74 - 1.65 (m, 2H), 1.65 - 1.55 (m, 4H), 1.13 - 1.00 (m, 1H), 0.59 - 0.50 (m, 2H), 0.28 (q, *J* = 4.7 Hz, 2H). ¹³C NMR (101 MHz, CDCl₃) δ 165.02, 163.41, 161.01, 156.35, 140.10, 128.88, 128.56, 126.22, 90.36, 51.80, 45.37, 44.07, 35.66, 33.94, 25.77, 24.95, 10.91, 3.48. HRMS [C₂₃H₃₁N₅O + H]⁺ : 394.2601 calculated, 394.2592 found (Δ = -2.33 ppm).

### 2-Chloro-N-(cyclopropylmethyl)-6-thiomorpholinopyrimidine-4-carboxamide (I-6)

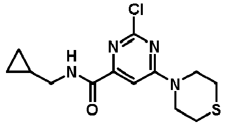

The title compound was prepared according to General Procedure B using dichloropyrimidine **I-4** (117 mg, 0.48 mmol, 1 eq), DiPEA (124 µL, 0.71 mmol, 1.5 eq) and thiomorpholine (51 µL, 0.50 mmol, 1.05 eq). Column chromatography (20% -> 50% EtOAc/pentane) afforded the product (48 mg, 0.15 mmol, 32%). TLC: R_{f} = 0.2 (20% EtOAc/pentane). ¹H NMR (400 MHz, CDCl₃) δ 7.91 (br s, 1H), 7.28 (s, 1H), 4.07 (br s, 4H), 3.36 - 3.23 (m, 2H), 2.74 - 2.63 (m, 4H), 1.12 - 0.99 (m, 1H), 0.65 - 0.48 (m, 2H), 0.29 (q, *J* = 4.8 Hz, 2H). ¹³C NMR (101 MHz, CDCl₃) δ 163.33, 162.30, 160.04, 158.07, 99.66, 44.55, 27.05, 10.73, 3.74. HRMS [C₁₄H₁₉ClN₄O₂ + H]⁺ : 295.1320 calculated, 295.1321 found (Δ = 0.20 ppm).

### N-(Cyclopropylmethyl)-2-(methyl(phenethyl)amino)-6-thiomorpholinopyrimidine-4-carboxamide (Example 5)

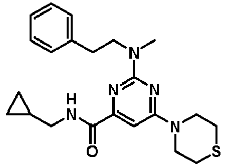

The title compound was prepared according to General Procedure E using 2-chloropyrimidine **I-6** (41 mg, 0.13 mmol, 1 eq), DiPEA (91 µL, 0.52 mmol, 4 eq) and N-methylphenethylamine HBr salt (43 mg, 0.20 mmol, 1.5 eq). Total heating time: 4 h at 160 °C with µW irradiation. Column chromatography (10% -> 40% EtOAc/pentane) afforded the product (51 mg, 0.12 mmol, 95%). TLC: R_{f} = 0.4 (25% EtOAc/pentane). ¹H NMR (400 MHz, CDCl₃) δ 8.05 (br s, 1H), 7.33 - 7.27 (m, 2H), 7.25 - 7.18 (m, 3H), 6.73 (s, 1H), 4.03 (br s, 4H), 3.84 - 3.74 (m, 2H), 3.35 - 3.26 (m, 2H), 3.13 (s, 3H), 2.96 - 2.85 (m, 2H), 2.70 - 2.58 (m, 4H), 1.13 - 0.99 (m, 1H), 0.55 (q, *J* = 5.7 Hz, 2H), 0.29 (q, *J* = 4.9 Hz, 2H). ¹³C NMR (101 MHz, CDCl₃) δ 164.67, 163.05, 156.85, 139.87, 128.80, 128.58, 126.29, 90.34, 51.74, 47.24, 44.08, 35.71, 33.90, 26.68, 10.88, 3.47. HRMS [C₂₂H₂₉N₅OS + H]⁺ : 412.2166 calculated, 412.2159 found (Δ = -1.60 ppm).

### Methyl (2,6-dichloropyrimidine-4-carbonyl)glycinate (I-7)

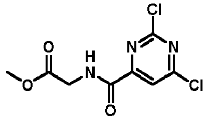

The title compound was prepared according to General Procedure A using 2,6-dichloropyrimidine-4-carbonyl chloride **I-1** (0.32 mL, 2.5 mmol, 1 eq), Et₃N (0.80 mL, 5.75 mmol, 2.3 eq) and glycine methylester HCl salt (0.32 g, 2.56 mmol, 1.025 eq). Column chromatography (5% -> 20% EtOAc/pentane) afforded the product (0.51 g, 1.95 mmol, 78%). TLC: R_{f} = 0.5 (30% EtOAc/pentane). ¹H NMR (500 MHz, CDCl₃) δ 8.31 (br s, 1H), 8.09 (s, 1H), 4.28 (d, *J* = 5.8 Hz, 2H), 3.81 (s, 3H). ¹³C NMR (126 MHz, CDCl₃) δ 169.30, 164.89, 160.65, 160.02, 159.55, 118.31, 52.66, 41.35. HRMS [C₁₄H₁₉ClN₄O₂ + H]⁺ : 295.1320 calculated, 295.1321 found (Δ = 0.20 ppm).

### Methyl (2-chloro-6-morpholinopyrimidine-4-carbonyl)glycinate (I-8)

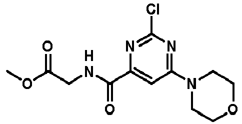

The title compound was prepared according to General Procedure B using dichloropyrimidine **I-7** (396 mg, 1.50 mmol, 1 eq), DiPEA (392 µL, 2.25 mmol, 1.5 eq) and morpholine (137 µL, 1.58 mmol, 1.05 eq). Column chromatography (60% -> 80% EtOAc/pentane) afforded the product (298 mg, 0.95 mmol, 63%). TLC: R_{f} = 0.5 (70% EtOAc/pentane). ¹H NMR (400 MHz, CDCl₃) δ 8.25 (t, *J* = 5.4 Hz, 1H), 7.25 (s, 1H), 4.22 (d, *J* = 5.9 Hz, 2H), 3.90 - 3.61 (m, 11H). ¹³C NMR (101 MHz, CDCl₃) δ 169.59, 163.83, 162.85, 160.06, 157.09, 99.50, 66.39, 52.51, 44.69, 41.29. HRMS [C₁₄H₁₉ClN₄O₂ + H]⁺ : 295.1320 calculated, 295.1321 found (Δ = 0.20 ppm).

### Sodium (2-chloro-6-morpholinopyrimidine-4-carbonyl) glycinate (I-9)

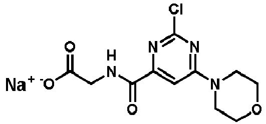

A round bottom flask was charged with methyl ester **I-8** (267 mg, 0.85 mmol, 1 eq) in 5 mL THF/MeOH (4:1, v/v). A 1.5M aqueous NaOH solution (0.57 mL, 0.85 mmol, 1 eq) was added together with 0.43 mL H₂O. The reaction was stirred overnight at rt after which the solvents where evaporated yielding the product which was used without further purification (270 mg, 0.85 mmol, quant.). TLC: R_{f} = 0.2 (5% MeOH/DCM). ¹H NMR (400 MHz, MeOD) δ 7.30 (s, 1H), 3.95 - 3.86 (m, 2H), 3.81 - 3.65 (m, 8H). ¹³C NMR (101 MHz, MeOD) δ 180.70, 159.60, 159.40, 158.60, 152.43, 99.06, 65.99, 43.24.

### (2-(Methyl(phenethyl)amino)-6-morpholinopyrimidine-4-carbonyl)glycine (I-10)

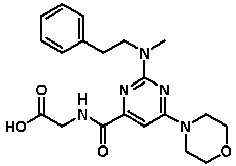

The title compound was prepared according to General Procedure E using 2-chloropyrimidine **I-9** (150 mg, 0.50 mmol, 1 eq), DiPEA (0.43 mL, 2.5 mmol, 5 eq) and N-methylphenethylamine HBr salt (162 mg, 0.76 mmol, 1.5 eq). Total heating time: 6 h at 160 °C with µW irradiation. Purification by HPLC (C18 reverse phase, 10% to 70% ACN/H₂O+50 mM NH₄HCO₃) afforded the product (40 mg, 0.10 mmol, 20%). TLC: R_{f} = 0.3 (5% MeOH/DCM). ¹H NMR (500 MHz, MeOD + CDCl₃) δ 7.32 - 7.14 (m, 5H), 6.65 (s, 1H), 4.01 (s, 2H), 3.83 (t, *J* = 7.3 Hz, 2H), 3.80 - 3.75 (m, 4H), 3.73 - 3.60 (m, 4H), 3.11 (s, 3H), 2.91 (t, *J* = 7.4 Hz, 2H). ¹³C NMR (126 MHz, MeOD + CDCl₃) δ 173.63, 164.96, 163.55, 160.59, 155.89, 139.54, 128.50, 128.00, 125.67, 89.33, 66.26, 51.10, 49.06, 43.96, 42.64, 39.58, 35.05, 33.47, 20.71. HRMS [C₂₀H₂₅N₅O₄ + H]⁺ : 400.1979 calculated, 400.1984 found (Δ = 1.17 ppm).

### Methyl (2-(methyl(phenethyl)amino)-6-morpholinopyrimidine-4-carbonyl)glycinate (Example 15)

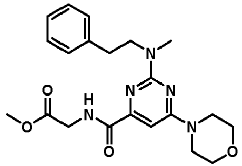

A round bottom flask was charged with carboxylic acid **I-10** (28 mg, 70 µmol, 1 eq) in dry DCM (1.5 mL). This was followed by addition of HOBt (15 mg, 0.11 mmol, 1.5 eq), EDC*HCl (20 mg, 0.11 mmol, 1.5 eq). The reaction was stirred for 1h at rt after which MeOH (11 µL, 0.28 mmol, 4 eq) was added and then stirred overnight at rt. The reaction was diluted with EtOAc (25 mL), washed with sat. aq. NaHCO₃ (2 x 25 mL), dried (Na₂SO₄), filtered and concentrated under reduced pressure. The residue was purified using silica gel column chromatography (60% -> 80% EtOAc/pentane) affording the product (18 mg, 44 µmol, 62%). TLC: R_{f} = 0.3 (70% EtOAc/pentane). ¹H NMR (400 MHz, CDCl₃) δ 8.40 (br s, 1H), 7.39 - 7.13 (m, 5H), 6.69 (s, 1H), 4.22 (d, *J =* 5.5 Hz, 2H), 3.86 - 3.71 (m, 9H), 3.71 - 3.59 (m, 4H), 3.11 (s, 3H), 2.96 - 2.85 (m, 2H). ¹³C NMR (101 MHz, CDCl₃) δ 170.25, 165.14, 163.91, 160.92, 155.99, 139.96, 128.98, 128.58, 126.27, 90.13, 66.75, 52.52, 51.68, 44.51, 41.37, 35.79, 34.01. HRMS [C₂₁H₂₇N₅O₄ + H]⁺ : 414.2136 calculated, 414.2144 found (Δ = 1.98 ppm).

### 2,6-Dichloro-N-isobutylpyrimidine-4-carboxamide (I-11)

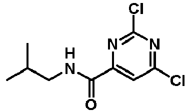

The title compound was prepared according to General Procedure A using 2,6-dichloropyrimidine-4-carbonyl chloride **I-1** (0.25 mL, 2.0 mmol, 1 eq), Et₃N (0.36 mL, 2.60 mmol, 1.3 eq) and isobutylamine (0.20 mL, 2.05 mmol, 1.025 eq). Column chromatography (5% -> 20% EtOAc/pentane) afforded the product (0.50 g, 2.0 mmol, 99%). TLC: R_{f} = 0.8 (20% EtOAc/pentane). ¹H NMR (400 MHz, CDCl₃) δ 8.11 (s, 1H), 7.94 (br s, 1H), 3.33 (t, *J* = 6.6 Hz, 2H), 2.07 - 1.87 (m, 1H), 1.00 (d, *J* = 6.8 Hz, 6H). ¹³C NMR (101 MHz, CDCl₃) δ 164.68, 160.41, 160.15, 159.70, 118.17, 47.10, 28.52, 20.07. HRMS [C₁₄H₁₉ClN₄O₂ + H]⁺ : 295.1320 calculated, 295.1321 found (Δ = 0.20 ppm).

### 2-Chloro-N-isobutyl-6-morpholinopyrimidine-4-carboxamide (I-12)

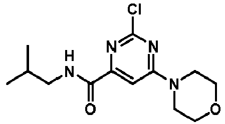

The title compound was prepared according to General Procedure B using dichloropyrimidine **I-11** (275 mg, 1.11 mmol, 1 eq), DiPEA (290 µL, 1.66 mmol, 1.5 eq) and morpholine (101 µL, 1.16 mmol, 1.05 eq). Column chromatography (40% -> 60% EtOAc/pentane) afforded the product (330 mg, 1.11 mmol, 99%). TLC: R_{f} = 0.5 (50% EtOAc/pentane). ¹H NMR (400 MHz, CDCl₃) δ 7.91 (t, *J* = 5.8 Hz, 1H), 7.28 (s, 1H), 4.01 - 3.52 (m, 8H), 3.26 (t, *J* = 6.7 Hz, 2H), 2.00 - 1.81 (m, 1H), 0.97 (d, *J* = 6.7 Hz, 6H). ¹³C NMR (101 MHz, CDCl₃) δ 163.75, 162.25, 159.71, 157.83, 99.23, 66.26, 46.82, 44.56, 28.58, 20.11. HRMS [C₁₄H₁₉ClN₄O₂ + H]⁺ : 295.1320 calculated, 295.1321 found (Δ = 0.20 ppm).

### N-Isobutyl-2-(methyl(phenethyl)amino)-6-morpholinopyrimidine-4-carboxamide (Example 6)

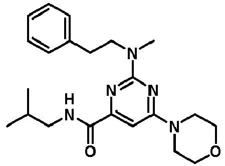

The title compound was prepared according to General Procedure E using 2-chloropyrimidine **I-12** (30 mg, 0.10 mmol, 1 eq), DiPEA (70 µL, 0.40 mmol, 4 eq) and N-methylphenethylamine HBr salt (32 mg, 0.15 mmol, 1.5 eq). Total heating time: 3 d at 120 °C. Column chromatography (40% -> 60% EtOAc/pentane) afforded the product (29 mg, 73 µmol, 73%). TLC: R_{f} = 0.7 (50% EtOAc/pentane). ¹H NMR (400 MHz, CDCl₃) δ 8.04 (br s, 1H), 7.33 - 7.25 (m, 2H), 7.25 - 7.16 (m, 3H), 6.72 (s, 1H), 3.88 - 3.73 (m, 6H), 3.73 - 3.62 (m, 4H), 3.26 (t, *J* = 6.5 Hz, 2H), 3.10 (s, 3H), 2.96 - 2.85 (m, 2H), 1.97 - 1.82 (m, 1H), 0.98 (d, *J* = 6.7 Hz, 6H). ¹³C NMR (101 MHz, CDCl₃) δ 164.77, 164.01, 160.84, 156.84, 139.94, 128.84, 128.58, 126.29, 90.07, 66.75, 51.63, 46.65, 44.52, 35.72, 33.98, 28.80, 20.28. HRMS [C₂₂H₃₁N₅O₂ + H]⁺ : 398.2551 calculated, 398.2552 found (Δ = 0.38 ppm).

### N-Butyl-2,6-dichloropyrimidine-4-carboxamide (I-13)

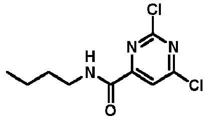

The title compound was prepared according to General Procedure A using 2,6-dichloropyrimidine-4-carbonyl chloride **I-1** (0.25 mL, 2.0 mmol, 1 eq), Et₃N (0.36 mL, 2.60 mmol, 1.3 eq) and n-butylamine (0.20 mL, 2.05 mmol, 1.025 eq). Column chromatography (5% -> 20% EtOAc/pentane) afforded the product (0.50 g, 2.0 mmol, 99%). TLC: R_{f} = 0.7 (20% EtOAc/pentane). ¹H NMR (400 MHz, CDCl₃) δ 8.10 (s, 1H), 7.85 (br s, 1H), 3.49 (q, *J* = 7.0 Hz, 2H), 1.74 - 1.56 (m, 2H), 1.53 - 1.35 (m, 2H), 0.97 (t, *J* = 7.4 Hz, 3H). ¹³C NMR (101 MHz, CDCl₃) δ 164.78, 160.46, 160.13, 159.78, 118.18, 39.64, 31.40, 20.09, 13.72.

### N-Butyl-2-chloro-6-morpholinopyrimidine-4-carboxamide (I-14)

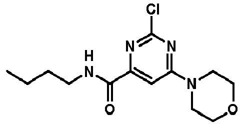

The title compound was prepared according to General Procedure B using dichloropyrimidine **I-13** (193 mg, 0.78 mmol, 1 eq), DiPEA (203 µL, 1.17 mmol, 1.5 eq) and morpholine (71 µL, 0.82 mmol, 1.05 eq). Column chromatography (40% -> 60% EtOAc/pentane) afforded the product (212 mg, 0.71 mmol, 91%). TLC: R_{f} = 0.4 (50% EtOAc/pentane). ¹H NMR (400 MHz, CDCl₃) δ 7.84 (br s, 1H), 7.27 (s, 1H), 3.91 - 3.61 (m, 8H), 3.43 (q, *J* = 7.1 Hz, 2H), 1.70 - 1.51 (m, 2H), 1.48 - 1.34 (m, 2H), 0.95 (t, *J =* 7.3 Hz, 3H). ¹³C NMR (101 MHz, CDCl₃) δ 163.79, 162.19, 159.75, 157.88, 99.21, 66.31, 44.57, 39.29, 31.48, 20.07, 13.72. HRMS [C₁₄H₁₉ClN₄O₂ + H]⁺ : 295.1320 calculated, 295.1321 found (Δ = 0.20 ppm).

### N-Butyl-2-(methyl(phenethyl)amino)-6-morpholinopyrimidine-4-carboxamide (Example 9)

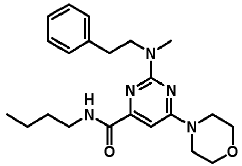

The title compound was prepared according to General Procedure E using 2-chloropyrimidine **I-14** (30 mg, 0.10 mmol, 1 eq), DiPEA (70 µL, 0.40 mmol, 4 eq) and N-methylphenethylamine HBr salt (32 mg, 0.15 mmol, 1.5 eq). Total heating time: 45h at 120°C. Column chromatography (40% -> 60% EtOAc/pentane) afforded the product (29 mg, 73 µmol, 73%). TLC: R_{f} = 0.6 (50% EtOAc/pentane). ¹H NMR (400 MHz, CDCl₃) δ 7.94 (br s, 1H), 7.33 - 7.25 (m, 2H), 7.24 - 7.16 (m, 3H), 6.72 (s, 1H), 3.84 - 3.72 (m, 6H), 3.66 (br s, 4H), 3.42 (q, *J* = 6.6 Hz, 2H), 3.11 (s, 3H), 2.95 - 2.84 (m, 2H), 1.60 (p, *J* = 7.1 Hz, 2H), 1.42 (h, *J* = 7.3 Hz, 2H), 0.96 (t, *J* = 7.3 Hz, 3H). ¹³C NMR (101 MHz, CDCl₃) δ 164.74, 164.00, 160.86, 156.83, 139.97, 128.84, 128.60, 126.31, 90.05, 66.76, 51.64, 44.52, 39.12, 35.72, 33.97, 31.81, 20.30, 13.94. HRMS [C₂₂H₃₁N₅O₂ + H]⁺ : 398.2551 calculated, 398.2560 found (Δ = 2.39 ppm).

### 2-Chloro-N-(cyclopropylmethyl)-6-(4-methylpiperazin-1-yl)pyrimidine-4-carboxamide (I-15)

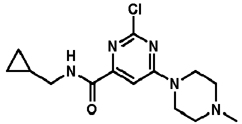

The title compound was prepared according to General Procedure B using dichloropyrimidine **I-4** (96 mg, 0.39 mmol, 1 eq), DiPEA (102 µL, 0.59 mmol, 1.5 eq) and 1-methylpiperazine (45 µL, 0.41 mmol, 1.05 eq). Column chromatography (5% -> 10% MeOH/DCM) afforded the product (77 mg, 0.25 mmol, 64%). TLC: R_{f} = 0.4 (5% MeOH/DCM). ¹H NMR (500 MHz, CDCl₃) δ 7.99 - 7.84 (m, 1H), 7.28 (s, 1H), 3.96 - 3.58 (m, 4H), 3.35 - 3.24 (m, 2H), 2.54 - 2.44 (m, 4H), 2.34 (s, 3H), 1.12 - 0.99 (m, 1H), 0.64 - 0.50 (m, 2H), 0.28 (q, *J* = 4.8 Hz, 2H). ¹³C NMR (126 MHz, CDCl₃) δ 163.61, 162.39, 159.88, 157.74, 99.52, 54.50, 46.06, 44.49, 43.44, 10.70, 3.71. HRMS [C₁₄H₁₉ClN₄O₂ + H]⁺ : 295.1320 calculated, 295.1321 found (Δ = 0.20 ppm).

### N-(Cyclopropylmethyl)-2-(methyl(phenethyl)amino)-6-(4-methylpiperazin-1-yl)pyrimidine-4-carboxamide (Example 7)

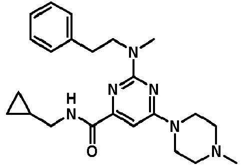

The title compound was prepared according to General Procedure E using 2-chloropyrimidine **I-15** (42 mg, 0.14 mmol, 1 eq), DiPEA (98 µL, 0.56 mmol, 4 eq) and N-methylphenethylamine HBr salt (44 mg, 0.20 mmol, 1.5 eq). Total heating time: 45 h at 120 °C. Purification by preparative HPLC (C18 reverse phase, 25% to 35% ACN/H₂O + 0.2% TFA, RT 8.98 min) afforded the product (21 mg, 51 µmol, 37%). TLC: R_{f} = 0.3 (5% MeOH/DCM). ¹H NMR (400 MHz, CDCl₃) δ 8.03 (t, *J* = 5.4 Hz, 1H), 7.33 - 7.26 (m, 2H), 7.25 - 7.18 (m, 3H), 6.74 (s, 1H), 3.87 - 3.73 (m, 6H), 3.34 - 3.25 (m, 2H), 3.13 (s, 3H), 2.94 - 2.86 (m, 2H), 2.64 - 2.51 (m, 4H), 2.42 (s, 3H), 1.12 - 1.00 (m, 1H), 0.60 - 0.50 (m, 2H), 0.29 (q, *J* = 4.9 Hz, 2H). ¹³C NMR (101 MHz, CDCl₃) δ 164.70, 163.60, 160.91, 156.82, 139.95, 128.85, 128.60, 126.30, 90.22, 54.58, 51.72, 45.88, 44.12, 43.58, 35.71, 33.94, 10.89, 3.49. HRMS [C₂₃H₃₂N₆O + H]⁺ : 409.2710 calculated, 409.2708 found (Δ = -0.58 ppm).

### Benzyl-4-(2-chloro-6-((cyclopropylmethyl)carbamoyl) pyrimidin-4-yl)piperazine-1-carboxylate (I-16)

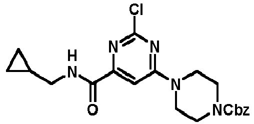

The title compound was prepared according to General Procedure B using dichloropyrimidine **I-4** (181 mg, 0.74 mmol, 1 eq), DiPEA (193 µL, 1.11 mmol, 1.5 eq) and 1-Cbz-piperazine (149 µL, 0.77 mmol, 1.05 eq). Column chromatography (30% -> 60% EtOAc/pentane) afforded the product (290 mg, 0.67 mmol, 91%). TLC: R_{f} = 0.5 (50% EtOAc/pentane). ¹H NMR (400 MHz, CDCl₃) δ 7.92 (t, *J* = 5.7 Hz, 1H), 7.40 - 7.30 (m, 5H), 7.28 (s, 1H), 5.17 (s, 2H), 3.74 (br s, 4H), 3.65 - 3.55 (m, 4H), 3.34 - 3.22 (m, 2H), 1.12 - 0.98 (m, 1H), 0.63 - 0.46 (m, 2H), 0.36 - 0.19 (m, 2H). ¹³C NMR (101 MHz, CDCl₃) δ 163.64, 162.10, 159.81, 158.01, 155.01, 136.26, 128.54, 128.20, 128.00, 99.44, 67.50, 44.40, 43.17, 10.63, 3.61. HRMS [C₁₄H₁₉ClN₄O₂ + H]⁺ : 295.1320 calculated, 295.1321 found (Δ = 0.20 ppm).

### Benzyl-4-(6-((cyclopropylmethyl)carbamoyl)-2-(methyl(phenethyl)amino) pyrimidin-4-yl)piperazine-1-carboxylate (Example 10)

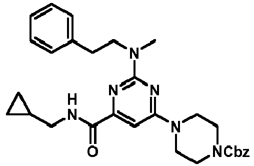

The title compound was prepared according to General Procedure E using 2-chloropyrimidine **I-16** (202 mg, 0.47 mmol, 1 eq), DiPEA (0.40 mL, 2.28 mmol, 5 eq) and N-methylphenethylamine HBr salt (161 mg, 0.74 mmol, 1.5 eq). Total heating time: 4 h at 160 °C with µW irradiation. Column chromatography (30% -> 60% EtOAc/pentane) afforded the product (199 mg, 0.38 mmol, 80%). TLC: R_{f} = 0.6 (50% EtOAc/pentane). ¹H NMR (400 MHz, CDCl₃) δ 8.02 (t, *J* = 5.4 Hz, 1H), 7.42 - 7.25 (m, 7H), 7.24 - 7.17 (m, 3H), 6.73 (s, 1H), 5.17 (s, 2H), 3.86 - 3.77 (m, 2H), 3.69 (br s, 4H), 3.62 - 3.53 (m, 4H), 3.34 - 3.26 (m, 2H), 3.12 (s, 3H), 2.94 - 2.85 (m, 2H), 1.12 - 1.00 (m, 1H), 0.61 - 0.50 (m, 2H), 0.28 (q, *J* = 4.7 Hz, 2H). ¹³C NMR (101 MHz, CDCl₃) δ 164.58, 163.64, 160.85, 156.90, 155.32, 139.84, 136.56, 128.79, 128.62, 128.56, 128.23, 128.08, 126.27, 90.16, 67.46, 51.66, 44.07, 43.86, 43.53, 35.67, 33.91, 10.85, 3.44. HRMS [C₃₀H₃₆N₆O₃ + H]⁺ : 529.2922 calculated, 529.2933 found (Δ = 2.13 ppm).

### N-(Cyclopropylmethyl)-2-(methyl(phenethyl)amino)-6-(piperazin-1-yl)pyrimidine-4-carboxamide (Example 11)

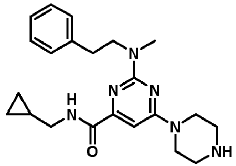

A round bottom flask was charged with Cbz-protected amine **Example 10** (175 mg, 0.33 mmol, 1 eq), dry MeOH (3 mL) and AcOH (0.3 mL). The flask was purged with N₂, followed by addition of Pd/C (10% w/w, 18 mg, 0.02 mmol, 5 mol%) and then purging with H₂ (balloon). The reaction was stirred for 2 days, then filtered over a cellulose filter (Whatman) which was washed with MeOH and concentrated under reduced pressure. The residue was purified using silica gel column chromatography (2.5% -> 5% MeOH/DCM with 5% Et₃N) affording the product (82 mg, 0.21 mmol, 63%). ¹H NMR (400 MHz, CDCl₃) δ 8.05 (t, *J* = 5.5 Hz, 1H), 7.35 - 7.26 (m, 2H), 7.26 - 7.17 (m, 3H), 6.74 (s, 1H), 3.84 - 3.76 (m, 2H), 3.75 - 3.58 (m, 4H), 3.30 (t, *J* = 6.5 Hz, 2H), 3.13 (s, 3H), 2.96 - 2.85 (m, 6H), 1.96 (s, 1H), 1.13 - 1.00 (m, 1H), 0.61 - 0.47 (m, 2H), 0.28 (q, *J* = 4.7 Hz, 2H). ¹³C NMR (101 MHz, CDCl₃) δ 164.78, 163.74, 160.85, 156.51, 139.95, 128.80, 128.52, 126.20, 90.16, 51.66, 45.94, 45.26, 44.02, 35.62, 33.88, 10.84, 3.42. HRMS [C₂₂H₃₀N₆O + H]⁺ : 395.2554 calculated, 395.2558 found (Δ = 1.04 ppm).

### 6-(4-Acetylpiperazin-1-yl)-N-(cyclopropylmethyl)-2-(methyl(phenethyl)amino) pyrimidine -4-carboxamide (Example 12)

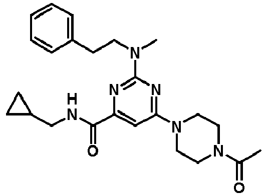

A round bottom flask was charged with amine Example 11 (22 mg, 56 µmol, 1 eq) in dry DCM (1.5 mL). This was followed by addition of DiPEA (49 µL, 0.28 mmol, 5 eq) and Ac₂O (10.5 µL, 0.11 mmol, 2 eq). The reaction was stirred for 3h at rt and then diluted with EtOAc (25 mL). The organics were washed with sat. aq. NaHCO₃ (1 x 25 mL) and brine (1 x 25 mL), dried (MgSO₄), filtered and concentrated under reduced pressure. The residue was purified by silica gel column chromatography (2.5% -> 10% MeOH/DCM) affording the product (19 mg, 44 µmol, 78%). TLC: R_{f} = 0.8 (10% MeOH/DCM). ¹H NMR (400 MHz, CDCl₃) δ 8.02 (t, *J* = 5.3 Hz, 1H), 7.34 - 7.26 (m, 2H), 7.26 - 7.17 (m, 3H), 6.74 (s, 1H), 3.87 - 3.77 (m, 2H), 3.77 - 3.65 (m, 6H), 3.59 - 3.50 (m, 2H), 3.34 - 3.26 (m, 2H), 3.13 (s, 3H), 2.95 - 2.86 (m, 2H), 2.15 (s, 3H), 1.14 - 0.99 (m, 1H), 0.61 - 0.50 (m, 2H), 0.29 (q, *J* = 4.7 Hz, 2H). ¹³C NMR (101 MHz, CDCl₃) δ 169.32, 164.57, 163.63, 160.85, 156.97, 139.84, 128.82, 128.61, 126.33, 90.13, 51.70, 45.94, 44.12, 43.82, 41.12, 35.73, 33.92, 21.57, 10.87, 3.48. HRMS [C₂₄H₃₂N₆O₂ + H]⁺ : 437.2660 calculated, 437.2661 found (Δ = 0.34 ppm).

### 6-(4-Benzoylpiperazin-1-yl)-N-(cyclopropylmethyl)-2-(methyl(phenethyl)amino) pyrimidine-4-carboxamide (Example 16)

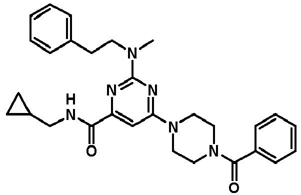

A round bottom flask was charged with amine **Example 11** (22 mg, 56 µmol, 1 eq) in dry DCM (1.5 mL). This was followed by addition of Et₃N (16 µL, 0.11 mmol, 2 eq) and benzoyl chloride (8 µL, 67 µmol, 1.2 eq). The reaction was stirred for 3h at rt and then diluted with EtOAc (25 mL). The organics were washed with sat. aq. NaHCO₃ (1 x 25 mL) and brine (1 x 25 mL), dried (Na₂SO₄), filtered and concentrated under reduced pressure. The residue was purified by silica gel column chromatography (60 -> 80% EtOAc/pentane) affording the product (20 mg, 40 µmol, 72%). TLC: R_{f} = 0.3 (60% EtOAc/pentane). ¹H NMR (400 MHz, CDCl₃) δ 8.03 (br s, 1H), 7.49 - 7.40 (m, 5H), 7.32 - 7.25 (m, 2H), 7.25 - 7.17 (m, 3H), 6.75 (s, 1H), 3.95 - 3.74 (m, 6H), 3.66 (br s, 2H), 3.52 (br s, 2H), 3.36 - 3.25 (m, 2H), 3.12 (s, 3H), 2.94 - 2.82 (m, 2H), 1.14 - 0.99 (m, 1H), 0.63 - 0.48 (m, 2H), 0.29 (q, *J* = 4.7 Hz, 2H). ¹³C NMR (101 MHz, CDCl₃) δ 170.77, 164.61, 163.71, 160.87, 156.99, 139.85, 135.48, 130.14, 128.83, 128.76, 128.62, 128.49, 127.23, 126.34, 90.21, 51.70, 47.55, 44.16, 42.05, 35.75, 33.94, 10.88, 3.50. HRMS [C₂₉H₃₄N₆O₂ + H]⁺ : 499.2816 calculated, 499.2825 found (Δ = 1.80 ppm).

### 2-Chloro-N-(cyclopropylmethyl)-6-(pyrrolidin-1-yl)pyrimidine-4-carboxamide (I-18)

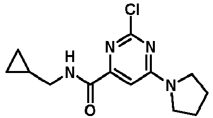

The title compound was prepared according to General Procedure B using dichloropyrimidine **I-4** (123 mg, 0.50 mmol, 1 eq), DiPEA (131 µL, 0.75 mmol, 1.5 eq) and pyrrolidine (43 µL, 0.53 mmol, 1.05 eq). Column chromatography (30% -> 60% EtOAc/pentane) afforded the product (150 mg, 0.40 mmol, 79%). TLC: R_{f} = 0.5 (40% EtOAc/pentane). ¹H NMR (400 MHz, CDCl₃) δ 7.93 (br s, 1H), 7.05 (s, 1H), 3.65 (t, *J =* 6.6 Hz, 2H), 3.46 (t, *J* = 6.7 Hz, 2H), 3.29 (t, *J* = 6.5 Hz, 2H), 2.09 (p, *J* = 6.3 Hz, 2H), 2.00 (p, *J* = 6.3 Hz, 2H), 1.13 - 0.99 (m, 1H), 0.56 (q, *J* = 5.2 Hz, 2H), 0.29 (q, *J* = 4.9 Hz, 2H). ¹³C NMR (101 MHz, CDCl₃) δ 162.52, 161.97, 159.61, 156.52, 100.53, 47.23, 46.98, 44.43, 25.52, 24.82, 10.69, 3.69.

### N-(Cyclopropylmethyl)-2-(methyl(phenethyl)amino)-6-(pyrrolidin-1-yl)pyrimidine-4-carboxamide (Example 17)

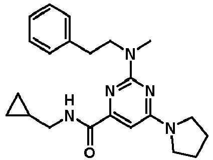

The title compound was prepared according to General Procedure E using 2-chloropyrimidine **I-18** (28 mg, 0.10 mmol, 1 eq), DiPEA (52 µL, 0.30 mmol, 3 eq) and N-methylphenethylamine (22 µL, 0.15 mmol, 1.5 eq). Total heating time: 4 h at 160 °C with µW irradiation. Column chromatography (30% -> 50% EtOAc/pentane) afforded the product (26 mg, 69 µmol, 69%). TLC: R_{f} = 0.7 (40% EtOAc/pentane). ¹H NMR (500 MHz, CDCl₃) δ 8.09 (t, *J* = 5.3 Hz, 1H), 7.33 - 7.27 (m, 2H), 7.27 - 7.18 (m, 3H), 6.54 (s, 1H), 3.89 - 3.71 (m, 2H), 3.62 (br s, 2H), 3.53 - 3.34 (m, 2H), 3.30 (dd, *J* = 6.9, 5.9 Hz, 2H), 3.14 (s, 3H), 2.97 - 2.85 (m, 2H), 2.15 - 1.85 (m, 4H), 1.12 - 1.01 (m, 1H), 0.63 - 0.47 (m, 2H), 0.35 - 0.22 (m, 2H). ¹³C NMR (126 MHz, CDCl₃) δ 165.03, 161.92, 160.90, 155.49, 140.19, 128.89, 128.54, 126.18, 91.37, 51.73, 46.43, 44.05, 35.55, 33.95, 25.78, 25.01, 10.91, 3.47. HRMS [C₂₂H₂₉N₅O + H]⁺ : 380.2445 calculated, 380.2452 found (Δ = 1.87 ppm).

### 2-Chloro-N-(cyclopropylmethyl)-6-(dimethylamino)pyrimidine-4-carboxamide (I-19)

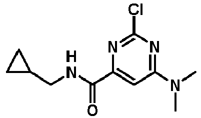

The title compound was prepared according to General Procedure B using dichloropyrimidine **I-4** (246 mg, 1.0 mmol, 1 eq), DiPEA (261 µL, 1.5 mmol, 1.5 eq) and dimethylamine (2 M in THF, 0.53 mL, 1.05 mmol, 1.05 eq). Column chromatography (40% -> 60% EtOAc/pentane) afforded the product (225 mg, 0.88 mmol, 88%). TLC: R_{f} = 0.5 (50% EtOAc/pentane). ¹H NMR (400 MHz, CDCl₃) δ 7.97 (br s, 1H), 7.19 (s, 1H), 3.33 - 3.27 (m, 2H), 3.27 - 3.06 (m, 6H), 1.13 - 0.99 (m, 1H), 0.62 - 0.49 (m, 2H), 0.29 (q, *J* = 4.8 Hz, 2H). ¹³C NMR (101 MHz, CDCl₃) δ 164.03, 162.29, 159.36, 156.93, 99.19, 44.26, 37.76, 37.29, 10.56, 3.53.

### N-(Cyclopropylmethyl)-6-(dimethylamino)-2-(methyl(phenethyl)amino) pyrimidine-4-carboxamide (Example 76)

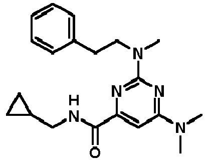

The title compound was prepared according to the general procedure E using 2-chloropyrimidine **I-19** (27 mg, 0.11 mmol, 1 eq), *N*-methylphenethylamine hydrobromide (35 mg, 0.16 mmol, 1.6 eq) and DiPEA (92 µL, 0.53 mmol, 5 eq). Total heating time: 8 h at 160 °C with µW irradiation. Column chromatography (20% -> 50% EtOAc/pentane) afforded the product (32 mg, 90 µmol, 90%). TLC: R_{f} = 0.6 (40% EtOAc/pentane). ¹H NMR (400 MHz, CDCl₃) δ 8.08 (t, *J* = 5.8 Hz, 1H), 7.34 - 7.27 (m, 2H), 7.26 - 7.17 (m, 3H), 6.69 (s, 1H), 3.92 - 3.70 (m, 2H), 3.30 (dd, *J* = 7.1, 5.8 Hz, 2H), 3.23 - 3.01 (m, 9H), 3.00 - 2.80 (m, 2H), 1.17 - 0.98 (m, 1H), 0.64 - 0.46 (m, 2H), 0.36 - 0.22 (m, 2H). ¹³C NMR (101 MHz, CDCl₃) δ 164.89, 164.01, 160.68, 155.88, 140.01, 128.79, 128.47, 126.12, 90.10, 51.66, 43.98, 37.16, 35.52, 33.84, 10.82, 3.39. HRMS [C₂₀H₂₇N₅O + H]⁺ : 354.2288 calculated, 354.2290 (found (Δ = 0.34 ppm).

### (S)-N-(Cyclopropylmethyl)-6-(dimethylamino)-2-(3-phenylpiperidin-1-yl)pyrimidine-4-carboxamide (Example 18)

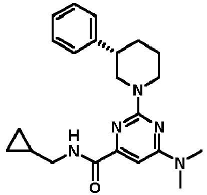

The title compound was prepared according to General Procedure E using 2-chloropyrimidine **I-19** (25 mg, 0.10 mmol, 1 eq), DiPEA (52 µL, 0.30 mmol, 3 eq) and (S)-3-phenylpiperidine (21 mg, 0.13 mmol, 1.3 eq). Total heating time: 4 h at 160 °C with µW irradiation. Column chromatography (30% -> 50% EtOAc/pentane) afforded the product (28 mg, 74 µmol, 74%). *ee*: >99% (as determined by chiral HPLC using 70:30 hexane/isopropanol, Chiralcell OD). TLC: R_{f} = 0.7 (50% EtOAc/pentane). ¹H NMR (400 MHz, CDCl₃) δ 8.02 (br s, 1H), 7.43 - 7.16 (m, 5H), 6.70 (s, 1H), 4.84 (t, *J* = 14.1 Hz, 2H), 3.39 - 3.19 (m, 2H), 3.10 (s, 6H), 2.89 (t, *J* = 12.1 Hz, 2H), 2.77 (t, *J* = 10.2 Hz, 1H), 2.07 (d, *J* = 13.1 Hz, 1H), 1.88 - 1.60 (m, 3H), 1.14 - 0.98 (m, 1H), 0.63 - 0.44 (m, 2H), 0.36 - 0.20 (m, 2H). ¹³C NMR (101 MHz, CDCl₃) δ 164.91, 164.18, 160.89, 156.13, 144.43, 128.61, 127.31, 126.58, 90.71, 51.38, 44.70, 44.02, 42.56, 37.30, 32.21, 25.58, 10.96, 3.51, 3.49. HRMS [C₂₂H₂₉N₅O + H]⁺ : 380.2445 calculated, 380.2452 found (Δ = 1.87 ppm).

### (R)-N-(Cyclopropylmethyl)-6-(dimethylamino)-2-(3-phenylpiperidin-1-yl) pyrimidine-4-carboxamide (Example 19)

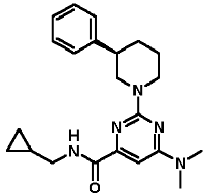

The title compound was prepared according to General Procedure E using 2-chloropyrimidine **I-19** (25 mg, 0.10 mmol, 1 eq), DiPEA (52 µL, 0.30 mmol, 3 eq) and (R)-3-phenylpiperidine (21 mg, 0.13 mmol, 1.3 eq). Total heating time: 4 h at 160 °C with µW irradiation. Column chromatography (30% -> 50% EtOAc/pentane) afforded the product (32 mg, 84 µmol, 84%). *ee*: >97% (as determined by chiral HPLC using 70:30 hexane/isopropanol, Chiralcell OD). TLC: R_{f} = 0.7 (50% EtOAc/pentane). ¹H NMR (400 MHz, CDCl₃) δ 8.01 (t, *J* = 5.4 Hz, 1H), 7.39 - 7.28 (m, 4H), 7.28 - 7.21 (m, 1H), 6.70 (s, 1H), 4.95 - 4.74 (m, 2H), 3.40 - 3.18 (m, 2H), 3.10 (s, 6H), 2.96 - 2.83 (m, 2H), 2.83 - 2.70 (m, 1H), 2.07 (d, *J* = 13.9 Hz, 1H), 1.90 - 1.81 (m, 1H), 1.81 - 1.62 (m, 2H), 1.11 - 0.99 (m, 1H), 0.59 - 0.46 (m, 2H), 0.27 (q, *J* = 4.7 Hz, 2H). ¹³C NMR (101 MHz, CDCl₃) δ 164.91, 164.18, 160.90, 156.12, 144.43, 128.61, 127.31, 126.58, 90.71, 51.38, 44.70, 44.02, 42.56, 37.30, 32.21, 25.58, 10.96, 3.51, 3.49. HRMS [C₂₂H₂₉N₅O + H]⁺ : 380.2445 calculated, 380.2452 found (Δ = 1.87 ppm).

### 2-Chloro-N-(cyclopropylmethyl)-6-(diethylamino)pyrimidine-4-carboxamide (I-20)

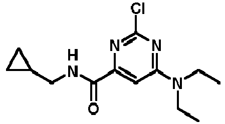

The title compound was prepared according to General Procedure B using dichloropyrimidine **I-4** (123 mg, 0.50 mmol, 1 eq), DiPEA (131 µL, 0.75 mmol, 1.5 eq) and diethylamine (55 µL, 0.53 mmol, 1.05 eq). Column chromatography (20% -> 50% EtOAc/pentane) afforded the product (94 mg, 0.33 mmol, 66%). TLC: R_{f} = 0.7 (50% EtOAc/pentane). ¹H NMR (400 MHz, CDCl₃) δ 7.94 (br s, 1H), 7.16 (s, 1H), 3.77 - 3.37 (m, 4H), 3.34 - 3.24 (m, 2H), 1.22 (t, *J* = 7.0 Hz, 6H), 1.12 - 1.00 (m, 1H), 0.61 - 0.51 (m, 2H), 0.29 (q, *J* = 4.8 Hz, 2H). ¹³C NMR (101 MHz, CDCl₃) δ 162.96, 162.57, 159.79, 157.11, 99.29, 44.41, 42.96, 42.73, 12.64, 10.69, 3.67.

### N-(Cyclopropylmethyl)-6-(diethylamino)-2-(methyl(phenethyl)amino)pyrimidine-4-carboxamide (Example 20)

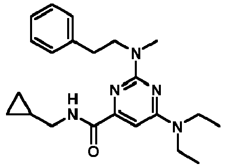

The title compound was prepared according to General Procedure E using 2-chloropyrimidine **I-20** (37 mg, 0.13 mmol, 1 eq), DiPEA (68 µL, 0.39 mmol, 3 eq) and N-methylphenethylamine (28 µL, 0.20 mmol, 1.5 eq). Total heating time: 4 h at 160 °C with µW irradiation. Column chromatography (20% -> 40% EtOAc/pentane) afforded the product (30 mg, 79 µmol, 60%). TLC: R_{f} = 0.8 (50% EtOAc/pentane). ¹H NMR (400 MHz, CDCl₃) δ 8.10 (br s, 1H), 7.40 - 7.14 (m, 5H), 6.65 (s, 1H), 3.92 - 3.71 (m, 2H), 3.54 (br s, 4H), 3.30 (t, *J* = 6.3 Hz, 2H), 3.14 (s, 3H), 3.02 - 2.85 (m, 2H), 1.20 (t, *J* = 6.9 Hz, 6H), 1.13 - 0.99 (m, 1H), 0.64 - 0.46 (m, 2H), 0.37 - 0.21 (m, 2H). ¹³C NMR (101 MHz, CDCl₃) δ 165.05, 162.59, 160.96, 155.94, 140.12, 128.87, 128.55, 126.21, 90.19, 51.79, 44.03, 42.44, 35.60, 34.04, 13.26, 10.92, 3.46. HRMS [C₂₂H₃₁N₅O + H]⁺ : 382.2601 calculated, 382.2599 found (Δ = 0.63 ppm).

### 2-Chloro-N-(cyclopropylmethyl)-6-(methylamino)pyrimidine-4-carboxamide (I-21)

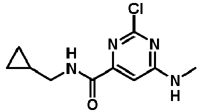

The title compound was prepared according to General Procedure B using dichloropyrimidine **I-4** (123 mg, 0.50 mmol, 1 eq), DiPEA (218 µL, 1.25 mmol, 2.5 eq) and methylamine HCl salt (35 mg, 0.53 mmol, 1.05 eq). Column chromatography (50% -> 70% EtOAc/pentane) afforded the product (77 mg, 0.32 mmol, 64%). TLC: R_{f} = 0.4 (60% EtOAc/pentane). ¹H NMR (500 MHz, CDCl₃) δ 7.93 (br s, 1H), 7.40 - 7.04 (m, 1H), 6.62 - 6.20 (m, 1H), 3.29 (br s, 2H), 3.04 (br s, 3H), 1.13 - 1.00 (m, 1H), 0.65 - 0.50 (m, 2H), 0.29 (q, *J* = 4.9 Hz, 2H). ¹³C NMR (126 MHz, CDCl₃) δ 175.67, 166.23, 165.44, 162.63, 162.22, 160.34, 158.43, 155.80, 103.55, 98.02, 44.55, 29.81, 29.06, 28.22, 3.76.

### N-(Cyclopropylmethyl)-2-(methyl(phenethyl)amino)-6-(methylamino)pyrimidine-4-carboxamide (Example 21)

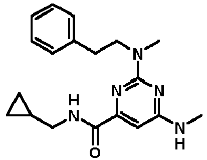

The title compound was prepared according to General Procedure E using 2-chloropyrimidine **I-21** (25 mg, 0.10 mmol, 1 eq), DiPEA (52 µL, 0.30 mmol, 3 eq) and N-methylphenethylamine (22 µL, 0.15 mmol, 1.5 eq). Total heating time: 4 h at 160 °C with µW irradiation. Column chromatography (50% -> 70% EtOAc/pentane) afforded the product (26 mg, 77 µmol, 77%). TLC: R_{f} = 0.5 (50% EtOAc/pentane). ¹H NMR (400 MHz, CDCl₃) δ 8.03 (br s, 1H), 7.33 - 7.27 (m, 2H), 7.26 - 7.18 (m, 3H), 6.54 (s, 1H), 5.13 - 4.72 (m, 1H), 3.93 - 3.66 (m, 2H), 3.34 - 3.24 (m, 2H), 3.13 (s, 3H), 2.98 (d, *J* = 4.9 Hz, 3H), 2.94 - 2.87 (m, 2H), 1.12 - 1.00 (m, 1H), 0.61 - 0.50 (m, 2H), 0.28 (q, *J* = 4.7 Hz, 2H). ¹³C NMR (101 MHz, CDCl₃) δ 164.71, 161.06, 156.00, 140.05, 128.89, 128.55, 126.24, 92.54, 51.66, 44.06, 35.62, 33.93, 28.23, 10.89, 3.48. HRMS [C₁₉H₂₅N₅O + H]⁺ : 340.2132 calculated, 340.2138 found (Δ = 1.79 ppm).

### 2-Chloro-N-(cyclopropylmethyl)-6-(azetidin-1-yl)pyrimidine-4-carboxamide (I-22)

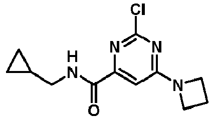

The title compound was prepared according to General Procedure B using dichloropyrimidine **I-4** (98 mg, 0.40 mmol, 1 eq), DiPEA (174 µL, 1.0 mmol, 2.5 eq) and azetidine HCl salt (39 mg, 0.42 mmol, 1.05 eq). Column chromatography (30% -> 50% EtOAc/pentane) afforded the product (79 mg, 0.30 mmol, 74%). TLC: R_{f} = 0.3 (50% EtOAc/pentane). ¹H NMR (400 MHz, CDCl₃) δ 7.90 (br s, 1H), 6.88 (s, 1H), 4.44 - 4.07 (m, 4H), 3.28 (dd, *J* = 7.0, 6.0 Hz, 2H), 2.49 (p, *J* = 7.6 Hz, 2H), 1.14 - 0.99 (m, 1H), 0.62 - 0.51 (m, 2H), 0.28 (q, *J* = 4.7 Hz, 2H). ¹³C NMR (101 MHz, CDCl₃) δ 163.91, 162.30, 159.95, 156.54, 98.54, 50.20, 44.44, 16.51, 10.69, 3.69.

### N-(Cyclopropylmethyl)-2-(methyl(phenethyl)amino)-6-(azetidin-1-yl)pyrimidine-4-carboxamide (Example 25)

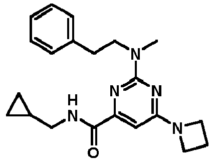

The title compound was prepared according to General Procedure E using 2-chloropyrimidine **I-22** (27 mg, 0.10 mmol, 1 eq), DiPEA (52 µL, 0.30 mmol, 3 eq) and N-methylphenethylamine (22 µL, 0.15 mmol, 1.5 eq). Total heating time: 4 h at 160 °C with µW irradiation. Column chromatography (40% -> 60% EtOAc/pentane) afforded the product (25 mg, 68 µmol, 68%). TLC: R_{f} = 0.6 (50% EtOAc/pentane). ¹H NMR (400 MHz, CDCl₃) δ 8.04 (br s, 1H), 7.33 - 7.27 (m, 2H), 7.26 - 7.16 (m, 3H), 6.38 (s, 1H), 4.11 (t, *J* = 7.5 Hz, 4H), 3.85 - 3.71 (m, 2H), 3.35 - 3.26 (m, 2H), 3.13 (s, 3H), 2.94 - 2.84 (m, 2H), 2.40 (p, *J* = 7.5 Hz, 2H), 1.14 - 1.00 (m, 1H), 0.60 - 0.48 (m, 2H), 0.28 (q, *J* = 4.7 Hz, 2H). ¹³C NMR (101 MHz, CDCl₃) δ 164.78, 164.75, 160.96, 155.60, 140.10, 128.90, 128.55, 126.22, 89.42, 51.73, 49.83, 44.07, 35.54, 33.88, 16.75, 10.90, 3.49. HRMS [C₂₁H₂₇N₅O + H]⁺ : 366.2288 calculated, 366.2296 found (Δ = 2.26 ppm).

### 2-Chloro-N-(cyclopropylmethyl)-6-(2-oxa-6-azaspiro[3.3]heptan-6-yl)pyrimidine-4-carboxamide (I-23)

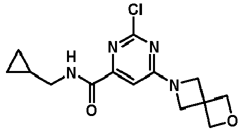

The title compound was prepared according to General Procedure B using dichloropyrimidine **I-4** (98 mg, 0.40 mmol, 1 eq), DiPEA (174 µL, 1.0 mmol, 2.5 eq) and 2-oxa-6-azaspiro[3.3]heptane hemioxalate salt (61 mg, 0.42 mmol, 1.05 eq). Column chromatography (40% -> 100% EtOAc/pentane) afforded the product (96 mg, 0.31 mmol, 78%). TLC: R_{f} = 0.2 (80% EtOAc/pentane). ¹H NMR (400 MHz, CDCl₃) δ 7.88 (br s, 1H), 6.92 (s, 1H), 4.86 (br s, 4H), 4.35 (s, 4H), 3.28 (dd, *J* = 7.0, 6.0 Hz, 2H), 1.11 - 0.98 (m, 1H), 0.63 - 0.50 (m, 2H), 0.34 - 0.23 (m, 2H). ¹³C NMR (101 MHz, CDCl₃) δ 164.01, 162.11, 160.06, 157.10, 98.96, 80.71, 59.73, 44.56, 39.16, 10.72, 3.76.

### N-(Cyclopropylmethyl)-2-(methyl(phenethyl)amino)-6-(2-oxa-6-azaspiro[3.3]heptan-6-yl)pyrimidine-4-carboxamide (Example 26)

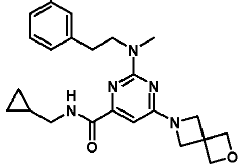

The title compound was prepared according to General Procedure E using 2-chloropyrimidine **I-23** (31 mg, 0.10 mmol, 1 eq), DiPEA (52 µL, 0.30 mmol, 3 eq) and N-methylphenethylamine (22 µL, 0.15 mmol, 1.5 eq). Total heating time: 4 h at 160 °C with µW irradiation. Column chromatography (70% -> 90% EtOAc/pentane) afforded the product (7 mg, 17 µmol, 17%). TLC: R_{f} = 0.4 (80% EtOAc/pentane). ¹H NMR (400 MHz, CDCl₃) δ 8.00 (br s, 1H), 7.35 - 7.27 (m, 2H), 7.26 - 7.19 (m, 3H), 6.39 (s, 1H), 4.85 (s, 4H), 4.24 (s, 4H), 3.84 - 3.71 (m, 2H), 3.36 - 3.22 (m, 2H), 3.12 (s, 3H), 2.94 - 2.82 (m, 2H), 1.13 - 0.98 (m, 1H), 0.60 - 0.48 (m, 2H), 0.28 (q, *J* = 4.7 Hz, 2H). ¹³C NMR (101 MHz, CDCl₃) δ 164.68, 164.52, 160.91, 156.07, 139.97, 128.89, 128.62, 126.32, 89.61, 81.23, 59.40, 51.71, 44.12, 39.24, 35.61, 33.89, 10.90, 3.51. HRMS [C₂₃H₂₉N₅O₂ + H]⁺ : 408.2394 calculated, 408.2396 found (Δ = 0.49 ppm).

### N-(Cyclopropylmethyl)-2,6-bis(methyl(phenethyl)amino)pyrimidine-4-carboxamide (Example 27)

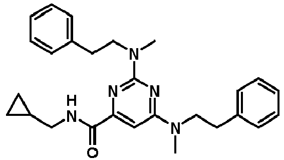

The title compound was prepared according to General Procedure E using dichloropyrimidine **I-4** (28 mg, 0.11 mmol, 1 eq), DiPEA (79 µL, 0.46 mmol, 4 eq) and *N*-methylphenethylamine (41 µL, 0.28 mmol, 2.5 eq). Total heating time: 4 h at 160 °C with µW irradiation. Column chromatography (20% -> 40% EtOAc/pentane) afforded the product (30 mg, 68 µmol, 61%). TLC: R_{f} = 0.6 (30% EtOAc/pentane). ¹H NMR (400 MHz, CDCl₃) δ 8.09 (t, *J* = 5.2 Hz, 1H), 7.32 - 7.24 (m, 4H), 7.24 - 7.12 (m, 6H), 6.68 (br s, 1H), 4.02 - 3.56 (m, 4H), 3.34 - 3.26 (m, 2H), 3.15 (s, 3H), 2.99 (br s, 3H), 2.96 - 2.87 (m, 4H), 1.15 - 1.01 (m, 1H), 0.60 - 0.48 (m, 2H), 0.29 (q, *J* = 4.8 Hz, 2H). ¹³C NMR (101 MHz, CDCl₃) δ 164.96, 163.42, 160.86, 156.14, 140.03, 128.97, 128.90, 128.68, 128.60, 126.43, 126.25, 90.24, 51.72, 44.09, 35.71, 34.01, 33.85, 10.93, 3.50. HRMS [C₂₇H₃₃N₅O + H]⁺ : 444.2758 calculated, 444.2765 found (Δ = 1.60 ppm).

### N-(Cyclopropylmethyl)-2-(methyl(phenethyl)amino)-6-(benzyl(methyl)amino) pyrimidine-4-carboxamide (Example 28)

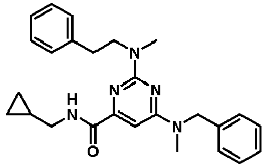

The title compound was prepared according to General Procedure H using dichloropyrimidine **I-4** (37 mg, 0.15 mmol, 1 eq), DiPEA (39 µL, 0.23 mmol, 1.5 eq) and N-methylbenzylamine (19 µL, 0.15 mmol, 1.0 eq) in MeOH (1.5 mL), followed by concentration and addition of DiPEA (78 µL, 0.45 mmol, 3 eq), N-methylphenethylamine (33 µL, 0.23 mmol, 1.5 eq) and n-BuOH (0.75 mL). Total heating time: 4 h at 160 °C with µW irradiation. Purification by HPLC (C18 reverse phase, 43% to 49% ACN/H₂O + 0.2% TFA) afforded the product (47 mg, 0.11 mmol, 73%). TLC: R_{f} = 0.6 (40% EtOAc/pentane). ¹H NMR (400 MHz, CDCl₃) δ 8.08 (br s, 1H), 7.36 - 7.28 (m, 2H), 7.28 - 7.03 (m, 8H), 6.75 (br s, 1H), 4.86 (br s, 2H), 3.77 (br s, 2H), 3.39 - 3.22 (m, 2H), 3.13 (s, 3H), 3.08 (br s, 2H), 2.86 (br s, 2H), 1.14 - 0.98 (m, 1H), 0.62 - 0.48 (m, 2H), 0.29 (q, *J* = 4.8 Hz, 2H). ¹³C NMR (101 MHz, CDCl₃) δ 164.88, 164.13, 160.83, 156.51, 139.96, 128.89, 128.74, 128.57, 127.24, 126.24, 90.07, 51.85, 44.14, 35.75, 33.95, 10.94, 3.52. HRMS [C₂₆H₃₁N₅O + H]⁺ : 430.2601 calculated, 430.2604 found (Δ = 0.60 ppm).

### N-(Cyclopropylmethyl)-2-(methyl(phenethyl)amino)-6-(3-phenylpyrrolidin-1-yl)pyrimidine-4-carboxamide (Example 29)

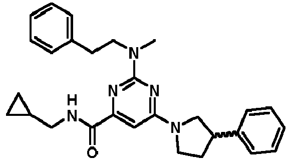

The title compound was prepared according to General Procedure H using dichloropyrimidine **I-4** (40 mg, 0.16 mmol, 1 eq), DiPEA (43 µL, 0.24 mmol, 1.5 eq) and (±)-3-phenylpyrrolidine (24 µL, 0.16 mmol, 1.0 eq) in MeOH (1.6 mL), followed by concentration and addition of DiPEA (84 µL, 0.48 mmol, 3 eq), N-methylphenethylamine (35 µL, 0.24 mmol, 1.5 eq) and n-BuOH (0.75 mL). Total heating time: 4 h at 160 °C with µW irradiation. Column chromatography (70% -> 90% EtOAc/pentane) afforded the product (35 mg, 91 µmol, 57%). TLC: R_{f} = 0.4 (30% EtOAc/pentane). ¹H NMR (400 MHz, CDCl₃) δ 8.08 (br s, 1H), 7.39 - 7.32 (m, 2H), 7.32 - 7.17 (m, 8H), 6.57 (s, 1H), 4.19 (br s, 1H), 3.94 (br s, 1H), 3.81 (br s, 2H), 3.74 - 3.38 (m, 3H), 3.30 (t, *J* = 6.4 Hz, 2H), 3.14 (s, 3H), 2.92 (br s, 2H), 2.42 (br s, 1H), 2.16 (br s, 1H), 1.14 - 1.00 (m, 1H), 0.62 - 0.46 (m, 2H), 0.29 (q, *J* = 4.9 Hz, 2H). ¹³C NMR (101 MHz, CDCl₃) δ 164.96, 161.90, 160.92, 155.72, 141.79, 140.15, 128.92, 128.80, 128.57, 127.19, 126.97, 126.23, 91.13, 52.89, 51.76, 46.36, 44.10, 43.39, 35.62, 33.98, 33.31, 10.93, 3.51. HRMS [C₂₈H₃₃N₅O + H]⁺ : 456.2758 calculated, 456.2757 found (Δ = -0.19 ppm).

### N-(Cyclopropylmethyl)-6-((2-hydroxyethyl)(methyl)amino)-2-(methyl(phenethyl) amino)pyrimidine-4-carboxamide (Example 30)

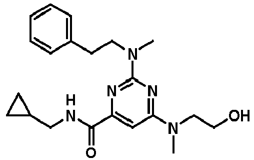

The title compound was prepared according to General Procedure H using dichloropyrimidine **I-4** (39 mg, 0.16 mmol, 1 eq), DiPEA (43 µL, 0.24 mmol, 1.5 eq) and *N*-methylethanolamine (13 µL, 0.16 mmol, 1.0 eq) in MeOH (1.6 mL), followed by concentration and addition of DiPEA (84 µL, 0.48 mmol, 3 eq), N-methylphenethylamine (35 µL, 0.24 mmol, 1.5 eq) and n-BuOH (0.75 mL). Total heating time: 4 h at 160 °C with µW irradiation. Column chromatography (20% -> 40% EtOAc/pentane) afforded the product (15 mg, 33 µmol, 21%). TLC: R_{f} = 0.4 (80% EtOAc/pentane). ¹H NMR (400 MHz, CDCl₃) δ 8.02 (br s, 1H), 7.33 - 7.27 (m, 2H), 7.26 - 7.18 (m, 3H), 6.68 (s, 1H), 4.12 - 3.52 (m, 7H), 3.33 - 3.26 (m, 2H), 3.14 (s, 3H), 3.11 (s, 3H), 2.93 - 2.86 (m, 2H), 1.12 - 1.00 (m, 1H), 0.60 - 0.51 (m, 2H), 0.29 (q, *J* = 4.7 Hz, 2H). ¹³C NMR (101 MHz, CDCl₃) δ 164.63, 160.52, 156.34, 139.80, 128.86, 128.60, 126.32, 90.28, 62.14, 52.62, 51.83, 44.12, 37.11, 35.91, 33.97, 10.86, 3.48. HRMS [C₂₁H₂₉N₅O₂ + H]⁺ : 384.2394 calculated, 384.2399 found (Δ = 1.30 ppm).

### 2-Chloro-N-(cyclopropylmethyl)-6-(3,3-difluoropyrrolidin-1-yl)pyrimidine-4-carboxamide (I-24)

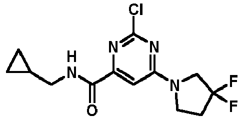

The title compound was prepared according to General Procedure B using dichloropyrimidine **I-4** (98 mg, 0.40 mmol, 1 eq), DiPEA (174 µL, 1.0 mmol, 2.5 eq) and 3,3-difluoropyrrolidine HCl salt (60 mg, 0.42 mmol, 1.05 eq). Column chromatography (20% -> 40% EtOAc/pentane) afforded the product (108 mg, 0.34 mmol, 85%). TLC: R_{f} = 0.6 (40% EtOAc/pentane). ¹H NMR (500 MHz, CDCl₃) δ 7.90 (br s, 1H), 7.22 - 6.87 (m, 1H), 4.19 - 3.59 (m, 4H), 3.36 - 3.22 (m, 2H), 2.55 (br s, 2H), 1.14 - 0.97 (m, 1H), 0.63 - 0.50 (m, 2H), 0.37 - 0.18 (m, 2H). ¹³C NMR (126 MHz, CDCl₃) δ 162.52, 162.11, 159.90, 157.70, 100.24, 99.83, 53.66 (t, *J* = 32.9 Hz), 44.62, 33.91, 3.78.

### N-(Cyclopropylmethyl)-6-(3,3-difluoropyrrolidin-1-yl)-2-(methyl(phenethyl)amino) pyrimidine-4-carboxamide (Example 31)

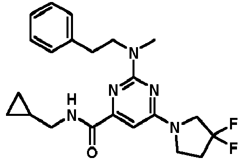

The title compound was prepared according to General Procedure E using 2-chloropyrimidine **I-24** (32 mg, 0.10 mmol, 1 eq), DiPEA (52 µL, 0.30 mmol, 3 eq) and N-methylphenethylamine (22 µL, 0.15 mmol, 1.5 eq). Total heating time: 4 h at 160 °C with µW irradiation. Column chromatography (20% -> 40% EtOAc/pentane) afforded the product (34 mg, 82 µmol, 82%). TLC: R_{f} = 0.8 (40% EtOAc/pentane). ¹H NMR (400 MHz, CDCl₃) δ 8.03 (br s, 1H), 7.34 - 7.27 (m, 2H), 7.25 - 7.18 (m, 3H), 6.52 (br s, 1H), 3.89 (br s, 2H), 3.84 - 3.78 (m, 2H), 3.74 (br s, 2H), 3.37 - 3.22 (m, 2H), 3.13 (s, 3H), 2.97 - 2.83 (m, 2H), 2.56 - 2.37 (m, 2H), 1.14 - 1.00 (m, 1H), 0.64 - 0.47 (m, 2H), 0.29 (q, *J* = 4.7 Hz, 2H). ¹³C NMR (101 MHz, CDCl₃) δ 164.50, 162.05, 160.76, 156.36, 139.91, 128.86, 128.61, 127.48 (t, *J* = 247.3 Hz), 126.31, 90.39, 53.35 (t, *J* = 32.2 Hz), 51.68, 44.12, 43.95, 35.66, 33.94 (t, *J* = 22.8 Hz), 10.88, 3.50. HRMS [C₂₂H₂₇F₂N₅O + H]⁺ : 416.2256 calculated, 416.2260 found (Δ = 0.86 ppm).

### 2-Chloro-N-(cyclopropylmethyl)-6-(3-hydroxypyrrolidin-1-yl)pyrimidine-4-carboxamide (I-25)

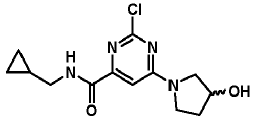

The title compound was prepared according to General Procedure B using dichloropyrimidine **I-4** (98 mg, 0.40 mmol, 1 eq), DiPEA (174 µL, 1.0 mmol, 2.5 eq) and (±)-3-hydroxypyrrolidine HCl salt (52 mg, 0.42 mmol, 1.05 eq). Column chromatography (70% -> 100% EtOAc/pentane) afforded the product (89 mg, 0.30 mmol, 75%). TLC: R_{f} = 0.2 (80% EtOAc/pentane). ¹H NMR (400 MHz, CDCl₃) δ 7.98 (t, *J* = 5.3 Hz, 1H), 7.08 - 6.89 (m, 1H), 4.73 - 4.55 (m, 1H), 3.87 - 3.45 (m, 4H), 3.33 - 3.20 (m, 2H), 2.25 - 2.07 (m, 2H), 1.12 - 0.98 (m, 1H), 0.63 - 0.50 (m, 2H), 0.29 (q, *J* = 4.9 Hz, 2H). ¹³C NMR (101 MHz, CDCl₃) δ 162.63, 162.32, 162.16, 159.59, 156.38, 100.55, 100.49, 70.46, 69.78, 55.58, 55.35, 45.26, 45.02, 44.58, 33.87, 33.29, 10.62, 3.75.

### N-(Cyclopropylmethyl)-6-(3-hydroxypyrrolidin-1-yl)-2-(methyl(phenethyl)amino) pyrimidine-4-carboxamide (Example 32)

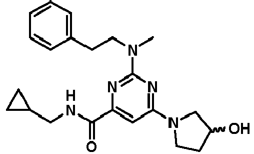

The title compound was prepared according to General Procedure E using 2-chloropyrimidine **I-25** (65 mg, 0.22 mmol, 1 eq), DiPEA (115 µL, 0.66 mmol, 3 eq) and *N*-methylphenethylamine (48 µL, 0.33 mmol, 1.5 eq). Total heating time: 4 h at 160 °C with µW irradiation. Column chromatography (70% -> 100% EtOAc/pentane) afforded the product (60 mg, 0.15 mmol, 69%). TLC: R_{f} = 0.4 (80% EtOAc/pentane). ¹H NMR (400 MHz, CDCl₃) 5 8.10 (t, *J* = 5.2 Hz, 1H), 7.32 - 7.26 (m, 2H), 7.25 - 7.17 (m, 3H), 6.50 (s, 1H), 4.58 (br s, 1H), 3.98 - 3.33 (m, 6H), 3.31 - 3.23 (m, 2H), 3.11 (s, 3H), 3.02 - 2.62 (m, 3H), 2.08 (br s, 2H), 1.10 - 0.98 (m, 1H), 0.63 - 0.45 (m, 2H), 0.27 (q, *J* = 4.7 Hz, 2H). ¹³C NMR (101 MHz, CDCl₃) δ 165.08, 162.07, 160.78, 155.35, 140.10, 128.87, 128.54, 126.19, 91.26, 54.89, 51.73, 44.36, 44.10, 35.57, 33.91, 10.83, 3.48. HRMS [C₂₂H₂₉N₅O₂ + H]⁺ : 396.2394 calculated, 396.2400 found (Δ = 1.51 ppm).

### N-(Cyclopropylmethyl)-6-(3-methoxypyrrolidin-1-yl)-2-(methyl(phenethyl)amino) pyrimidine-4-carboxamide (Example 34)

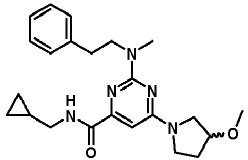

A round bottom flask was charged with hydroxyl **Example 31** (35 mg, 88 µmol, 1 eq) in dry DMF (0.5 mL) and cooled to 0 °C. NaH (60% in mineral oil, 4 mg, 106 µmol, 1.2 eq) was added and the mixture was stirred for 15 min followed by addition of methyl iodide (6.0 µL, 97 µmol, 1.1 eq). The reaction was allowed to warm to rt while stirring overnight. The reaction was quenched with H₂O (20 mL) followed by extraction with DCM (3 x 20 mL), drying (Na₂SO₄), filtering and concentration under reduced pressure. The residue was purified by silica gel column chromatography (60 -> 70% EtOAc/pentane) affording the product (16 mg, 39 µmol, 44%). TLC: R_{f} = 0.4 (60% EtOAc/pentane). ¹H NMR (400 MHz, CDCl₃) δ 8.07 (br s, 1H), 7.33 - 7.27 (m, 2H), 7.26 - 7.17 (m, 3H), 6.54 (s, 1H), 4.07 (br s, 1H), 3.88 - 3.72 (m, 3H), 3.72 - 3.45 (m, 3H), 3.37 (s, 3H), 3.33 - 3.23 (m, 2H), 3.13 (s, 3H), 2.98 - 2.84 (m, 2H), 2.29 - 1.96 (m, 2H), 1.14 - 0.99 (m, 1H), 0.62 - 0.48 (m, 2H), 0.29 (q, *J* = 4.7 Hz, 2H). ¹³C NMR (101 MHz, CDCl₃) δ 207.96, 164.96, 162.10, 160.86, 155.62, 140.16, 128.91, 128.57, 126.22, 91.25, 79.95, 79.15, 56.76, 51.75, 51.42, 44.42, 44.09, 35.62, 33.94, 30.45, 10.91, 3.50. HRMS [C₂₃H₃₁N₅O₂ + H]⁺ : 410.2551 calculated, 410.2549 found (Δ = -0.37 ppm).

### 2-Chloro-N-(cyclopropylmethyl)-6-(3-(dimethylamino)pyrrolidin-1-yl)pyrimidine-4-carboxamide (I-26)

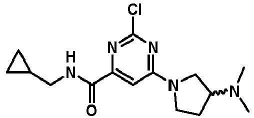

The title compound was prepared according to General Procedure B using dichloropyrimidine **I-4** (98 mg, 0.40 mmol, 1 eq), DiPEA (244 µL, 1.4 mmol, 3.5 eq) and (±)-3-dimethylaminepyrrolidine double HCl salt (79 mg, 0.42 mmol, 1.05 eq). Column chromatography (2.5% -> 10% MeOH/DCM) afforded the product (50 mg, 0.15 mmol, 39%). TLC: R_{f} = 0.4 (5% MeOH/DCM). ¹H NMR (400 MHz, CDCl₃) δ 8.10 - 7.77 (m, 1H), 7.06 (s, 1H), 4.06 - 3.91 (m, 2H), 3.90 - 3.60 (m, 1H), 3.59 - 3.32 (m, 2H), 3.32 - 3.25 (m, 2H), 2.95 - 2.70 (m, 1H), 2.40 - 2.30 (m, 6H), 2.30 - 2.17 (m, 1H), 2.08 - 1.78 (m, 1H), 1.13 - 0.99 (m, 1H), 0.66 - 0.45 (m, 2H), 0.35 - 0.21 (m, 2H). ¹³C NMR (101 MHz, CDCl₃) δ 171.69, 163.73, 162.56, 162.47, 162.15, 161.99, 159.76, 159.69, 159.50, 158.70, 158.27, 156.79, 106.57, 100.46, 100.22, 93.93, 65.41, 64.76, 53.71, 51.47, 51.33, 50.76, 46.42, 46.22, 45.79, 44.54, 44.51, 44.42, 44.28, 44.22, 44.02, 30.41, 29.81, 10.90, 10.85, 10.74, 3.76, 3.51, 3.47.

### N-(Cyclopropylmethyl)-6-(3-(dimethylamino)pyrrolidin-1-yl)-2-(methyl(phenethyl) amino)pyrimidine-4-carboxamide (Example 33)

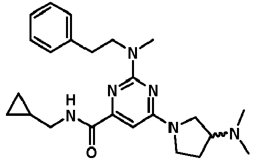

The title compound was prepared according to General Procedure E using 2-chloropyrimidine **I-26** (50 mg, 0.15 mmol, 1 eq), DiPEA (78 µL, 0.60 mmol, 4 eq) and N-methylphenethylamine (33 µL, 0.23 mmol, 1.5 eq). Total heating time: 4 h at 160 °C with µW irradiation. Column chromatography (2.5% -> 10% MeOH/DCM) afforded the product (3 mg, 7 µmol, 5%). TLC: R_{f} = 0.5 (5% MeOH/DCM). ¹H NMR (600 MHz, CDCl₃) δ 8.10 (br s, 1H), 7.31 - 7.27 (m, 2H), 7.24 - 7.18 (m, 3H), 6.69 (s, 1H), 3.98 (dd, *J* = 10.6, 7.2 Hz, 1H), 3.92 - 3.64 (m, 3H), 3.58 - 3.50 (m, 1H), 3.42 (br s, 1H), 3.35 - 3.25 (m, 2H), 2.99 (br s, 3H), 2.89 (t, *J* = 7.7 Hz, 2H), 2.41 (br s, 6H), 2.24 (br s, 1H), 2.06 - 1.89 (m, 1H), 1.80 - 1.51 (m, 1H), 1.12 - 1.02 (m, 1H), 0.59 - 0.49 (m, 2H), 0.34 - 0.24 (m, 2H). ¹³C NMR (151 MHz, CDCl₃) δ 164.89, 163.37, 159.54, 156.17, 128.99, 128.68, 126.44, 90.62, 65.56, 51.98, 45.61, 44.02, 33.80, 28.71, 11.00, 3.52. HRMS [C₂₄H₃₄N₆O + H]⁺ : 423.2867 calculated, 423.2868 found (Δ = 0.26 ppm).

### (R)-2-Chloro-N-(cyclopropylmethyl)-6-(3-hydroxypyrrolidin-1-yl)pyrimidine-4-carboxamide (I-27)

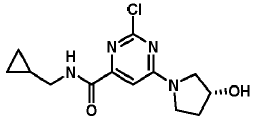

The title compound was prepared according to General Procedure B using dichloropyrimidine **I-4** (98 mg, 0.40 mmol, 1 eq), DiPEA (174 µL, 1.0 mmol, 2.5 eq) and (R)-3-hydroxypyrrolidine HCl salt (52 mg, 0.42 mmol, 1.05 eq). Column chromatography (70% -> 100% EtOAc/pentane) afforded the product (105 mg, 0.35 mmol, 88%). TLC: R_{f} = 0.2 (80% EtOAc/pentane). ¹H NMR (400 MHz, CDCl₃) δ 7.98 (t, *J* = 5.4 Hz, 1H), 7.08 - 6.91 (m, 1H), 4.74 - 4.54 (m, 1H), 3.90 - 3.39 (m, 5H), 3.34 - 3.20 (m, 2H), 2.24 - 2.07 (m, 2H), 1.13 - 0.98 (m, 1H), 0.63 - 0.48 (m, 2H), 0.36 - 0.17 (m, 2H). ¹³C NMR (101 MHz, CDCl₃) δ 162.64, 162.34, 162.18, 159.73, 159.60, 156.40, 100.57, 100.52, 70.50, 69.81, 55.59, 55.36, 45.27, 45.03, 44.59, 33.87, 33.32, 10.63, 3.76.

### (R)-N-(Cyclopropylmethyl)-6-(3-hydroxypyrrolidin-1-yl)-2-(methyl(phenethyl)amino) pyrimidine-4-carboxamide (Example 38)

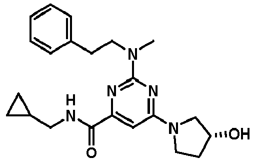

The title compound was prepared according to General Procedure E using 2-chloropyrimidine **I-27** (38 mg, 0.13 mmol, 1 eq), DiPEA (67 µL, 0.38 mmol, 3 eq) and N-methylphenethylamine (28 µL, 0.19 mmol, 1.5 eq). Total heating time: 4 h at 160 °C with µW irradiation. Column chromatography (80% -> 100% EtOAc/pentane) afforded the product (37 mg, 94 µmol, 78%). *ee*: >99% (as determined by chiral HPLC using 75:25 hexane/ethanol, Chiralcell OD). TLC: R_{f} = 0.3 (80% EtOAc/pentane). ¹H NMR (400 MHz, CDCl₃) δ 8.09 (br s, 1H), 7.33 - 7.27 (m, 2H), 7.26 - 7.17 (m, 3H), 6.52 (s, 1H), 4.59 (br s, 1H), 3.79 (dd, *J* = 8.7, 5.8 Hz, 2H), 3.74 - 3.37 (m, 4H), 3.33 - 3.22 (m, 2H), 3.12 (s, 3H), 2.96 - 2.82 (m, 2H), 2.70 - 2.21 (m, 1H), 2.09 (br s, 2H), 1.12 - 0.99 (m, 1H), 0.61 - 0.49 (m, 2H), 0.28 (q, *J* = 4.7 Hz, 2H). ¹³C NMR (101 MHz, CDCl₃) δ 165.04, 162.12, 160.82, 155.49, 140.13, 128.90, 128.58, 126.22, 91.28, 70.52, 54.92, 51.75, 44.34, 44.12, 35.60, 33.93, 10.87, 3.50. HRMS [C₂₂H₂₉N₅O₂ + H]⁺ : 396.2394 calculated, 396.2394 found (Δ = 0.0 ppm).

### N-(Cyclopropylmethyl)-6-((R)-3-hydroxypyrrolidin-1-yl)-2-((R)-3-phenylpiperidin-1-yl)pyrimidine-4-carboxamide (Example 42)

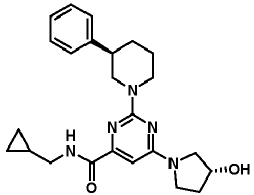

The title compound was prepared according to General Procedure E using 2-chloropyrimidine **I-27** (22 mg, 74 µmol, 1 eq), DiPEA (39 µL, 0.22 mmol, 3 eq) and (R)-3-phenylpiperidine (16 mg, 96 µmol, 1.3 eq). Total heating time: 4 h at 160 °C with µW irradiation. Column chromatography (70% -> 90% EtOAc/pentane) afforded the product (23 mg, 55 µmol, 74%). TLC: R_{f} = 0.4 (80% EtOAc/pentane). ¹H NMR (400 MHz, CDCl₃) δ 8.03 (t, *J* = 5.7 Hz, 1H), 7.40 - 7.19 (m, 5H), 6.53 (s, 1H), 4.84 (t, *J* = 14.3 Hz, 2H), 4.57 (s, 1H), 3.90 - 3.37 (m, 4H), 3.37 - 3.17 (m, 2H), 2.94 - 2.81 (m, 2H), 2.81 - 2.69 (m, 1H), 2.56 (s, 1H), 2.07 (d, *J* = 12.7 Hz, 3H), 1.89 - 1.57 (m, 4H), 1.10 - 0.98 (m, 1H), 0.60 - 0.42 (m, 2H), 0.26 (q, *J* = 4.7 Hz, 2H). ¹³C NMR (101 MHz, CDCl₃) δ 164.96, 162.19, 160.91, 155.67, 144.43, 128.62, 127.32, 126.59, 91.75, 70.36, 54.95, 51.36, 44.68, 44.38, 44.06, 42.54, 32.20, 25.60, 10.92, 3.51. HRMS [C₂₄H₃₁N₅O₂ + H]⁺ : 422.2551 calculated, 422.2552 found (Δ = 0.36 ppm).

### N-(cyclopropylmethyl)-6-((R)-3-hydroxypyrrolidin-1-yl)-2-((S)-3-phenylpiperidin-1-yl)pyrimidine-4-carboxamide (Example 43)

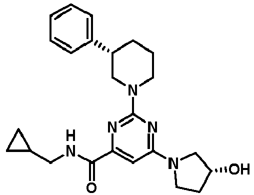

The title compound was prepared according to General Procedure E using 2-chloropyrimidine **I-27** (24 mg, 81 µmol, 1 eq), DiPEA (42 µL, 0.24 mmol, 3 eq) and (S)-3-phenylpiperidine (17 mg, 0.11 mmol, 1.3 eq). Total heating time: 4 h at 160 °C with µW irradiation. Column chromatography (70% -> 90% EtOAc/pentane) afforded the product (23 mg, 55 µmol, 67%). TLC: R_{f} = 0.4 (80% EtOAc/pentane). ¹H NMR (400 MHz, CDCl₃) δ 8.02 (br s, 1H), 7.39 - 7.22 (m, 5H), 6.54 (s, 1H), 4.84 (t, *J* = 14.0 Hz, 2H), 4.57 (s, 1H), 3.86 - 3.37 (m, 4H), 3.37 - 3.17 (m, 2H), 2.87 (t, *J* = 12.0 Hz, 2H), 2.81 - 2.70 (m, 1H), 2.16 - 1.93 (m, 3H), 1.90 - 1.54 (m, 4H), 1.11 -0.97 (m, 1H), 0.59 - 0.45 (m, 2H), 0.27 (q, *J* = 4.7 Hz, 2H). ¹³C NMR (101 MHz, CDCl₃) δ 164.93, 162.20, 160.91, 155.71, 144.42, 128.64, 127.33, 126.60, 91.76, 54.96, 51.30, 44.67, 44.38, 44.07, 42.59, 32.24, 25.59, 10.93, 3.52. HRMS [C₂₄H₃₁N₅O₂ + H]⁺ : 422.2551 calculated, 422.2551 found (Δ = 0.12 ppm).

### (S)-2-Chloro-N-(cyclopropylmethyl)-6-(3-hydroxypyrrolidin-1-yl)pyrimidine-4-carboxamide (I-28)

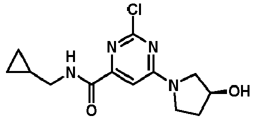

The title compound was prepared according to General Procedure B using dichloropyrimidine **I-4** (98 mg, 0.40 mmol, 1 eq), DiPEA (174 µL, 1.0 mmol, 2.5 eq) and (S)-3-hydroxypyrrolidine HCl salt (52 mg, 0.42 mmol, 1.05 eq). Column chromatography (70% -> 100% EtOAc/pentane) afforded the product (97 mg, 0.33 mmol, 82%). TLC: R_{f} = 0.2 (80% EtOAc/pentane). ¹H NMR (300 MHz, CDCl₃) δ 7.98 (t, *J* = 5.2 Hz, 1H), 7.11 - 6.86 (m, 1H), 4.81 - 4.47 (m, 1H), 3.93 - 3.35 (m, 5H), 3.33 - 3.21 (m, 2H), 2.25 - 2.06 (m, 2H), 1.16 - 0.96 (m, 1H), 0.57 (q, *J* = 5.5 Hz, 2H), 0.29 (q, *J* = 4.7 Hz, 2H). ¹³C NMR (75 MHz, CDCl₃) δ 162.65, 162.34, 162.19, 159.73, 159.61, 156.41, 100.55, 100.51, 70.48, 69.79, 55.58, 55.35, 45.27, 45.03, 44.58, 33.87, 33.31, 10.62, 3.74.

### (S)-N-(Cyclopropylmethyl)-6-(3-hydroxypyrrolidin-1-yl)-2-(methyl(phenethyl)amino) pyrimidine-4-carboxamide (Example 39)

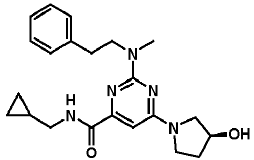

The title compound was prepared according to General Procedure E using 2-chloropyrimidine **I-28** (36 mg, 0.12 mmol, 1 eq), DiPEA (63 µL, 0.36 mmol, 3 eq) and N-methylphenethylamine (27 µL, 0.18 mmol, 1.5 eq). Total heating time: 4 h at 160 °C with µW irradiation. Column chromatography (80% -> 100% EtOAc/pentane) afforded the product (36 mg, 91 µmol, 70%). *ee*: 97% (as determined by chiral HPLC using 75:25 hexane/ethanol, Chiralcell OD). TLC: R_{f} = 0.3 (80% EtOAc/pentane). ¹H NMR (400 MHz, CDCl₃) 5 8.10 (t, *J* = 5.1 Hz, 1H), 7.35 - 7.27 (m, 2H), 7.25 - 7.15 (m, 3H), 6.51 (s, 1H), 4.58 (br s, 1H), 3.82 - 3.74 (m, 2H), 3.74 - 3.35 (m, 4H), 3.32 - 3.22 (m, 2H), 3.12 (s, 3H), 2.94 - 2.84 (m, 2H), 2.80 - 2.48 (m, 1H), 2.08 (br s, 2H), 1.12 - 0.98 (m, 1H), 0.60 - 0.48 (m, 2H), 0.27 (q, *J* = 4.7 Hz, 2H). ¹³C NMR (101 MHz, CDCl₃) δ 165.07, 162.07, 160.77, 155.36, 140.10, 128.88, 128.55, 126.20, 91.26, 70.88, 70.32, 54.89, 51.74, 44.35, 44.11, 35.58, 33.91, 10.83, 3.48. HRMS [C₂₂H₂₉N₅O₂ + H]⁺ : 396.2394 calculated, 396.2398 found (Δ = 1.01 ppm).

### N-(Cyclopropylmethyl)-6-((S)-3-hydroxypyrrolidin-1-yl)-2-((S)-3-phenylpiperidin-1-yl)pyrimidine-4-carboxamide (Example 40)

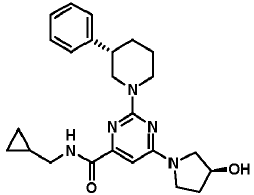

The title compound was prepared according to General Procedure E using 2-chloropyrimidine **I-28** (37 mg, 0.12 mmol, 1 eq), DiPEA (65 µL, 0.37 mmol, 3 eq) and (S)-3-phenylpiperidine (26 mg, 0.16 mmol, 1.3 eq). Total heating time: 4 h at 160 °C with µW irradiation. Column chromatography (70% -> 100% EtOAc/pentane) afforded the product (26 mg, 62 µmol, 51%). TLC: R_{f} = 0.4 (80% EtOAc/pentane). ¹H NMR (400 MHz, CDCl₃) δ 8.03 (br s, 1H), 7.40 - 7.20 (m, 5H), 6.53 (s, 1H), 4.84 (t, *J* = 14.3 Hz, 2H), 4.57 (s, 1H), 3.91 - 3.37 (m, 4H), 3.36 - 3.18 (m, 2H), 2.96 - 2.81 (m, 2H), 2.81 - 2.70 (m, 1H), 2.17 - 1.94 (m, 3H), 1.93 - 1.50 (m, 4H), 1.11 - 0.97 (m, 1H), 0.59 - 0.43 (m, 2H), 0.26 (q, *J* = 4.7 Hz, 2H). ¹³C NMR (101 MHz, CDCl₃) δ 164.97, 162.17, 160.90, 155.63, 144.41, 128.62, 127.31, 126.58, 91.75, 71.02, 70.38, 54.95, 51.36, 44.68, 44.39, 44.05, 42.53, 32.19, 25.59, 10.91, 3.50. HRMS [C₂₄H₃₁N₅O₂ + H]⁺ : 422.2551 calculated, 422.2555 found (Δ = 1.07 ppm).

### N-(Cyclopropylmethyl)-6-((S)-3-hydroxypyrrolidin-1-yl)-2-((R)-3-phenylpiperidin-1-yl)pyrimidine-4-carboxamide (Example 41)

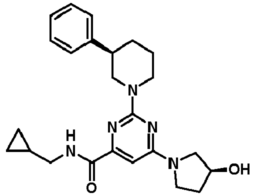

The title compound was prepared according to General Procedure E using 2-chloropyrimidine **I-28** (32 mg, 0.11 mmol, 1 eq), DiPEA (56 µL, 0.32 mmol, 3 eq) and (R)-3-phenylpiperidine (23 mg, 0.14 mmol, 1.3 eq). Total heating time: 4 h at 160 °C with µW irradiation. Column chromatography (70% -> 100% EtOAc/pentane) afforded the product (26 mg, 62 µmol, 56%). TLC: R_{f} = 0.4 (80% EtOAc/pentane). ¹H NMR (400 MHz, CDCl₃) δ 8.03 (t, *J* = 5.7 Hz, 1H), 7.40 - 7.19 (m, 5H), 6.53 (s, 1H), 4.84 (t, *J =* 14.3 Hz, 2H), 4.57 (s, 1H), 3.90 - 3.37 (m, 4H), 3.37 - 3.17 (m, 2H), 2.94 - 2.81 (m, 2H), 2.81 - 2.69 (m, 1H), 2.16 - 1.96 (m, 3H), 1.89 - 1.57 (m, 4H), 1.10 - 0.98 (m, 1H), 0.60 - 0.42 (m, 2H), 0.26 (q, *J* = 4.7 Hz, 2H). ¹³C NMR (101 MHz, CDCl₃) δ 164.84, 162.10, 160.81, 155.58, 144.32, 128.52, 127.22, 126.48, 91.64, 70.84, 70.16, 54.84, 51.19, 44.55, 44.27, 43.95, 42.47, 32.13, 25.48, 10.82, 3.40. HRMS [C₂₄H₃₁N₅O₂ + H]⁺ : 422.2551 calculated, 422.2552 found (Δ = 0.36 ppm).

### 2-Chloro-N-(cyclopropylmethyl)-6-(4,4-difluoropiperidin-1-yl)pyrimidine-4-carboxamide (I-29)

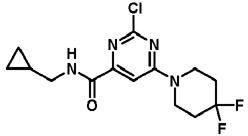

The title compound was prepared according to General Procedure B using dichloropyrimidine **I-4** (0.12 g, 0.49 mmol, 1 eq), DiPEA (0.21 mL, 1.23 mmol, 2.5 eq) and 4,4-difluoropiperidine HCl salt (86 mg, 0.52 mmol, 1.05 eq). Column chromatography (5% -> 25% EtOAc/pentane) afforded the product (90 mg, 0.27 mmol, 55%). TLC: R_{f} = 0.4 (15% EtOAc/pentane). ¹H NMR (300 MHz, CDCl₃) δ 7.91 (br s, 1H), 7.35 (s, 1H), 3.89 (br s, 4H), 3.38 - 3.20 (m, 2H), 2.06 (tt, *J* = 13.2, 5.9 Hz, 4H), 1.13 - 0.98 (m, 1H), 0.64 - 0.48 (m, 2H), 0.29 (q, *J* = 4.8 Hz, 2H). ¹³C NMR (75 MHz, CDCl₃) δ 163.47, 162.18, 160.08, 158.40, 121.29 (t, *J* = 242.4 Hz), 99.44, 44.55, 41.49, 33.82 (t, *J* = 23.7 Hz), 10.72, 3.72.

### N-(Cyclopropylmethyl)-6-(4,4-difluoropiperidin-1-yl)-2-(methyl(phenethyl)amino) pyrimidine-4-carboxamide (Example 2)

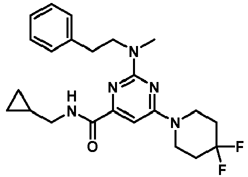

The title compound was prepared according to General Procedure E using 2-chloropyrimidine **I-29** (45 mg, 0.14 mmol, 1 eq), DiPEA (73 µL, 0.42 mmol, 3 eq) and N-methylphenethylamine (31 µL, 0.21 mmol, 1.5 eq). Total heating time: 4 h at 160 °C with µW irradiation. Column chromatography (5% -> 30% EtOAc/pentane) afforded the product (27 mg, 63 µmol, 45%). TLC: R_{f} = 0.5 (15% EtOAc/pentane). ¹H NMR (400 MHz, CDCl₃) δ 8.03 (t, *J* = 5.2 Hz, 1H), 7.33 - 7.27 (m, 2H), 7.25 - 7.18 (m, 3H), 6.79 (s, 1H), 3.88 - 3.74 (m, 6H), 3.36 - 3.25 (m, 2H), 3.13 (s, 3H), 2.95 - 2.85 (m, 2H), 2.06 - 1.92 (m, 4H), 1.13 - 0.99 (m, 1H), 0.63 - 0.50 (m, 2H), 0.29 (q, *J* = 4.7 Hz, 2H). ¹³C NMR (101 MHz, CDCl₃) δ 164.58, 163.23, 160.99, 157.16, 139.85, 128.83, 128.62, 126.35, 122.10 (t, *J* = 242.0 Hz), 90.06, 51.73, 44.13, 41.27, 35.74, 33.95, 33.84 (t, *J =* 23.0 Hz), 10.89, 3.49. HRMS [C₂₃H₂₉F₂N₅O + H]⁺ : 430.2413 calculated, 430.2422 found (Δ = 2.26 ppm).

### 2-Chloro-N-(cyclopropylmethyl)-6-(3,3-difluoropiperidin-1-yl)pyrimidine-4-carboxamide (I-30)

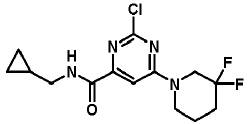

The title compound was prepared according to General Procedure B using dichloropyrimidine **I-4** (0.10 g, 0.41 mmol, 1 eq), DiPEA (100 µL, 0.57 mmol, 2.4 eq) and 3,3-difluoropiperidine HCl salt (68 mg, 0.43 mmol, 1.05 eq). Column chromatography (10% -> 40% EtOAc/pentane) afforded the product (88 mg, 0.26 mmol, 50%). TLC: R_{f} = 0.4 (20% EtOAc/pentane). ¹H NMR (300 MHz, CDCl₃) δ 7.91 (br s, 1H), 7.35 (s, 1H), 3.99 (br s, 2H), 3.74 (br s, 2H), 3.29 (t, *J* = 6.5 Hz, 2H), 2.13 (tt, *J* = 13.3, 6.3 Hz, 2H), 2.02 - 1.74 (m, 2H), 1.17 - 0.95 (m, 1H), 0.67 - 0.44 (m, 2H), 0.28 (q, *J* = 5.1 Hz, 2H). ¹³C NMR (75 MHz, CDCl₃) δ 164.08, 162.15, 159.87, 158.33, 119.17 (t, *J* = 244.8 Hz), 99.62, 44.54, 44.05, 32.63 (t, *J* = 23.5 Hz), 29.76, 21.70 (t, *J* = 4.5 Hz). 10.72, 3.71.

### N-(Cyclopropylmethyl)-6-(3,3-difluoropiperidin-1-yl)-2-(methyl(phenethyl)amino )pyrimidine-4-carboxamide (Example 86)

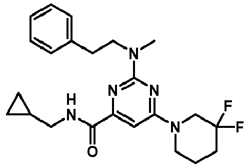

The title compound was prepared according to General Procedure E using 2-chloropyrimidine **I-30** (50 mg, 0.15 mmol, 1 eq), DiPEA (78 µL, 0.45 mmol, 3 eq) and N-methylphenethylamine (33 µL, 0.27 mmol, 1.5 eq). Total heating time: 4 h at 160 °C with µW irradiation. Column chromatography (10% -> 30% EtOAc/pentane) afforded the product (10 mg, 23 µmol, 15%). TLC: R_{f} = 0.5 (20% EtOAc/pentane). ¹H NMR (400 MHz, CDCl₃) δ 8.02 (br s, 1H), 7.34 - 7.27 (m, 2H), 7.26 - 7.18 (m, 3H), 6.78 (s, 1H), 4.01 (t, *J* = 11.7 Hz, 2H), 3.86 - 3.73 (m, 2H), 3.71 - 3.58 (m, 2H), 3.35 - 3.25 (m, 2H), 3.13 (s, 3H), 2.95 - 2.86 (m, 2H), 2.17 - 2.03 (m, 2H), 1.88 - 1.78 (m, 2H), 1.12 - 1.01 (m, 1H), 0.61 - 0.50 (m, 2H), 0.29 (q, *J* = 4.7 Hz, 2H). ¹³C NMR (101 MHz, CDCl₃) δ 164.61, 163.70, 160.80, 157.10, 139.92, 128.91, 128.63, 126.32, 119.79 (t, *J* = 244.2 Hz), 90.16, 51.89, 49.40 (t, *J* = 32.8 Hz), 44.14, 43.73, 35.80, 33.92, 33.03 (t, *J* = 23.5 Hz), 22.07 (t, *J* = 4.4 Hz), 10.91, 3.50. HRMS [C₂₃H₂₉F₂N₅O + H]⁺ : 430.2413 calculated, 430.2419 found (Δ = 1.42 ppm).

### 2-Chloro-N-(cyclopropylmethyl)-6-(1H-pyrazol-1-yl)pyrimidine-4-carboxamide (I-31)

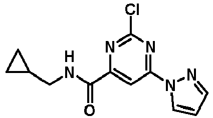

The title compound was prepared according to General Procedure G using dichloropyrimidine **I-4** (123 mg, 0.50 mmol, 1 eq), K₂CO₃ (104 mg, 0.75 mmol, 1.5 eq) and pyrazole (36 mg, 0.53 mmol, 1.05 eq). Column chromatography (10% -> 30% EtOAc/pentane) afforded the product (76 mg, 0.27 mmol, 55%). TLC: R_{f} = 0.7 (50% EtOAc/pentane). TLC: R_{f} = 0.8 (30% EtOAc/pentane). ¹H NMR (400 MHz, CDCl₃) δ 8.61 (s, 1H), 8.56 (d, *J* = 2.7 Hz, 1H), 7.92 (br s, 1H), 7.85 (d, *J* = 1.3 Hz, 1H), 6.55 (dd, *J* = 2.8, 1.6 Hz, 1H), 3.36 (dd, *J* = 7.1, 5.9 Hz, 2H), 1.19 - 1.00 (m, 1H), 0.71 - 0.49 (m, 2H), 0.32 (q, *J* = 4.7 Hz, 2H). ¹³C NMR (101 MHz, CDCl₃) δ 161.28, 160.88, 160.48, 159.64, 145.18, 128.34, 110.22, 105.51, 44.73, 3.76.

### N-(Cyclopropylmethyl)-2-(methyl(phenethyl)amino)-6-(1H-pyrazol-1-yl)pyrimidine-4-carboxamide (Example 24)

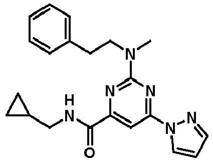

The title compound was prepared according to General Procedure E using 2-chloropyrimidine **I-31** (28 mg, 0.10 mmol, 1 eq), DiPEA (52 µL, 0.30 mmol, 3 eq) and N-methylphenethylamine (22 µL, 0.15 mmol, 1.5 eq). Total heating time: 4 h at 160 °C with µW irradiation. Column chromatography (20% -> 40% EtOAc/pentane) afforded the product (30 mg, 80 µmol, 80%). TLC: R_{f} = 0.5 (30% EtOAc/pentane). ¹H NMR (400 MHz, CDCl₃) δ 8.51 (s, 1H), 7.98 - 7.81 (m, 2H), 7.81 - 7.73 (m, 1H), 7.33 - 7.27 (m, 2H), 7.26 - 7.18 (m, 3H), 6.56 - 6.39 (m, 1H), 3.97 - 3.83 (m, 2H), 3.33 (dd, *J* = 6.9, 6.0 Hz, 2H), 3.21 (s, 3H), 3.01 - 2.91 (m, 2H), 1.18 - 1.00 (m, 1H), 0.57 (q, *J* = 5.4 Hz, 2H), 0.30 (q, *J* = 4.8 Hz, 2H). ¹³C NMR (101 MHz, CDCl₃) δ 163.17, 160.57, 159.73, 159.48, 143.40, 139.40, 128.84, 128.69, 127.30, 126.52, 108.54, 94.69, 51.83, 44.24, 36.01, 33.79, 10.87, 3.52. HRMS [C₂₁H₂₄N₆O + H]⁺ : 377.2084 calculated, 377.2088 found (Δ = 0.95 ppm).

### 2-Chloro-N-(cyclopropylmethyl)-6-(1H-imidazol-1-yl)pyrimidine-4-carboxamide (I-32)

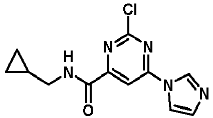

The title compound was prepared according to General Procedure G using dichloropyrimidine **I-4** (123 mg, 0.50 mmol, 1 eq), K₂CO₃ (104 mg, 0.75 mmol, 1.5 eq) and imidazole (36 mg, 0.53 mmol, 1.05 eq). Column chromatography (60% -> 80% EtOAc/pentane) afforded the product as an inseperable mixture of regioisomers (2.5:1, 6-imidazolyl : 2-imidazolyl), that were used as such for the following step (76 mg, 0.27 mmol, 55%). TLC: R_{f} = 0.4 (5% MeOH/DCM). ¹H NMR (400 MHz, CDCl₃) δ 8.56 (s, 1H), 8.10 (s, 1H), 7.98 (br s, 1H), 7.77 (t, *J* = 1.4 Hz, 1H), 7.26 (s, 1H), 3.47 - 3.26 (m, 2H), 1.18 - 1.03 (m, 1H), 0.68 - 0.53 (m, 2H), 0.41 - 0.27 (m, 2H). ¹³C NMR (101 MHz, CDCl₃) δ 161.93, 160.55, 159.94, 158.14, 135.73, 132.38, 116.02, 104.68, 44.84, 10.66, 3.76.

### N-(Cyclopropylmethyl)-6-(1H-imidazol-1-yl)-2-(methyl(phenethyl)amino) pyrimidine-4-carboxamide (Example 23)

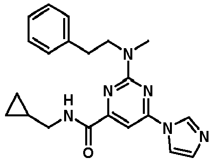

The title compound was prepared according to General Procedure E using 2-chloropyrimidine **I-32** (42 mg, 0.15 mmol, 1 eq), DiPEA (78 µL, 0.45 mmol, 3 eq) and N-methylphenethylamine (33 µL, 0.23 mmol, 1.5 eq). Total heating time: 4 h at 160 °C with µW irradiation. Purification by preparative HPLC (C18 reverse phase, 35% to 45% ACN/H₂O + 0.2% TFA, RT 8.87 min) afforded the product (27 mg, 72 µmol, 48%). TLC: R_{f} = 0.5 (5% MeOH/DCM). ¹H NMR (400 MHz, CDCl₃) δ 8.46 (s, 1H), 7.99 - 7.72 (m, 1H), 7.68 (s, 1H), 7.33 - 7.27 (m, 3H), 7.25 - 7.17 (m, 4H), 4.00 - 3.81 (m, 2H), 3.38 - 3.29 (m, 2H), 3.22 (s, 3H), 3.02 - 2.90 (m, 2H), 1.16 - 1.03 (m, 1H), 0.69 - 0.51 (m, 2H), 0.32 (q, *J* = 4.9 Hz, 2H). ¹³C NMR (101 MHz, CDCl₃) δ 162.88, 160.89, 160.19, 157.03, 139.14, 135.37, 131.10, 128.82, 128.73, 126.61, 116.00, 93.80, 51.89, 44.36, 36.06, 33.78, 10.84, 3.56. HRMS [C₂₁H₂₄N₆O + H]⁺ : 377.2084 calculated, 377.2087 found (Δ = 0.69 ppm).

### 2-Chloro-N-(cyclopropylmethyl)-6-phenoxypyrimidine-4-carboxamide (I-33)

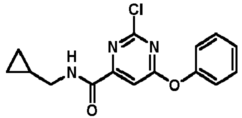

The title compound was prepared according to General Procedure G using dichloropyrimidine **I-4** (100 mg, 0.41 mmol, 1 eq), K₂CO₃ (86 mg, 0.62 mmol, 1.5 eq) and phenol (44 mg, 0.43 mmol, 1.05 eq). Column chromatography (5% -> 30% EtOAc/pentane) afforded the product (100 mg, 0.33 mmol, 80%). TLC: R_{f} = 0.4 (20% EtOAc/pentane). ¹H NMR (300 MHz, CDCl₃) δ 7.90 (br s, 1H), 7.54 (s, 1H), 7.50 - 7.39 (m, 2H), 7.36 - 7.28 (m, 1H), 7.20 - 7.08 (m, 2H), 3.41 - 3.17 (m, 2H), 1.15 - 0.96 (m, 1H), 0.66 - 0.47 (m, 2H), 0.29 (q, *J* = 4.7 Hz, 2H). ¹³C NMR (75 MHz, CDCl₃) δ 172.25, 161.17, 161.02, 159.73, 151.84, 130.19, 126.62, 121.27, 104.58, 44.68, 10.70, 3.75.

### N-(Cyclopropylmethyl)-2-(methyl(phenethyl)amino)-6-phenoxypyrimidine-4-carboxamide (Example 87)

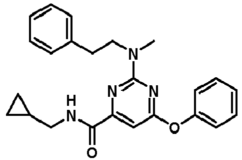

The title compound was prepared according to General Procedure E using 2-chloropyrimidine **I-33** (49 mg, 0.16 mmol, 1 eq), DiPEA (84 µL, 0.48 mmol, 3 eq) and N-methylphenethylamine (35 µL, 0.24 mmol, 1.5 eq). Total heating time: 4 h at 160 °C with µW irradiation. Column chromatography (5% -> 25% EtOAc/pentane) afforded the product (40 mg, 99 µmol, 62%). TLC: R_{f} = 0.5 (20% EtOAc/pentane). ¹H NMR (400 MHz, CDCl₃) δ 7.91 (br s, 1H), 7.46 - 7.36 (m, 2H), 7.35 - 7.05 (m, 7H), 7.04 - 6.68 (m, 2H), 4.00 - 3.39 (m, 2H), 3.30 (t, *J* = 6.4 Hz, 2H), 3.10 (br s, 3H), 2.97 - 2.53 (m, 2H), 1.13 - 1.01 (m, 1H), 0.56 (q, *J* = 5.6 Hz, 2H), 0.35 - 0.23 (m, 2H). ¹³C NMR (101 MHz, CDCl₃) δ 171.62, 163.46, 160.96, 159.59, 152.85, 139.32, 129.64, 128.89, 128.50, 126.33, 125.47, 122.04, 51.85, 44.18, 35.78, 33.63, 10.88, 3.51. HRMS [C₂₄H₂₅N₄O₂ + H]⁺ : 403.2129 calculated, 403.2137 found (Δ = 2.11 ppm).

### 2-Chloro-N-(cyclopropylmethyl)-6-(pyridin-3-yloxy)pyrimidine-4-carboxamide (I-34)

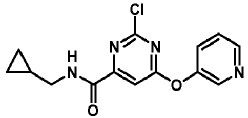

The title compound was prepared according to General Procedure G using dichloropyrimidine **I-4** (0.11 g, 0.45 mmol, 1 eq), K₂CO₃ (93 mg, 0.68 mmol, 1.5 eq) and pyridine-3-ol (45 mg, 0.47 mmol, 1.05 eq). Column chromatography (60% -> 100% EtOAc/pentane) afforded the product (70 mg, 0.23 mmol, 51%). TLC: R_{f} = 0.4 (80% EtOAc/pentane). ¹H NMR (300 MHz, CDCl₃) δ 8.56 (dd, *J* = 9.3, 3.2 Hz, 2H), 7.94 (br s, 1H), 7.69 (s, 1H), 7.63 - 7.50 (m, 1H), 7.43 (dd, *J* = 8.3, 4.7 Hz, 1H), 3.46 - 3.19 (m, 2H), 1.17 - 0.99 (m, 1H), 0.66 - 0.51 (m, 2H), 0.31 (q, *J* = 4.8 Hz, 2H). ¹³C NMR (75 MHz, CDCl₃) δ 171.32, 161.44, 160.74, 159.43, 148.48, 147.52, 143.41, 129.09, 124.31, 105.26, 44.66, 10.66, 3.71.

### N-(Cyclopropylmethyl)-2-(methyl(phenethyl)amino)-6-(pyridin-3-yloxy)pyrimidine-4-carboxamide (Example 1)

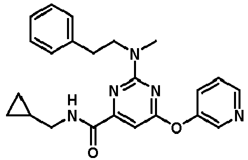

The title compound was prepared according to General Procedure E using 2-chloropyrimidine **I-34** (38 mg, 0.12 mmol, 1 eq), DiPEA (62 µL, 0.36 mmol, 3 eq) and N-methylphenethylamine (26 µL, 0.18 mmol, 1.5 eq). Total heating time: 4 h at 160 °C with µW irradiation. Column chromatography (60% -> 100% EtOAc/pentane) afforded the product (26 mg, 64 µmol, 54%). TLC: R_{f} = 0.5 (80% EtOAc/pentane). ¹H NMR (400 MHz, CDCl₃) δ 8.66 - 8.42 (m, 2H), 7.90 (br s, 1H), 7.65 - 7.44 (m, 1H), 7.41 - 7.32 (m, 1H), 7.31 - 7.12 (m, 4H), 7.07 - 6.71 (m, 2H), 3.82 (br s, 1H), 3.51 (br s, 1H), 3.38 - 3.26 (m, 2H), 3.13 (br s, 2H), 2.93 (br s, 2H), 2.65 (br s, 1H), 1.15 - 1.02 (m, 1H), 0.57 (q, *J* = 5.1 Hz, 2H), 0.30 (q, *J* = 4.8 Hz, 2H). ¹³C NMR (101 MHz, CDCl₃) δ 170.88, 163.22, 160.69, 160.01, 149.45, 146.41, 144.26, 129.68, 128.80, 128.56, 126.44, 123.96, 51.74, 44.23, 35.78, 33.59, 10.88, 3.53. HRMS [C₂₃H₂₅N₅O₂ + H]⁺ : 404.2081 calculated, 404.2088 found (Δ = 1.73 ppm).

### 2-Chloro-N-(cyclopropylmethyl)-6-morpholinopyrimidine-4-carboxamide (I-35)

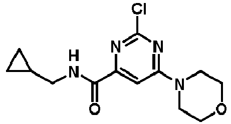

The title compound was prepared according to General Procedure B using dichloropyrimidine **I-4** (1.7 g, 7.07 mmol, 1 eq), DiPEA (1.85 mL, 10.6 mmol, 1.5 eq) and morpholine (0.64 mL, 7.42 mmol, 1.05 eq). Column chromatography (30% -> 60% EtOAc/pentane) afforded the product (1.7 g, 6.3 mmol, 89%). R_{f} = 0.5 in 40% EtOAc/pentane. ¹H NMR (400 MHz, CDCl₃) δ 7.94 (t, *J* = 5.9 Hz, 1H), 7.28 (s, 1H), 3.87 - 3.63 (m, 8H), 3.33 - 3.25 (m, 2H), 1.12 - 1.00 (m, 1H), 0.61 - 0.52 (m, 2H), 0.32 - 0.24 (m, 2H). ¹³C NMR (101 MHz, CDCl₃) δ 163.83, 162.21, 159.84, 157.90, 99.36, 66.35, 44.45, 10.65, 3.64. Regioselectivity was confirmed by NOESY analysis. HRMS [C₁₃H₁₇ClN₄O₂ + H]⁺ : 297.1113 calculated, 297.1116 found (Δ = 1.11 ppm).

**Regioisomer 6-chloro-*N*-(cyclopropylmethyl)-2-morpholinopyrimidine-4-carboxamide (I-35B)** was also obtained (99 mg, 0.33 mmol, 5%). R_{f} = 0.6 in 40% EtOAc/pentane. ¹H NMR (400 MHz, CDCl₃) δ 7.80 - 7.65 (m, 1H), 7.32 (s, 1H), 3.90 - 3.72 (m, 8H), 3.30 (t, *J* = 6.5 Hz, 2H), 1.14 - 0.98 (m, 1H), 0.69 - 0.46 (m, 2H), 0.39 - 0.17 (m, 2H). ¹³C NMR (101 MHz, CDCl₃) δ 163.48, 162.31, 160.68, 158.86, 107.51, 66.64, 44.42, 44.30, 10.80, 3.55.

### N-(Cyclopropylmethyl)-6-morpholino-2-(phenethylamino)pyrimidine-4-carboxamide (Example 47)

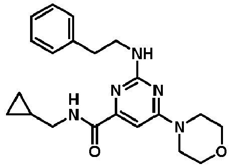

The title compound was prepared according to the general procedure E using 2-chloropyrimidine **I-35** (59 mg, 0.20 mmol, 1 eq), 2-phenethylamine (30 µL, 0.24 mmol, 1.2 eq) and DiPEA (70 µL, 0.40 mmol, 2 eq). Total heating time: 8 h at 160 °C with µW irradiation. Column chromatography (2% -> 5% MeOH/DCM) afforded the product (40 mg, 0.10 mmol, 52%). TLC: R_{f} = 0.4 (4% MeOH/DCM). ¹H NMR (400 MHz, CDCl₃) δ 8.02 (br s, 1H), 7.39 - 7.28 (m, 2H), 7.28 - 7.15 (m, 3H), 6.78 (s, 1H), 4.96 (br s, 1H), 3.84 - 3.71 (m, 4H), 3.71 - 3.49 (m, 6H), 3.27 (t, *J* = 6.4 Hz, 2H), 2.92 (t, *J* = 7.2 Hz, 2H), 1.13 - 0.95 (m, 1H), 0.64 - 0.41 (m, 2H), 0.37 - 0.18 (m, 2H). ¹³C NMR (101 MHz, CDCl₃) δ 164.46, 163.98, 161.46, 156.84, 139.40, 128.89, 128.71, 126.53, 91.72, 66.68, 44.50, 43.00, 36.13, 10.79, 3.63. HRMS [C₂₁H₂₇N₅O₂ + H]⁺ : 382.2238 calculated, 382.2241 found (Δ = 0.89 ppm).

### 2-(Benzyl(methyl)amino)-N-(cyclopropylmethyl)-6-morpholinopyrimidine-4-carboxamide (Example 48)

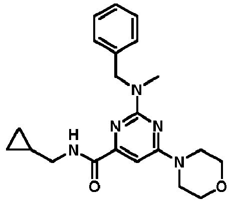

The title compound was prepared according to the general procedure E using 2-chloropyrimidine **I-35** (59 mg, 0.20 mmol, 1 eq), *N*-methylbenzylamine (38 µL, 0.30 mmol, 1.5 eq) and DiPEA (140 µL, 0.80 mmol, 4 eq). Total heating time: 8 h at 160 °C with µW irradiation. Column chromatography (2% -> 5% MeOH/DCM) afforded the product (40 mg, 0.10 mmol, 52%). TLC: R_{f} = 0.5 (4% MeOH/DCM). ¹H NMR (500 MHz, CDCl₃) δ 7.96 (br s, 1H), 7.34 - 7.28 (m, 2H), 7.28 - 7.21 (m, 3H), 6.75 (s, 1H), 4.85 (s, 2H), 3.82 - 3.69 (m, 4H), 3.69 - 3.54 (m, 4H), 3.26 (t, *J* = 6.5 Hz, 2H), 3.15 (s, 3H), 1.10 - 0.95 (m, 1H), 0.56 - 0.44 (m, 2H), 0.29 - 0.17 (m, 2H). ¹³C NMR (101 MHz, CDCl₃) δ 164.58, 163.97, 161.35, 156.85, 139.11, 128.56, 127.34, 127.03, 90.41, 66.68, 52.76, 44.47, 44.00, 35.18, 10.85, 3.43. HRMS [C₂₁H₂₇N₅O₂ + H]⁺ : 382.2238 calculated, 382.2241 found (Δ = 0.86 ppm).

### N-Methylphenethylamine hydrobromide (I-36)

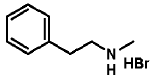

A round bottom flask was charged with methylamine (33 wt% in ethanol, 8.83 mL, 73.0 mmol, 10 eq) and cooled to 0 °C. (2-bromoethyl)benzene (1.00 mL, 7.3 mmol, 1 eq) was added and the reaction was stirred and allowed to warm up to room temperature. After 40 h the reaction showed complete conversion on TLC and the solvents were concentrated under reduced pressure. The product was obtained as a mixture with the di-substituted byproduct, *N*-methyl-*N*-phenethyl-2-phenylethan-1-amine hydrobromide (9:1) and used without further purification (1.4 g, 6.6 mmol, 90%). TLC: R_{f} = 0.35 (6% MeOH/DCM). ¹H NMR (400 MHz, MeOD) δ 7.52 - 6.99 (m, 5H), 3.42 - 3.10 (m, 2H), 3.10 - 2.85 (m, 2H), 2.67 (s, 3H). ¹³C NMR (101 MHz, MeOD) δ 137.75, 129.86, 129.78, 128.10, 51.50, 33.92, 33.31.

### N-(Cyclopropylmethyl)-2-(methyl(phenethyl)amino)-6-morpholinopyrimidine-4-carboxamide (Example 49)

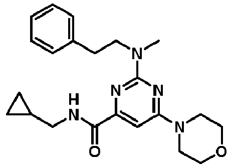

The title compound was prepared according to the general procedure E using 2-chloropyrimidine **I-35** (59 mg, 0.20 mmol, 1 eq), *N*-methylphenethylamine hydrobromide (66 µL, 0.30 mmol, 1.5 eq) and DiPEA (140 µL, 0.80 mmol, 4 eq). Total heating time: 8 h at 160 °C with µW irradiation. Column chromatography (40% -> 60% EtOAc/pentane) afforded the product (40 mg, 0.10 mmol, 52%). TLC: R_{f} = 0.3 (40% EtOAc/pentane). ¹H NMR (400 MHz, CDCl₃) δ 8.03 (br s, 1H), 7.34 - 7.25 (m, 2H), 7.25 - 7.12 (m, 3H), 6.72 (s, 1H), 3.88 - 3.72 (m, 6H), 3.72 - 3.55 (m, 4H), 3.30 (t, *J* = 6.5 Hz, 2H), 3.13 (s, 3H), 2.90 (t, *J* = 7.7 Hz, 2H), 1.14 - 0.99 (m, 1H), 0.64 - 0.44 (m, 2H), 0.38 - 0.19 (m, 2H). ¹³C NMR (101 MHz, CDCl₃) δ 164.66, 163.97, 160.86, 156.78, 139.92, 128.95, 128.58, 126.29, 90.08, 66.74, 51.68, 44.50, 44.11, 35.70, 33.93, 10.88, 3.48. HRMS [C₂₂H₂₉N₅O₂+H]⁺ : 396.2394 calculated, 396.2387 found (Δ = -1.77 ppm).

### N-(Cyclopropylmethyl)-2-(methyl(2-(thiophen-2-yl)ethyl)amino)-6-morpholino pyrimidine-4-carboxamide (Example 22)

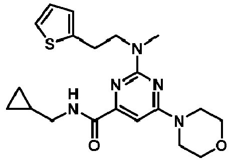

The title compound was prepared according to General Procedure E using 2-chloropyrimidine **I-35** (30 mg, 0.10 mmol, 1 eq), DiPEA (52 µL, 0.30 mmol, 3 eq) and N-methyl-2-thiopheneethylamine (18 µL, 0.13 mmol, 1.3 eq). Total heating time: 4 h at 160 °C with µW irradiation. Column chromatography (40% -> 60% EtOAc/pentane) afforded the product (16 mg, 40 µmol, 40%). TLC: R_{f} = 0.4 (50% EtOAc/pentane). ¹H NMR (400 MHz, CDCl₃) δ 8.04 (t, *J* = 5.3 Hz, 1H), 7.15 (dd, *J* = 5.1, 1.1 Hz, 1H), 6.94 (dd, *J* = 5.1, 3.4 Hz, 1H), 6.83 (d, *J* = 2.9 Hz, 1H), 6.73 (s, 1H), 3.88 - 3.80 (m, 2H), 3.80 - 3.72 (m, 4H), 3.71 - 3.62 (m, 4H), 3.33 - 3.26 (m, 2H), 3.20 - 3.08 (m, 5H), 1.13 - 0.99 (m, 1H), 0.60 - 0.48 (m, 2H), 0.28 (q, *J* = 4.7 Hz, 2H). ¹³C NMR (101 MHz, CDCl₃) δ 164.62, 163.97, 160.84, 156.81, 142.18, 127.06, 125.03, 123.68, 90.26, 66.75, 51.90, 44.51, 44.13, 35.75, 27.94, 10.91, 3.55. HRMS [C₂₀H₂₇N₅O₂S + H]⁺ : 402.1958 calculated, 402.1956 found (Δ = -0.55 ppm).

### N-(Cyclopropylmethyl)-6-morpholino-2-(2-phenylmorpholino)pyrimidine-4-carboxamide (Example 37)

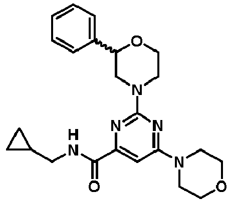

The title compound was prepared according to General Procedure E using 2-chloropyrimidine **I-35** (30 mg, 0.10 mmol, 1 eq), DiPEA (52 µL, 0.30 mmol, 3 eq) and (±)-2-phenylmorpholine (21 µL, 0.13 mmol, 1.3 eq). Total heating time: 4 h at 160 °C with µW irradiation. Column chromatography (40% -> 60% EtOAc/pentane) afforded the product (37 mg, 87 µmol, 87%). TLC: R_{f} = 0.4 (50% EtOAc/pentane). ¹H NMR (400 MHz, CDCl₃) δ 7.92 (br s, 1H), 7.50 - 7.31 (m, 5H), 6.80 (s, 1H), 4.66 (d, *J* = 13.3 Hz, 1H), 4.59 - 4.48 (m, 2H), 4.20 - 4.13 (m, 1H), 3.80 (td, *J* = 11.8, 2.8 Hz, 1H), 3.77 - 3.70 (m, 4H), 3.70 - 3.59 (m, 4H), 3.36 - 3.22 (m, 2H), 3.22 - 3.12 (m, 1H), 2.97 (dd, *J* = 13.3, 10.6 Hz, 1H), 1.12 - 0.99 (m, 1H), 0.60 - 0.46 (m, 2H), 0.28 (q, *J* = 4.7 Hz, 2H). ¹³C NMR (101 MHz, CDCl₃) δ 164.34, 163.92, 160.97, 156.85, 139.91, 128.62, 128.23, 126.51, 91.50, 78.26, 67.02, 66.67, 50.63, 44.47, 44.11, 43.99, 10.94, 3.53. HRMS [C₂₃H₂₉N₅O₃ + H]⁺ : 424.2343 calculated, 424.2340 found (Δ = -0.75 ppm).

### (S)-N-(Cyclopropylmethyl)-6-morpholino-2-(3-phenylpiperidin-1-yl)pyrimidine-4-carboxamide (Example 46)

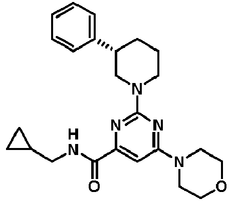

The title compound was prepared according to General Procedure E using 2-chloropyrimidine **I-35** (30 mg, 0.10 mmol, 1 eq), DiPEA (52 µL, 0.30 mmol, 3 eq) and (S)-3-phenylpiperidine (21 mg, 0.13 mmol, 1.3 eq). Total heating time: 4 h at 160 °C with µW irradiation. Column chromatography (40% -> 60% EtOAc/pentane) afforded the product (41 mg, 97 µmol, 97%). TLC: R_{f} = 0.6 (50% EtOAc/pentane). ¹H NMR (500 MHz, CDCl₃) δ 7.97 (br s, 1H), 7.40 - 7.32 (m, 2H), 7.32 - 7.21 (m, 3H), 6.73 (s, 1H), 4.90 - 4.70 (m, 2H), 3.83 - 3.70 (m, 4H), 3.69 - 3.56 (m, 4H), 3.36 - 3.19 (m, 2H), 2.97 - 2.83 (m, 2H), 2.76 (tt, *J* = 11.5, 3.6 Hz, 1H), 2.13 - 2.02 (m, 1H), 1.90 - 1.72 (m, 2H), 1.72 - 1.59 (m, 1H), 1.12 - 0.98 (m, 1H), 0.59 - 0.45 (m, 2H), 0.27 (q, *J* = 4.7 Hz, 2H). ¹³C NMR (126 MHz, CDCl₃) δ 164.63, 164.11, 160.97, 156.96, 144.30, 128.66, 127.30, 126.67, 90.60, 66.73, 51.27, 44.71, 44.52, 44.06, 42.64, 32.28, 25.57, 10.94, 3.50, 3.48. HRMS [C₂₄H₃₁N₅O₂ + H]⁺ : 422.2551 calculated, 422.2549 found (Δ = -0.36 ppm).

### N-(Cyclopropylmethyl)-6-morpholino-2-(3-phenylpiperidin-1-yl)pyrimidine-4-carboxamide (Example 52)

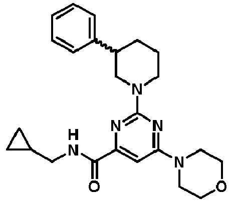

The title compound was prepared according to the general procedure E using 2-chloropyrimidine **I-35** (30 mg, 0.10 mmol, 1 eq), (±)-3-phenylpiperidine (24 µL, 0.15 mmol, 1.5 eq) and DiPEA (70 µL, 0.40 mmol, 4 eq). Total heating time: 4 h at 160 °C with µW irradiation. Column chromatography (40% -> 70% EtOAc/pentane) afforded the product (37 mg, 90 µmol, 88%). TLC: R_{f} = 0.3 (40% EtOAc/pentane). ¹H NMR (500 MHz, CDCl₃) δ 7.97 (t, *J* = 5.9 Hz, 1H), 7.35 (t, *J* = 7.5 Hz, 2H), 7.32 - 7.22 (m, 3H), 6.73 (s, 1H), 4.89 - 4.70 (m, 2H), 3.74 (t, *J* = 4.8 Hz, 4H), 3.69 - 3.56 (m, 4H), 3.37 - 3.16 (m, 2H), 2.97 - 2.83 (m, 2H), 2.76 (tt, *J* = 11.5, 3.7 Hz, 1H), 2.12 - 2.02 (m, 1H), 1.91 - 1.83 (m, 1H), 1.82 - 1.72 (m, 1H), 1.72 - 1.57 (m, 1H), 1.11 - 0.99 (m, 1H), 0.61 - 0.43 (m, 2H), 0.27 (q, *J* = 5.1 Hz, 2H). ¹³C NMR (101 MHz, CDCl₃) δ 164.60, 164.07, 160.94, 156.93, 144.28, 128.65, 127.30, 126.66, 90.57, 66.71, 51.24, 44.67, 44.48, 44.04, 42.63, 32.27, 25.56, 10.94, 3.50, 3.48. HRMS [C₂₄H₃₁N₅O₂ + H]⁺ : 422.2551 calculated, 422.2548 found (Δ = -0.52 ppm).

### N-(Cyclopropylmethyl)-6-morpholino-2-(3-phenylpyrrolidin-1-yl)pyrimidine-4-carboxamide (Example 51)

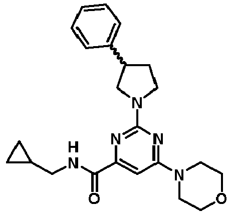

The title compound was prepared according to the general procedure E using 2-chloropyrimidine **I-35** (30 mg, 0.10 mmol, 1 eq), (±)-3-phenylpyrrolidine (22 µL, 0.15 mmol, 1.5 eq) and DiPEA (70 µL, 0.40 mmol, 4 eq). Total heating time: 4 h at 160 °C with µW irradiation. Column chromatography (30% -> 70% EtOAc/pentane) afforded the product (39 mg, 90 µmol, 98%). TLC: R_{f} = 0.4 (40% EtOAc/pentane). ¹H NMR (500 MHz, CDCl₃) δ 8.09 (br s, 1H), 7.40 - 7.29 (m, 4H), 7.29 - 7.24 (m, 1H), 6.75 (s, 1H), 4.18 - 4.03 (m, 1H), 3.88 (t, *J* = 9.5 Hz, 1H), 3.79 - 3.71 (m, 4H), 3.71 - 3.59 (m, 5H), 3.58 - 3.51 (m, 1H), 3.51 - 3.43 (m, 1H), 3.35 - 3.20 (m, 2H), 2.43 - 2.33 (m, 1H), 2.18 - 2.06 (m, 1H), 1.13 - 0.97 (m, 1H), 0.61 - 0.44 (m, 2H), 0.34 - 0.20 (m, 2H). ¹³C NMR (101 MHz, CDCl₃) δ 164.65, 163.89, 159.58, 156.77, 142.22, 128.70, 127.29, 126.84, 90.15, 66.73, 53.27, 46.49, 44.42, 44.19, 44.02, 33.28, 10.90, 3.49. HRMS [C₂₃H₂₉N₅O₂ + H]⁺ : 408.2394 calculated, 408.2391 found (Δ = -0.83 ppm).

### 2-(2-Benzylpiperidin-1-yl)-N-(cyclopropylmethyl)-6-morpholinopyrimidine-4-carboxamide (Example 54)

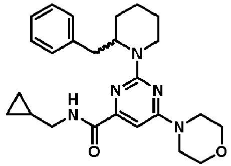

The title compound was prepared according to the general procedure E using 2-chloropyrimidine **I-35** (30 mg, 0.10 mmol, 1 eq), (±)-2-benzylpiperidine hydrochloride (52 mg, 0.24 mmol, 2.4 eq) and DiPEA (104 µL, 0.60 mmol, 6 eq). Total heating time: 6 d at 120 °C. Column chromatography (30% -> 60% EtOAc/pentane) afforded the product (12 mg, 30 µmol, 27%). TLC: R_{f} = 0.4 (50% EtOAc/pentane). ¹H NMR (600 MHz, CDCl₃) δ 7.98 (t, *J* = 5.2 Hz, 1H), 7.26 (t, *J* = 7.4 Hz, 2H), 7.24 - 7.16 (m, 3H), 6.70 (s, 1H), 5.13 - 4.97 (m, 1H), 4.77 - 4.60 (m, 1H), 3.82 - 3.73 (m, 4H), 3.66 (br s, 4H), 3.40 - 3.21 (m, 2H), 3.07 - 2.98 (m, 1H), 2.95 (dd, *J* = 13.1, 10.0 Hz, 1H), 2.81 (dd, *J* = 13.1, 5.1 Hz, 1H), 1.85 - 1.73 (m, 2H), 1.73 - 1.67 (m, 2H), 1.57 - 1.46 (m, 2H), 1.13 - 1.04 (m, 1H), 0.63 - 0.53 (m, 2H), 0.31 (q, *J* = 4.7 Hz, 2H). ¹³C NMR (151 MHz, CDCl₃) δ 164.75, 163.97, 160.84, 156.81, 140.16, 129.26, 128.47, 126.16, 90.34, 66.78, 52.27, 44.53, 44.24, 39.47, 35.32, 26.29, 25.82, 19.30, 10.95, 3.65, 3.61. HRMS [C₂₅H₃₃N₅O₂ + H]⁺ : 436.2707 calculated, 436.2706 found (Δ = -0.30 ppm).

### N-(Cyclopropylmethyl)-2-(2-(4-methoxybenzyl)piperidin-1-yl)-6-morpholinopyrimidine-4-carboxamide (Example 73)

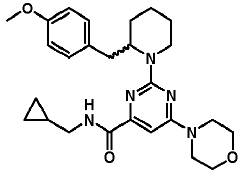

The title compound was prepared according to the general procedure E using 2-chloropyrimidine **I-35** (30 mg, 0.10 mmol, 1 eq), (±)-2-(4-methoxybenzyl)piperidine (46 mg, 0.22 mmol, 2.2 eq) and DiPEA (70 µL, 0.40 mmol, 4 eq). Total heating time: 28 h at 160 °C with µW irradiation. Purification by HPLC (C18 reverse phase, 5% -> 90% ACN/H₂O + 0.2% TFA, RT 9.3 min) afforded the product (13 mg, 29 µmol, 30%). TLC: R_{f} = 0.3 (40% EtOAc/pentane). ¹H NMR (400 MHz, CDCl₃) δ 7.98 (t, *J* = 5.8 Hz, 1H), 7.16 - 7.10 (m, 2H), 6.85 - 6.75 (m, 2H), 6.69 (s, 1H), 5.00 (dt, *J* = 10.4, 4.9 Hz, 1H), 4.67 (dd, *J* = 13.5, 3.7 Hz, 1H), 3.84 - 3.72 (m, 7H), 3.65 (t, *J* = 4.8 Hz, 4H), 3.40 - 3.21 (m, 2H), 3.00 (td, *J* = 13.2, 2.8 Hz, 1H), 2.90 (dd, *J* = 13.2, 10.0 Hz, 1H), 2.74 (dd, *J* = 13.2, 5.1 Hz, 1H), 1.85 - 1.71 (m, 2H), 1.65 (s, 2H), 1.59 - 1.42 (m, 2H), 1.15 - 1.01 (m, 1H), 0.64 - 0.50 (m, 2H), 0.36 - 0.25 (m, 2H). ¹³C NMR (101 MHz, CDCl₃) δ 164.78, 164.00, 160.88, 158.03, 156.84, 132.18, 130.15, 113.89, 90.31, 66.79, 55.40, 52.38, 44.54, 44.23, 39.47, 34.37, 26.18, 25.83, 19.29, 10.97, 3.65, 3.61. HRMS [C₂₅H₃₅N₅O₃ + H]⁺ : 466.2813 calculated, 466.2809 found (Δ = -0.71 ppm).

### 2-(2-Benzylpyrrolidin-1-yl)-N-(cyclopropylmethyl)-6-morpholinopyrimidine-4-carboxamide (Example 59)

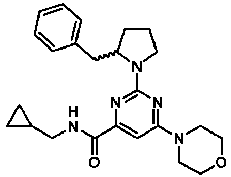

The title compound was prepared according to the general procedure E using 2-chloropyrimidine **I-35** (30 mg, 0.10 mmol, 1 eq), (±)-2-benzylpyrrolidine (24 µL, 0.15 mmol, 1.5 eq) and DiPEA (70 µL, 0.40 mmol, 4 eq). Total heating time: 3 d at 120 °C. Column chromatography (40% -> 60% EtOAc/pentane) afforded the product (42 mg, 0.10 mmol, 99%). TLC: R_{f} = 0.5 (50% EtOAc/pentane). ¹H NMR (400 MHz, CDCl₃) δ 8.11 (t, *J* = 5.8 Hz, 1H), 7.35 - 7.25 (m, 2H), 7.26 - 7.18 (m, 3H), 6.76 (s, 1H), 4.48 - 4.34 (m, 1H), 3.80 - 3.73 (m, 4H), 3.73 - 3.65 (m, 4H), 3.65 - 3.59 (m, 1H), 3.59 - 3.50 (m, 1H), 3.37 - 3.24 (m, 3H), 2.59 (dd, *J* = 13.1, 9.7 Hz, 1H), 1.91 - 1.82 (m, 4H), 1.14 - 0.98 (m, 1H), 0.65 - 0.46 (m, 2H), 0.37 - 0.21 (m, 2H). ¹³C NMR (101 MHz, CDCl₃) δ 164.71, 164.02, 159.56, 156.78, 139.83, 129.39, 128.49, 126.24, 90.22, 66.76, 59.06, 47.38, 44.54, 44.18, 39.30, 29.42, 23.16, 10.87, 3.51. HRMS [C₂₄H₃₁N₅O₂ + H]⁺ : 422.2551 calculated, 422.2549 found (Δ = -0.26 ppm).

### N-(Cyclopropylmethyl)-2-(3,4-dihydroisoquinolin-2(1H)-yl)-6-morpholinopyrimidine-4-carboxamide (Example 63)

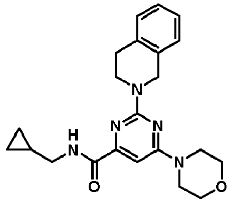

The title compound was prepared according to the general procedure E using 2-chloropyrimidine **I-35** (30 mg, 0.10 mmol, 1 eq), 1,2,3,4-tetrahydroisoquinoline (19 µL, 0.15 mmol, 1.5 eq) and DiPEA (70 µL, 0.40 mmol, 4 eq). Total heating time: 18 h at 120 °C. Column chromatography (20% -> 60% EtOAc/pentane) afforded the product (34 mg, 90 µmol, 93%). TLC: R_{f} = 0.5 (50% EtOAc/pentane). ¹H NMR (400 MHz, CDCl₃) δ 8.04 (t, *J* = 6.0 Hz, 1H), 7.25 - 7.13 (m, 4H), 6.76 (s, 1H), 4.90 (s, 2H), 4.04 (t, *J* = 5.9 Hz, 2H), 3.81 - 3.62 (m, 8H), 3.36 - 3.28 (m, 2H), 2.93 (t, *J* = 5.8 Hz, 2H), 1.16 - 1.01 (m, 1H), 0.61 - 0.51 (m, 2H), 0.31 (dt, *J* = 6.1, 4.6 Hz, 2H). ¹³C NMR (101 MHz, CDCl₃) δ 164.61, 163.99, 160.78, 156.88, 135.40, 134.49, 128.77, 126.58, 126.40, 126.23, 90.80, 66.76, 46.46, 44.54, 44.10, 41.63, 29.14, 10.99, 3.54. HRMS [C₂₂H₂₇N₅O₂ + H]⁺ : 394.2238 calculated, 394.2231 found (Δ = -1.67 ppm).

### N-(Cyclopropylmethyl)-2-(methyl(2-(pyridin-3-yl)ethyl)amino)-6-morpholinopyrimidine-4-carboxamide (Example 80)

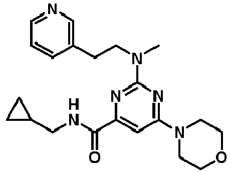

The title compound was prepared according to the general procedure E using 2-chloropyrimidine **I-35** (30 mg, 0.10 mmol, 1 eq), *N*-methyl-2-(pyridin-3-yl)ethan-1-amine (21 µL, 0.15 mmol, 1.5 eq) and DiPEA (70 µL, 0.40 mmol, 4 eq). Total heating time: 17 h at 120 °C. Column chromatography (2% -> 6% MeOH/DCM) afforded the product (11 mg, 29 µmol, 29%). TLC: R_{f} = 0.15 (4% MeOH/DCM). ¹H NMR (400 MHz, CDCl₃) δ 8.65 - 8.28 (m, 2H), 7.95 (br s, 1H), 7.51 (d, *J* = 7.9 Hz, 1H), 7.21 (dd, *J* = 7.9, 4.8 Hz, 1H), 6.72 (s, 1H), 3.89 - 3.69 (m, 6H), 3.69 - 3.53 (m, 4H), 3.29 (t, *J* = 6.4 Hz, 2H), 3.11 (s, 3H), 2.92 (t, *J* = 7.4 Hz, 2H), 1.12 - 0.99 (m, 1H), 0.66 - 0.45 (m, 2H), 0.36 - 0.19 (m, 2H). ¹³C NMR (101 MHz, CDCl₃) δ 164.57, 163.95, 160.86, 156.77, 150.27, 147.86, 136.33, 135.30, 123.50, 90.36, 66.74, 51.19, 44.49, 44.15, 35.84, 31.23, 10.91, 3.51. HRMS [C₂₁H₂₈N₆O₂ + H]⁺ : 397.2347 calculated, 397.2345 (found (Δ = -0.38 ppm).

### N-(Cyclopropylmethyl)-2-(methyl(2-(pyridin-4-yl)ethyl)amino)-6-morpholinopyrimidine-4-carboxamide (Example 81)

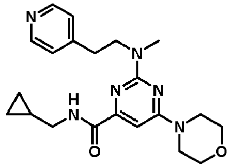

The title compound was prepared according to the general procedure E using 2-chloropyrimidine **I-35** (30 mg, 0.10 mmol, 1 eq), *N*-methyl-2-(pyridin-4-yl)ethan-1-amine (21 µL, 0.15 mmol, 1.5 eq) and DiPEA (70 µL, 0.40 mmol, 4 eq). Total heating time: 17 h at 120 °C. Column chromatography (2% -> 6% MeOH/DCM) afforded the product (8 mg, 20 µmol, 21%). TLC: R_{f} = 0.2 (4% MeOH/DCM). ¹H NMR (400 MHz, CDCl₃) δ 8.51 (d, *J* = 5.7 Hz, 2H), 7.96 (br s, 1H), 7.15 (d, *J* = 5.7 Hz, 2H), 6.74 (s, 1H), 3.90 - 3.80 (m, 2H), 3.80 - 3.72 (m, 4H), 3.72 - 3.53 (m, 4H), 3.36 - 3.24 (m, 2H), 3.11 (s, 3H), 2.98 - 2.85 (m, 2H), 1.12 - 0.99 (m, 1H), 0.64 - 0.47 (m, 2H), 0.28 (q, *J* = 4.7 Hz, 2H). ¹³C NMR (101 MHz, CDCl₃) δ 164.54, 163.96, 160.83, 156.80, 149.96, 148.99, 124.32, 90.46, 66.74, 50.59, 44.50, 44.13, 35.87, 33.44, 10.91, 3.51. HRMS [C₂₁H₂₈N₆O₂ + H]⁺ : 397.2347 calculated, 397.2345 (found (Δ = -0.38 ppm).

### N-(Cyclopropylmethyl)-2-(methyl(2-(pyridin-2-yl)ethyl)amino)-6-morpholinopyrimidine-4-carboxamide (Example 84)

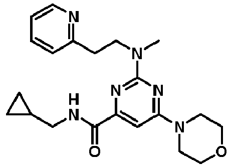

The title compound was prepared according to the general procedure E using 2-chloropyrimidine **I-35** (30 mg, 0.10 mmol, 1 eq), *N*-methyl-2-(pyridin-2-yl)ethan-1-amine (21 µL, 0.15 mmol, 1.5 eq) and DiPEA (70 µL, 0.40 mmol, 4 eq). Total heating time: 8 h at 160 °C with µW irradiation. Column chromatography (3% -> 4% MeOH/DCM) afforded the product (26 mg, 66 µmol, 66%). TLC: R_{f} = 0.3 (3% MeOH/DCM). ¹H NMR (400 MHz, CDCl₃) δ 8.66 - 8.44 (m, 1H), 8.18 (br s, 1H), 7.58 (td, *J* = 7.6, 1.7 Hz, 1H), 7.21 - 7.03 (m, 2H), 6.72 (s, 1H), 3.99 (t, *J* = 7.2 Hz, 2H), 3.88 - 3.71 (m, 4H), 3.71 - 3.54 (m, 4H), 3.31 (t, *J* = 6.4 Hz, 2H), 3.21 - 3.03 (m, 5H), 1.16 - 1.00 (m, 1H), 0.62 - 0.43 (m, 2H), 0.29 (q, *J* = 4.8 Hz, 2H). ¹³C NMR (101 MHz, CDCl₃) δ 164.62, 163.91, 160.79, 159.86, 156.60, 149.21, 136.68, 123.60, 121.48, 90.12, 66.72, 49.98, 44.48, 44.01, 36.15, 35.62, 10.94, 3.45. HRMS [C₂₁H₂₈N₆O₂ + H]⁺ : 397.2347 calculated, 397.2345 (found (Δ = -0.38 ppm).

### N-(Cyclopropylmethyl)-6-(methyl(phenethyl)amino)-2-morpholinopyrimidine-4-carboxamide (Example 55)

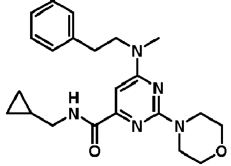

The title compound was prepared according to the general procedure E using 4-chloropyrimidine **I-35B** (21 mg, 70 µmol, 1.0 eq), *N*-methylphenethylamine hydrobromide (16 mg, 70 µmol, 1 eq) and DiPEA (36.6 µL, 0.21 mmol, 3 eq) in MeOH. Total heating time: 6 h at 70 °C. Column chromatography (30% -> 60% EtOAc/pentane) afforded the product (20 mg, 50 µmol, 71%). TLC: R_{f} = 0.4 (30% EtOAc/pentane). ¹H NMR (400 MHz, CDCl₃) δ 8.44 (br s, 1H), 7.33 - 7.27 (m, 2H), 7.25 - 7.13 (m, 3H), 6.84 (s, 1H), 3.79 (br s, 10H), 3.38 - 3.22 (m, 2H), 3.02 (s, 3H), 2.90 (t, *J* = 7.4 Hz, 2H), 1.14 - 1.00 (m, 1H), 0.62 - 0.46 (m, 2H), 0.36 - 0.21 (m, 2H). ¹³C NMR (101 MHz, CDCl₃) δ 162.97, 159.05, 128.91, 128.82, 126.75, 93.22, 66.81, 44.92, 44.69, 10.71, 3.65. HRMS [C₂₂H₂₉N₅O₂ + H]⁺ : 396.2394 calculated, 396.2385 found (Δ = -1.77 ppm).

### N-Phenethylpropan-2-amine (I-37)

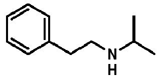

A round bottom flask was charged with NaBH₄ (218 mg, 5.76 mmol, 1.9 eq) and dry DCM (5 mL). The solution was stirred in an ice bath for 3 minutes and glacial acetic acid (1 mL, 14.4 mmol, 5.8 eq) was added. The mixture was stirred for 1 h at 0 °C and 30 minutes at room temperature. Separately, a solution of 2-phenethylamine (377 µL, 3 mmol, 1 eq) was prepared in dry DCM (2.5 mL) and acetone (198 µL, 2.7 mmol, 0.9 eq). This solution was added dropwise to the NaBH(OAc)₃ mixture and was stirred at rt overnight. After 20 hours the reaction mixture was acidified to pH 2 with 0.1 M HCl (aq.) and washed with DCM (2 x 30 mL) to remove by-products. Then, the solution is basified with 1 M NaOH (aq.) until pH 10 and extracted with DCM (3 x 50 mL). The combined organic layers were dried (Na₂SO₄), filtered, concentrated under reduced pressure and the residue was purified by silica gel column chromatography (isocratic, 0.5% Et₃N/EtOAc) to provide the product (277 mg, 1.7 mmol, 63%). TLC: R_{f} = 0.3 (100% EtOAc with 3 drops of Et₃N). ¹H NMR (400 MHz, CDCl₃) δ 7.30 - 7.23 (m, 2H), 7.22 - 7.14 (m, 3H), 2.88 - 2.82 (m, 2H), 2.81 - 2.74 (m, 3H), 1.08 (br s, 1H), 1.03 (d, *J* = 6.4 Hz, 6H). ¹³C NMR (101 MHz, CDCl₃) δ 139.98, 128.52, 128.28, 125.95, 48.70, 48.38, 36.48, 22.80. HRMS [C₁₁H₁₆N + H]⁺ : 164.1434 calculated, 164.1434 found (Δ = -0.18 ppm).

### N-(cyclopropylmethyl)-2-(isopropyl(phenethyl)amino)-6-morpholinopyrimidine-4-carboxamide (Example 50)

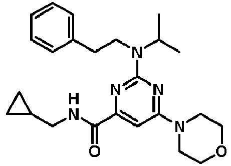

The title compound was prepared according to the general procedure E using 2-chloropyrimidine **I-35** (59 mg, 0.20 mmol, 1 eq), amine **I-37** (50 mg, 0.30 mmol, 1.5 eq) and DiPEA (140 µL, 0.80 mmol, 4 eq). Total heating time: 12 h at 160 °C with µW irradiation. Column chromatography (50% -> 60% EtOAc/pentane) afforded the product (19 mg, 40 µmol, 22%). TLC: R_{f} = 0.4 (50% EtOAc/pentane). ¹H NMR (400 MHz, CDCl₃) δ 8.05 (br s, 1H), 7.37 - 7.29 (m, 2H), 7.29 - 7.20 (m, 3H), 6.75 (s, 1H), 4.96 (hept, *J =* 6.8 Hz, 1H), 3.82 - 3.74 (m, 4H), 3.72 - 3.64 (m, 4H), 3.64 - 3.55 (m, 2H), 3.35 - 3.26 (m, 2H), 3.01 - 2.88 (m, 2H), 1.24 (d, *J* = 6.8 Hz, 6H), 1.11 - 1.00 (m, 1H), 0.60 - 0.49 (m, 2H), 0.33 - 0.23 (m, 2H). ¹³C NMR (101 MHz, CDCl₃) δ 164.79, 164.06, 160.40, 156.81, 140.36, 128.68, 128.65, 126.35, 90.26, 66.77, 46.38, 44.57, 44.48, 44.17, 36.29, 20.65, 10.90, 3.51. HRMS [C₂₄H₃₃N₅O₂ + H]⁺ : 424.2707 calculated, 424.2705 found (Δ = -0.52 ppm).

### N-(Cyclopropylmethyl)-6-(dimethylamino)-2-(isopropyl(phenethyl)amino) pyrimidine-4-carboxamide (Example 85)

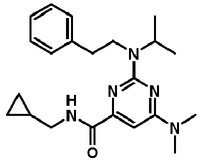

The title compound was prepared according to the general procedure E using 2-chloropyrimidine **I-19** (29 mg, 0.11 mmol, 1 eq), amine **I-37** (28 µL, 0.17 mmol, 1.5 eq) and DiPEA (76 µL, 0.44 mmol, 4 eq). Total heating time: 24 h at 160 °C with µW irradiation. Column chromatography (20% -> 50% EtOAc/pentane) afforded the product (6 mg, 16 µmol, 14%). TLC: R_{f} = 0.6 (40% EtOAc/pentane) and recovered starting material (10 mg, 40 µmol, 36%). ¹H NMR (500 MHz, CDCl₃) δ 8.09 (br s, 1H), 7.34 - 7.21 (m, 5H), 6.71 (s, 1H), 5.00 (hept, *J* = 6.7 Hz, 1H), 3.68 - 3.53 (m, 2H), 3.30 (dd, *J* = 6.9, 6.0 Hz, 2H), 3.14 (s, 6H), 3.02 - 2.90 (m, 2H), 1.24 (d, *J* = 6.8 Hz, 6H), 1.10 - 1.01 (m, 1H), 0.58 - 0.48 (m, 2H), 0.28 (q, *J* = 4.7 Hz, 2H). ¹³C NMR (126 MHz, CDCl₃) δ 165.14, 164.20, 160.40, 156.05, 140.60, 128.78, 128.64, 126.28, 90.36, 46.32, 44.61, 44.14, 37.36, 36.45, 20.70, 10.96, 3.51. HRMS [C₂₂H₃₀N₅O + H]⁺ : 382.2601 calculated, 382.2604 (found (Δ = 0.58 ppm).

### Diphenethylamine (I-38).

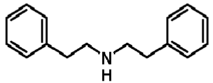

A round bottom flask was charged with phenylacetaldehyde (125 µL, 1 mmol, 1 eq), 2-phenethylamine (251 µL, 2 mmol, 2 eq) and dry DCM (5 mL). The solution was stirred for 10 minutes and then NaHB(OAc)₃ (425 mg, 2 mmol, 2 eq) and glacial AcOH (114 µL, 2 mmol, 2 eq) were added. After 19 h the reaction mixture was diluted with DCM (5 mL) washed with sat. aq. NaHCO₃ (1 x 10 mL), brine (1 x 10 mL), dried (Na₂SO₄), filtered and concentrated under reduced pressure. The residue was purified by silica gel column chromatography (isocratic, 5% MeOH/DCM with 0.5% Et₃N) affording the product (128 mg, 0.57 mmol, 57%). TLC: R_{f} = 0.3 (6% MeOH/DCM with 3 drops of Et₃N). ¹H NMR (400 MHz, CDCl₃) δ 7.73 - 6.67 (m, 10H), 3.05 - 2.61 (m, 8H), 1.96 (br s, 1H). ¹³C NMR (101 MHz, CDCl₃) δ 139.91, 128.67, 128.45, 126.12, 51.00, 36.25. HRMS [C₁₆H₁₉N + H]⁺ : 226.1590 calculated, 226.1591 found (Δ = 0.31 ppm).

### N-(Cyclopropylmethyl)-2-(diphenethylamino)-6-morpholinopyrimidine-4-carboxamide (Example 62)

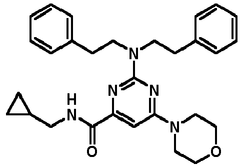

The title compound was prepared according to the general procedure E using 2-chloropyrimidine **I-35** (30 mg, 0.10 mmol, 1 eq), amine **I-38** (37 mg, 0.16 mmol, 1.6 eq) and DiPEA (70 µL, 0.40 mmol, 4 eq). Total heating time: 12h at 160 °C with µW irradiation. Column chromatography (20% -> 50% EtOAc/pentane) afforded the product (24 mg, 50 µmol, 50%). TLC: R_{f} = 0.5 (30% EtOAc/pentane). ¹H NMR (400 MHz, CDCl₃) δ 8.02 (t, *J* = 5.8 Hz, 1H), 7.34 - 7.26 (m, 4H), 7.24 - 7.15 (m, 6H), 6.75 (s, 1H), 3.83 - 3.60 (m, 12H), 3.30 (dd, *J* = 7.1, 5.8 Hz, 2H), 2.97 - 2.82 (m, 4H), 1.12 - 1.00 (m, 1H), 0.63 - 0.49 (m, 2H), 0.35 - 0.23 (m, 2H). ¹³C NMR (101 MHz, CDCl₃) δ 164.66, 164.08, 160.34, 156.84, 139.91, 128.82, 128.62, 126.35, 90.34, 66.75, 50.69, 44.57, 44.23, 34.58, 10.86, 3.52. HRMS [C₂₉H₃₅N₅O₂ + H]⁺ : 486.2864 calculated, 486.2861 found (Δ = -0.53 ppm).

### N-Benzyl-2-phenylethan-1-amine (I-39)

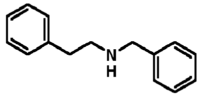

A round bottom flask was charged with phenylacetaldehyde (125 µL, 1 mmol, 1 eq), benzylamine (218 µL, 2 mmol, 2eq) and DCM (10 mL). The solution was stirred at room temperature for 3 minutes and then NaHB(OAc)₃ (424 mg, 2 mmol, 2 eq) and glacial AcOH (114 µL, 2 mmol, 2 eq) were added. After 18 h the reaction mixture was diluted with DCM (20 mL), washed with sat. aq. NaHCO₃ (1 x 30 mL), brine (1 x 30 mL), dried (Na₂SO₄), filtered and concentrated with reduced pressure. The residue was purified by silica gel column chromatography with (4% -> 5% MeOH/DCM with 0.5% Et₃N) affording the product (125 mg, 0.59 mmol, 59%). TLC: R_{f} = 0.4 (5% MeOH/DCM with 3 drops of Et₃N). ¹H NMR (400 MHz, CDCl₃) δ 7.31 - 7.21 (m, 7H), 7.21 - 7.17 (m, 3H), 3.77 (s, 2H), 2.91 - 2.85 (m, 2H), 2.84 - 2.78 (m, 2H), 1.97 (br s, 1H). ¹³C NMR (101 MHz, CDCl₃) δ 140.21, 140.04, 128.77, 128.51, 128.44, 128.15, 126.98, 126.20, 53.88, 50.57, 36.36.

### 2-(Benzyl(phenethyl)amino)-N-(cyclopropylmethyl)-6-morpholinopyrimidine-4-carboxamide (Example 65)

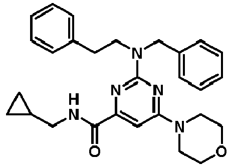

The title compound was prepared according to the general procedure E using 2-chloropyrimidine **I-35** (30 mg, 0.10 mmol, 1 eq), amine **I-39** (28 mg, 0.15 mmol, 1.5 eq) and DiPEA (44 µL, 0.25 mmol, 2.5 eq). Total heating time: 7 d at 120 °C. Column chromatography (30% -> 50% EtOAc/pentane) afforded the product (20 mg, 40 µmol, 48%). TLC: R_{f} = 0.6 (40% EtOAc/pentane). ¹H NMR (400 MHz, MeOD + CDCl₃) δ 7.36 - 7.04 (m, 10H), 6.67 (s, 1H), 4.74 (s, 2H), 3.91 - 3.50 (m, 10H), 3.19 (br s, 2H), 2.94 - 2.79 (m, 2H), 0.98 (br s, 1H), 0.50 (br s, 2H), 0.21 (br s, 2H). ¹³C NMR (101 MHz, CDCl₃) δ 164.60, 164.09, 160.81, 156.93, 139.90, 139.50, 128.82, 128.62, 128.57, 127.31, 127.04, 126.34, 90.66, 66.72, 51.39, 49.88, 44.56, 44.13, 34.22, 10.84, 3.47. HRMS [C₂₃H₃₂N₅O₂ + H]⁺ : 472.2707 calculated, 472.2704 found (Δ = -0.70 ppm).

### N-Phenethylcyclopropanamine (I-40)

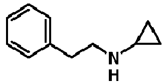

A round bottom flask was charged with phenylacetaldehyde (125 µL, 1 mmol, 1 eq), cyclopropanamine (139 µL, 2 mmol, 2 eq) and dry DCM (5 mL). The solution was stirred at room temperature for 10 minutes and then NaHB(OAc)₃ (425 mg, 2 mmol, 2 eq) and glacial AcOH (114 µL, 2 mmol, 2 eq) were added. After 21 h the reaction mixture was diluted with DCM (10 mL), washed with sat. aq. NaHCO₃ (1 x 15 mL), brine (1 x 15 mL), dried (Na₂SO₄), filtered and concentrated under reduced pressure. The residue was purified by silica gel column chromatography (30% -> 50% EtOAc/pentane with 0.5% Et₃N) affording the product (85 mg, 0.53 mmol, 53%), R_{f} = 0.1 (6% MeOH/DCM). ¹H NMR (400 MHz, CDCl₃) δ 7.42 - 7.07 (m, 5H), 3.01 - 2.93 (m, 2H), 2.80 (t, *J* = 7.2 Hz, 2H), 2.26 - 2.02 (m, 2H), 0.47 - 0.40 (m, 2H), 0.37 - 0.31 (m, 2H). ¹³C NMR (101 MHz, CDCl₃) δ 140.23, 128.78, 128.54, 126.19, 50.84, 36.37, 30.24, 6.33. HRMS [C₁₁H₁₅N + H]⁺ : 162.1277 calculated, 162.1277 found (Δ = -0.25 ppm).

### 2-(Cyclopropyl(phenethyl)amino)-N-(cyclopropylmethyl)-6-morpholinopyrimidine-4-carboxamide (Example 70)

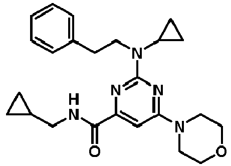

The title compound was prepared according to the general procedure E using 2-chloropyrimidine **I-35** (30 mg, 0.10 mmol, 1 eq), amine **I-40** (25 mg, 0.16 mmol, 1.6 eq) and DiPEA (115 µL, 0.60 mmol, 6 eq). Total heating time: 36 h at 160 °C with µW irradiation. Purification by HPLC (C18 reverse phase, 37% -> 47% ACN/H₂O +0.2% TFA) afforded the product (7 mg, 20 µmol, 20%). TLC: R_{f} = 0.4 (40% EtOAc/pentane). ¹H NMR (400 MHz, CDCl₃) δ 8.17 (s, 1H), 7.34 - 7.24 (m, 3H), 7.25 - 7.16 (m, 3H), 6.79 (s, 1H), 3.87 - 3.73 (m, 6H), 3.69 (t, *J* = 4.7 Hz, 4H), 3.30 (dd, *J* = 7.1, 5.7 Hz, 2H), 2.98 - 2.85 (m, 2H), 2.73 - 2.64 (m, 1H), 1.14 - 0.98 (m, 1H), 0.90 - 0.77 (m, 2H), 0.68 - 0.59 (m, 2H), 0.59 - 0.49 (m, 2H), 0.35 - 0.22 (m, 2H). ¹³C NMR (101 MHz, CDCl₃) δ 164.62, 163.99, 162.12, 156.64, 140.18, 128.88, 128.59, 126.31, 91.08, 66.78, 50.52, 44.57, 44.11, 34.58, 30.41, 10.86, 8.67, 3.42. HRMS [C₂₄H₃₁N₅O₂ + H]⁺ : 422.2551 calculated, 422.2549 found (Δ = -0.43 ppm).

### N-Ethyl-2-phenylethan-1-amine (I-41)

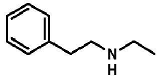

A round bottom flask was charged with acetaldehyde (56 µL, 1 mmol, 1 eq), 2-phenethylamine (189 µL, 1.5 mmol, 1.5 eq) and dry DCM (10 mL). The solution was stirred at room temperature for 10 minutes and then NaHB(OAc)₃ (424 mg, 2 mmol, 2 eq) was added. After 16 h the reaction was complete as judged by TLC. The mixture was diluted with DCM (20 mL), washed with sat. aq. NaHCO₃ (1 x 30 mL), brine (1 x 30 mL), dried (MgSO₄), filtered and concentrated under reduced pressure. The residue was purified by silica gel column chromatography (5% -> 7% MeOH/DCM with 0.5% Et₃N) affording the product (27 mg, 0.18 mmol, 18%). TLC: R_{f} = 0.3 (6% MeOH/DCM). ¹H NMR (400 MHz, CDCl₃) δ 7.34 - 7.24 (m, 2H), 7.24 - 7.16 (m, 3H), 2.96 - 2.77 (m, 4H), 2.68 (q, *J* = 7.1 Hz, 2H), 1.10 (t, *J* = 7.2 Hz, 3H). ¹³C NMR (101 MHz, CDCl₃) δ 140.14, 128.83, 128.59, 126.27, 51.11, 46.98, 44.09, 15.24. HRMS [C₁₀H₁₅N + H]⁺ : 150.1277 calculated, 150.1277 found (Δ = -0.47 ppm).

### N-(Cyclopropylmethyl)-2-(ethyl(phenethyl)amino)-6-morpholinopyrimidine-4-carboxamide (Example 72)

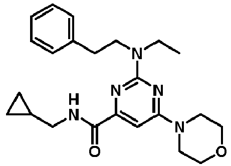

The title compound was prepared according to the general procedure E using 2-chloropyrimidine **I-35** (30 mg, 0.10 mmol, 1 eq), amine **I-41** (21 mg, 0.15 mmol, 1.5 eq) and DiPEA (70 µL, 0.40 mmol, 4 eq). Total heating time: 12 h at 160 °C with µW irradiation. Column chromatography (20% -> 50% EtOAc/pentane) afforded the product (15 mg, 40 µmol, 43%). TLC: R_{f} = 0.3 (30% EtOAc/pentane). ¹H NMR (400 MHz, CDCl₃) δ 8.04 (t, *J* = 5.9 Hz, 1H), 7.35 - 7.27 (m, 2H), 7.25 - 7.20 (m, 3H), 6.72 (s, 1H), 3.80 - 3.73 (m, 6H), 3.70 - 3.63 (m, 4H), 3.58 (q, *J* = 7.0 Hz, 2H), 3.30 (dd, *J* = 7.1, 5.8 Hz, 2H), 2.97 - 2.89 (m, 2H), 1.18 (t, *J* = 7.0 Hz, 3H), 1.11 - 1.01 (m, 1H), 0.59 - 0.51 (m, 2H), 0.31 - 0.25 (m, 2H). ¹³C NMR (101 MHz, CDCl₃) δ 164.74, 164.12, 160.29, 156.86, 140.06, 128.83, 128.62, 126.32, 90.06, 66.78, 49.83, 44.53, 44.09, 43.04, 34.76, 13.24, 10.90, 3.44. HRMS [C₂₃H₃₁N₅O₂ + H]⁺ : 410.2551 calculated, 410.2549 found (Δ = -0.49 ppm).

### N-Phenethylaniline (I-42)

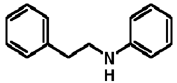

A round bottom flask with a stir bar under N₂ atmosphere was charged with phenylboronic acid (488 mg, 4 mmol, 2 eq), Cu(OAc)₂ x H₂O (40 mg, 0.2 mmol, 0.1 eq), powdered 4 Å molecular sieves (1.5 g) and dry dichloroethane (16 mL). The suspension was stirred for 5 minutes at room temperature followed by addition of 2-phenethylamine (252 µL, 2 mmol, 1 eq). The blue mixture was purged using a balloon of O₂ causing a colorshift to purple and stirred under an O₂ atmosphere for 26h. The mixture was then filtered through a plug of Celite and the filtrate concentrated under reduced pressure. The residue was purified by silica gel column chromatography (2% - > 8% EtOAc/pentane) affording the product (127 mg, 0.64 mmol, 32%). TLC: R_{f} = 0.6 (5% EtOAc/pentane). ¹H NMR (400 MHz, CDCl₃) δ 7.34 - 7.25 (m, 2H), 7.24 - 7.12 (m, 5H), 6.74 - 6.66 (m, 1H), 6.61 - 6.54 (m, 2H), 3.63 (br s, 1H), 3.36 (t, *J* = 7.1 Hz, 2H), 2.87 (t, *J* = 7.0 Hz, 2H). ¹³C NMR (101 MHz, CDCl₃) δ 148.06, 139.38, 129.35, 128.86, 128.67, 126.48, 117.50, 113.04, 45.08, 35.55.

### N-(Cyclopropylmethyl)-6-morpholino-2-(phenethyl(phenyl)amino)pyrimidine-4-carboxamide (Example 77)

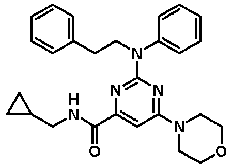

A microwave vial with a magnetic stir bar under N₂ was charged with 2-chloropyrimidine **I-35** (29 mg, 98 µmol, 1eq), amine **I-42** (24 mg, 0.12 mmol, 1.2 eq) and dry toluene (0.1 mL). The vial was capped and the solution purged with N₂. This was followed by the addition of RuPhosPd G3 (0.01 M THF solution, 100 µL, 1 µmol, 0.01 eq) and sodium *tert*-butoxide (2 M THF solution, 60 µL, 0.12 mmol, 1.2 eq) and the mixture was purged again with N₂ and stirred in a preheated oil bath at 110 °C. After 24 h the reaction was complete as judged by LC/MS. The mixture was filtered through a plug of Celite and the filtrate concentrated under reduced pressure to provide the crude material. Purification by HPLC (C18 reverse phase, 5% -> 50% ACN/H₂O + 0.2% TFA, RT 12.0 min) afforded the product (12 mg, 26 µmol, 27%). TLC: R_{f} = 0.4 (40% EtOAc/pentane). ¹H NMR (400 MHz, CDCl₃) δ 7.79 (t, *J* = 4.9 Hz, 1H), 7.43 - 7.33 (m, 2H), 7.32 - 7.17 (m, 8H), 6.79 (s, 1H), 4.28 - 4.11 (m, 2H), 3.79 - 3.67 (m, 4H), 3.60 (t, *J* = 4.8 Hz, 4H), 3.20 (dd, *J* = 7.1, 5.7 Hz, 2H), 3.09 - 2.96 (m, 2H), 1.02 - 0.89 (m, 1H), 0.55 - 0.41 (m, 2H), 0.27 - 0.12 (m, 2H). ¹³C NMR (101 MHz, CDCl₃) δ 164.24, 163.97, 160.50, 156.64, 144.67, 139.63, 128.84, 128.62, 127.72, 126.40, 125.73, 91.42, 66.71, 52.33, 44.51, 43.96, 34.64, 10.70, 3.34. HRMS [C₂₇H₃₁N₅O₂ + H]⁺ : 458.2551 calculated, 458.2547 (found (Δ = -0.72 ppm).

### Methyl (4-chlorophenethyl)carbamate (I-43)

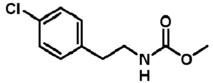

The title compound was prepared according to general procedure C using 2-(4-chlorophenyl)ethan-1-amine (70 µL, 0.50 mmol, 1 eq), methylchloroformate (58 µL, 0.75 mmol, 1.5 eq) and DiPEA (174 µL, 1.0 mmol, 2 eq). Column chromatography (10% -> 50% EtOAc/pentane) afforded the product (104 mg, 0.50 mmol, 99%). TLC: R_{f} = 0.7 (50% EtOAc/pentane). ¹H NMR (400 MHz, CDCl₃) δ 7.35 - 7.21 (m, 2H), 7.16 - 7.05 (m, 2H), 5.01 - 4.39 (m, 1H), 3.65 (s, 3H), 3.41 (q, *J* = 6.7 Hz, 2H), 2.78 (t, *J* = 7.0 Hz, 2H). ¹³C NMR (101 MHz, CDCl₃) δ 157.04, 137.32, 132.39, 130.22, 128.80, 52.20, 42.17, 35.62. HRMS [C₁₀H₁₂ClNO₂ + H]⁺ : 214.0629 calculated, 214.0631 found (Δ = 0.61 ppm).

### 2-(4-Chlorophenyl)-N-methylethan-1-amine (I-44).

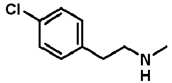

The title compound was prepared according to general procedure D using carbamate **I-43** (104 mg, 0.48 mmol, 1 eq), LiAlH₄ (2 M THF solution, 0.39 mL, 0.77 mmol, 1.6 eq). Column chromatography (isocratic, 5% MeOH/DCM + 0.5% Et₃N) afforded the product (36 mg, 0.21 mmol, 44%). TLC: R_{f} = 0.1 (2% MeOH/DCM with 3 drops of Et₃N). ¹H NMR (500 MHz, CDCl₃) δ 7.28 - 7.20 (m, 2H), 7.19 - 7.10 (m, 2H), 2.92 - 2.74 (m, 4H), 2.66 - 2.52 (m, 1H), 2.45 (s, 3H). ¹³C NMR (126 MHz, CDCl₃) δ 138.30, 132.03, 130.14, 128.66, 52.87, 36.12, 35.26. HRMS [C₉H₁₂ClN + H]⁺ : 170.0731 calculated, 170.0732 found (Δ = 0.53 ppm).

### 2-((4-Chlorophenethyl)(methyl)amino)-N-(cyclopropylmethyl)-6-morpholinopyrimidine-4-carboxamide (Example 57)

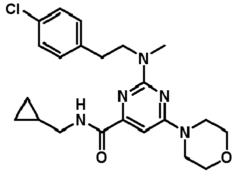

The title compound was prepared according to the general procedure E using using 2-chloropyrimidine **I-35** (30 mg, 0.10 mmol, 1 eq), amine **I-44** (34 mg, 0.20 mmol, 2 eq) and DiPEA (70 µL, 0.40 mmol, 4 eq). Total heating time: 25 h at 120 °C. Column chromatography (30% -> 70% EtOAc/pentane) afforded the product (10 mg, 30 µmol, 30%). TLC: R_{f} = 0.4 (50% EtOAc/pentane). ¹H NMR (400 MHz, CDCl₃) δ 7.98 (br s, 1H), 7.28 - 7.23 (m, 2H), 7.17 - 7.09 (m, 2H), 6.73 (s, 1H), 3.82 - 3.72 (m, 6H), 3.65 (t, *J =* 4.8 Hz, 4H), 3.35 - 3.24 (m, 2H), 3.09 (s, 3H), 2.94 - 2.82 (m, 2H), 1.12 - 1.01 (m, 1H), 0.60 - 0.49 (m, 2H), 0.36 - 0.23 (m, 2H). ¹³C NMR (101 MHz, CDCl₃) δ 164.64, 163.98, 156.81, 138.45, 132.12, 130.22, 128.72, 90.28, 66.76, 51.50, 44.53, 44.15, 35.86, 33.39, 10.91, 3.51. HRMS [C₂₂H₂₈ClN₅O₂ + H]⁺ : 430.2004 calculated, 430.2004 found (Δ = -0.16 ppm).

### Methyl (3-chlorophenethyl)carbamate (I-45)

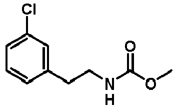

The title compound was prepared according to the general procedure C using 2-(3-chlorophenyl)ethan-1-amine (70 µL, 0.40 mmol, 1 eq), methylchloroformate (58 µL, 0.75 mmol, 1.5 eq) and DiPEA (174 µL, 2.0 mmol, 2 eq). Column chromatography (20% -> 40% EtOAc/pentane) afforded the product (67 mg, 0.31 mmol, 77%). TLC: R_{f} = 0.7 (30% EtOAc/pentane). ¹H NMR (400 MHz, CDCl₃) δ 7.25 - 7.15 (m, 3H), 7.11 - 7.03 (m, 1H), 4.89 (br s, 1H), 3.65 (s, 3H), 3.41 (q, *J* = 6.8 Hz, 2H), 2.78 (t, *J* = 7.1 Hz, 2H). ¹³C NMR (101 MHz, CDCl₃) δ 157.05, 140.94, 134.37, 129.90, 128.93, 127.04, 126.75, 52.15, 42.04, 35.90. HRMS [C₁₀H₁₂ClNO₂ + H]⁺ : 214.0629 calculated, 214.0630 found (Δ = 0.14 ppm).

### 2-(3-Chlorophenyl)-N-methylethan-1-amine (I-46)

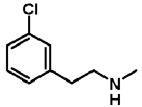

The title compound was prepared according to the general procedure D using carbamate **I-45** (67 mg, 0.31 mmol, 1 eq), LiAlH₄ (2 M THF solution, 0.250 mL, 0.50 mmol, 1.6 eq) and was used without further purification (33 mg, 0.14 mmol, 63%). R_{f} = 0.15 (6% MeOH/DCM). ¹H NMR (400 MHz, CDCl₃) δ 7.26 - 7.16 (m, 3H), 7.13 - 7.04 (m, 1H), 2.92 - 2.76 (m, 4H), 2.47 (s, 3H), 2.45 (br s, 1H). ¹³C NMR (101 MHz, CDCl₃) δ 141.75, 134.40, 129.90, 128.92, 127.06, 126.63, 52.69, 36.06, 35.51. HRMS [C₉H₁₂ClN + H]⁺ : 170.0731 calculated, 170.0730 found (Δ = -0.41 ppm).

### 2-((3-Chlorophenethyl)(methyl)amino)-N-(cyclopropylmethyl)-6-morpholinopyrimidine-4-carboxamide (Example 66)

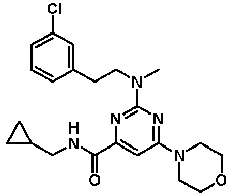

The title compound was prepared according to the general procedure E using 2-chloropyrimidine **I-35** (20 mg, 70 µmol, 1 eq), amine **I-46** (20 mg, 0.12 mmol, 1.5 eq) and DiPEA (49 µL, 0.28 mmol, 4 eq). Total heating time: 5 d at 120 °C. Column chromatography (30% -> 50% EtOAc/pentane) afforded the product (29 mg, 70 µmol, 87%). TLC: R_{f} = 0.6 (40% EtOAc/pentane). ¹H NMR (400 MHz, CDCl₃) δ 8.00 (t, *J* = 5.9 Hz, 1H), 7.25 - 7.14 (m, 3H), 7.14 - 7.02 (m, 1H), 6.73 (s, 1H), 3.86 - 3.72 (m, 6H), 3.66 (t, *J* = 4.9 Hz, 4H), 3.30 (dd, *J* = 7.1, 5.8 Hz, 2H), 3.12 (s, 3H), 2.93 - 2.83 (m, 2H), 1.12 - 1.01 (m, 1H), 0.60 - 0.50 (m, 2H), 0.33 - 0.24 (m, 2H). ¹³C NMR (101 MHz, CDCl₃) δ 164.62, 163.98, 160.86, 156.78, 141.98, 134.32, 129.82, 128.99, 127.06, 126.50, 90.27, 66.74, 51.34, 44.51, 44.12, 35.72, 33.68, 10.90, 3.48. HRMS [C₂₂H₂₈ClN₅O₂ + H]⁺ : 430.2004 calculated, 430.2004 found (Δ = -0.16 ppm).

### Methyl (2-chlorophenethyl)carbamate (I-47).

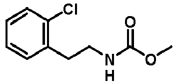

The title compound was prepared according to the general procedure C using 2-(2-chlorophenyl)ethan-1-amine (141 µL, 1.0 mmol, 1 eq), methylchloroformate (116 µL, 1.5 mmol, 1.5 eq) and DiPEA (348 µL, 2.0 mmol, 2 eq). Column chromatography (30% - > 50% EtOAc/pentane) afforded the product (183 mg, 0.85 mmol, 86%). TLC: R_{f} = 0.7 (50% EtOAc/pentane). ¹H NMR (400 MHz, CDCl₃) δ 7.39 - 7.30 (m, 1H), 7.25 - 7.12 (m, 3H), 4.94 (br s, 1H), 3.65 (s, 3H), 3.44 (q, *J* = 6.7 Hz, 2H), 2.95 (t, *J* = 7.1 Hz, 2H). ¹³C NMR (101 MHz, CDCl₃) δ 157.10, 136.50, 134.17, 131.06, 129.65, 128.06, 126.97, 52.10, 40.67, 33.93. HRMS [C₁₀H₁₂ClNO₂ + H]⁺ : 214.0629 calculated, 214.0630 found (Δ = 0.14 ppm).

### 2-(2-Chlorophenyl)-N-methylethan-1-amine (I-48).

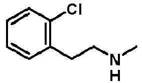

The title compound was prepared according to the general procedure D using carbamate **I-47** (183 mg, 0.85 mmol, 1 eq), LiAlH₄ (2 M THF solution, 0.68 mL, 1.36 mmol, 1.6 eq) and was used without further purification (130 mg, 0.77 mmol, 90%). TLC: R_{f} = 0.1 (6% MeOH/DCM). ¹H NMR (400 MHz, CDCl₃) δ 7.38 - 7.26 (m, 1H), 7.26 - 7.08 (m, 3H), 3.02 - 2.73 (m, 4H), 2.50 - 2.26 (m, 4H). ¹³C NMR (101 MHz, CDCl₃) δ 137.55, 134.08, 130.82, 129.58, 127.67, 126.81, 51.32, 36.18, 33.82. HRMS [C₉H₁₂ClN + H]⁺ : 170.0731 calculated, 170.0732 found (Δ = 0.47 ppm).

### 2-((2-Chlorophenethyl)(methyl)amino)-N-(cyclopropylmethyl)-6-morpholino pyrimidine-4-carboxamide (Example 68)

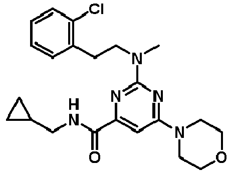

The title compound was prepared according to the general procedure E using 2-chloropyrimidine **I-35** (30 mg, 0.10 mmol, 1 eq), amine **I-48** (28 mg, 0.17 mmol, 1.7 eq) and DiPEA (70 µL, 0.40 mmol, 4 eq). Total heating time: 3 d at 120 °C. Purification by HPLC (C18 reverse phase, 35% -> 45% ACN/H₂O + 0.2% TFA, RT 10.8 min) afforded the product (31 mg, 70 µmol, 72%). TLC: R_{f} = 0.7 (50% EtOAc/pentane). ¹H NMR (500 MHz, CDCl₃) δ 8.06 (br s, 1H), 7.37 - 7.32 (m, 1H), 7.21 - 7.12 (m, 3H), 6.70 (s, 1H), 3.89 - 3.83 (m, 2H), 3.78 - 3.74 (m, 4H), 3.69 - 3.62 (m, 4H), 3.29 (dd, *J* = 7.0, 5.9 Hz, 2H), 3.14 (s, 3H), 3.06 - 3.02 (m, 2H), 1.13 - 1.01 (m, 1H), 0.59 - 0.51 (m, 2H), 0.31 - 0.27 (m, 2H). ¹³C NMR (101 MHz, CDCl₃) δ 164.70, 163.95, 160.97, 156.79, 137.46, 134.14, 131.17, 129.60, 127.93, 127.01, 90.11, 66.78, 49.36, 44.51, 44.14, 35.52, 31.93, 10.97, 3.59. HRMS [C₂₂H₂₈ClN₅O₂ + H]⁺ : 430.2004 calculated, 430.2003 found (Δ = -0.33 ppm).

### Methyl (4-phenoxyphenethyl)carbamate (I-49).

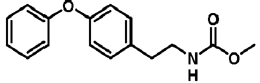

The title compound was prepared according to general procedure C using 2-(4-phenoxyphenyl)ethan-1-amine TFA salt (211 mg, 0.64 mmol, 1 eq), methylchloroformate (74 µL, 0.96 mmol, 1.5 eq) and DiPEA (245 µL, 1.41 mmol, 2.2 eq). Column chromatography (10% -> 40% EtOAc/pentane) afforded the product (145 mg, 0.53 mmol, 83%). TLC: R_{f} = 0.8 (100% EtOAc). ¹H NMR (400 MHz, CDCl₃) δ 7.40 - 7.27 (m, 2H), 7.22 - 7.04 (m, 3H), 7.03 - 6.89 (m, 4H), 4.96 - 4.65 (m, 1H), 3.65 (s, 3H), 3.51 - 3.29 (m, 2H), 2.77 (t, *J* = 7.1 Hz, 2H). ¹³C NMR (101 MHz, CDCl₃) δ 157.36, 157.05, 155.85, 133.69, 130.06, 129.77, 123.20, 119.12, 118.76, 52.08, 42.36, 35.48. HRMS [C₁₆H₁₇NO₃ + H]⁺ : 272.1281 calculated, 272.1281 found (Δ = -0.15 ppm).

### N-Methyl-2-(4-phenoxyphenyl)ethan-1-amine (I-50).

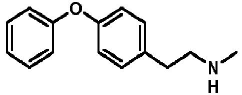

The title compound was prepared according to general procedure D using carbamate **I-49** (144 mg, 0.53 mmol, 1 eq), LiAlH₄ (2 M THF solution, 0.42 mL, 0.85 mmol, 1.6 eq). Column chromatography (isocratic, 5% MeOH/DCM + 0.5% Et₃N) afforded the product (65 mg, 0.29 mmol, 55%). TLC: R_{f} = 0.1 (5% MeOH/DCM with 3 drops of Et₃N). ¹H NMR (500 MHz, CDCl₃) δ 7.35 - 7.27 (m, 2H), 7.20 - 7.14 (m, 2H), 7.12 - 7.03 (m, 1H), 7.02 - 6.96 (m, 2H), 6.96 - 6.91 (m, 2H), 3.08 (br s, 1H), 2.92 - 2.80 (m, 4H), 2.48 (s, 3H). ¹³C NMR (126 MHz, CDCl₃) δ 157.46, 155.65, 134.48, 130.01, 129.77, 123.15, 119.13, 118.73, 53.02, 36.00, 35.00. HRMS [C₁₅H₁₇NO + H]⁺ : 228.1383 calculated, 228.1385 found (Δ = 0.92 ppm).

### N-(Cyclopropylmethyl)-2-(methyl(4-phenoxyphenethyl)amino)-6-morpholinopyrimidine-4-carboxamide (Example 53)

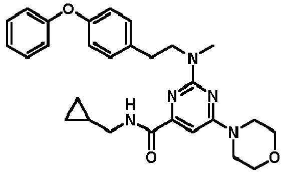

The title compound was prepared according to the general procedure E using 2-chloropyrimidine **I-35** (30 mg, 0.10 mmol, 1 eq), amine **I-50** (34 mg, 0.15 mmol, 1.5 eq) and DiPEA (70 µL, 0.40 mmol, 4 eq). Total heating time: 48 h at 120 °C. Column chromatography (30% -> 70% EtOAc/pentane) afforded the product (26 mg, 50 µmol, 53%). TLC: R_{f} = 0.4 (50% EtOAc/pentane). ¹H NMR (600 MHz, CDCl₃) δ 8.00 (br s, 1H), 7.35 - 7.28 (m, 2H), 7.19 - 7.13 (m, 2H), 7.10 - 7.06 (m, 1H), 6.99 - 6.89 (m, 4H), 6.72 (s, 1H), 3.84 - 3.78 (m, 2H), 3.78 - 3.73 (m, 4H), 3.69 - 3.63 (m, 4H), 3.31 - 3.26 (m, 2H), 3.13 (s, 3H), 2.93 - 2.86 (m, 2H), 1.11 - 0.98 (m, 1H), 0.56 - 0.51 (m, 2H), 0.29 - 0.25 (m, 2H). ¹³C NMR (151 MHz, CDCl₃) δ 164.67, 164.00, 160.92, 157.63, 156.80, 155.60, 134.93, 130.09, 129.83, 123.17, 119.27, 118.63, 90.12, 66.75, 51.67, 44.54, 44.12, 35.73, 33.25, 10.90, 3.49. HRMS [C₂₃H₃₃N₅O₃ + H]⁺ : 488.2656 calculated, 488.2656 found (Δ = -0.02 ppm).

### 2-(3-Phenoxyphenyl)acetonitrile (I-51).

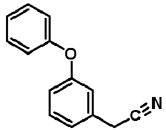

A round bottom flask was charged with 1-(chloromethyl)-3-phenoxybenzene (0.37 mL, 2 mmol, 1 eq) in dioxane/EtOH/H₂O (2:2:1, 6 mL) and KCN (260 mg, 4 mmol, 2 eq). The reaction mixture was stirred overnight at reflux. The mixture was diluted with water and extracted with EtOAc (3x). The combined organics are washed with brine, dried (Na₂SO₄), filtered and concentrated under reduced pressure. The crude material was purified by silica gel column chromatography (5% -> 7% EtOAc/pentane) to provide the product (352 mg, 1.68 mmol, 84%). TLC: R_{f} = 0.6 (10% EtOAc/pentane). ¹H NMR (500 MHz, CDCl₃) δ 7.37 - 7.19 (m, 3H), 7.15 - 7.03 (m, 1H), 7.04 - 6.94 (m, 3H), 6.95 - 6.84 (m, 2H), 3.56 (s, 2H). ¹³C NMR (126 MHz, CDCl₃) δ 157.73, 156.32, 131.81, 130.23, 129.71, 123.58, 122.34, 118.97, 117.96, 117.78, 117.51, 23.00.

### N-Methyl-2-(3-phenoxyphenyl)ethan-1-amine (I-52).

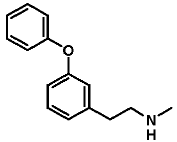

*Nitrile reduction:* A round bottom flask was charged with nitrile **I-51** (211 mg, 1 mmol, 1 eq), EtOH (5 mL) and 37% w/w aqueous HCl (0.16 mL, 2 mmol, 2 eq). N₂ is bubbled through the solution for 5 minutes and Pd/C 10 wt.% (52 mg, 50 µmol, 5 mol%) is added. The mixture is sparged with N₂ and then with H₂ and is kept under H₂ atmosphere (balloon). After 50 hours the reaction is complete as judged by TLC. The mixture is filtered over a Whatman filter and the filtrate is concentrated under reduced pressure to afford the product, which was used without further purification (246 mg, 0.98 mmol, 98%). TLC: R_{f} = 0.05 (6% MeOH/DCM). *Carbamoylation:* The carbamate was prepared according to general procedure C using amine (246 mg, 0.98 mmol, 1 eq), methylchloroformate (115 µL, 1.48 mmol, 1.5 eq) and DiPEA (517 µL, 3.0 mmol, 3 eq). Column chromatography (5% -> 40% EtOAc/pentane) afforded the product (176 mg, 0.65 mmol, 66%). TLC: R_{f} = 0.3 (10% EtOAc/pentane). *Carbamate reduction:* The title compound was prepared according to the general procedure D using carbamate (170 mg, 0.62 mmol, 1 eq), LiAlH₄ (2 M THF solution, 0.52 mL, 1.04 mmol, 1.6 eq) and was used without further purification (125 mg, 0.55 mmol, 88%). TLC: R_{f} = 0.1 (6% MeOH/DCM). ¹H NMR (400 MHz, CDCl₃) δ 7.35 - 7.28 (m, 2H), 7.25 - 7.20 (m, 1H), 7.12 - 7.05 (m, 1H), 7.03 - 6.97 (m, 2H), 6.95 - 6.91 (m, 1H), 6.89 - 6.81 (m, 2H), 2.91 - 2.67 (m, 5H), 2.41 (s, 3H). ¹³C NMR (101 MHz, CDCl₃) δ 157.45, 157.19, 142.10, 129.77, 129.74, 123.65, 123.26, 119.12, 118.94, 116.55, 52.94, 36.27, 35.99. HRMS [C₁₅H₁₇NO + H]⁺ : 228.1383 calculated, 228.1384 found (Δ = 0.26 ppm).

### N-(Cyclopropylmethyl)-2-(methyl(3-phenoxyphenethyl)amino)-6-morpholinopyrimidine-4-carboxamide (Example 61)

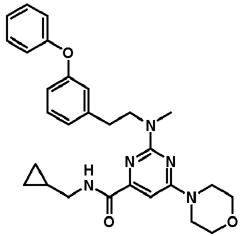

The title compound was prepared according to the general procedure E using 2-chloropyrimidine **I-35** (25 mg, 84 µmol, 1 eq), amine **I-52** (28 mg, 0.12 mmol, 1.5 eq) and DiPEA (60 µL, 0.34 mmol, 4 eq). Total heating time: 5 d at 120 °C. Purification by HPLC (C18 reverse phase, 40% -> 50% ACN/H₂O +0.2% TFA, RT 11.2 min) afforded the product (14 mg, 29 µmol, 34%). TLC: R_{f} = 0.5 (30% EtOAc/pentane). ¹H NMR (400 MHz, CDCl₃) δ 8.00 (br s, 1H), 7.35 - 7.28 (m, 2H), 7.29 - 7.20 (m, 1H), 7.13 - 7.06 (m, 1H), 6.99 - 6.94 (m, 3H), 6.92 - 6.87 (m, 1H), 6.86 - 6.83 (m, 1H), 6.71 (s, 1H), 3.84 - 3.77 (m, 2H), 3.76 - 3.70 (m, 4H), 3.67 - 3.61 (m, 4H), 3.30 - 3.24 (m, 2H), 3.12 (s, 3H), 2.93 - 2.83 (m, 2H), 1.11 - 0.98 (m, 1H), 0.57 - 0.49 (m, 2H), 0.30 - 0.24 (m, 2H). ¹³C NMR (101 MHz, CDCl₃) δ 164.67, 163.98, 160.89, 157.41, 157.32, 156.81, 142.03, 129.86, 123.91, 123.26, 119.57, 118.70, 116.91, 90.19, 66.75, 51.48, 44.52, 44.14, 35.69, 33.87, 10.90, 3.51. HRMS [C₂₃H₃₃N₅O₃ + H]⁺ : 488.2656 calculated, 488.2653 found (Δ = -0.57 ppm).

### Methyl (2-phenoxyphenethyl) carbamate (I-53)

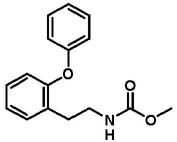

The title compound was prepared according to the general procedure C using 2-(2-phenoxyphenyl)ethan-1-amine (99 µL, 0.50 mmol, 1 eq), methylchloroformate (58 µL, 0.75 mmol, 1.5 eq) and DiPEA (174 µL, 1.0 mmol, 2 eq). Column chromatography (20% -> 40% EtOAc/pentane) afforded the product (115 mg, 0.42 mmol, 84%). TLC: R_{f} = 0.7 (30% EtOAc/pentane). ¹H NMR (400 MHz, CDCl₃) δ 7.36 - 7.22 (m, 3H), 7.21 - 7.15 (m, 1H), 7.10 - 7.04 (m, 2H), 6.96 - 6.91 (m, 2H), 6.89 - 6.83 (m, 1H), 4.89 (br s, 1H), 3.62 (s, 3H), 3.44 (q, *J* = 6.6 Hz, 2H), 2.85 (t, *J* = 6.9 Hz, 2H). ¹³C NMR (101 MHz, CDCl₃) δ 157.49, 157.08, 154.97, 131.27, 130.31, 129.85, 128.11, 123.97, 123.02, 119.26, 118.07, 52.05, 41.35, 30.73. HRMS [C₁₆H₁₇NO₃ + H]⁺ : 272.1281 calculated, 272.1281 found (Δ = -0.11 ppm).

### N-Methyl-2-(2-phenoxyphenyl)ethan-1-amine (I-54)

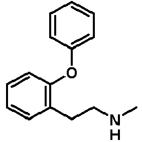

The title compound was prepared according to the general procedure D using carbamate **I-53** (115 mg, 0.42 mmol, 1 eq), LiAlH₄ (2 M THF solution, 0.34 mL, 0.67 mmol, 1.6 eq). Column chromatography (4% -> 7% MeOH/DCM + 0.5% Et₃N) afforded the product (72 mg, 0.32 mmol, 76%). TLC: R_{f} = 0.3 (4% MeOH/DCM with 3 drops of Et₃N). ¹H NMR (400 MHz, CDCl₃) δ 7.34 - 7.23 (m, 3H), 7.18 (td, *J* = 7.8, 1.7 Hz, 1H), 7.11 - 7.01 (m, 2H), 6.97 - 6.83 (m, 3H), 3.04 (br s, 1H), 2.87 (s, 4H), 2.42 (s, 3H). ¹³C NMR (101 MHz, CDCl₃) δ 157.79, 154.81, 131.17, 131.12, 129.80, 127.88, 123.99, 122.82, 119.58, 117.88, 51.55, 35.80, 30.23. HRMS [C₁₅H₁₇NO + H]⁺ : 228.1383 calculated, 228.1383 found (Δ = -0.18 ppm).

### N-(Cyclopropylmethyl)-2-(methyl(2-phenoxyphenethyl)amino)-6-morpholinopyrimidine-4-carboxamide (Example 67)

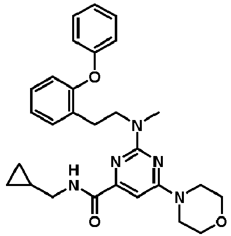

The title compound was prepared according to the general procedure E using 2-chloropyrimidine **I-35** (34 mg, 0.10 mmol, 1 eq), amine **I-54** (37 mg, 0.16 mmol, 1.6 eq) and DiPEA (70 µL, 0.40 mmol, 4 eq). Total heating time: 3 d at 120 °C. Column chromatography (30% -> 50% EtOAc/pentane) afforded the product (45 mg, 90 µmol, 92%). TLC: R_{f} = 0.5 (40% EtOAc/pentane). ¹H NMR (400 MHz, CDCl₃) δ 8.06 (t, *J* = 5.4 Hz, 1H), 7.34 - 7.28 (m, 2H), 7.26 (dd, *J* = 7.4, 1.5 Hz, 1H), 7.17 (td, *J* = 7.8, 1.7 Hz, 1H), 7.12 - 7.00 (m, 2H), 6.93 (dd, *J* = 8.6, 0.9 Hz, 2H), 6.86 (d, *J* = 8.0 Hz, 1H), 6.69 (s, 1H), 3.90 - 3.79 (m, 2H), 3.76 - 3.54 (m, 8H), 3.27 (t, *J* = 6.4 Hz, 2H), 3.07 (s, 3H), 2.99 - 2.88 (m, 2H), 1.11 - 0.98 (m, 1H), 0.60 - 0.44 (m, 2H), 0.27 (q, *J* = 4.7 Hz, 2H). ¹³C NMR (101 MHz, CDCl₃) δ 164.74, 163.94, 160.90, 157.74, 156.72, 155.09, 131.31, 129.89, 127.84, 123.92, 122.99, 119.36, 118.07, 89.98, 66.70, 50.17, 44.44, 44.03, 35.57, 28.69, 10.92, 3.49. HRMS [C₂₃H₃₃N₅O₃ + H]⁺ : 488.2656 calculated, 488.2653 found (Δ = -0.72 ppm).

### Methyl (4-methoxyphenethyl)carbamate (I-55)

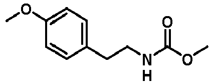

The title compound was prepared according to the general procedure C using 2-(4-methoxyphenyl)ethan-1-amine (73 µL, 0.50 mmol, 1 eq), methylchloroformate (58 µL, 0.75 mmol, 1.5 eq) and DiPEA (174 µL, 1.0 mmol, 2 eq). Column chromatography (10% -> 40% EtOAc/pentane) afforded the product (108 mg, 0.50 mmol, 99%). TLC: R_{f} = 0.6 (50% EtOAc/pentane). ¹H NMR (400 MHz, CDCl₃) δ 7.09 (d, *J* = 8.5 Hz, 2H), 6.83 (d, 2H), 4.93 (br s, 1H), 3.77 (s, 3H), 3.63 (s, 3H), 3.38 (q, *J* = 6.8 Hz, 2H), 2.73 (t, *J* = 7.1 Hz, 2H). ¹³C NMR (101 MHz, CDCl₃) δ 158.21, 157.04, 130.80, 129.69, 113.99, 55.21, 51.96, 42.42, 35.21. HRMS [C₁₁H₁₅NO₃ + H]⁺ : 210.1125 calculated, 210.1125 found (Δ = 0.0 ppm).

### 2-(4-Methoxyphenyl)-N-methylethan-1-amine (I-56)

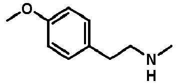

The title compound was prepared according to the general procedure D using carbamate **I-55** (105 mg, 0.50 mmol, 1 eq), LiAlH₄ (2 M THF solution, 0.40 mL, 0.80 mmol, 1.6 eq) and was used without further purification (78 mg, 0.47 mmol, 93%). TLC: R_{f} = 0.1 (5% MeOH/DCM with 3 drops of Et₃N). ¹H NMR (400 MHz, CDCl₃) δ 7.17 - 7.05 (m, 2H), 6.87 - 6.77 (m, 2H), 3.77 (s, 3H), 2.87 - 2.65 (m, 4H), 2.49 - 2.19 (m, 4H). ¹³C NMR (101 MHz, CDCl₃) δ 158.08, 132.00, 129.68, 113.96, 55.30, 53.37, 36.29, 35.18. HRMS [C₁₀H₁₅NO + H]⁺ : 166.1226 calculated, 166.1226 found (Δ = -0.24 ppm).

### N-(Cyclopropylmethyl)-2-((4-methoxyphenethyl)(methyl)amino)-6-morpholinopyrimidine-4-carboxamide (Example 64)

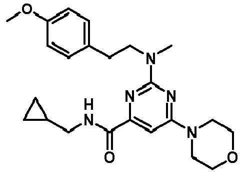

The title compound was prepared according to the general procedure E using 2-chloropyrimidine **I-35** (30 mg, 0.10 mmol, 1 eq), amine **I-56** (36 mg, 0.20 mmol, 2 eq) and DiPEA (70 µL, 0.40 mmol, 4 eq). Total heating time: 7 d at 120 °C. Column chromatography (30% -> 70% EtOAc/pentane) afforded the product (38 mg, 90 µmol, 90%). TLC: R_{f} = 0.4 (30% EtOAc/pentane). ¹H NMR (400 MHz, CDCl₃) δ 8.04 (t, *J* = 5.6 Hz, 1H), 7.18 - 7.07 (m, 2H), 6.87 - 6.80 (m, 2H), 6.72 (s, 1H), 3.79 (s, 3H), 3.79 - 3.72 (m, 6H), 3.66 (t, *J* = 4.8 Hz, 4H), 3.30 (dd, *J* = 7.1, 5.8 Hz, 2H), 3.11 (s, 3H), 2.88 - 2.81 (m, 2H), 1.11 - 1.02 (m, 1H), 0.58 - 0.52 (m, 2H), 0.32 - 0.27 (m, 2H). ¹³C NMR (101 MHz, CDCl₃) δ 164.71, 164.00, 160.88, 158.16, 156.79, 131.97, 129.76, 114.01, 90.06, 66.76, 55.39, 51.86, 44.51, 44.11, 35.75, 33.01, 10.90, 3.49. HRMS [C₂₃H₃₁N₅O₃ + H]⁺ : 426.2500 calculated, 426.2497 found (Δ = -0.54 ppm).

### Methyl (2-methoxyphenethyl)carbamate (I-57)

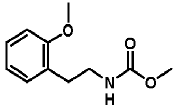

The title compound was prepared according to the general procedure C using 2-(2-methoxyphenyl)ethan-1-amine (145 µL, 1.0 mmol, 1 eq), methylchloroformate (116 µL, 1.5 mmol, 1.5 eq) and DiPEA (348 µL, 2.0 mmol, 2 eq). Column chromatography (20% - > 40% EtOAc/pentane) afforded the product (230 mg, 1.0 mmol, 99%). TLC: R_{f} = 0.5 (30% EtOAc/pentane). ¹H NMR (400 MHz, CDCl₃) δ 7.19 (td, *J* = 8.0, 1.7 Hz, 1H), 7.11 (d, *J* = 6.9 Hz, 1H), 6.94 - 6.78 (m, 2H), 5.19 - 4.71 (m, 1H), 3.79 (s, 3H), 3.61 (s, 3H), 3.39 (q, *J* = 6.6 Hz, 2H), 2.81 (t, *J* = 6.9 Hz, 2H). ¹³C NMR (101 MHz, CDCl₃) δ 157.48, 157.04, 130.51, 127.75, 127.20, 120.51, 110.27, 55.14, 51.81, 41.08, 30.65. HRMS [C₁₁H₁₅NO₃ + H]⁺ : 210.1125 calculated, 210.1125 found (Δ = 0.24 ppm).

### 2-(2-Methoxyphenyl)-N-methylethan-1-amine (I-58)

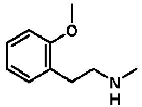

The title compound was prepared according to the general procedure D using carbamate **I-57** (230 mg, 1.0 mmol, 1 eq), LiAlH₄ (1 M THF solution, 1.6 mL, 1.6 mmol, 1.6 eq) and was used without further purification (160 mg, 0.97 mmol, 88%). TLC: R_{f} = 0.3 (2% MeOH/DCM with 3 drops of Et₃N). ¹H NMR (400 MHz, CDCl₃) δ 7.24 - 7.07 (m, 2H), 6.95 - 6.74 (m, 2H), 3.80 (s, 3H), 2.90 - 2.70 (m, 4H), 2.43 (s, 3H), 1.26 (br s, 1H). ¹³C NMR (101 MHz, CDCl₃) δ 157.59, 130.33, 128.44, 127.36, 120.39, 110.30, 55.20, 51.85, 36.37, 30.70. HRMS [C₁₀H₁₅NO + H]⁺ : 166.1226 calculated, 166.1225 found (Δ = -0.60 ppm).

### N-(Cyclopropylmethyl)-2-((2-methoxyphenethyl)(methyl)amino)-6-morpholinopyrimidine-4-carboxamide (Example 83)

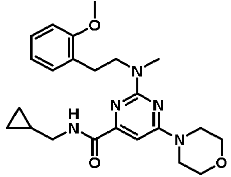

The title compound was prepared according to the general procedure E using 2-chloropyrimidine **I-35** (30 mg, 0.10 mmol, 1 eq), amine **I-58** (24 mg, 0.15 mmol, 1.5 eq) and DiPEA (70 µL, 0.40 mmol, 4 eq). Total heating time: 8 h at 160 °C with µW irradiation. Column chromatography (30% -> 70% EtOAc/pentane) afforded the product (48 mg, 0.11 mmol, 99%). TLC: R_{f} = 0.3 (30% EtOAc/pentane). ¹H NMR (400 MHz, CDCl₃) δ 8.08 (t, *J* = 6.0 Hz, 1H), 7.20 (td, *J* = 7.8, 1.8 Hz, 1H), 7.13 (dd, *J* = 7.3, 1.7 Hz, 1H), 6.94 - 6.82 (m, 2H), 6.71 (s, 1H), 3.83 (s, 3H), 3.81 - 3.72 (m, 6H), 3.66 (t, *J =* 4.8 Hz, 4H), 3.30 (dd, *J* = 7.0, 5.9 Hz, 2H), 3.12 (s, 3H), 3.03 - 2.84 (m, 2H), 1.13 - 0.97 (m, 1H), 0.65 - 0.44 (m, 2H), 0.35 - 0.20 (m, 2H). ¹³C NMR (101 MHz, CDCl₃) δ 164.81, 163.99, 160.95, 157.81, 156.77, 130.55, 128.37, 127.64, 120.64, 110.48, 89.88, 66.79, 55.47, 49.93, 44.49, 44.04, 35.65, 28.70, 10.94, 3.47. HRMS [C₂₃H₃₁N₅O₃ + H]⁺ : 426.2500 calculated, 426.2496 (found (Δ = -0.77 ppm).

### Methyl (3-phenylpropyl)carbamate (I-59)

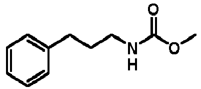

The title compound was prepared according to general procedure C using 3-phenylpropan-1-amine (71 µL, 0.50 mmol, 1 eq), methylchloroformate (58 µL, 0.75 mmol, 1.5 eq) and DiPEA (174 µL, 1.0 mmol, 2 eq). Column chromatography (20% -> 50% EtOAc/pentane) afforded the product (91 mg, 0.47 mmol, 94%). TLC: R_{f} = 0.7 (50% EtOAc/pentane). ¹H NMR (400 MHz, CDCl₃) δ 7.33 - 7.22 (m, 2H), 7.22 - 7.13 (m, 3H), 4.82 (br s, 1H), 3.65 (s, 3H), 3.26 - 3.07 (m, 2H), 2.69 - 2.57 (m, 2H), 1.82 (p, *J =* 7.3 Hz, 2H). ¹³C NMR (101 MHz, CDCl₃) δ 157.18, 141.47, 128.49, 128.40, 126.02, 52.08, 40.68, 33.06, 31.68. HRMS [C₁₁H₁₅NO₂ + H]⁺ : 194.1176 calculated, 194.1175 found (Δ = -0.41 ppm).

### N-Methyl-3-phenylpropan-1-amine (I-60)

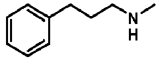

The title compound was prepared according to general procedure D using carbamate **I-59** (91 mg, 0.47 mmol, 1 eq), LiAlH₄ (2 M THF solution, 0.75 mL, 1.54 mmol, 3.3 eq) and was used without further purification (39 mg, 0.13 mmol, 27%). TLC: R_{f} = 0.1 (5% MeOH/DCM). ¹H NMR (500 MHz, CDCl₃) δ 7.31 - 7.13 (m, 5H), 2.68 - 2.63 (m, 2H), 2.63 - 2.58 (m, 2H), 2.42 (s, 3H), 1.90 - 1.76 (m, 2H), 1.71 - 1.58 (m, 1H). HRMS [C₁₀H₁₅N + H]⁺ : 150.1277 calculated, 150.1278 found (Δ = 0.33 ppm).

### N-(Cyclopropylmethyl)-2-(methyl(3-phenylpropyl)amino)-6-morpholinopyrimidine-4-carboxamide (Example 58)

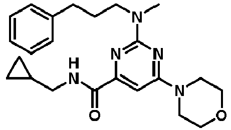

The title compound was prepared according to the general procedure E using 2-chloropyrimidine **I-35** (39 mg, 0.13 mmol, 1 eq), amine **I-60** (30 mg, 0.20 mmol, 1.5 eq) and DiPEA (87 µL, 0.53 mmol, 4 eq). Total heating time: 48 h at 120 °C. Column chromatography (30% -> 60% EtOAc/pentane) afforded the product (38 mg, 93 µmol, 71%). TLC: R_{f} = 0.4 (50% EtOAc/pentane). ¹H NMR (400 MHz, CDCl₃) δ 7.98 (br s, 1H), 7.32 - 7.22 (m, 2H), 7.22 - 7.14 (m, 3H), 6.69 (s, 1H), 3.76 - 3.68 (m, 4H), 3.65 - 3.59 (m, 2H), 3.59 - 3.51 (m, 4H), 3.28 (t, *J* = 7.0, 5.9 Hz, 2H), 3.14 (s, 3H), 2.66 (t, *J* = 7.5 Hz, 2H), 1.95 (p, *J* = 9.0, 7.5 Hz, 2H), 1.10 - 0.98 (m, 1H), 0.57 - 0.49 (m, 2H), 0.30 - 0.24 (m, 2H). ¹³C NMR (101 MHz, CDCl₃) δ 164.69, 163.93, 161.04, 156.75, 142.00, 128.49, 128.43, 125.93, 89.96, 66.73, 48.99, 44.42, 43.97, 35.31, 33.52, 28.92, 10.93, 3.43. HRMS [C₂₃H₃₂N₅O₂ + H]⁺ : 410.2551 calculated, 410.2548 found (Δ = -0.59 ppm).

### Methyl (4-phenylbutyl)carbamate (I-61)

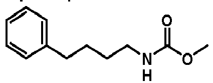

The title compound was prepared according to general procedure C using 4-phenylbutan-1-amine (79 µL, 0.50 mmol, 1 eq), methylchloroformate (58 µL, 0.75 mmol, 1.5 eq) and DiPEA (174 µL, 1.0 mmol, 2 eq). Column chromatography (10% -> 40% EtOAc/pentane) afforded the product (97 mg, 0.47 mmol, 94%). TLC: R_{f} = 0.6 (50% EtOAc/pentane). ¹H NMR (400 MHz, CDCl₃) δ 7.26 (t, *J* = 7.5 Hz, 2H), 7.17 (t, *J* = 7.9 Hz, 3H), 4.80 (br s, 1H), 3.64 (s, 3H), 3.29 - 3.00 (m, 2H), 2.61 (t, *J* = 7.6 Hz, 2H), 1.69 - 1.57 (m, 2H), 1.51 (p, *J* = 6.9 Hz, 2H). ¹³C NMR (101 MHz, CDCl₃) δ 157.15, 142.15, 128.42, 128.37, 125.84, 52.01, 40.93, 35.54, 29.65, 28.55. HRMS [C₁₂H₁₇NO₂ + H]⁺ : 208.1332 calculated, 208.1333 found (Δ = 0.19 ppm).

### N-Methyl-4-phenylbutan-1-amine (I-62)

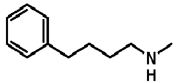

The title compound was prepared according to general procedure D using carbamate **I-61** (97 mg, 0.47 mmol, 1 eq), LiAlH₄ (2 M THF solution, 0.38 mL, 0.75 mmol, 1.6 eq) and was used without further purification (64 mg, 0.39 mmol, 83%). TLC: R_{f} = 0.1 (5% MeOH/DCM). ¹H NMR (400 MHz, CDCl₃) δ 7.31 - 7.21 (m, 2H), 7.20 - 7.11 (m, 3H), 2.62 (t, *J* = 7.6 Hz, 2H), 2.59 - 2.54 (m, 2H), 2.40 (s, 3H), 1.77 - 1.57 (m, 3H), 1.57 - 1.46 (m, 2H). ¹³C NMR (101 MHz, CDCl₃) δ 142.51, 128.45, 128.31, 125.73, 52.05, 36.57, 35.90, 29.61, 29.24. HRMS [C₁₁H₁₇N + H]⁺ : 164.1434 calculated, 164.1433 found (Δ = -0.30 ppm).

### N-(Cyclopropylmethyl)-2-(methyl(4-phenylbutyl)amino)-6-morpholinopyrimidine-4-carboxamide (Example 60)

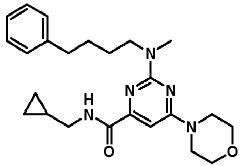

The title compound was prepared according to the general procedure E using 2-chloropyrimidine **I-35** (30 mg, 0.10 mmol, 1 eq), amine **I-62** (23 mg, 0.15 mmol, 1.5 eq) and DiPEA (70 µL, 0.40 mmol, 4 eq). Total heating time: 24 h at 120 °C. Column chromatography (30% -> 60% EtOAc/pentane) afforded the product (40 mg, 94 µmol, 98%). TLC: R_{f} = 0.5 (50% EtOAc/pentane). ¹H NMR (400 MHz, CDCl₃) δ 8.03 (t, *J* = 5.8 Hz, 1H), 7.31 - 7.22 (m, 2H), 7.21 - 7.12 (m, 3H), 6.70 (s, 1H), 3.77 - 3.70 (m, 4H), 3.67 - 3.58 (m, 6H), 3.31 - 3.24 (m, 2H), 3.11 (s, 3H), 2.73 - 2.59 (m, 2H), 1.76 - 1.56 (m, *J* = 3.5, 2.9 Hz, 4H), 1.11 - 0.95 (m, 1H), 0.56 - 0.44 (m, 2H), 0.28 - 0.18 (m, 2H). ¹³C NMR (101 MHz, CDCl₃) δ 164.70, 163.96, 161.10, 156.74, 142.44, 128.42, 128.40, 125.87, 89.89, 66.72, 49.12, 44.47, 44.00, 35.88, 35.32, 29.03, 27.20, 10.86, 3.39. HRMS [C₂₄H₃₃N₅O₂ + H]⁺ : 424.2707 calculated, 424.2706 found (Δ = -0.35 ppm).

### Methyl (4-(trifluoromethyl)phenethyl)carbamate (I-63)

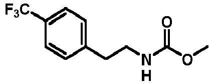

The title compound was prepared according to general procedure C using 2-(4-(trifluoromethyl)phenyl)ethan-1-amine (80 µL, 0.50 mmol, 1 eq), methylchloroformate (58 µL, 0.75 mmol, 1.5 eq) and DiPEA (174 µL, 1.0 mmol, 2 eq). Column chromatography (5% -> 40% EtOAc/pentane) afforded the product (113 mg, 0.45 mmol, 91%). TLC: R_{f} = 0.7 (50% EtOAc/pentane). ¹H NMR (400 MHz, CDCl₃) δ 7.56 (d, *J =* 8.0 Hz, 2H), 7.31 (d, *J* = 8.0 Hz, 2H), 4.89 - 4.64 (m, 1H), 3.66 (s, 3H), 3.45 (q, *J* = 6.7 Hz, 2H), 2.88 (t, *J* = 7.0 Hz, 2H). ¹³C NMR (101 MHz, CDCl₃) δ 157.06, 143.06, 129.25, 128.82, 128.37, 125.62 (q, *J* = 3.8 Hz), 122.97, 52.25, 42.03, 36.14. HRMS [C₁₁H₁₂F₃NO₂ + H]⁺ : 248.0893 calculated, 248.0896 found (Δ = 1.29 ppm).

### N-Methyl-2-(4-(trifluoromethyl)phenyl)ethan-1-amine (I-64)

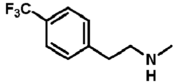

The title compound was prepared according to general procedure D using carbamate **I-63** (113 mg, 0.45 mmol, 1 eq), LiAlH₄ (2 M THF solution, 0.36 mL, 0.72 mmol, 1.6 eq). Column chromatography (isocratic, 5% MeOH/DCM + 0.5% Et₃N) afforded the product (40 mg, 0.20 mmol, 44%). TLC: R_{f} = 0.3 (2% MeOH/DCM with 3 drops of Et₃N). ¹H NMR (500 MHz, CDCl₃) δ 7.55 (d, *J* = 8.0 Hz, 2H), 7.34 (d, *J* = 7.9 Hz, 2H), 3.80 (br s, 1H), 2.99 - 2.91 (m, 4H), 2.51 (s, 3H). ¹³C NMR (126 MHz, CDCl₃) δ 143.51, 129.13, 125.57 (q, *J* = 3.7 Hz), 125.41, 123.25, 52.36, 35.73, 35.29. HRMS [C₁₀H₁₂F₃N + H]⁺ : 204.0995 calculated, 204.0996 found (Δ = 0.59 ppm).

### N-(Cyclopropylmethyl)-2-(methyl(4-(trifluoromethyl)phenethyl)amino)-6-morpholino-pyrimidine-4-carboxamide (Example 56)

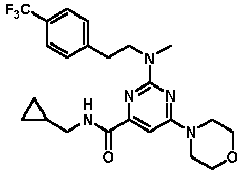

The title compound was prepared according to the general procedure E using 2-chloropyrimidine **I-35** (22 mg, 73 µmol, 1 eq), amine **I-64** (29 mg, 0.10 mmol, 1.5 eq) and DiPEA (51 µL, 0.29 mmol, 4 eq). Total heating time: 25 h at 120 °C. Purification by HPLC (C18 reverse phase, 47% -> 55% ACN/H₂O + 0.2% TFA, RT 12 min) afforded the product (9 mg, 20 µmol, 28%). TLC: R_{f} = 0.4 (50% EtOAc/pentane). ¹H NMR (400 MHz, CDCl₃) δ 7.98 (br s, 1H), 7.54 (d, *J* = 8.0 Hz, 2H), 7.32 (d, *J* = 7.9 Hz, 2H), 6.73 (s, 1H), 3.87 - 3.79 (m, 2H), 3.79 - 3.71 (m, 4H), 3.65 (t, *J* = 4.8 Hz, 4H), 3.35 - 3.24 (m, 2H), 3.10 (s, 3H), 3.02 - 2.93 (m, 2H), 1.12 - 0.99 (m, 1H), 0.65 - 0.48 (m, 2H), 0.39 - 0.22 (m, 2H). ¹³C NMR (101 MHz, CDCl₃) δ 164.61, 163.98, 160.88, 156.81, 144.18 (q, *J* = 1.4 Hz), 129.21, 128.89, 128.57, 125.73, 125.51 (q, *J* = 3.7 Hz), 123.03, 90.37, 66.75, 51.30, 44.51, 44.13, 35.87, 33.91, 10.92, 3.50. HRMS [C₂₃H₂₇F₃N₅O₂ + H]⁺ : 464.2268 calculated, 464.2267 found (Δ = -0.24 ppm).

### Methyl (4-methylphenethyl)carbamate (I-65)

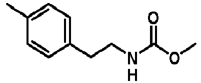

The title compound was prepared according to the general procedure C using 2-(p-tolyl)ethan-1-amine (145 µL, 1.0 mmol, 1 eq), methylchloroformate (116 µL, 1.5 mmol, 1.5 eq) and DiPEA (348 µL, 2.0 mmol, 2 eq). Column chromatography (30% -> 50% EtOAc/pentane) afforded the product (198 mg, 1.0 mmol, 99%). TLC: R_{f} = 0.7 (50% EtOAc/pentane). ¹H NMR (400 MHz, CDCl₃) δ 7.18 - 6.91 (m, 4H), 5.02 (br s, 1H), 3.61 (s, 3H), 3.38 (q, *J* = 6.8 Hz, 2H), 2.74 (t, *J* = 7.2 Hz, 2H), 2.30 (s, 3H). ¹³C NMR (101 MHz, CDCl₃) δ 157.00, 135.82, 135.66, 129.18, 128.55, 51.85, 42.26, 35.60, 20.91. HRMS [C₁₁H₁₅NO₂ + H]⁺ : 194.1176 calculated, 194.1176 found (Δ = 0.00 ppm).

### N-Methyl-2-(p-tolyl)ethan-1-amine (I-66)

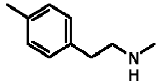

The title compound was prepared according to the general procedure D using carbamate **I-65** (198 mg, 1.0 mmol, 1 eq), LiAlH₄ (2 M THF solution, 0.80 mL, 1.0 mmol, 1.6 eq) and was used without further purification (130 mg, 0.87 mmol, 87%). TLC: R_{f} = 0.2 (5% MeOH/DCM). ¹H NMR (400 MHz, CDCl₃) δ 7.11 - 7.07 (m, 4H), 2.83 - 2.72 (m, 4H), 2.41 (s, 3H), 2.33 - 2.28 (m, 4H). ¹³C NMR (101 MHz, CDCl₃) δ 136.87, 135.59, 129.17, 128.60, 80.66, 53.26, 36.28, 35.65, 21.02. HRMS [C₁₀H₁₅N + H]⁺ : 150.1277 calculated, 150.1277 found (Δ = -0.33 ppm).

### N-(Cyclopropylmethyl)-2-(methyl(4-methylphenethyl)amino)-6-morpholinopyrimidine-4-carboxamide (Example 69)

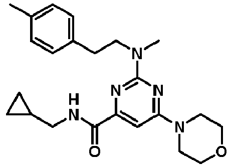

The title compound was prepared according to the general procedure E using 2-chloropyrimidine **I-35** (30 mg, 0.10 mmol, 1 eq), amine **I-66** (23 mg, 0.15 mmol, 1.5 eq) and DiPEA (70 µL, 0.40 mmol, 4 eq). Total heating time: 3 d at 120 °C. Column chromatography (30% -> 50% EtOAc/pentane) afforded the product (16 mg, 40 µmol, 40%). TLC: R_{f} = 0.8 (50% EtOAc/pentane). ¹H NMR (400 MHz, CDCl₃) δ 8.04 (t, *J* = 5.8 Hz, 1H), 7.11 (s, 4H), 6.72 (s, 1H), 3.82 - 3.72 (m, 6H), 3.66 (t, *J* = 4.8 Hz, 4H), 3.30 (dd, *J* = 7.1, 5.8 Hz, 2H), 3.13 (s, 3H), 2.90 - 2.82 (m, 2H), 2.33 (s, 3H), 1.14 - 0.99 (m, 1H), 0.61 - 0.49 (m, 2H), 0.33 - 0.25 (m, 2H). ¹³C NMR (101 MHz, CDCl₃) δ 164.72, 164.01, 160.89, 156.79, 136.81, 135.80, 129.28, 128.72, 90.06, 66.76, 51.81, 44.52, 44.12, 35.69, 33.46, 21.16, 10.90, 3.49. HRMS [C₂₃H₃₁N₅O₂ + H]⁺ : 410.2551 calculated, 410.2549 found (Δ = -0.41 ppm).

### Methyl (2-methylphenethyl)carbamate (I-67)

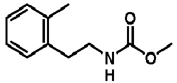

The title compound was prepared according to the general procedure C using 2-(*o-*tolyl)ethan-1-amine (70 µL, 0.50 mmol, 1 eq), methylchloroformate (58 µL, 0.75 mmol, 1.5 eq) and DiPEA (174 µL, 1.0 mmol, 2 eq). Column chromatography (30% -> 50% EtOAc/pentane) afforded the product (102 mg, 0.50 mmol, 99%). TLC: R_{f} = 0.7 (50% EtOAc/pentane). ¹H NMR (400 MHz, CDCl₃) δ 7.19 - 7.07 (m, 4H), 5.00 - 4.79 (m, 1H), 3.65 (s, 3H), 3.37 (q, *J* = 6.9 Hz, 2H), 2.81 (t, *J* = 7.4 Hz, 2H), 2.32 (s, 3H). ¹³C NMR (101 MHz, CDCl₃) δ 157.12, 136.93, 136.37, 130.44, 129.34, 126.62, 126.11, 52.03, 41.16, 33.59, 19.29. HRMS [C₁₁H₁₅NO₂ + H]⁺ : 194.1176 calculated, 194.1176 found (Δ = -0.05 ppm).

### N-Methl-2-(o-tolyl)ethan-1-amine (I-68)

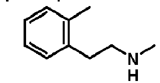

The title compound was prepared according to the general procedure D using carbamate **I-67** (102 mg, 0.5 mmol, 1 eq), LiAlH₄ (1 M THF solution, 0.85 mL, 0.85 mmol, 1.6 eq) and was used without further purification (74 mg, 0.50 mmol, 94%). TLC: R_{f} = 0.1 (6% MeOH/DCM). ¹H NMR (400 MHz, CDCl₃) δ 7.21 - 7.02 (m, 4H), 2.80 (s, 4H), 2.45 (s, 3H), 2.32 (s, 3H), 1.71 (br s, 1H). ¹³C NMR (101 MHz, CDCl₃) δ 138.15, 136.21, 130.34, 129.25, 126.29, 126.01, 52.11, 36.44, 33.56, 19.42. HRMS [C₁₀H₁₅N + H]⁺ : 150.1277 calculated, 150.1277 found (Δ = -0.40 ppm).

### N-(Cyclopropylmethyl)-2-(methyl(2-methylphenethyl)amino)-6-morpholinopyrimidine-4-carboxamide (Example 78)

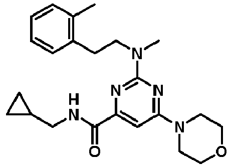

The title compound was prepared according to the general procedure E using 2-chloropyrimidine **I-35** (30 mg, 0.10 mmol, 1 eq), amine **I-68** (23 mg, 0.15 mmol, 1.5 eq) and DiPEA (70 µL, 0.40 mmol, 4 eq). Total heating time: 4 d at 120 °C. Column chromatography (30% -> 60% EtOAc/pentane) afforded the product (39 mg, 95 µmol, 96%). TLC: R_{f} = 0.4 (40% EtOAc/pentane). ¹H NMR (400 MHz, CDCl₃) δ 8.02 (t, *J* = 5.9 Hz, 1H), 7.23 - 7.06 (m, 4H), 6.73 (s, 1H), 3.85 - 3.71 (m, 6H), 3.66 (t, *J* = 4.8 Hz, 4H), 3.29 (dd, *J* = 7.1, 5.9 Hz, 2H), 3.15 (s, 3H), 2.97 - 2.83 (m, 2H), 2.39 (s, 3H), 1.15 - 0.98 (m, 1H), 0.62 - 0.47 (m, 2H), 0.34 - 0.22 (m, 2H). ¹³C NMR (101 MHz, CDCl₃) δ 164.69, 163.99, 160.91, 156.84, 137.97, 136.13, 130.38, 129.54, 126.51, 126.22, 90.12, 66.74, 50.23, 44.50, 44.12, 35.58, 31.31, 19.46, 10.91, 3.52. HRMS [C₂₃H₃₁N₅O₂ + H]⁺ : 410.2551 calculated, 410.2546 (found (Δ = -1.22 ppm).

### 2-([1,1'-Biphenyl]-2-yl)acetonitrile (I-69)

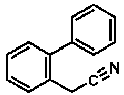

A round bottom flask was charged with 2-(bromomethyl)-1,1'-biphenyl (0.37 mL, 2 mmol, 1 eq), solvent mixture dioxane/EtOH/H₂O (2:2:1, 6 mL) and KCN (260 mg, 4 mmol, 2 eq). The reaction mixture was stirred overnight at reflux. The mixture was diluted with water and extracted with EtOAc (3x). The combined organics are washed with brine, dried (Na₂SO₄), filtered and concentrated under reduced pressure. The crude material was purified by silica gel column chromatography (0.5% -> 5% EtOAc/pentane) to provide the product (400 mg, 2.0 mmol, 99%). TLC: R_{f} = 0.6 (3% EtOAc/pentane). ¹H NMR (400 MHz, CDCl₃) δ 7.59 - 7.12 (m, 9H), 3.57 (s, 2H). ¹³C NMR (101 MHz, CDCl₃) δ 141.72, 139.78, 130.35, 128.86, 128.84, 128.59, 128.12, 127.68, 118.23, 21.92.

### Methyl (2-([1,1'-biphenyl]-2-yl)ethyl)carbamate (I-70)

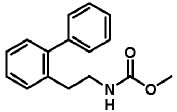

*Nitrile reduction:* A round bottom flask was charged with nitrile **I-69** (195 mg, 1 mmol, 1 eq), EtOH (5 mL) and 37% w/w aqueous HCl (0.16 mL, 2 mmol, 2 eq). N₂ was bubbled through the solution for 5 minutes and Pd/C 10 wt.% (52 mg, 50 µmol, 5 mol%) is added. The mixture was sparged with N₂ and then with H₂ and was kept under an H₂ atmosphere (balloon). After 3.5 days the reaction was complete as judged by TLC. The mixture is filtered over a Whatman filter and the filtrate was concentrated under reduced pressure to afford the product, which was used without further purification (235 mg, 1 mmol, 99%). TLC: R_{f} = 0.15 (6% MeOH/DCM). *Carbamoylation:* The title compound was prepared according to the general procedure C using the amine HCl salt (235 mg, 1.0 mmol, 1 eq), methylchloroformate (116 µL, 1.5 mmol, 1.5 eq) and DiPEA (522 µL, 3.0 mmol, 3 eq). Column chromatography (10% -> 40% EtOAc/pentane) afforded the product (210 mg, 0.82 mmol, 82%). TLC: R_{f} = 0.5 (20% EtOAc/pentane). ¹H NMR (400 MHz, CDCl₃) δ 7.48 - 7.16 (m, 9H), 4.86 - 4.47 (m, 1H), 3.56 (s, 3H), 3.28 - 3.08 (m, 2H), 2.79 (t, *J* = 7.2 Hz, 2H). ¹³C NMR (101 MHz, CDCl₃) δ 156.89, 142.40, 141.50, 136.16, 130.32, 129.68, 129.19, 128.26, 127.64, 127.05, 126.46, 51.98, 41.83, 33.26. HRMS [C₁₆H₁₇NO₂ + H]⁺ : 256.1332 calculated, 256.1333 found (Δ = 0.16 ppm).

### 2-([1,1'-Bipheny)]-2-yl)-N-methylethan-1-amine (I-71)

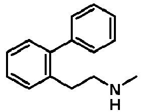

The title compound was prepared according to the general procedure D using carbamate **I-70** (210 mg, 0.82 mmol, 1 eq), LiAlH₄ (2 M THF solution, 0.66 mL, 1.32 mmol, 1.6 eq) and was used without further purification (157 mg, 0.74 mmol, 90%). TLC: R_{f} = 0.3 (6% MeOH/DCM). ¹H NMR (400 MHz, CDCl₃) δ 7.42 - 7.36 (m, 2H), 7.35 - 7.19 (m, 7H), 2.83 - 2.73 (m, 2H), 2.70 - 2.58 (m, 2H), 2.26 (s, 3H), 2.11 (br s, 1H). ¹³C NMR (101 MHz, CDCl₃) δ 142.24, 141.70, 137.29, 130.25, 129.56, 129.22, 128.16, 127.50, 126.93, 126.13, 52.77, 36.06, 33.16. HRMS [C₁₅H₁₇N + H]⁺ : 212.1434 calculated, 212.1434 found (Δ = 0.19 ppm).

### 2-((2-([1,1'-Biphenyl]-2-yl)ethyl)(methyl)amino)-N-(cyclopropylmethyl)-6-morpholino-pyrimidine-4-carboxamide (Example 71)

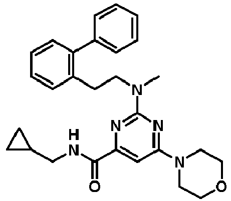

The title compound was prepared according to the general procedure E using 2-chloropyrimidine **I-35** (30 mg, 0.10 mmol, 1 eq), amine **I-71** (31 mg, 0.15 mmol, 1.5 eq) and DiPEA (70 µL, 0.40 mmol, 4 eq). Total heating time: 24 h at 160 °C with µW irradiation. Column chromatography (30% -> 60% EtOAc/pentane) afforded the product (43 mg, 91 µmol, 95%). TLC: R_{f} = 0.4 (30% EtOAc/pentane). ¹H NMR (400 MHz, CDCl₃) δ 7.97 (t, *J* = 5.9 Hz, 1H), 7.41 - 7.19 (m, 9H), 6.68 (s, 1H), 3.77 - 3.70 (m, 4H), 3.66 - 3.57 (m, 6H), 3.28 (dd, *J* = 7.0, 5.8 Hz, 2H), 2.94 - 2.89 (m, 2H), 2.77 (s, 3H), 1.10 - 1.00 (m, 1H), 0.59 - 0.49 (m, 2H), 0.31 - 0.25 (m, 2H). ¹³C NMR (101 MHz, CDCl₃) δ 164.69, 163.87, 160.70, 156.65, 142.44, 141.63, 137.33, 130.26, 129.83, 129.28, 128.15, 127.56, 126.94, 126.29, 89.99, 66.72, 51.00, 44.44, 44.04, 35.31, 30.93, 10.90, 3.47. HRMS [C₂₃H₃₃N₅O₂ + H]⁺ : 472.2707 calculated, 472.2703 found (Δ = -0.78 ppm).

### 1-Benzyl 4-(tert-butyl) 2-phenylpiperazine-1,4-dicarboxylate (I-72)

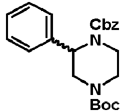

A round bottom flask was charged with *tert*-butyl 3-phenylpiperazine-1-carboxylate (290 mg, 1.1 mmol, 1 eq), NaHCO₃ (462 mg, 5.5 mmol, 5 eq) in THF/H₂O (8 mL, 1:1). The mixture was cooled to 0 °C and CbzCl (189 µL, 1.3 mmol, 1.2 eq) was added dropwise. The reaction mixture was allowed to warm to room temperature and stirred for 1.5 h. The mixture was extracted with EtOAc (3 x 20 mL), the combined organic layers were washed with brine (50 mL), dried (Na₂SO₄), filtered and concentrated under reduced pressure. The residue was purified by silica gel column chromatography (5% -> 40% EtOAc/pentane) affording the product (396 mg, 1.00 mmol, 90%). TLC: R_{f} = 0.4 (20% EtOAc/pentane). ¹H NMR (400 MHz, CDCl₃) δ 7.55 - 7.04 (m, 10H), 5.36 (br s, 1H), 5.27 - 5.12 (m, 2H), 4.50 (d, *J* = 13.9 Hz, 1H), 4.13 - 3.68 (m, 2H), 3.33 (dd, *J* = 14.0, 4.3 Hz, 1H), 3.19 - 2.79 (m, 2H), 1.42 (s, 9H). ¹³C NMR (101 MHz, CDCl₃) δ 155.77, 154.43, 138.40, 136.48, 128.66, 128.61, 128.21, 128.00, 127.39, 126.88, 80.32, 67.64, 54.15, 46.04, 42.81, 39.80, 28.42.

### Benzyl 2-phenylpiperazine-1-carboxylate hydrochloride (I-73)

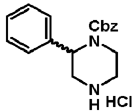

A round bottom flask was charged with compound **I-72** (103 mg, 0.26 mmol, 1 eq) and HCl (4 M solution in 1,4-dioxane, 1 ml, 4 mmol, 15.5 eq) and the reaction was stirred at room temperature. After 1 h the reaction was concentrated under reduced pressure to afford the product as the hydrochloride salt (87 mg, 0.26 mmol, 99%). TLC: R_{f} = 0.3 (50% EtOAc/pentane). ¹H NMR (400 MHz, CDCl₃) δ 10.24 (br s, 1H), 9.33 - 8.80 (m, 1H), 7.51 - 7.02 (m, 10H), 5.55 (br s, 1H), 5.29 - 4.95 (m, 2H), 4.22 (d, *J* = 14.1 Hz, 1H), 3.99 - 3.77 (m, 1H), 3.49 - 3.22 (m, 3H), 3.06 (br s, 1H). ¹³C NMR (101 MHz, CDCl₃) δ 155.26, 135.78, 135.54, 129.31, 128.64, 128.42, 128.28, 128.11, 126.34, 68.19, 51.77, 44.38, 42.59, 36.98.

### Benzyl-4-(4-((cyclopropylmethyl)carbamoyl)-6-morpholinopyrimidin-2-yl)-2-phenylpiperazine-1-carboxylate (Example 74)

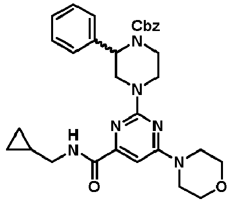

The title compound was prepared according to the general procedure E using 2-chloropyrimidine **I-35** (30 mg, 0.10 mmol, 1 eq), amine **IK91** HCl salt (45 mg, 0.12 mmol, 1.2 eq) and DiPEA (70 µL, 0.40 mmol, 4 eq). Total heating time: 41 h at 120 °C. Column chromatography (40% -> 70% EtOAc/pentane) afforded the product (56 mg, 0.1 mmol, 99%). TLC: R_{f} = 0.3 (50% EtOAc/pentane). ¹H NMR (400 MHz, CDCl₃) δ 7.90 (t, *J* = 5.9 Hz, 1H), 7.45 - 7.19 (m, 10H), 6.77 (s, 1H), 5.47 (br s, 1H), 5.31 - 5.12 (m, 2H), 5.04 (d, *J =* 13.8 Hz, 1H), 4.52 - 4.31 (m, 1H), 4.17 (d, *J* = 9.4 Hz, 1H), 3.75 (t, *J =* 4.8 Hz, 4H), 3.71 - 3.60 (m, 4H), 3.53 (d, *J* = 13.4 Hz, 1H), 3.29 (t, *J* = 6.5 Hz, 2H), 3.17 (d, *J* = 9.2 Hz, 2H), 1.13 - 0.97 (m, 1H), 0.61 - 0.46 (m, 2H), 0.34 - 0.21 (m, 2H). ¹³C NMR (101 MHz, CDCl₃) δ 164.36, 163.88, 160.76, 156.82, 155.79, 139.31, 136.59, 128.68, 128.65, 128.23, 128.03, 127.44, 127.04, 91.44, 67.65, 66.70, 45.36, 44.54, 44.13, 43.82, 39.89, 10.93, 3.53. HRMS [C₃₁H₃₆N₆O₄ + H]⁺ : 557.2871 calculated, 557.2869 found (Δ = -0.39 ppm).

### N-(Cyclopropylmethyl)-6-morpholino-2-(3-phenylpiperazin-1-yl)pyrimidine-4-carboxamide (Example 82)

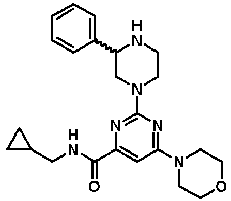

A round bottom flask was charged with Cbz-protected amine **Example 74** (56 mg, 0.1 mmol, 1 eq) and MeOH (0.5 mL). The solution was purged with N₂ and Pd/C 10 wt.% (50 mg, 50 µmol, 0.5 eq) was added. The mixture was purged with N₂ and then with H₂ and stirred for 2 h under an H₂ atmosphere (balloon). The mixture was filtered through a plug of Celite and the filtrate concentrated under reduced pressure. The residue was purified by silica gel column chromatography (1% -> 4% MeOH/DCM) to afford the product (38 mg, 90 µmol, 90%). TLC: R_{f} = 0.3 (2% MeOH/DCM). ¹H NMR (400 MHz, CDCl₃) δ 7.94 (t, *J* = 5.9 Hz, 1H), 7.51 - 7.44 (m, 2H), 7.43 - 7.30 (m, 3H), 6.76 (s, 1H), 4.69 (d, *J* = 12.5 Hz, 2H), 3.88 - 3.78 (m, 1H), 3.78 - 3.71 (m, 4H), 3.68 - 3.61 (m, 4H), 3.32 - 3.23 (m, 2H), 3.21 (d, *J* = 10.6 Hz, 1H), 3.13 - 2.94 (m, 2H), 2.90 (t, *J* = 11.7 Hz, 1H), 2.11 (br s, 1H), 1.09 - 1.00 (m, 1H), 0.57 - 0.47 (m, 2H), 0.30 - 0.24 (m, 2H).¹³C NMR (101 MHz, CDCl₃) δ 164.51, 163.99, 161.01, 156.89, 142.01, 128.73, 127.97, 127.32, 91.05, 66.72, 60.57, 51.48, 46.27, 44.49, 44.26, 44.10, 10.97, 3.55. HRMS [C₂₃H₃₆N₆O₂ + H]⁺ : 423.2503 calculated, 423.2501 (found (Δ = -0.47 ppm).

### 2-(4-Benzyl-3-phenylpiperazin-1-yl)-N-(cyclopropylmethyl)-6-morpholinopyrimidine-4-carboxamide (Example 35)

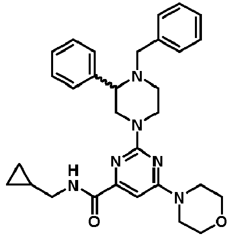

A round bottom flask was charged with amine Example 82 (19 mg, 45 µmol, 1 eq) in dry CH₃CN (0.5 mL). This was followed by DiPEA (16 µL, 90 µmol, 2 eq) and benzyl bromide (6.4 µL, 54 µmol, 1.2 eq). The reaction was stirred for 4 h at rt after which the solvents were concentrated under reduced pressure. The residue was purified by silica gel column chromatography (1 -> 5% MeOH/DCM) affording the product (17 mg, 33 µmol, 74%). TLC: R_{f} = 0.5 (2% MeOH/DCM). ¹H NMR (400 MHz, CDCl₃) δ 7.90 (t, *J* = 5.7 Hz, 1H), 7.57 (d, *J* = 7.2 Hz, 2H), 7.41 (t, *J* = 7.4 Hz, 2H), 7.36 - 7.27 (m, 5H), 7.25 - 7.18 (m, 1H), 6.74 (s, 1H), 4.70 - 4.54 (m, 2H), 3.83 (d, *J* = 13.4 Hz, 1H), 3.77 - 3.67 (m, 4H), 3.66 - 3.54 (m, 4H), 3.35 (dd, *J* = 10.6, 3.1 Hz, 1H), 3.32 - 3.18 (m, 2H), 3.08 (td, *J* = 12.7, 2.7 Hz, 1H), 3.03 - 2.92 (m, 2H), 2.87 (d, *J* = 13.4 Hz, 1H), 2.17 (td, *J* = 11.8, 3.0 Hz, 1H), 1.09 - 0.95 (m, 1H), 0.57 - 0.44 (m, 2H), 0.25 (q, *J* = 4.6 Hz, 2H). ¹³C NMR (101 MHz, CDCl₃) δ 164.48, 164.02, 160.74, 156.91, 141.70, 138.93, 128.92, 128.87, 128.29, 128.20, 127.87, 127.00, 90.97, 67.34, 66.71, 59.23, 51.90, 51.73, 44.47, 44.36, 44.08, 10.92, 3.53, 3.51. HRMS [C₃₆H₃₆N₆O₂ + H]⁺ : 513.2973 calculated, 513.2973 found (Δ = 0.01 ppm).

### 2,6-Dichloro-N-(prop-2-yn-1-yl)pyrimidine-4-carboxamide (I-74)

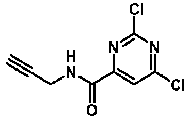

The title compound was prepared according to General Procedure A using 2,6-dichloropyrimidine-4-carbonyl chloride **I-1** (0.25 mL, 2.0 mmol, 1 eq), Et₃N (0.36 mL, 2.60 mmol, 1.3 eq) and propargylamine (0.13 mL, 2.05 mmol, 1.025 eq). Column chromatography (5% -> 20% EtOAc/pentane) afforded the product (0.44 g, 1.91 mmol, 96%). TLC: R_{f} = 0.6 (20% EtOAc/pentane). ¹H NMR (400 MHz, CDCl₃) δ 8.24 - 8.00 (m, 2H), 4.30 (dd, *J* = 5.7, 2.6 Hz, 2H), 2.36 (t, *J* = 2.6 Hz, 1H). ¹³C NMR (101 MHz, CDCl₃) δ 164.92, 160.08, 159.94, 159.63, 118.35, 78.30, 72.42, 29.52. HRMS [C₁₄H₁₉ClN₄O₂ + H]⁺ : 295.1320 calculated, 295.1321 found (Δ = 0.20 ppm).

### 2-Chloro-6-morpholino-N-(prop-2-yn-1-yl)pyrimidine-4-carboxamide (I-75)

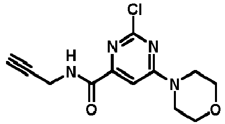

The title compound was prepared according to General Procedure B using dichloropyrimidine **I-74** (221 mg, 0.96 mmol, 1 eq), DiPEA (251 µL, 1.44 mmol, 1.5 eq) and morpholine (88 µL, 1.01 mmol, 1.05 eq). Column chromatography (40% -> 60% EtOAc/pentane) afforded the product (249 mg, 0.89 mmol, 92%). TLC: R_{f} = 0.5 (50% EtOAc/pentane). ¹H NMR (400 MHz, CDCl₃) δ 8.00 (br s, 1H), 7.26 (s, 1H), 4.22 (dd, *J* = 5.7, 2.6 Hz, 2H), 3.88 - 3.59 (m, 8H), 2.29 (t, *J* = 2.6 Hz, 1H). ¹³C NMR (101 MHz, CDCl₃) δ 163.84, 162.23, 160.04, 157.20, 99.53, 78.84, 72.05, 66.41, 29.32. HRMS [C₁₄H₁₉ClN₄O₂ + H]⁺ : 295.1320 calculated, 295.1321 found (Δ = 0.20 ppm).

### 2-(Methyl(phenethyl)amino)-6-morpholino-N-(prop-2-yn-1-yl)pyrimidine-4-carboxamide (Example 8)

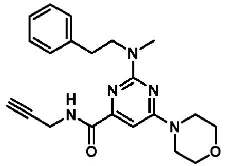

The title compound was prepared according to General Procedure E using 2-chloropyrimidine **I-75** (42 mg, 0.15 mmol, 1 eq), DiPEA (105 µL, 0.60 mmol, 4 eq) and N-methylphenethylamine HBr salt (49 mg, 0.225 mmol, 1.5 eq). Total heating time: 45 h at 120 °C. Column chromatography (30% -> 50% EtOAc/pentane) afforded the product (43 mg, 0.11 mmol, 76%). TLC: R_{f} = 0.7 (50% EtOAc/pentane). ¹H NMR (400 MHz, CDCl₃) δ 8.03 (br s, 1H), 7.33 - 7.25 (m, 2H), 7.25 - 7.17 (m, 3H), 6.69 (s, 1H), 4.22 (dd, *J* = 5.6, 2.5 Hz, 2H), 3.86 - 3.72 (m, 6H), 3.72 - 3.60 (m, 4H), 3.11 (s, 3H), 2.96 - 2.85 (m, 2H), 2.29 (t, *J* = 2.4 Hz, 1H). ¹³C NMR (101 MHz, CDCl₃) δ 164.60, 163.89, 160.88, 156.03, 139.91, 128.90, 128.60, 126.30, 90.15, 79.57, 71.67, 66.72, 51.70, 44.50, 35.75, 33.98, 29.21. HRMS [C₂₁H₂₅N₅O₂ + H]⁺ : 380.2081 calculated, 380.2089 found (Δ = -3.16 ppm).

### (S)-2-Chloro-6-(3-hydroxypyrrolidin-1-yl)-N-(prop-2-yn-1-yl)pyrimidine-4-carboxamide (I-76)

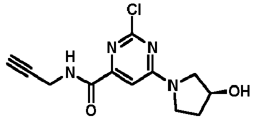

The title compound was prepared according to General Procedure B using dichloropyrimidine **I-74** (138 mg, 0.60 mmol, 1 eq), DiPEA (261 µL, 1.5 mmol, 2.5 eq) and (S)-3-hydroxypyrrolidine HCl salt (78 mg, 0.63 mmol, 1.05 eq). Column chromatography (80% -> 100% EtOAc/pentane) afforded the product (139 mg, 0.50 mmol, 83%). TLC: R_{f} = 0.2 (90% EtOAc/pentane). ¹H NMR (400 MHz, CDCl₃) δ 8.13 (t, *J* = 5.6 Hz, 1H), 7.06 - 6.89 (m, 1H), 4.73 - 4.57 (m, 1H), 4.29 - 4.15 (m, 2H), 3.88 - 3.19 (m, 5H), 2.33 (t, *J* = 2.5 Hz, 1H), 2.23 - 2.07 (m, 2H). ¹³C NMR (101 MHz, CDCl₃) δ 162.58, 162.16, 162.01, 159.71, 159.59, 155.59, 100.70, 100.64, 78.74, 72.13, 70.43, 69.73, 55.57, 55.28, 45.29, 45.03, 33.79, 33.26, 29.28.

### tert-Butyl (S)-4-(4-(3-hydroxypyrrolidin-1-yl)-6-(prop-2-yn-1-ylcarbamoyl)pyrimidin-2-yl)piperazine-1-carboxylate (I-77)

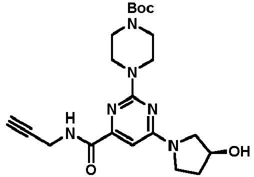

The title compound was prepared according to General Procedure E using 2-chloropyrimidine **I-76** (117 mg, 0.42 mmol, 1 eq), DiPEA (218 µL, 1.25 mmol, 3 eq) and 1-Boc-piperazine (110 mg, 0.59 mmol, 1.4 eq). Stirring at 100 °C for 48 h. Column chromatography (80% -> 100% EtOAc/pentane) afforded the product (163 mg, 0.38 mmol, 90%). TLC: R_{f} = 0.3 (90% EtOAc/pentane). ¹H NMR (400 MHz, CDCl₃) δ 8.10 (t, *J* = 5.4 Hz, 1H), 6.63 - 6.27 (m, 1H), 4.56 (s, 1H), 4.20 (dd, *J* = 5.5, 2.2 Hz, 2H), 3.88 - 3.69 (m, 5H), 3.69 - 3.51 (m, 3H), 3.51 - 3.35 (m, 5H), 2.31 (t, *J* = 2.4 Hz, 1H), 2.19 - 1.95 (m, 2H), 1.49 (s, 9H). ¹³C NMR (101 MHz, CDCl₃) δ 164.62, 161.73, 160.68, 154.88, 154.51, 92.43, 79.97, 79.37, 71.64, 70.56, 69.81, 54.74, 44.36, 43.67, 34.00, 33.16, 28.99, 28.42.

### (S)-6-(3-Hydroxypyrrolidin-1-yl)-N-(prop-2-yn-1-yl)-2-(4-(4-(3-(trifluoromethyl)-3H-diazirin-3-yl)benzyl)piperazin-1-yl)pyrimidine-4-carboxamide (Example 44)

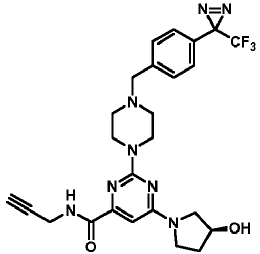

*Boc deprotection:* A round bottom flask was charged with Boc-piperazine **I-77** (160 mg, 0.37 mmol, 1 eq) and 4M HCl in dioxane (2 mL, 8 mmol, 21 eq). After stirring for 2h at rt the solvents were evaporated under reduced pressure affording the HCl salt, which was used for the next step without further purification (135 mg, 0.37 mmol, quant.) *Alkylation*: A round bottom flask was charged with amine HCl salt (26 mg, 45 µmol, 1 eq) in dry CH₃CN (1 mL). This was followed by addition of DiPEA (37 µL, 0.21 mmol, 3 eq) and 4-[3-(trifluoromethyl)-3H-diazirin-3-yl]benzyl bromide (14 µL, 85 µmol, 1.2 eq). The reaction was stirred in the dark for 3 h at rt after which the solvents were evaporated under reduced pressure. The residue was purified by silica gel column chromatography (2.5 -> 7.5% MeOH/DCM) affording the product (12 mg, 23 µmol, 32%). TLC: R_{f} = 0.4 (5% MeOH/DCM). ¹H NMR (500 MHz, CDCl₃) δ 7.99 (t, *J* = 5.5 Hz, 1H), 7.40 (d, *J* = 8.2 Hz, 2H), 7.16 (d, *J* = 8.0 Hz, 2H), 6.54 (s, 1H), 4.58 (br s, 1H), 4.20 (dd, *J* = 5.6, 2.5 Hz, 2H), 3.80 (br s, 4H), 3.62 (br s, 2H), 3.56 (s, 2H), 3.43 (s, 1H), 2.58 - 2.34 (m, 4H), 2.25 (t, *J* = 2.5 Hz, 1H), 2.08 (br s, 2H), 1.71 (br s, 2H). ¹³C NMR (126 MHz, CDCl₃) δ 122.28 (q, *J* = 274.7, 273.4 Hz). ¹³C NMR (126 MHz, CDCl₃) δ 164.73, 162.05, 160.92, 154.93, 140.07, 129.66, 128.09, 126.55, 122.28 (q, *J* = 274.4 Hz), 92.22, 79.56, 71.65, 70.36, 62.62, 54.92, 53.17, 44.36, 43.91, 34.25, 29.15, 28.51 (q, *J* = 40.4 Hz). HRMS [C₂₅H₂₇F₃N₃O₂ + H]⁺ : 529.2282 calculated, 529.2296 found (Δ = 2.68 ppm).

### (S)-6-(3-Hydroxypyrrolidin-1-yl)-N-(prop-2-yn-1-yl)-2-(4-(4-(3-(trifluoromethyl)-3H-diazirin-3-yl)benzoyl)piperazin-1-yl)pyrimidine-4-carboxamide (Example 45)

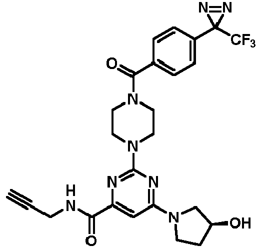

*Boc deprotection:* A round bottom flask was charged with Boc-piperazine **I-77** (160 mg, 0.37 mmol, 1 eq) and 4M HCl in dioxane (2 mL, 8 mmol, 21 eq). After stirring for 2h at rt the solvents were evaporated under reduced pressure affording the HCl salt, which was used for the next step without further purification (135 mg, 0.37 mmol, quant.) *Amide coupling:* A round bottom flask was charged with amine HCl salt (30 mg, 82 µmol, 1 eq) in dry DMF (1 mL) at 0 °C. This was followed by addition of DiPEA (57 µL, 0.33 mmol, 4 eq), 4-[3-(trifluoromethyl)-3H-diazirin-3-yl]benzoic acid (19 mg, 82 µmol, 1.2 eq) and PyBOP (47 mg, 90 µmol, 1.1 eq). The reaction was stirred in the dark overnight warming up to rt after which the solvents were evaporated under reduced pressure and the residue coevaporated with toluene. The residue was purified by silica gel column chromatography (2.5 -> 7.5% MeOH/DCM) affording the product (8 mg, 15 µmol, 18%). TLC: R_{f} = 0.5 (100% EtOAc). ¹H NMR (500 MHz, MeOD) δ 7.58 (d, *J* = 8.5 Hz, 2H), 7.38 (d, *J* = 8.1 Hz, 2H), 6.60 - 6.40 (m, 1H), 4.59 - 4.41 (m, 1H), 4.18 - 4.07 (m, 2H), 3.97 (br s, 2H), 3.83 (br s, 4H), 3.76 - 3.49 (m, 4H), 3.49 - 3.34 (m, 2H), 2.58 (t, *J* = 2.5 Hz, 1H), 2.22 - 1.91 (m, 2H). ¹³C NMR (126 MHz, MeOD) δ 171.27, 166.81, 163.30, 162.25, 156.47, 138.57, 131.69, 128.99, 127.98, 123.47 (q, *J* = 273.7 Hz), 93.19, 80.63, 79.48, 71.91, 70.77, 55.53, 45.34, 44.64, 43.49, 34.73, 34.02, 29.44 (d, *J* = 40.5 Hz), 29.43. HRMS [C₂₅H₂₅F₃N₃O₃ + H]⁺ : 543.2075 calculated, 543.2085 found (Δ = 1.93 ppm).

### Methyl 2-chloro-6-morpholinopyrimidine-4-carboxylate (I-78)

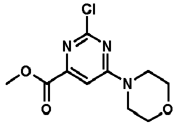

The title compound was prepared according to General Procedure B using methyl 2,6-dichloropyrimidine-4-carboxylate (621 mg, 3.0 mmol, 1 eq), DiPEA (0.78 mL, 4.5 mmol, 1.5 eq) and morpholine (0.27 mL, 3.15 mmol, 1.05 eq). Column chromatography (40% - > 60% EtOAc/pentane) afforded the product (684 mg, 2.65 mmol, 88%). TLC: R_{f} = 0.3 (50% EtOAc/pentane). ¹H NMR (400 MHz, CDCl3) δ 7.19 (s, 1H), 3.96 (s, 3H), 3.91 - 3.55 (m, 8H). ¹³C NMR (101 MHz,) δ 163.80, 163.09, 160.65, 155.10, 101.89, 65.83, 52.84, 44.18.

### 2-(Methyl(phenethyl)amino)-6-morpholinopyrimidine-4-carboxylic acid (I-79)

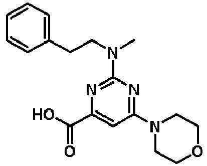

*Ester hydrolysis:* A round bottom flask was charged with methyl ester **I-78** (680 mg, 2.64 mmol, 1 eq) in 12.5 mL THF/MeOH (4:1). A 1.5M aqueous NaOH solution (1.76 mL, 2.64 mmol, 1 eq) was added together with 0.75 mL H₂O. The reaction was stirred overnight at rt after which the solvents where evaporated yielding the product which was used without further purification (779 mg, 2.64 mmol, quant.). *S_{N}Ar reaction:* The title compound was prepared according to General Procedure E using 2-chloropyrimidine (244 mg, 1.0 mmol, 1 eq), DiPEA (0.52 mL, 3.0 mmol, 3 eq) and N-methylphenethylamine (189 µL, 1.3 mmol, 1.3 eq). Total heating time: 6 d at 120 °C. Column chromatography (2.5% -> 15% MeOH/DCM) afforded the product (175 mg, 0.51 mmol, 51%). TLC: R_{f} = 0.5 (100% EtOAc with 3 drops of AcOH). ¹H NMR (400 MHz, MeOD + CDCl₃) δ 7.35 - 7.26 (m, 2H), 7.26 - 7.15 (m, 3H), 6.87 (s, 1H), 3.91 (t, *J* = 7.0 Hz, 2H), 3.81 (br s, 8H), 3.17 (s, 3H), 2.97 (t, *J* = 7.0 Hz, 2H). ¹³C NMR (101 MHz, MeOD + CDCl₃) δ 161.70, 152.17, 148.40, 137.57, 133.96, 128.50, 128.38, 126.57, 93.89, 66.02, 51.86, 45.10, 33.13. HRMS [C₁₈H₂₂N₄O₃ + H]⁺ : 343.1765 calculated, 343.1772 found (Δ = 2.13 ppm).

### N-Cyclopropyl-2-(methyl(phenethyl)amino)-6-morpholinopyrimidine-4-carboxamide (Example 13)

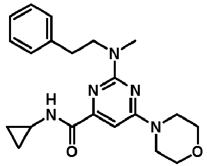

The title compound was prepared according to General Procedure F using the carboxylic acid **I-79** (34 mg, 0.10 mmol, 1 eq), DiPEA (52 µL, 0.30 mmol, 3 eq), PyBOP (78 mg, 0.12 mmol, 1.2 eq) and cyclopropylamine (8.3 µL, 0.12 mmol, 1.2 eq). Column chromatography (50% -> 80% EtOAc/pentane) afforded the product (8 mg, 44 µmol, 66%). TLC: R_{f} = 0.3 (60% EtOAc/pentane). ¹H NMR (400 MHz, CDCl₃) δ 7.92 (br s, 1H), 7.34 - 7.27 (m, 2H), 7.25 - 7.15 (m, 3H), 6.70 (s, 1H), 3.84 - 3.72 (m, 6H), 3.66 (br s, 4H), 3.09 (s, 3H), 2.94 - 2.82 (m, 3H), 1.36 - 1.21 (m, 1H), 0.92 - 0.82 (m, 2H), 0.67 - 0.58 (m, 2H). ¹³C NMR (101 MHz, CDCl₃) δ 166.25, 163.97, 160.84, 156.51, 139.98, 128.84, 128.64, 126.34, 89.86, 66.78, 51.67, 44.52, 35.72, 33.98, 22.53, 6.78. HRMS [C₂₁H₂₇N₅O₂ + H]⁺ : 382.2238 calculated, 382.2241 found (Δ = 0.92 ppm).

### N-Propyl-2-(methyl(phenethyl)amino)-6-morpholino-pyrimidine-4-carboxamide (Example 14)

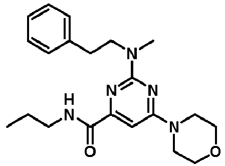

The title compound was prepared according to General Procedure F using the carboxylic acid **I-79** (23 mg, 67 µmol, 1 eq), DiPEA (60 µL, 0.34 mmol, 3 eq), PyBOP (52 mg, 0.10 mmol, 1.5 eq) and propylamine HCl salt (8 mg, 80 µmol, 1.2 eq). Column chromatography (40% -> 60% EtOAc/pentane) afforded the product (17 mg, 44 µmol, 66%). TLC: R_{f} = 0.3 (50% EtOAc/pentane). ¹H NMR (400 MHz, CDCl₃) δ 7.99 (br s, 1H), 7.37 - 7.28 (m, 2H), 7.28 - 7.19 (m, 3H), 6.74 (s, 1H), 3.90 - 3.75 (m, 6H), 3.69 (br s, 4H), 3.42 (q, *J* = 6.7 Hz, 2H), 3.13 (s, 3H), 2.92 (t, *J* = 7.5 Hz, 2H), 1.71 - 1.62 (m, 2H), 1.02 (t, *J* = 7.3 Hz, 3H). ¹³C NMR (101 MHz, CDCl₃) δ 164.80, 164.01, 160.86, 156.82, 139.98, 128.85, 128.61, 126.31, 90.06, 66.77, 51.65, 44.52, 41.08, 35.73, 33.98, 23.01, 11.61. HRMS [C₂₁H₂₉N₅O₂ + H]⁺ : 384.2394 calculated, 384.2394 found (Δ = 0.0 ppm).

### N-(2-Hydroxyethyl)-2-(methyl(phenethyl)amino)-6-morpholinopyrimidine-4-carboxamide (Example 36)

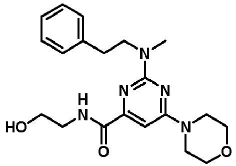

The title compound was prepared according to General Procedure F using the carboxylic acid **I-79** (39 mg, 0.11 mmol, 1 eq), DiPEA (60 µL, 0.34 mmol, 3 eq), PyBOP (89 mg, 0.17 mmol, 1.5 eq) and ethanolamine (34 µL, 0.57 mmol, 5 eq). Column chromatography (70% -> 100% EtOAc/pentane to 5% MeOH/EtOAc) afforded the product (25 mg, 65 µmol, 59%). TLC: R_{f} = 0.3 (80% EtOAc/pentane). ¹H NMR (400 MHz, CDCl₃) δ 8.29 (br s, 1H), 7.33 - 7.27 (m, 2H), 7.24 - 7.16 (m, 3H), 6.70 (s, 1H), 3.87 - 3.78 (m, 4H), 3.78 - 3.74 (m, 4H), 3.70 - 3.62 (m, 4H), 3.62 - 3.55 (m, 2H), 3.10 (s, 3H), 2.98 - 2.80 (m, 3H). ¹³C NMR (101 MHz, CDCl₃) δ 166.19, 163.93, 160.87, 156.29, 139.96, 128.89, 128.61, 126.32, 90.14, 66.74, 62.71, 51.61, 44.51, 42.71, 35.77, 33.98. HRMS [C₂₆H₂₇N₅O₃ + H]⁺ : 386.2187 calculated, 386.2191 found (Δ = 1.11 ppm).

### Methyl 2-chloro-6-morpholinoisonicotinate (I-80)

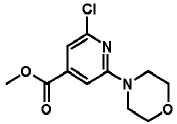

A round bottom flask was charged with methyl-2,6-dichloroisonicotinate (410 mg, 1.99 mmol, 1 eq), K₂CO₃ (549 mg, 3.97 mmol, 2 eq) morpholine (259 µL, 3.00 mmol, 1.5 eq) and dry MeCN (10 mL). The mixture was heated to reflux. After 45 h the reaction was complete as judged by TLC and cooled to room temperature. The mixture was filtered and the filtrate concentrated under reduced pressure. The crude material was purified by silica gel column chromatography (10% -> 30% EtOAc/pentane) affording the product (323 mg, 1.26 mmol, 66%). TLC: R_{f} = 0.6 (30% EtOAc/pentane). ¹H NMR (400 MHz, CDCl₃) δ 7.13 (d, *J* = 0.9 Hz, 1H), 7.07 (d, *J* = 1.0 Hz, 1H), 3.92 (s, 3H), 3.86 - 3.76 (m, 4H), 3.64 - 3.52 (m, 4H). ¹³C NMR (101 MHz, CDCl₃) δ 165.16, 159.40, 150.36, 141.33, 111.77, 104.69, 66.55, 52.83, 45.26. HRMS [C₁₁H₁₃ClN₂O₃ + H]⁺ : 257.0688 calculated, 257.0690 found (Δ = 0.86 ppm).

### 2-Chloro-6-morpholinoisonicotinic acid (I-81)

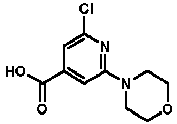

A round bottom flask was charged with methyl ester **I-80** (323 mg, 1.26 mmol, 1 eq) and THF (5 mL). A 1 M aqueous solution of NaOH (2.52 mL, 2.52 mmol, 2 eq) was added dropwise. After stirring 20 minutes at room temperature the reaction mixture was acidified carefully with 37% w/w HCl to pH 1 and THF was removed under reduced pressure. The residue was extracted with DCM (3 x 10 mL), the combined organic layers were washed with brine (1 x 15 mL), dried (MgSO₄), filtered and concentrated under reduced pressure affording the product (325 mg, 1.26 mmol, 99%). TLC: R_{f} = 0.2 (30% EtOAc/pentane with 3 drops of AcOH). ¹H NMR (400 MHz, MeOD + CDCl₃) δ 7.12 (s, 1H), 7.09 (s, 1H), 3.78 (t, *J* = 4.9 Hz, 4H), 3.54 (t, *J* = 4.9 Hz, 4H). ¹³C NMR (101 MHz, MeOD + CDCl₃) δ 167.10, 160.29, 150.79, 143.21, 112.56, 105.74, 67.18, 45.94. HRMS [C₁₀H₁₁ClN₂O₃ + H]⁺ : 243.0531 calculated, 243.0533 found (Δ = 0.70 ppm).

### 2-Chloro-N-(cyclopropylmethyl)-6-morpholinoisonicotinamide (I-82)

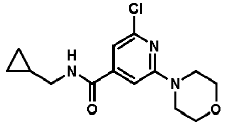

A round bottom flask was charged with carboxylic acid **I-81** (325 mg, 1.26 mmol, 1 eq), EDC hydrochloride (302 mg, 1.95 mmol, 1.5 eq) and HOBt (298 mg, 1.95 mmol, 1.5 eq) and dry DCM (7 mL). The suspension was stirred for 1 h at room temperature followed by addition of cyclopropylmethanamine (139 µL, 1.6 mmol, 1.2 eq). After 20 h DCM was removed under reduced pressure and the residue was dissolved in EtOAc (15 mL) and sequentially washed with 1M HCl (aq) (2 x 15 mL), sat. aq. NaHCO₃ (2 x 15 mL) and brine (1 x 20 mL). The organic layer was dried (MgSO₄), filtered and concentrated under reduced pressure. The resulting crude material is purified by silica gel column chromatography (30% -> 60% EtOAc/pentane) affording the product (296 mg, 1 mmol, 80%). TLC: R_{f} = 0.3 (40% EtOAc/pentane). ¹H NMR (400 MHz, CDCl₃) δ 6.91 (s, 1H), 6.81 (s, 1H), 6.45 - 6.31 (m, 1H), 3.84 - 3.73 (m, 4H), 3.66 - 3.51 (m, 4H), 3.28 (dd, *J* = 7.2, 5.4 Hz, 2H), 1.12 - 0.98 (m, 1H), 0.64 - 0.52 (m, 2H), 0.38 - 0.21 (m, 2H). ¹³C NMR (101 MHz, CDCl₃) δ 165.28, 159.52, 150.25, 146.27, 109.10, 103.25, 66.61, 45.27, 45.25, 10.68, 3.73.

### N-(Cyclopropylmethyl)-2-(methyl(phenethyl)amino)-6-morpholino isonicotinamide (Example 75)

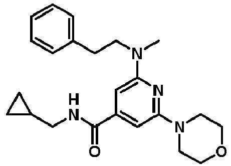

A microwave vial with a magnetic stir bar under N₂ was charged with 2-chloropyridine **I-82** (31 mg, 0.1 mmol, 1 eq), *N*-methylphenethylamine hydrobromide (28 mg, 0.13 mmol, 1.3 eq) and dry toluene (0.1 mL). The vial was capped and the solution purged with N₂. This was followed by the addition of RuPhosPd G3 (0.01 M THF solution, 100 µL, 1 µmol, 0.01 eq) and sodium *tert*-butoxide (2 M THF solution, 120 µL, 0.24 mmol, 2.4 eq) and the mixture is purged again with N₂ and stirred in a preheated oil bath at 110 °C. After 24 h the reaction was complete as judged by LC/MS. The mixture was filtered through a plug of Celite and the filtrate concentrated under reduced pressure to provide the crude material. Purification by HPLC (C18 reverse phase, 45% -> 55% ACN/H₂O + 0.2% TFA, RT 12.3 min) afforded the product (16 mg, 40 µmol, 41%). TLC: R_{f} = 0.5 (60% EtOAc/pentane). ¹H NMR (400 MHz, CDCl₃) δ 7.35 - 7.24 (m, 2H), 7.25 - 7.14 (m, 3H), 6.24 - 6.13 (m, 2H), 6.10 (s, 1H), 3.87 - 3.78 (m, 4H), 3.78 - 3.67 (m, 2H), 3.59 - 3.47 (m, 4H), 3.28 (dd, *J* = 7.2, 5.4 Hz, 2H), 2.98 (s, 3H), 2.93 - 2.82 (m, 2H), 1.15 - 0.95 (m, 1H), 0.64 - 0.48 (m, 2H), 0.35 - 0.19 (m, 2H). ¹³C NMR (101 MHz, CDCl3) δ 168.07, 159.06, 157.76, 145.86, 140.05, 128.98, 128.60, 126.26, 92.90, 91.48, 66.94, 52.51, 45.80, 44.97, 36.87, 33.92, 10.82, 3.68. HRMS [C₂₃H₃₆N₄O₂ + H]⁺ : 395.2442 calculated, 395.2434 found (Δ = -1.85 ppm).

### Methyl 4-chloro-6-(methyl(phenethyl)amino)picolinate (I-83)

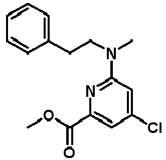

A round bottom flask was charged with methyl 4,6-dichloro-picolinate (206 mg, 0.99 mmol, 1 eq), *N*-methylphenethylamine hydrobromide (218 mg , 1.01 mmol, 1.02 eq), DiPEA (436 µL, 2.5 mmol, 2.5 eq) and dry MeOH (2 mL). The solution was stirred at rt for 3 d and then refluxed for 24 h. The reaction mixture was concentrated under reduced pressure and the residue was purified by silica gel column chromatography (5% -> 35% EtOAc/pentane) affording the product (122 mg, 0.40 mmol, 41%). TLC: R_{f} = 0.2 (5% EtOAc/pentane). ¹H NMR (400 MHz, CDCl₃) δ 7.35 - 7.26 (m, 3H), 7.23 (t, *J* = 7.3 Hz, 1H), 7.16 (d, *J* = 7.0 Hz, 2H), 6.56 (d, *J* = 2.3 Hz, 1H), 3.96 (s, 3H), 3.63 (t, *J* = 7.3 Hz, 2H), 2.93 - 2.81 (m, 5H). ¹³C NMR (101 MHz, CDCl₃) δ 165.68, 155.45, 152.41, 147.85, 138.19, 128.84, 128.80, 126.84, 107.83, 107.79, 53.84, 53.05, 38.50, 33.14. The regioselectivity was confirmed by HMBC-NMR. HRMS [C₁₆H₁₇N₂O₂ + H]⁺ : 305.1051 calculated, 305.1054 (found (Δ = 0.72 ppm).

### 4-Chloro-6-(methyl(phenethyl)amino)picolinic acid (I-84)

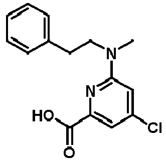

A round bottom flask was charged with methyl ester **I-83** (122 mg, 0.4 mmol, 1 eq) and THF (2 mL). An aqueous 1.5 M NaOH solution (0.53 mL, 0.8 mmol, 2 eq) was added dropwise and the reaction was stirred for 1.5 h at rt. The mixture was cooled to 0 °C and acidified to pH 1 by dropwise addition of 37% w/w aq. HCl. The mixture was then extracted with DCM (3 x 5 mL), the combined organic layers washed with brine (1 x 15 mL), dried (MgSO₄), filtered and concentrated under reduced pressure to afford the product (104 mg, 0.36 mmol, 89%). TLC R_{f} = 0.1 (5% MeOH/DCM with 3 drops of AcOH). ¹H NMR (400 MHz, CDCl₃) δ 8.92 (br s, 1H), 7.37 - 7.20 (m, 4H), 7.20 - 7.12 (m, 2H), 6.57 (d, *J* = 2.4 Hz, 1H), 3.66 (t, *J* = 7.2 Hz, 2H), 2.96 - 2.81 (m, 5H). ¹³C NMR (101 MHz, CDCl₃) δ 164.25, 156.33, 150.90, 146.25, 138.01, 128.96, 128.86, 127.01, 108.27, 105.87, 54.04, 38.78, 33.21.

### 4-Chloro-N-(cyclopropylmethyl)-6-(methyl(phenethyl)amino) picolinamide (I-85)

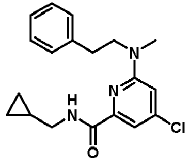

A round bottom flask was charged with carboxylic acid **I-84** (104 mg, 0.36 mmol, 1 eq), HOBt (73 mg, 0.47 mmol, 1.3 eq), EDC hydrochloride (102 mg, 0.53 mmol, 1.5 eq) and dry DCM (1.8 mL). The suspension was stirred for 1 h at rt followed by the addition of cyclopropylmethanamine (37 µL, 0.43 mmol, 1.2 eq). After stirring for 20 h the solvent was removed under reduced pressure and the residue was dissolved in EtOAc (10 mL) and washed with 1 M aq. HCl (1 x 10 mL), sat. aq. NaHCO₃ (1 x 10 mL) and brine (1 x 10 mL). The organic layer was dried (MgSO₄), filtered and concentrated under reduced pressure. The crude material was purified by silica gel column chromatography (isocratic, 30% EtOAc/pentane) affording the product (30 mg, 87 µmol, 9%), R_{f} = 0.35 (30% EtOAc/pentane). ¹H NMR (400 MHz, CDCl₃) δ 7.99 (t, *J* = 5.9 Hz, 1H), 7.43 (d, *J* = 2.4 Hz, 1H), 7.35 - 7.26 (m, 2H), 7.28 - 7.18 (m, 1H), 7.20 - 7.15 (m, 2H), 6.50 (d, *J* = 2.4 Hz, 1H), 3.64 (t, *J* = 7.3 Hz, 2H), 3.30 (dd, *J* = 7.1, 5.9 Hz, 2H), 2.91 - 2.84 (m, 5H), 1.13 - 1.01 (m, 1H), 0.58 - 0.52 (m, 2H), 0.31 - 0.26 (m, 2H). ¹³C NMR (101 MHz, CDCl₃) δ 163.90, 155.94, 151.08, 150.38, 138.41, 128.93, 128.90, 126.86, 106.84, 104.87, 54.02, 44.51, 38.71, 33.25, 10.92, 3.76.

### N-(Cyclopropylmethyl)-6-(methyl(phenethyl)amino)-4-morpholinopicolinamide (Example 79)

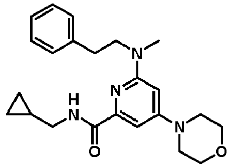

A microwave vial with a magnetic stir bar under N₂ was charged with 4-chloropyridine **I-85** (30 mg, 87 µmol, 1 eq), morpholine (9 µL, 0.1 mmol, 1.2 eq) and dry toluene (87 µL). The vial was capped and the solution purged with N₂. This was followed by the addition of RuPhosPd G3 (0.01 M THF solution, 237 µL, 2.37 µmol, 0.027 eq) and sodium *tert*-butoxide (2 M THF solution, 97 µL, 0.19 mmol, 2.2 eq) and the mixture was purged again with N₂ and stirred in a preheated oil bath at 110 °C for 44 h. The mixture was filtered through a plug of Celite and the filtrate concentrated under reduced pressure. The crude material was purified by silica gel column chromatography (30% -> 60% EtOAc/pentane) affording the product (5 mg, 13 µmol, 15%). TLC: R_{f} = 0.2 (30% EtOAc/pentane) and recovered starting material (11 mg, 32 µmol, 37%). ¹H NMR (500 MHz, CDCl₃) δ 8.03 (t, *J* = 5.7 Hz, 1H), 7.29 (t, *J* = 7.3 Hz, 2H), 7.24 - 7.13 (m, 4H), 5.77 (d, *J* = 2.0 Hz, 1H), 3.91 - 3.81 (m, 4H), 3.63 (t, *J* = 7.4 Hz, 2H), 3.51 - 3.40 (m, 4H), 3.34 - 3.29 (m, 2H), 2.92 - 2.83 (m, 5H), 1.11 - 1.05 (m, 1H), 0.58 - 0.50 (m, 2H), 0.29 (q, *J* = 4.7 Hz, 2H). ¹³C NMR (126 MHz, CDCl₃) δ 165.54, 160.20, 156.01, 148.69, 139.16, 129.00, 128.80, 126.61, 99.04, 90.28, 66.98, 54.09, 46.37, 44.16, 38.55, 33.55, 11.08, 3.57. HRMS [C₂₃H₃₆N₄O₂ + H]⁺ : 395.2442 calculated, 395.2438 (found (Δ = - 0.78 ppm).

### Biological Examples

### Example 2-1: In vitro NAPE-PLD activity assay

### NAPE-PLD plasmids

Full length human cDNA NAPE-PLD was obtained from Natsuo Ueda¹ and cloned into mammalian expression vector pcDNA3.1, containing a C-terminal Flag-tag and genes for ampicillin and neomycin resistance. All plasmids were grown in XL-10 Z-competent cells and prepped (Maxi Prep, Qiagen). Sequence analysis for the confirmation of the sequences was performed at the Leiden Genome Technology Centre.

### Cell culture

HEK293T were cultured at 37 °C and 7% CO₂ in DMEM (Sigma Aldrich, D6546) with glutamax, penicillin (100 µg/ml), streptomycin (100 µg/ml) and 10% New Born Calf Serum iron supplemented (Hyclone SH30072.03). Cells were passaged twice a week to appropriate confluence.

### Transient transfection

24 hours before transfection 10⁷ cells are seeded on a 15 cm dish. Two hours before transfection medium is refreshed, low volume. Transfection is performed with PEI in a ratio of 3:1 with human NAPE-PLD or Mock pcDNA3.1 Neo, 20 µg per dish. Medium is refreshed after 24 hours and cells are harvested after 72 hours.

### Membrane preparation

Cell pellets are re-suspended in lysis buffer: 20 mM Hepes, 2 mM DTT, 0.25 M sucrose, 1 mM MgCl₂, 2.5 U/ml benzonase and incubated 30 minutes on ice. The cytosolic fraction (supernatant) is separated from the membranes by ultra-centrifugation (32000 rpm for 30 minutes 100,000g). The pellet is resuspended in storage buffer: 20 mM Hepes, 2 mM DTT (membrane fraction). All samples are stored at -80°C. Enzyme concentrations are determined using a Bradford assay.

### NAPE-PLD activity assay

The NAPE-PLD activity assay is based on a previously reported method with small alterations.² The membrane protein fraction from transient overexpression of human NAPE-PLD in HEK293T cells was diluted to 0.4 mg/ml in assay buffer: 50 mM Tris-HCl (pH 7.5), 0.02% Triton X-100, 150 mM NaCl. The substrate PED6 (Invitrogen) 1 mM stock was consecutively diluted in DMSO (10x) and in assay buffer (10x) to make a 10 µM working solution. Relevant concentrations of compounds (100x working solution) are prepared in DMSO. The assay is performed in a dark Greiner 96 wells plate, end volume 100 µl. The compound or DMSO is incubated with membrane protein lysate (final concentration 0.04 mg/ml) for 30 minutes at 37 °C. Then, substrate PED6 is added (final concentration 1 µM) and the measurement is started immediately on a TECAN infinite M1000 pro at 37 °C (excitation 485 nm, emission 535 nm), scanning every 2 minutes for 1 hour. Mock lysate with DMSO is used for background substraction. IC₅₀ curves are generated used Graphpad Prism v6.

**Table 1.**

| **Compound** | **pIC₅₀** | **SD** |
|---|---|---|
| **1** | 5.28 | 0.05 |
| **2** | 5.05 | 0.08 |
| **3** | 6.19 | 0.08 |
| **4** | 5.34 | 0.11 |
| **5** | 5.58 | 0.08 |
| **6** | 5.15 | 0.09 |
| **7** | 5.80 | 0.04 |
| **8** | 6.04 | 0.06 |
| **9** | 5.17 | 0.08 |
| **11** | 5.27 | 0.07 |
| **12** | 5.92 | 0.05 |
| **13** | 5.43 | 0.07 |
| **14** | 5.74 | 0.09 |
| **15** | 6.08 | 0.03 |
| **16** | 5.11 | 0.09 |
| **17** | 6.65 | 0.09 |
| **18** | 6.95 | 0.10 |
| **19** | 6.39 | 0.11 |
| **20** | 6.30 | 0.10 |
| **21** | 6.07 | 0.06 |
| **22** | 5.97 | 0.03 |
| **23** | 5.65 | 0.04 |
| **24** | 5.92 | 0.08 |
| **25** | 6.37 | 0.05 |
| **26** | 5.78 | 0.03 |
| **27** | 6.81 | 0.06 |
| **28** | 5.96 | 0.05 |
| **29** | 5.85 | 0.08 |
| **30** | 6.00 | 0.03 |
| **31** | 5.93 | 0.10 |
| **32** | 6.65 | 0.04 |
| **33** | 5.02 | 0.03 |
| **34** | 6.15 | 0.11 |
| **35** | 6.00 | 0.12 |
| **36** | 5.30 | 0.04 |
| **37** | 5.91 | 0.03 |
| **38** | 6.52 | 0.03 |
| **39** | 6.63 | 0.05 |
| **40** | 7.14 | 0.04 |
| **41** | 6.60 | 0.05 |
| **42** | 6.49 | 0.04 |
| **43** | 6.96 | 0.04 |
| **44** | 6.32 | 0.07 |
| **45** | 6.21 | 0.08 |
| **46** | 6.68 | 0.09 |
| **47** | 5.56 | 0.06 |
| **48** | 5.04 | 0.08 |
| **49** | 6.15 | 0.04 |
| **50** | 6.55 | 0.07 |
| **51** | 5.66 | 0.10 |
| **52** | 6.42 | 0.11 |
| **53** | 5.52 | 0.10 |
| **54** | 6.13 | 0.06 |
| **55** | 5.39 | 0.11 |
| **56** | 5.21 | 0.08 |
| **57** | 5.20 | 0.08 |
| **58** | 5.50 | 0.07 |
| **59** | 6.42 | 0.09 |
| **60** | 5.00 | 0.04 |
| **61** | 5.46 | 0.08 |
| **62** | 6.28 | 0.16 |
| **63** | 5.65 | 0.09 |
| **64** | 5.46 | 0.07 |
| **65** | 5.95 | 0.09 |
| **66** | 5.61 | 0.14 |
| **67** | 6.22 | 0.06 |
| **68** | 6.01 | 0.07 |
| **69** | 5.74 | 0.07 |
| **70** | 5.96 | 0.07 |
| **71** | 6.31 | 0.10 |
| **72** | 6.19 | 0.08 |
| **73** | 5.59 | 0.09 |
| **74** | 5.49 | 0.11 |
| **75** | 5.84 | 0.03 |
| **76** | 6.54 | 0.05 |
| **77** | 6.30 | 0.09 |
| **78** | 6.05 | 0.07 |
| **79** | 4.98 | 0.03 |
| **80** | 4.97 | 0.10 |
| **81** | 4.94 | 0.07 |
| **82** | 5.11 | 0.06 |
| **83** | 6.11 | 0.04 |
| **84** | 4.89 | 0.04 |
| **85** | 6.69 | 0.05 |
| **86** | 6.41 | 0.12 |
| **87** | 4.95 | 0.11 |

### Example 2-2: Targeted lipidomics assay

The targeted lipidomics experiments are based on previously reported methods with small alterations.³

### Sample preparation

Neuro2a cells (grown at 37 °C, 7% CO₂) were treated for 2 h, n = 4 per condition, with compound (10 µM, 0.1% DMSO) or vehicle in medium (DMEM high glucose with 2% fetal calf serum). Washing with cold PBS (3x) followed by harvesting in 1.5 mL epp and centrifugation (10 min, 1500 rpm). PBS was removed and cell pellets frozen with liquid nitrogen and stored at -80 °C.

### Lipid extraction

Lipids extraction was performed on ice. In brief, cell pellets with 2 million cells were transferred to 1.5 mL Eppendorf tubes, spiked with 10 µL each of deuterated labeled internal standard mix for endocannabinoids (*N*-arachidonoyl ethanolamine (AEA)-d8, *N*-docosahexaenoylethanolamide (DHEA)-d4, 2-arachidonoylglycerol (2-AG)-d8, N-stearoylethanolamine (SEA)-d3, N-palmitoylethanolamine (PEA)-d4, *N-*linoleoylethanolamine (LEA)-d3 and *N*-oleoylethanolamine (OEA)-d4), and negative polar lipids (fatty acid (FA)17:0-d33), followed by the addition of ammonium acetate buffer (100 µL, 0.1 M, pH 4). After extraction with methyl *tert*-butyl ether (1 mL), the tubes were thoroughly mixed for 4 min using a bullet blender at medium speed (Next Advance, Inc., Averill park, NY, USA), followed by a centrifugation step (5000 g, 12 min, 4 °C). Then 925 µL of the upper layer methyl *tert*-butyl ether was transferred into clean 1.5 mL Eppendorf tubes. Samples were dried in a speed-vac followed by reconstitution in acetonitrile/water (50 µL, 90 : 10, v/v). The reconstituted samples were centrifuged (14000 g, 3 min, 4°C) before transferring into LC-MS vials. Each sample was injected on two different lipidomics platforms: endocannabinoids (5 µL) and negative polar lipids (8 µL).

### LC-MS/MS Analysis for endocannabinoids

A targeted analysis of 21 endocannabinoids and related NAEs (N-acylethanolamines) were measured using an Acquity UPLC I class binary solvent manager pump (Waters, Milford, USA) in conjugation with AB SCIEX 6500 quadrupole ion trap (QTRAP) (AB Sciex, Massachusetts, USA). Separation was performed with Acquity HSS T3 column (1.2 x 100 mm, 1.8 µm) maintained at 40 °C. The aqueous mobile phase A consisted of 2 mM ammonium formate and 10 mM formic acid, and the organic mobile phase B was acetonitrile. The flow rate was set to 0.4 ml/min; initial gradient conditions were 55% B held for 2 min and linearly ramped to 100% B over 6 minutes and held for 2 min; after 10 s the system returned to initial conditions and held 2 min before next injection. Electrospray ionization-MS was operated in positive mode for measurement of 21 endocannabinoids and NAEs, and a selective Multiple Reaction Mode (sMRM) was used for quantification.

### LC-MS/MS analysis for negative polar lipids

This method is measured on an Acquity UPLC binary solvent manager 8 pump (Waters) coupled to an Agilent 6530 electrospray ionization quadrupole time-of-flight (ESI-Q-TOF, Agilent, Jose, CA, USA) high resolution mass spectrometer using reference mass correction. The chromatographic separation was achieved on an Acquity HSS T3 column (1.2 x 100 mm, 1.8 µm) maintained at 40 °C for both methods. The negative apolar lipids that constitute free fatty acids were separated with a flow of 0.4 mL/min over 15 min gradient. In negative mode, the aqueous mobile phase A consisted of 5:95 (v/v) acetonitrile:H₂O with 10 mM ammonium formate, and the organic mobile phase B consisted of 99% (v/v) methanol with 10 mM ammonium formate.

## Claims

1. A compound of the general formula (I) 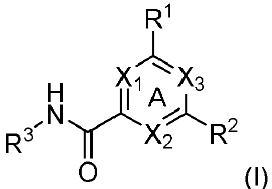 wherein
in a ring **A, X₁** is N or C**R⁴**; **X₂** is N or C**R⁵**; **X₃** is N or CH; with the proviso that at least one of **X₁** and **X₃** is N;
**R¹** represents -**R⁶**, -O**R⁷**, -S**R⁷**, -N**R⁸R⁹**, or -CON**R⁸R⁹**, or **R¹** and **R⁴** form 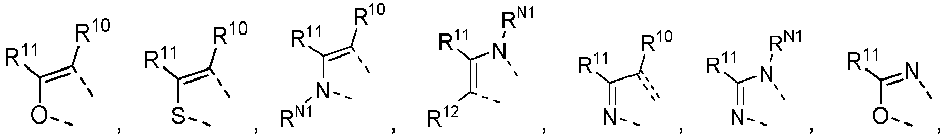 or 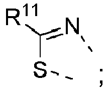
**R²** represents -**R¹⁶**, -O**R¹⁷**, -S**R¹⁷**, -N**R¹⁸R¹⁹**, -CON**R¹⁸R¹⁹**, or -N**R¹⁸**CH**R'**CO₂**R¹⁹**,
or **R²** and **R⁵** form 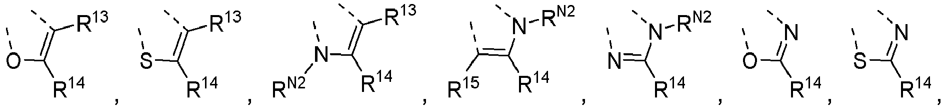 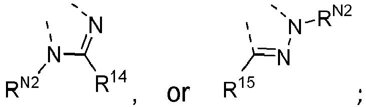
**R³** represents C₁-C₆ alkyl, C₁-C₆ alkyl-OH, C₁-C₆ alkyl-CO₂**R²⁰**, C₁-C₆ alkyl-CONH**R²⁰**, C₂-C₆ alkenyl, C₂-C₆ alkynyl, 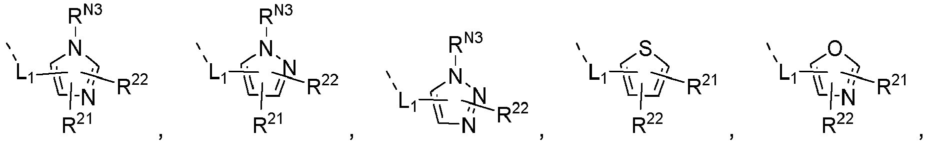 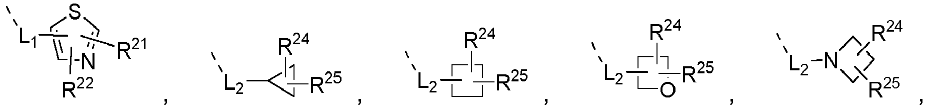 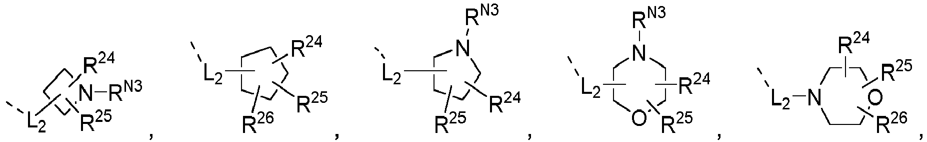 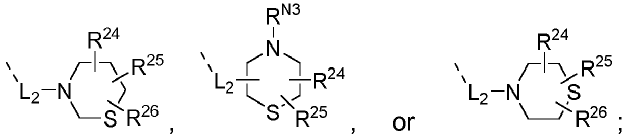
**R⁴** and **R⁵** represents -H, -F, -Cl, -Br, -I, C₁-C₃ alkyl, C₁-C₃ alkoxy, -CF₃, -OCF₂H or -OCF₃;
**R⁶**, **R⁷** and **R⁸** represent independently of each other, C₁-C₆ alkyl, C₃-C₆ cycloalkyl, -L₃-**R^{A}**, -L₄-**R^{B}**;
**R^{8'}**, **R⁹**, **R^{9'}**, **R¹⁰**, and **R¹¹** represent independently of each other -H, C₁-C₆ alkyl, C₃-C₆ cycloalkyl, -L₃-**R^{A}**, -L₄-**R^{B}**;
**R^{A}** and **R^{B}** represent independently of each other 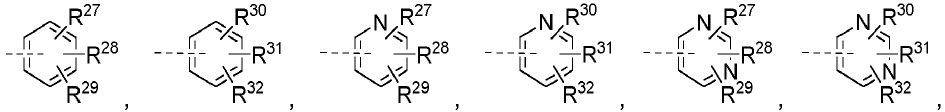 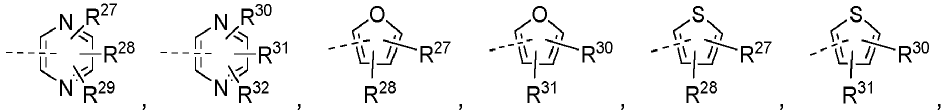 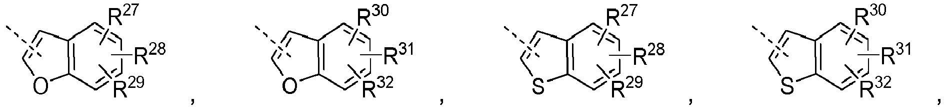 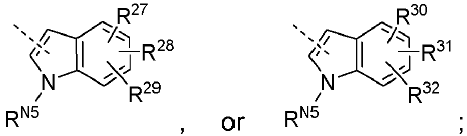
or -N**R⁸R⁹** represents 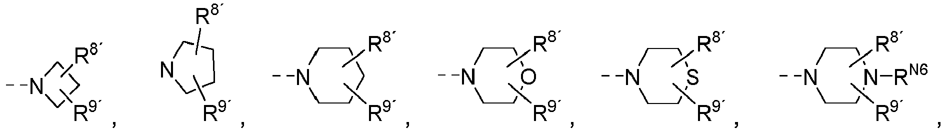 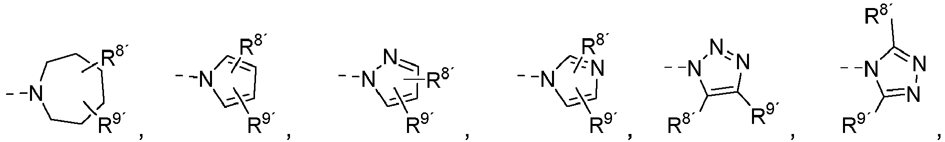 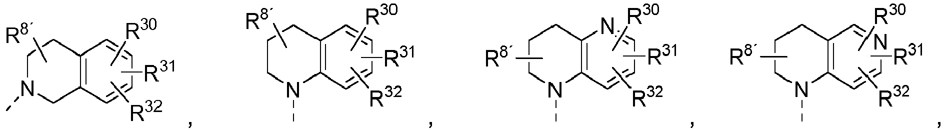 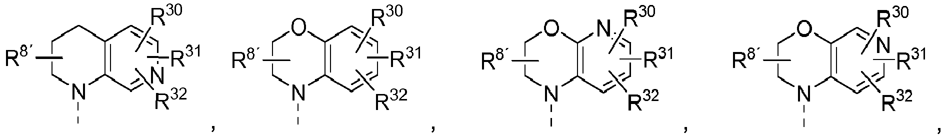 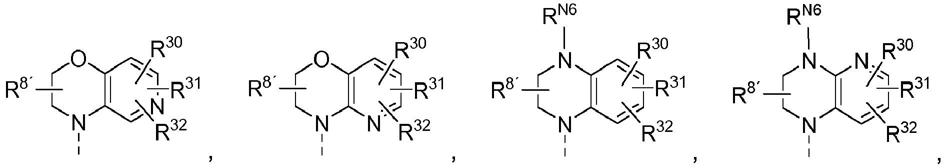 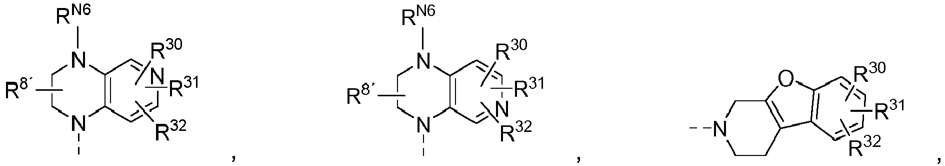 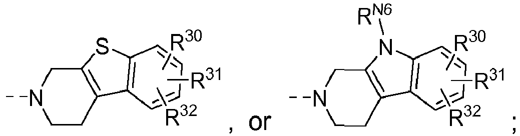
**R¹²**, **R¹³**, **R¹⁴** and **R¹⁵** represent independently of each other -H, -CI, -F, -CF₃, C₁-C₃ alkyl, or C₁-C₃ alkoxy;
**R¹⁶**, **R¹⁷**, **R¹⁸** and **R¹⁹** represent independently of each other C₁-C₆ alkyl or C₃-C₆ cycloalkyl;
or -N**R¹⁸R¹⁹** represents 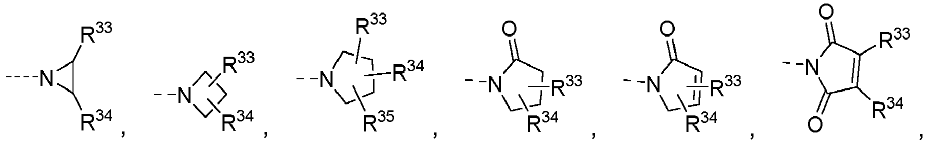 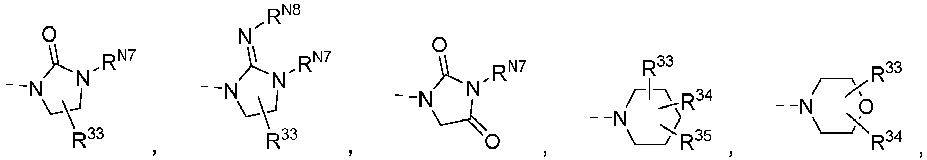 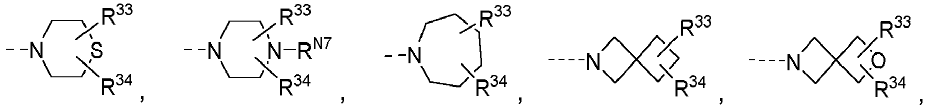 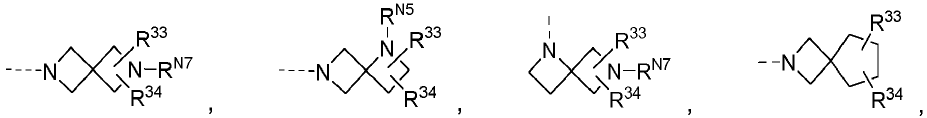 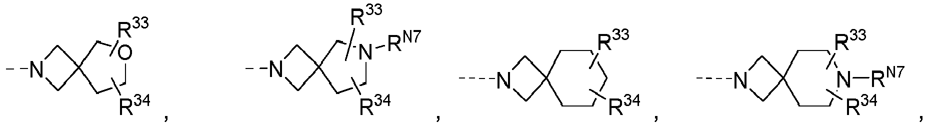 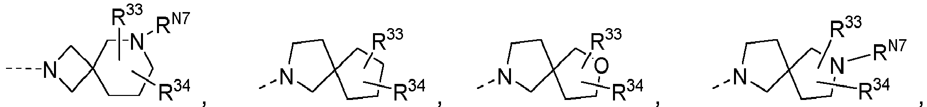 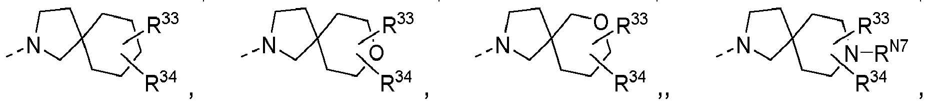 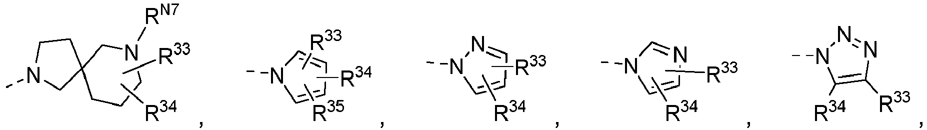 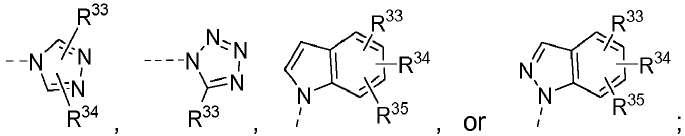
**R'** represents -H, -CH₃, -CH₂OH, -CH₂SH, -CH₂CO₂H, -CH₂CONH₂, -CH(OH)CH₃, -CH(CH₃)₂, -CH₂CH(CH₃)₂, -CH(CH₃)CH₂CH₃, -CH₂CH₂SCH₃, -CH₂CH₂CO₂H, -CH₂CH₂CONH₂, -CH₂Ph, -CH₂CH₂CH₂NHC(=NH)NH₂, -CH₂CH₂CH₂CH₂NH₂, 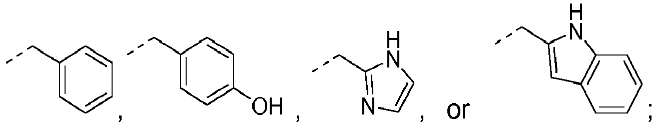
**L¹**, **L²**, **L³**, and **L⁴** represent independently of each other a covalent bond, C₁-C₆ alkyl group, linear C₂-C₆ alkenyl group, wherein one or more of the -CH₂- groups present in the C₁-C₆ alkyl or C₂-C₆ alkenyl group are optionally and independently of each other replaced by -O-, -S-, -N(R^{N9})-, -CO-, -COO-, -OOC-, -CON(R^{N9})-, -N(R^{N9})CO-, -NHCON(R^{N9})-, -N(R^{N9})CONH-, -SO-, -SO₂-, -SO₂N(R^{N9})-, or -N(R^{N9})SO₂-;
**R^{N1}**, **R^{N2}**, **R^{N3}**, **R^{N4}**, **R^{N5}**, **R^{N6}**, **R^{N7}**, **R^{N8}**, and **R^{N9}** represent independently of each other -H, -CH₃, -C₂H₅, -C₃H₇, -CH(CH₃)₂, -C₄H₉, -CH₂-CH(CH₃)₂, -CH(CH₃)-C₂H₅, -C(CH₃)₃, -cyclo-C₃H₅, -CH₂-cyclo-C₃H₅, -Ph, -CH₂Ph, -CH₂F, -CHF₂, -CF₃, -CH₂Cl, -CH₂Br, -CH₂-CH₂F, -CH₂-CHF₂, -CH₂-CF₃, -CH₂-CH₂Cl, -CH₂-CH₂Br, -CH₂-CH₂I, -CH₂-CH=CH₂, -CH₂-C≡CH, -OH, -OCH₃, -CHO, -COCH₃, -COC₂H₅, -COC₃H₇, -COCH(CH₃)₂, -COC(CH₃)₃, -COOCH₃, -COOC₂H₅, -COOC₃H₇, -COOCH(CH₃)₂, -COOC(CH₃)₃, 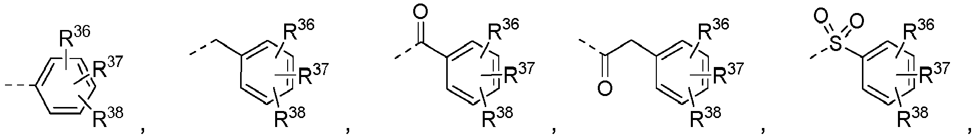 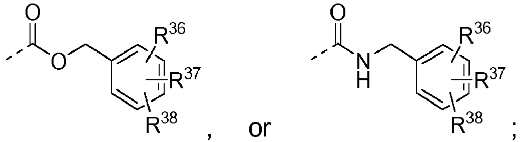
**R²⁰** represents -H, -CH₃, -C₂H₅, -C₃H₇, -CH(CH₃)₂, -C₄H₉, -CH₂-CH(CH₃)₂, -CH(CH₃)-C₂H₅, -C(CH₃)₃, -cyclo-C₃H₅, or -CH₂-cyclo-C₃H₅;
**R²¹**, **R²²**, **R²³**, **R²⁴**, **R²⁵**, **R²⁶**, **R²⁷**, **R²⁸**, **R²⁹**, **R³⁰**, **R³¹**, **R³²**, **R³³**, **R³⁴**, **R³⁵**, **R³⁶**, **R³⁷**, and **R³⁸** represent independently of each other -H, -OH, -OCH₃, -OC₂H₅, -OC₃H₇, -O-cyclo-C₃H₅, -OCH(CH₃)₂, -OC(CH₃)₃, -OC₄H₉, -OPh, -OCH₂-Ph, -OCPh₃, -CH₂-OCH₃, -C₂H₄-OCH₃, -C₃H₆-OCH₃, -CH₂-OC₂H₅, -C₂H₄-OC₂H₅, -C₃H₆-OC₂H₅, -CH₂-OC₃H₇, -C₂H₄-OC₃H₇, -C₃H₆-OC₃H₇, -CH₂-O-cyclo-C₃H₅, -C₂H₄-O-cyclo-C₃H₅, -C₃H₆-O-cyclo-C₃H₅, -CH₂-OCH(CH₃)₂, -C₂H₄-OCH(CH₃)₂, -C₃H₆-OCH(CH₃)₂, -CH₂-OC(CH₃)₃, -C₂H₄-OC(CH₃)₃, -C₃H₆-OC(CH₃)₃, -CH₂-OC₄H₉, -C₂H₄-OC₄H₉, -C₃H₆--OC₄H₉, -CH₂-OPh, -C₂H₄-OPh, -C₃H₆-OPh, -CH₂-OCH₂-Ph, -C₂H₄-OCH₂-Ph, -C₃H₆-OCH₂-Ph, -SH, -SCH₃, -SC₂H₅, -SC₃H₇, -S-cyc!o-C₃H₅, -SCH(CH₃)₂, -SC(CH₃)₃, -F, -Cl, -Br, -I, -CN, -COCH₃, -COC₂H₅, -COC₃H₇, -CO-cyclo-C₃H₅, -COCH(CH₃)₂, -COC(CH₃)₃, -COOH, -COOCH₃, -COOC₂H₅, -COOC₃H₇, -COO-cyclo-C₃H₅, -COOCH(CH₃)₂, -COOC(CH₃)₃, -OOC-CH₃, -OOC-C₂H₅, -OOC-C₃H₇, -OOC-cyclo-C₃H₅, -OOC-CH(CH₃)₂, -OOC-C(CH₃)₃, -CONH₂, -CONHCH₃, -CONHC₂H₅, -CONHC₃H₇, -CONH-cyclo-C₃H₅, -CONH[CH(CH₃)₂], -CONH[C(CH₃)₃], -CON(CH₃)₂, -CON(C₂H₅)₂, -CON(C₃H₇)₂, -CON(cyclo-C₃H₅)₂, -CON[CH(CH₃)₂]₂, -CON[C(CH₃)₃]₂, -NHCOCH₃, -NHCOC₂H₅, -NHCOC₃H₇, -NHCO-cyclo-C₃H₅, -NHCO-CH(CH₃)₂, -NHCO-C(CH₃)₃, -NHCO-OCH₃, -NHCO-OC₂H₅, -NHCO-OC₃H₇ⱼ -NHCO-O-cyclo-C₃H₅, -NHCO-OCH(CH₃)₂, -NHCO-OC(CH₃)₃, -NH₂, -NHCH₃, -NHC₂H₅, -NHC₃H₇, -NH-cyclo-C₃H₅, -NHCH(CH₃)₂, -NHC(CH₃)₃, -N(CH₃)₂, -N(C₂H₅)₂, -N(C₃H₇)₂, -N(cyclo-C₃H₅)₂, -N[CH(CH₃)₂]₂, -N[C(CH₃)₃]₂, -SOCH₃, -SOC₂H₅, -SOC₃H₇, -SO-cyclo-C₃H₅, -SOCH(CH₃)₂, -SOC(CH₃)₃, -SO₂CH₃, -SO₂C₂H₅, -SO₂C₃H₇, -SO₂-cyclo-C₃H₅, -SO₂CH(CH₃)₂, -SO₂C(CH₃)₃, -SO₃H, -SO₃CH₃, -SO₃C₂H₅, -SO₃C₃H₇, -SO₃-cyclo-C₃H₅, -SO₃CH(CH₃)₂, -SO₃C(CH₃)₃, -SO₂NH₂, -SO₂NHCH₃, -SO₂NHC₂H₅, -SO₂NHC₃H₇, -SO₂NH-cyclo-C₃H₅, -SO₂NHCH(CH₃)₂, -SO₂NHC(CH₃)₃, -SO₂N(CH₃)₂, -SO₂N(C₂H₅)₂, -SO₂N(C₃H₇)₂, -SO₂N(cyclo-C₃H₅)₂, -SO₂N[CH(CH₃)₂]₂, -SO₂N[C(CH₃)₃]₂, -O-S(=O)CH₃, -O-S(=O)C₂H₅, -O-S(=O)C₃H₇, -O-S(=O)-cyclo-C₃H₅, -O-S(=O)CH(CH₃)₂, -O-S(=O)C(CH₃)₃, -S(=O)(=NH)CH₃, -S(=O)(=NH)C₂H₅, -S(=O)(=NH)C₃H₇, -S(=O)(=NH)-cyclo-C₃H₅, -S(=O)(=NH)CH(CH₃)₂, -S(=O)(=NH)C(CH₃)₃, -NH-SO₂-CH₃, -NH-SO₂-C₂H₅, -NH-SO₂-C₃H₇, -NH-SO₂-cyclo-C₃H₅, -NH-SO₂-CH(CH₃)₂, -NH-SO₂-C(CH₃)₃, -O-SO₂-CH₃, -O-SO₂-C₂H₅, -O-SO₂-C₃H₇, -O-SO₂-cyclo-C₃H₅, -O-SO₂-CH(CH₃)₂, -O-SO₂-C(CH₃)₃, -OCH₂F, -OCHF₂ -OCF₃, -CH₂-OCF₃, -C₂H₄-OCF₃, -C₃H₆-OCF₃, -OC₂F₅, -CH₂-OC₂F₅, -C₂H₄-OC₂F₅, -C₃H₆-OC₂F₅, -O-COOCH₃, -O-COOC₂H₅, -O-COOC₃H₇, -O-COO-cyclo-C₃H₅, -O-COOCH(CH₃)₂, -O-COOC(CH₃)₃, -NH-CO-NH₂, -NH-CO-NHCH₃, -NH-CO-NHC₂H₅, -NH-CO-NHC₃H₇, -NH-C(=NH)-NH₂, -NH-CO-N(C₃H₇)₂, -NH-CO-NH[CH(CH₃)₂], -NH-CO-NH[C(CH₃)₃], -NH-CO-N(CH₃)₂, -NH-CO-N(C₂H₅)₂, -NH-CO-NH-cyclo-C₃H₅, -NH-CO-N(cyclo-C₃H₅)₂, -NH-CO-N[CH(CH₃)₂]₂, -NH-C(=NH)-NHCH₃, -NH-C(=NH)-NHC₂H₅, -NH-C(=NH)-NHC₃H₇, -O-CO-NH-cyclo-C₃H₅, -NH-C(=NH)-NH-cyclo-C₃H₅, -NH-C(=NH)-NH[CH(CH₃)₂], -O-CO-NH[CH(CH₃)₂], -NH-C(=NH)-NH[C(CH₃)₃], -NH-C(=NH)-N(CH₃)₂, -NH-C(=NH)-N(C₂H₅)₂, -NH-C(=NH)-N(C₃H₇)₂, -NH-C(=NH)-N(cyclo-C₃H₅)₂, -O-CO-NHC₃H₇, -NH-C(=NH)-N[CH(CH₃)₂]₂, -NH-C(=NH)-N[C(CH₃)₃]₂, -O-CO-NH₂, -O-CO-NHCH₃, -O-CO-NHC₂H₅, -O-CO-NH[C(CH₃)₃], -O-CO-N(CH₃)₂, -O-CO-N(C₂H₅)₂, -O-CO-N(C₃H₇)₂, -O-CO-N(cyclo-C₃H₅)₂, -O-CO-N[CH(CH₃)₂]₂, -O-CO-N[C(CH₃)₃]₂, -O-CO-OCH₃, -O-CO-OC₂H₅, -O-CO-OC₃H₇, -O-CO-O-cyclo-C₃H₅, -O-CO-OCH(CH₃)₂, -O-CO-OC(CH₃)₃, -CH₂F, -CHF₂, -CF₃, -CH₂-CH₂F, -CH₂-CHF₂, -CH₂-CF₃, cyclo-C₈H₁₅, -Ph, -CH₂-Ph, -CH₂-CH₂-Ph, -CH=CH-Ph, -CPh₃, -CH₃, -C₂H₅, -C₃H₇, -CH(CH₃)₂, -C₄H₉, -CH₂-CH(CH₃)₂, -CH(CH₃)-C₂H₅, -C(CH₃)₃, -C₅H₁₁, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, -C₂H₄-CH(CH₃)₂, -C₆H₁₃, -C₇H₁₅, -C₈H₁₇, -C₃H₆-CH(CH₃)₂, -C₂H₄-CH(CH₃)-C₂H₅, -CH(CH₃)-C₄H₉, -CH₂-CH(CH₃)-C₃H₇, -CH(CH₃)-CH₂-CH(CH₃)₂, -CH(CH₃)-CH(CH₃)-C₂H₅, -CH₂-CH(CH₃)-CH(CH₃)₂, -CH₂-C(CH₃)₂-C₂H₅, -C(CH₃)₂-C₃H₇, -C(CH₃)₂-CH(CH₃)₂, -C₂H₄-C(CH₃)₃, -CH(CH₃)-C(CH₃)₃, -CH=CH₂, -CH₂-CH=CH₂, -C(CH₃)=CH₂, -CH=CH-CH₃, -C₂H₄-CH=CH₂, -CH₂-CH=CH-CH₃, -CH=CH-C₂H₅, -CH₂-C(CH₃)=CH₂, -CH(CH₃)-CH=CH, -CH=C(CH₃)₂, -C(CH₃)=CH-CH₃, -CH=CH-CH=CH₂, -C₃H₆-CH=CH₂, -C₂H₄-CH=CH-CH₃, -CH₂-CH=CH-C₂H₅, -CH=CH-C₃H₇, -CH=CH-CH=CH-CH₃, -C₂H₄-C(CH₃)=CH₂, -CH₂-CH(CH₃)-CH=CH₂, -CH(CH₃)-CH₂-CH=CH₂, -CH₂-CH=C(CH₃)₂, -CH₂-C(CH₃)=CH-CH₃, -CH(CH₃)-CH=CH-CH₃, -CH=CH-CH(CH₃)₂, -CH=C(CH₃)-C₂H₅, -C(CH₃)=CH-C₂H₅, -C(CH₃)=C(CH₃)₂, -C(CH₃)₂-CH=CH₂, -CH(CH₃)-C(CH₃)=CH₂, -C₄H₈-CH=CH₂, -C₃H₆-CH=CH-CH₃, -C₂H₄-CH=CH-C₂H₅, -CH₂-CH=CH-C₃H₇, -CH=CH-C₄H₉, -C₃H₆-C(CH₃)=CH₂, -C₂H₄-CH(CH₃)-CH=CH₂, -CH₂-CH(CH₃)-CH₂-CH=CH₂, -C₂H₄-CH=C(CH₃)₂, -CH(CH₃)-C₂H₄-CH=CH₂, -C₂H₄-C(CH₃)=CH-CH₃, -CH₂-CH(CH₃)-CH=CH-CH₃, -CH(CH₃)-CH₂-CH=CH-CH₃, -CH₂-CH=CH-CH(CH₃)₂, -CH₂-CH=C(CH₃)-C₂H₅, -CH₂-C(CH₃)=CH-C₂H₅, -CH(CH₃)-CH=CH-C₂H₅, -CH=CH-CH₂-CH(CH₃)₂, -CH=CH-CH(CH₃)-C₂H₅, -CH=C(CH₃)-C₃H₇, -C(CH₃)=CH-C₃H₇, -CH₂-CH(CH₃)-C(CH₃)=CH₂, -C[C(CH₃)₃]=CH₂, -CH(CH₃)-CH₂-C(CH₃)=CH₂, -CH(CH₃)-CH(CH₃)-CH=CH₂, -CH=CH-C₂H₄-CH=CH₂, -C(CH₃)₂-CH₂-CH=CH₂, -CH₂-C(CH₃)=C(CH₃)₂, -CH(CH₃)-CH=C(CH₃)₂, -C(CH₃)₂-CH=CH-CH₃, -CH=CH-CH₂-CH=CH-CH₃, -CH(CH₃)-C(CH₃)=CH-CH₃, -CH=C(CH₃)-CH(CH₃)₂, -C(CH₃)=CH-CH(CH₃)₂, -C(CH₃)=C(CH₃)-C₂H₅, -CH=CH-C(CH₃)₃, -C(CH₃)₂-C(CH₃)=CH₂, -CH(C₂H₅)-C(CH₃)=CH₂, -C(CH₃)(C₂H₅)-CH=CH₂, -CH(CH₃)-C(C₂H₅)=CH₂, -CH₂-C(C₃H₇)=CH₂, -CH₂-C(C₂H₅)=CH-CH₃, -CH(C₂H₅)-CH=CH-CH₃, -C(C₄H₉)=CH₂, -C(C₃H₇)=CH-CH₃, -C(C₂H₅)=CH-C₂H₅, -C(C₂H₅)=C(CH₃)₂, -C[CH(CH₃)(C₂H₅)]=CH₂, -C[CH₂-CH(CH₃)₂]=CH₂, -C₂H₄-CH=CH-CH=CH₂, -CH₂-CH=CH-CH₂-CH=CH₂, -C₃H₆-C≡C-CH₃, -CH₂-CH=CH-CH=CH-CH₃, -CH=CH-CH=CH-C₂H₅, -CH(CH₃)-CH₂-C≡CH, -CH(CH₃)-C≡C-CH₃, -C₂H₄-CH(CH₃)-C≡CH, -CH=CH-CH=C(CH₃)₂, -CH₂-CH(CH₃)-CH₂-C≡CH, -CH=CH-C(CH₃)=CH-CH₃, -CH=C(CH₃)-CH=CH-CH₃, -CH₂-CH(CH₃)-C≡CH, -C(CH₃)=CH-CH=CH-CH₃, -C=CH, -C≡C-CH₃, -CH₂-C≡CH, -C₂H₄-C≡CH, -CH₂-C≡C-CH₃, -C≡C-C₂H₅, -C₃H₆-C≡CH, -C₂H₄-C≡C-CH₃, -CH₂-C≡C-C₂H₅, -C≡C-C₃H₇, -CH(CH₃)-C≡CH, -C₄H₈-C≡CH, -C₂H₄-C≡C-C₂H₅, -CH₂-C≡C-C₃H₇, -C≡C-C₄H₉, -C≡C-CH₂-CH(CH₃)₂, -CH(CH₃)-C₂H₄-C≡CH, -CH₂-CH(CH₃)-C≡C-CH₃, -C(CH₃)(C₂H₅)-C≡CH, -CH(CH₃)-CH₂-C≡C-CH₃, -CH(CH₃)-C≡C-C₂H₅, -CH₂-C≡C-CH(CH₃)₂, -C≡C-CH(CH₃)-C₂H₅, -CH₂-C≡C-C≡C-CH₃, -CH(C₂H₅)-C≡C-CH₃, -C(CH₃)₂-C≡C-CH₃, -CH(C₂H₅)-CH₂-C≡CH, -CH₂-CH(C₂H₅)-C≡CH, -C(CH₃)₂-CH₂-C≡CH, -CH₂-C(CH₃)₂-C≡CH, -CH(CH₃)-CH(CH₃)-C≡CH, -CH(C₃H₇)-C≡CH, -CH₂-CH(C≡CH)₂, -C=C-C=CH, -CH₂-C≡C-C≡CH, -C≡C-C≡C-CH₃, -CH(C≡CH)₂, -C₂H₄-C≡C-C≡CH, -CH₂-C≡C-CH₂-C≡CH, -C≡C-C₂H₄-C≡CH, -C≡C-C(CH₃)₃, -C≡C-CH₂-C≡C-CH₃, -C≡C-C≡C-C₂H₅, 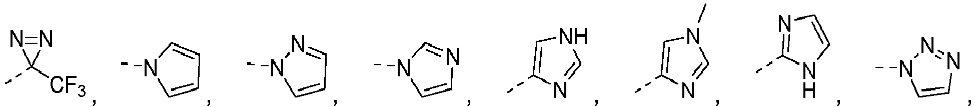 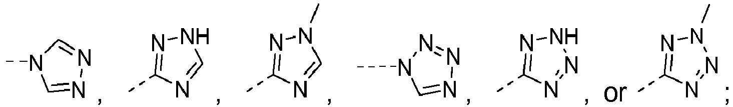
or tautomer, enantiomer, diastereomer, a mixture of tautomers, a mixture of enantiomers, a mixture of diastereomers, or a pharmaceutically acceptable salt thereof.

2. The compound according to Claim 1, wherein the ring A represents 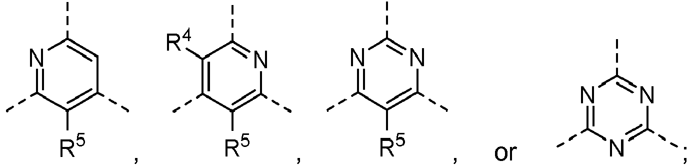 and **R⁴** and **R⁵** have the same meanings as defined in Claim 1.

3. The compound according to Claim 1 or 2, wherein the compound has any one of the formulae **(IV-1) - (IV-8):** 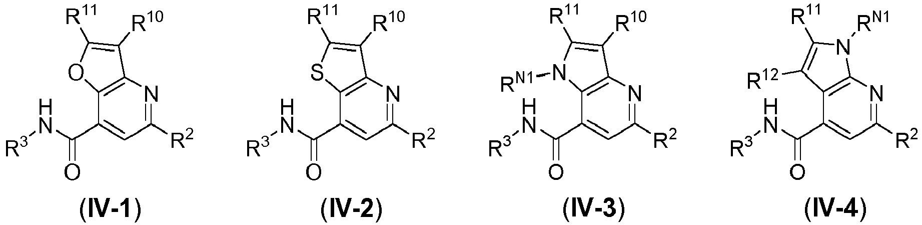 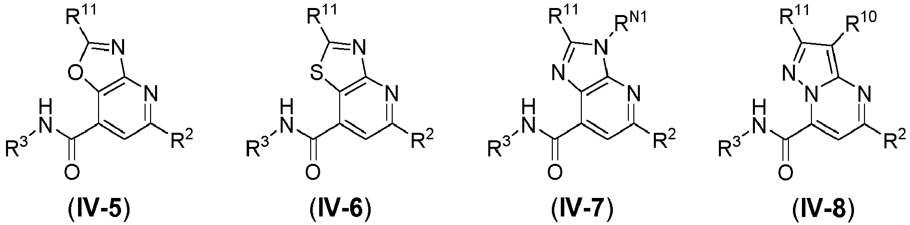 wherein **R²**, **R³**, **R¹⁰**, **R¹¹**, **R¹²**, and **R^{N1}** have the same meanings as defined in claim 1.

4. The compound according to Claims 1 or 2, wherein the compound has any one of the formulae **(V-1)** - (**V-8**):
| | | | |
|---|---|---|---|
| 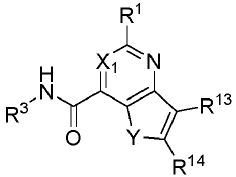 | 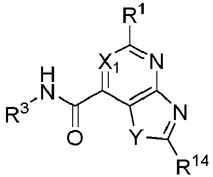 | 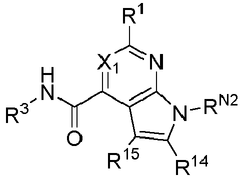 | 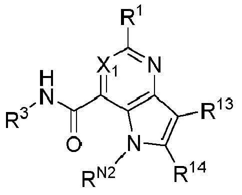 |
| (**V-1**) | (**V-2**) | (**V-3**) | **(V-4)** |
| 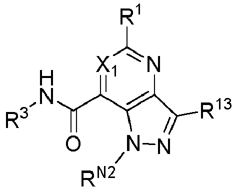 | 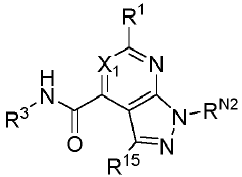 | 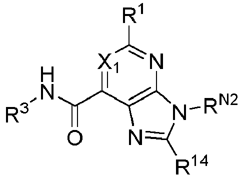 | 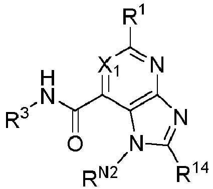 |
| **(V-5)** | **(V-6)** | **(V-7)** | (**V-8**) |
wherein X₁ represent N or CH;
Y represents O or S; and
**R¹**, **R³**, **R¹³**, **R¹⁴**, **R¹⁵** and **R^{N2}** have the same meanings as defined in claim 1.

5. The compound according to Claims 1 or 2, wherein the compound has any one of the formulae **(VI-1)** - (**VI-9**):
| | | |
|---|---|---|
| 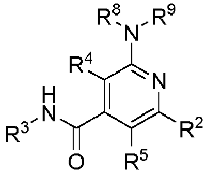 | 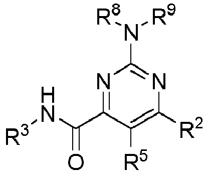 | 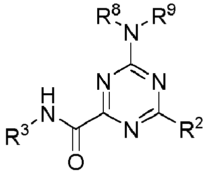 |
| (**VI-1**) | (**VI-2**) | (**VI-3**) |
| 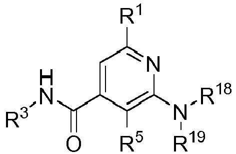 | 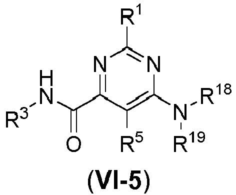 | 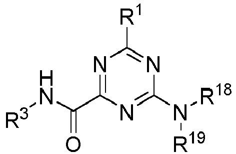 |
| (**VI-4**) | (**VI-5**) | (**VI-6**) |
| 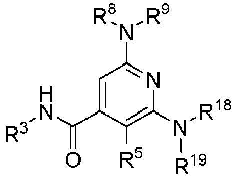 | 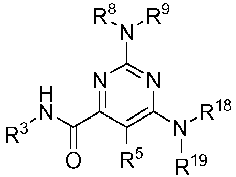 | 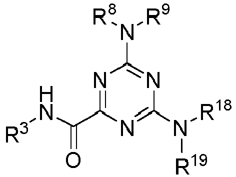 |
| (**VI-7**) | (**VI-8**) | (**VI-9**) |
wherein R¹, R², R³, R⁴, R⁵, R⁸, R⁹, R¹⁸ and R¹⁹ have the same meanings as defined in Claim 1.

6. The compound according to any one of Claims 1, 2, 4 and 5, wherein the **R¹** presents 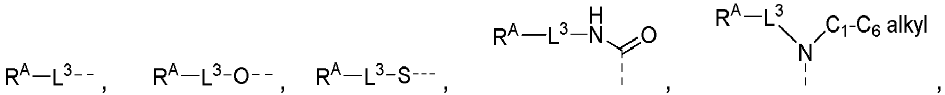 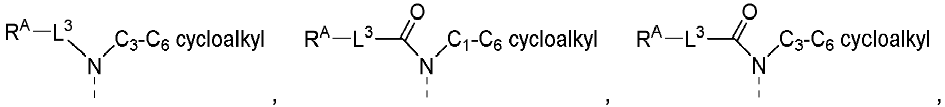 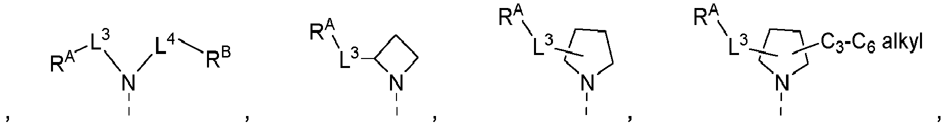 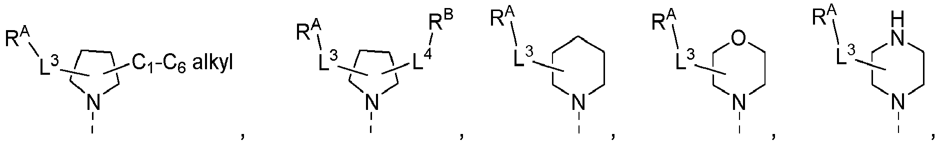 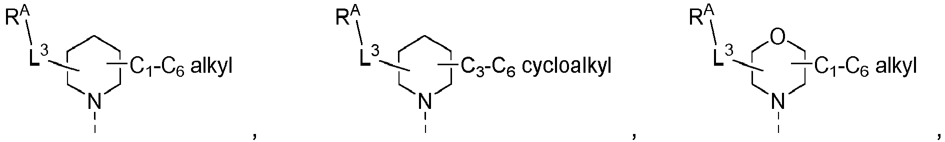 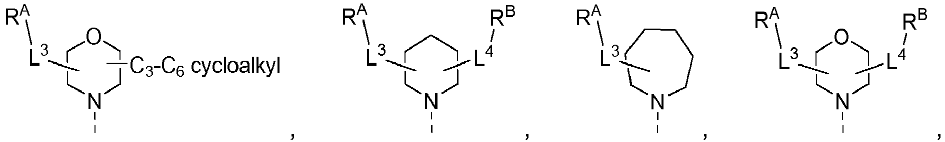 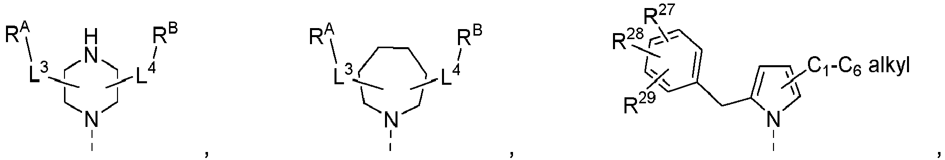 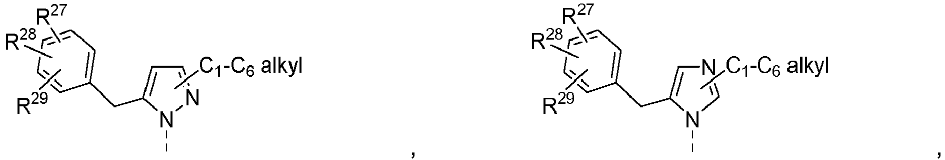 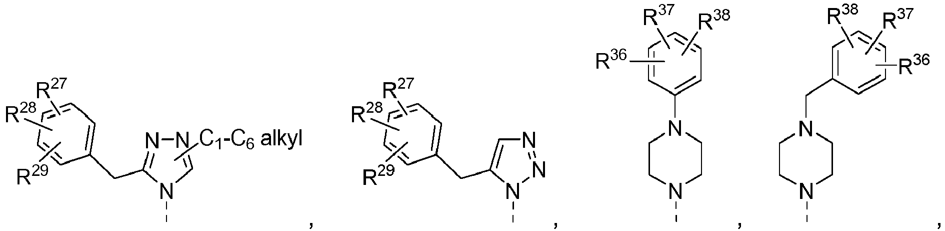 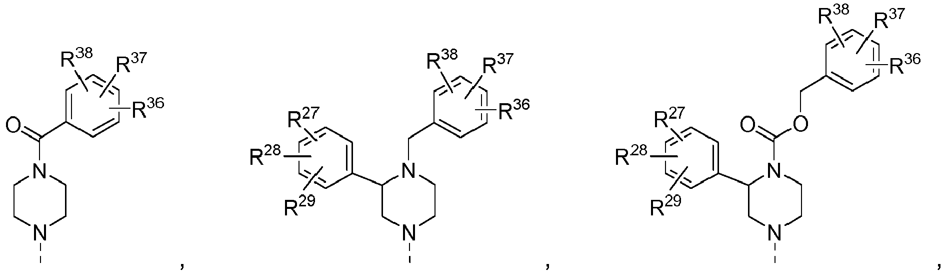 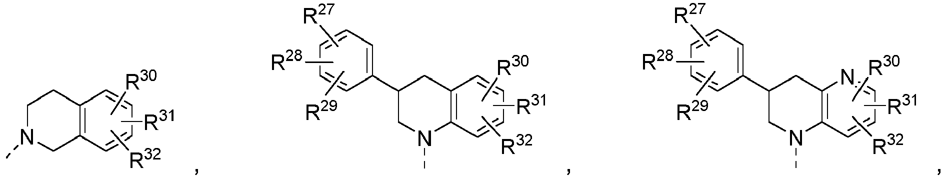 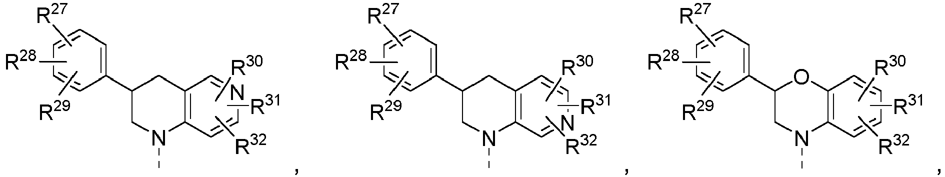 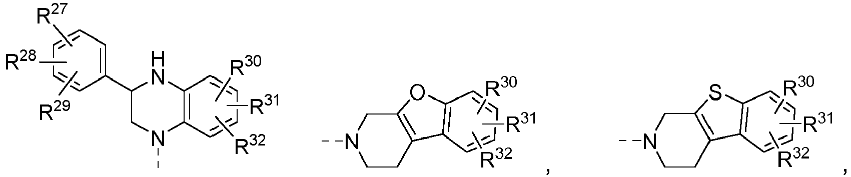 or 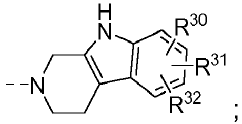 wherein R^{A}, represents 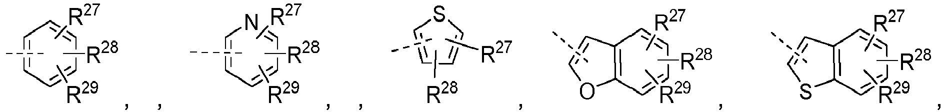 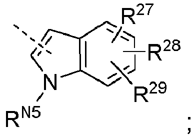 R^{B} represents 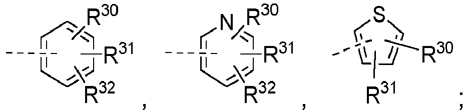 **L³**, and **L⁴** represent independently of each other a covalent bond, -CH₂-, 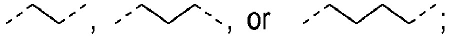 and **R²⁷**, **R²⁸**, **R²⁹**, **R³⁰**, **R³¹**, and **R³²** have the same meanings defined in Claim 1.

7. The compound according to any one of Claims 1, 2 and 4 - 6, wherein **R¹** represents 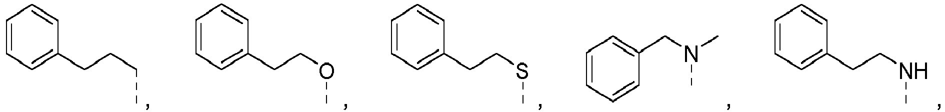 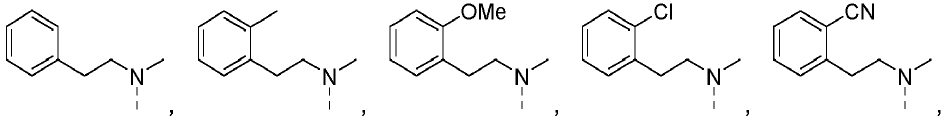 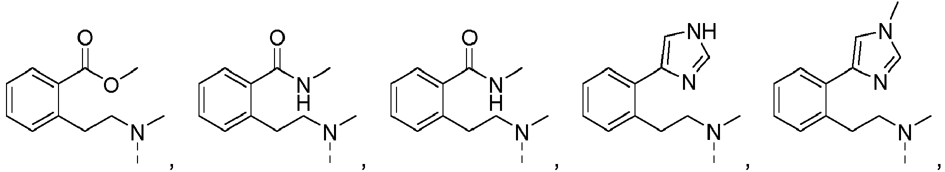 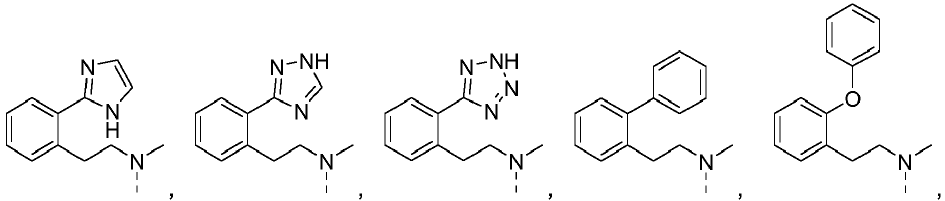 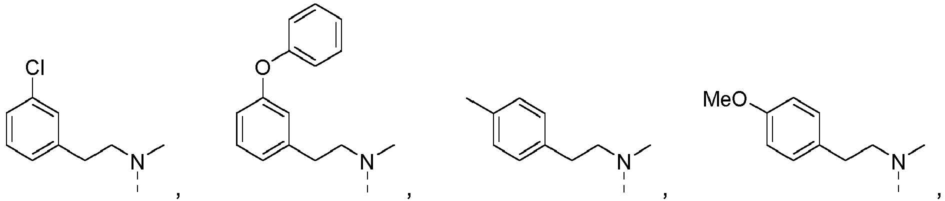 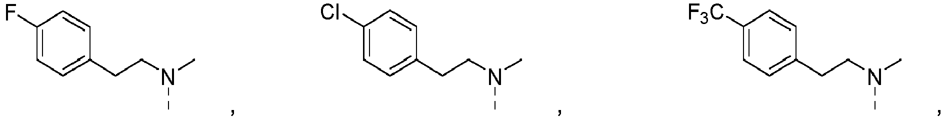 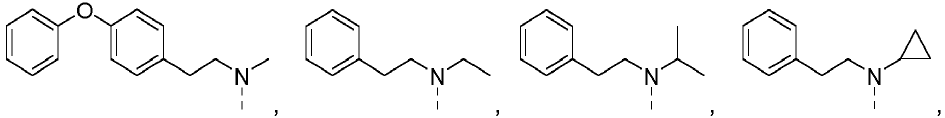 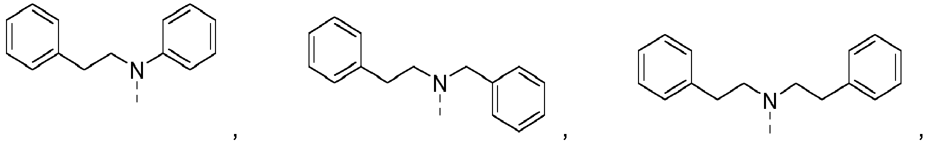 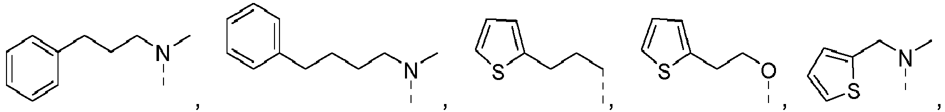 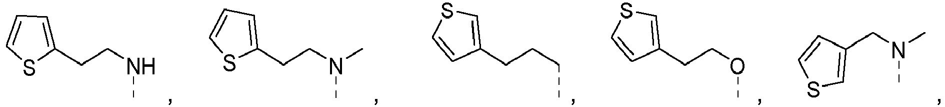 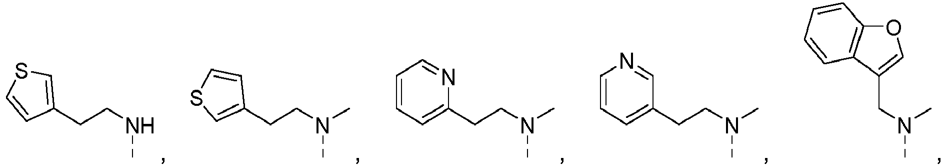 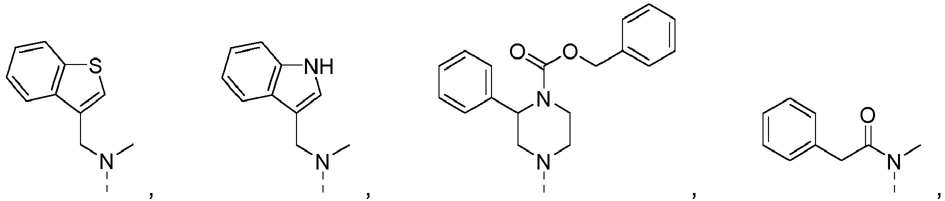 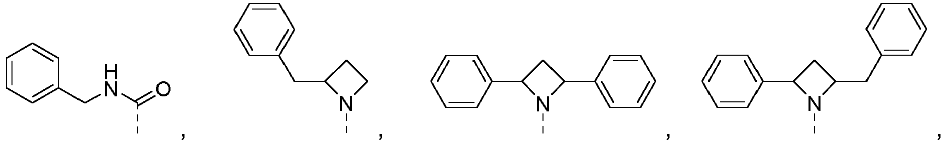 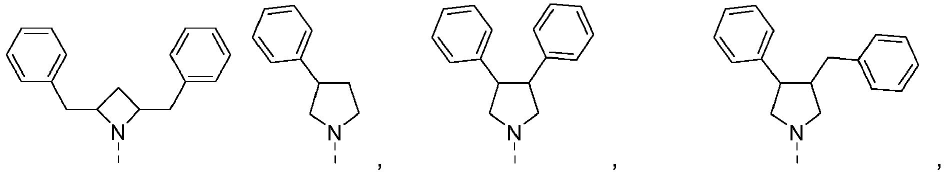 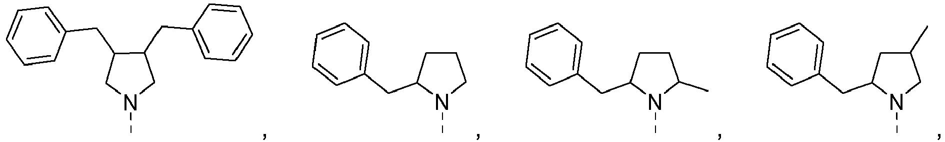 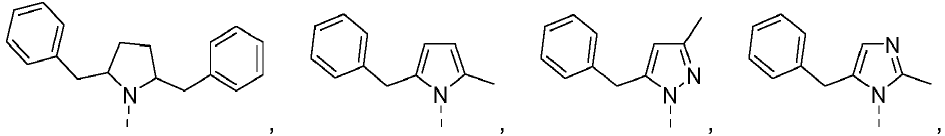 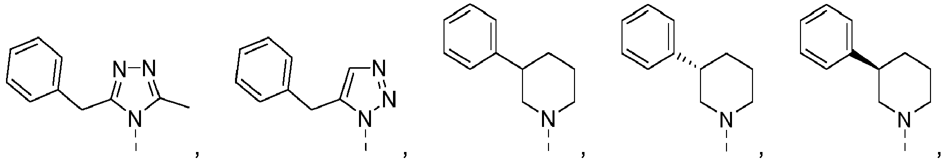 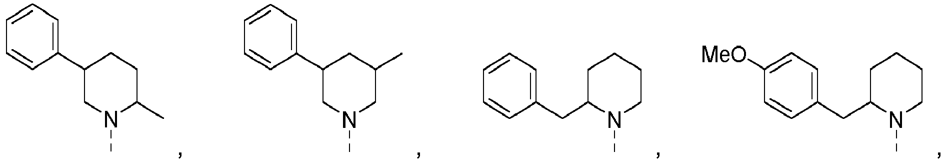 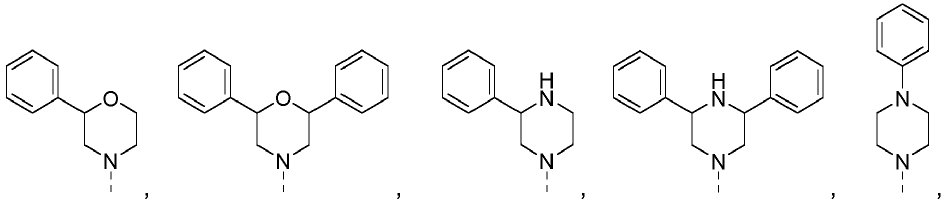 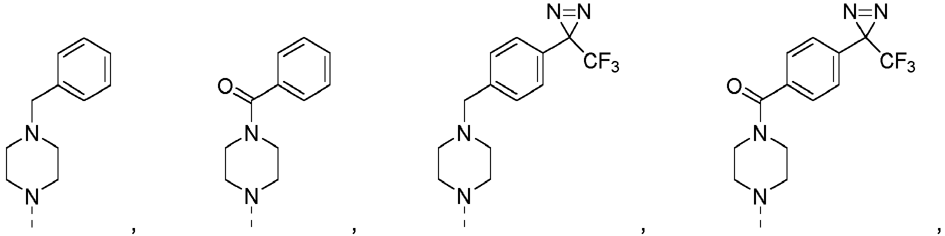 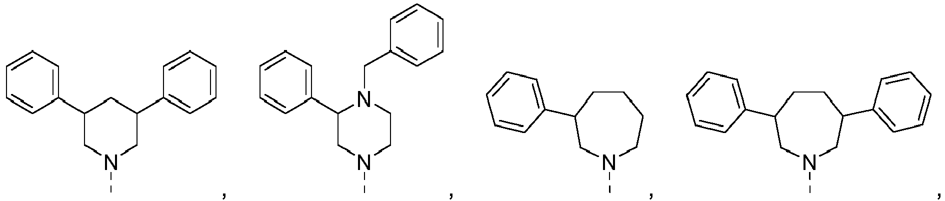 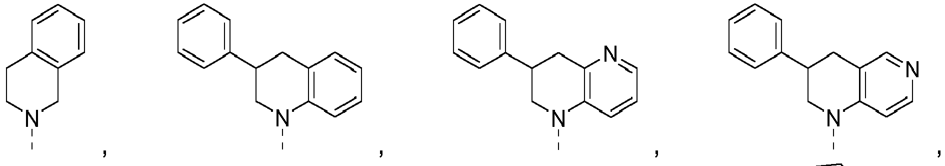 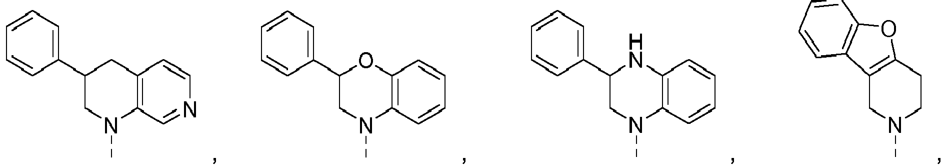 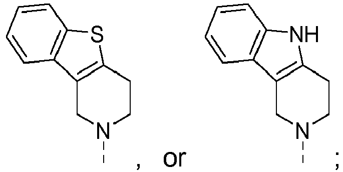 **R²** represents -CH₃, -Ph, -OCH₃, -SCH₃, -NHCH₃, -N(CH₃)₂, -CONHCH₃, -N(CH₂CH₃)₂, -N(CH₃)(CH₂CH₂OH), -N(CH₃)COCH₃, -N(CH₃)COCH₂OH, -N(CH₃)COCH(CH₃)CO₂CH₃, -N(CH₃)(CH₂Ph), -N(CH₃)(CH₂CH₂Ph), -NHCH(CH₃)CO₂CH₃, -NHCH(CH₂Ph)CO₂CH₃, -NHCH(CH₂SH)CO₂CH₃, -N(CH₃)CH(CH₃)CO₂CH₃, -N(CH₃)CH(CH₂Ph)CO₂CH₃, -N(CH₃)CH(CH₂SH)CO₂CH₃, 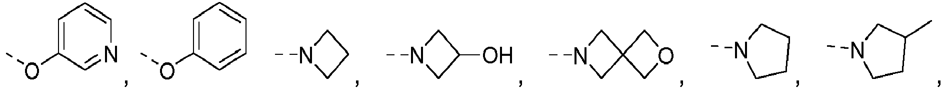 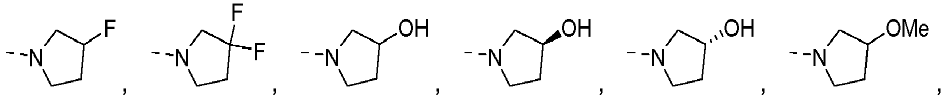 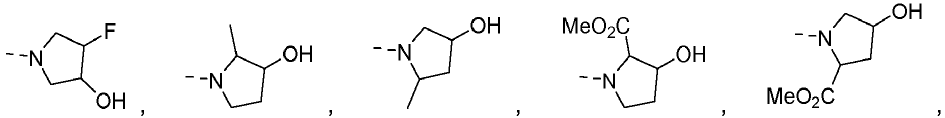 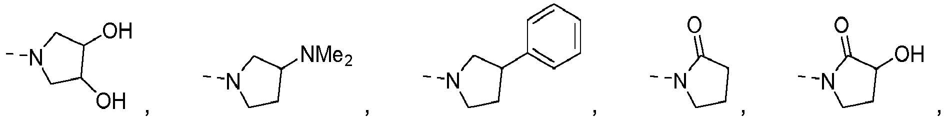 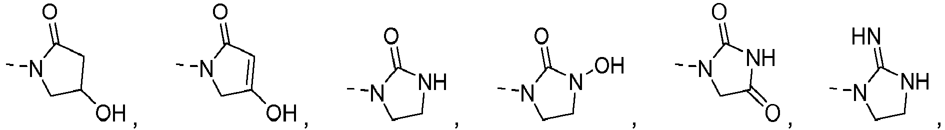 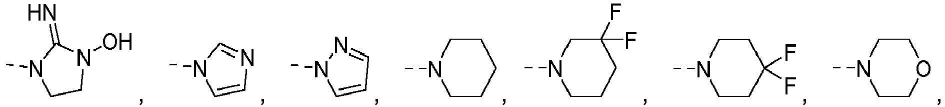 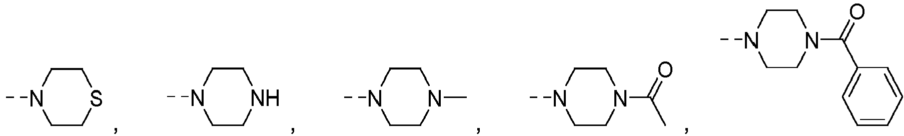 or 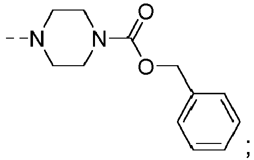 and **R³** represents -CH₃, -CH₂CH₃, -CH₂CH₂CH₃, -CH₂CH₂CH₂CH₃, -CH₂CH(CH₃)₂, -CH₂CH₂OH, -CH₂CO₂CH₃, -CH₂C≡CH, 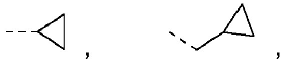 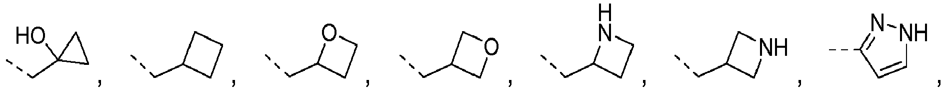 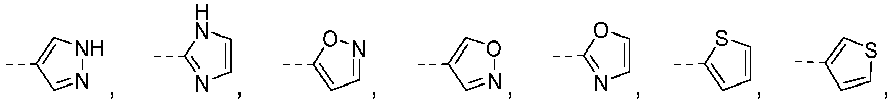 or

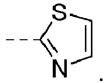

8. A compound of the general formula (I) 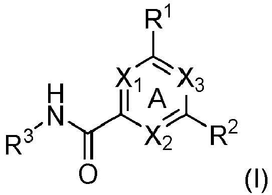 wherein
in a ring **A**, **X₁** is N or C**R⁴**; **X₂** is N or C**R⁵**; **X₃** is N or CH; with the proviso that at least one of **X₁** and **X₃** is N;
**R¹** represents -**R⁶**, -O**R⁷**, -S**R⁷**, -N**R⁸R⁹**, or -CON**R⁸R⁹**,
or **R¹** and **R⁴** form 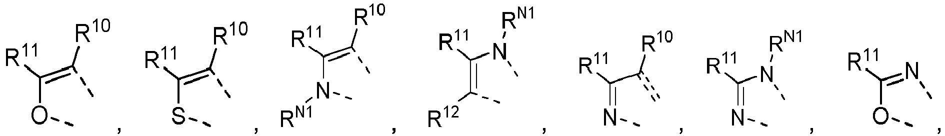 or 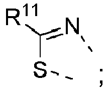
**R²** represents -**R¹⁶**, -O**R¹⁷**, -S**R¹⁷**, -N**R¹⁸R¹⁹**, -CON**R¹⁸R¹⁹**, or -N**R¹⁸**CH**R'**CO₂**R¹⁹**,
or **R²** and **R⁵** form 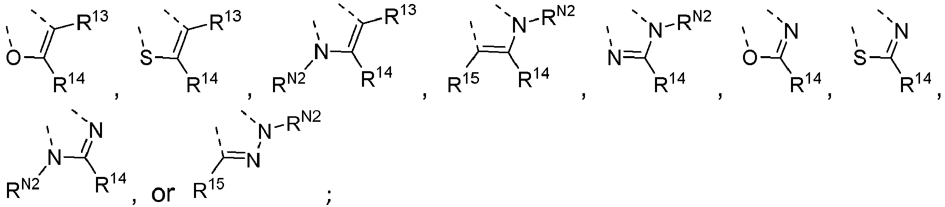
**R³** represents C₁-C₆ alkyl, C₁-C₆ alkyl-OH, C₁-C₆ alkyl-CO₂**R²⁰**, C₁-C₆ alkyl-CONH**R²⁰**, C₂-C₆ alkenyl, C₂-C₆ alkynyl, 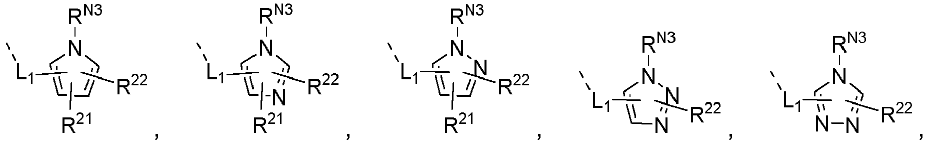 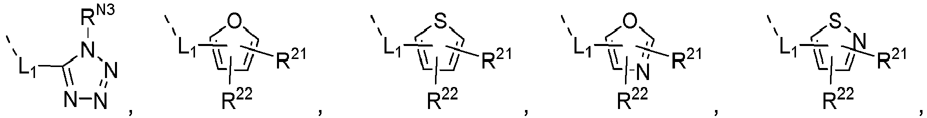 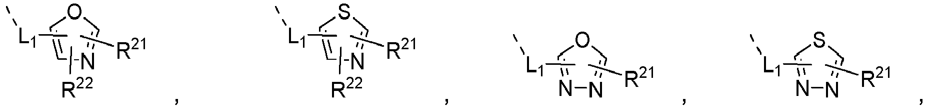 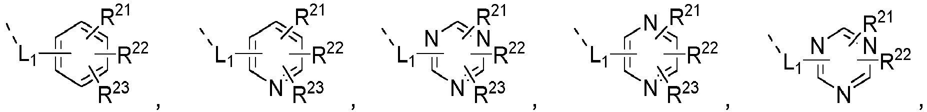 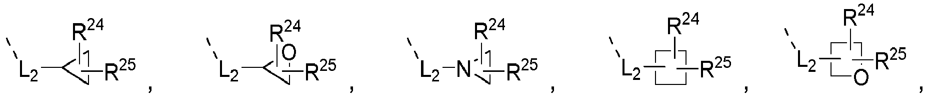 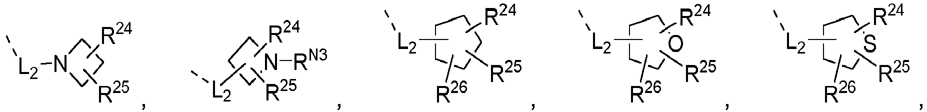 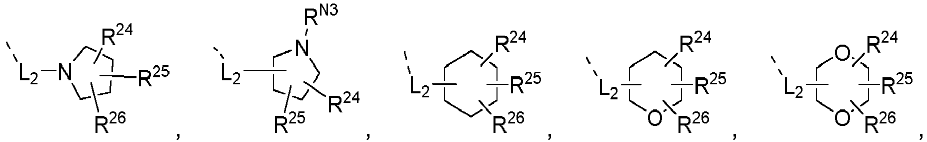 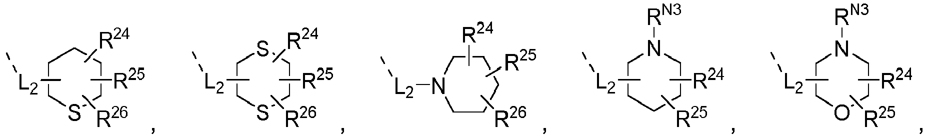 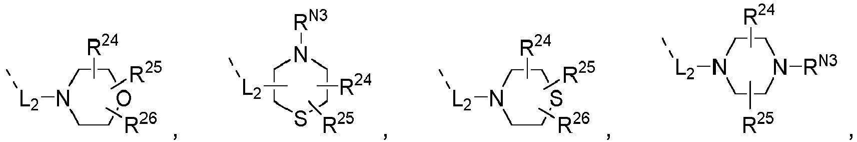 or 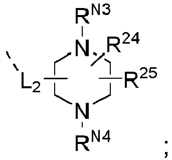
**R⁴** and **R⁵** represents -H, -F, -Cl, -Br, -I, C₁-C₃alkyl, C₁-C₃alkoxy, -CF₃, -OCF₂H or -OCF₃;
**R⁶**, **R⁷** and **R⁸** represent independently of each other, C₁-C₆ alkyl, C₃-C₆ cycloalkyl, -L₃-**R^{A}**, -L₄-**R^{B}**;
**R^{8'}**, **R⁹**, **R^{9'}** and **R¹¹** represent independently of each other -H, C₁-C₆ alkyl, C₃-C₆ cycloalkyl, -L₃-**R^{A}**, -L₄-**R^{B}**;
**R^{A}** and **R^{B}** represent independently of each other 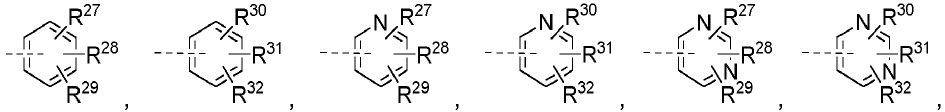 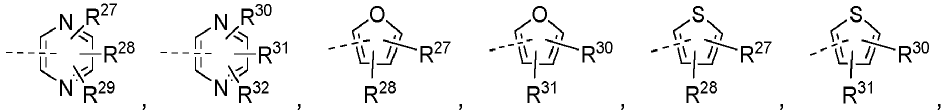 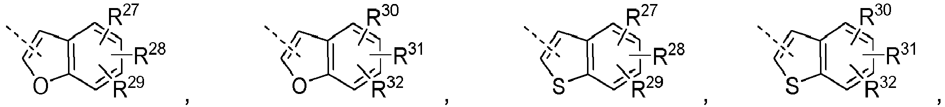 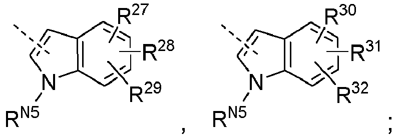
or -N**R⁸R⁹** represents 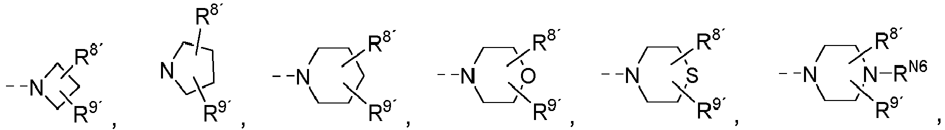 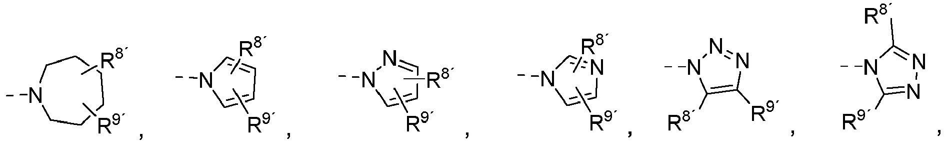 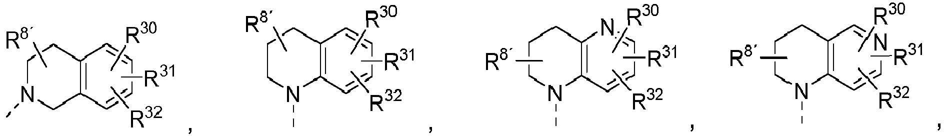 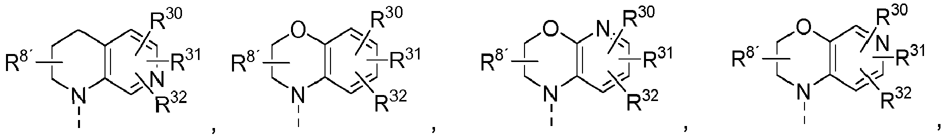 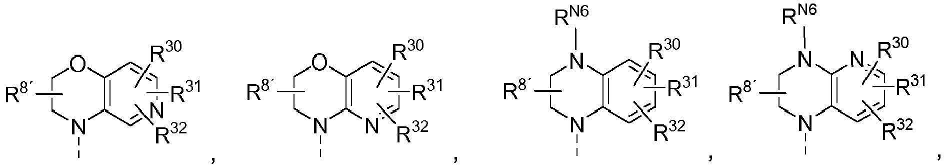 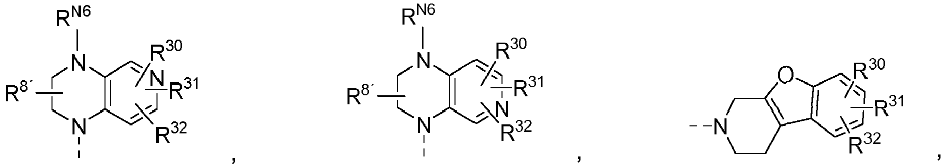 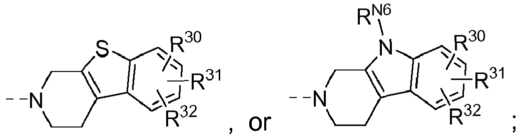
**R¹⁰**, **R¹²**, **R¹³**, **R¹⁴** and **R¹⁵** represent independently of each other -H, C₁-C₃ alkyl, or C₁-C3 alkoxy;
**R¹⁶**, **R¹⁷**, **R¹⁸** and **R¹⁹** represent independently of each other C₁-C₆ alkyl or C₃-C₆ cycloalkyl;
or -N**R¹⁸R¹⁹** represents 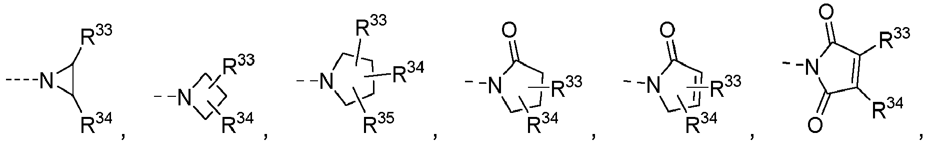 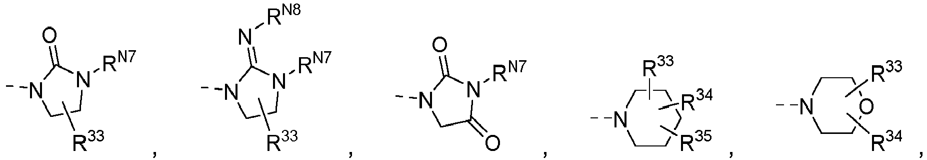 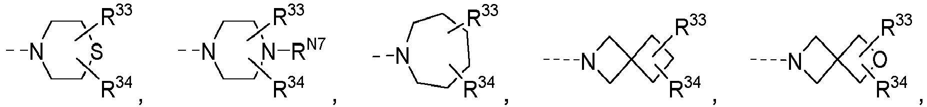 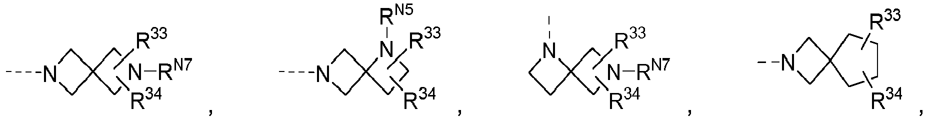 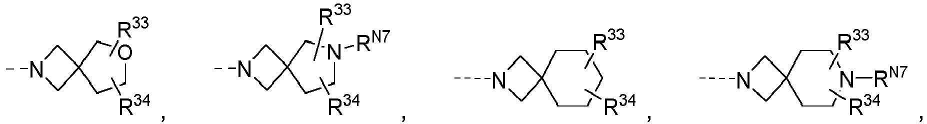 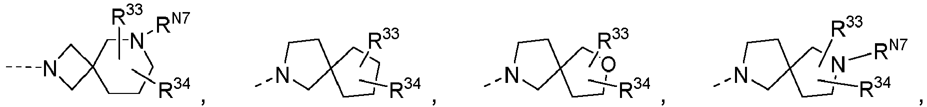 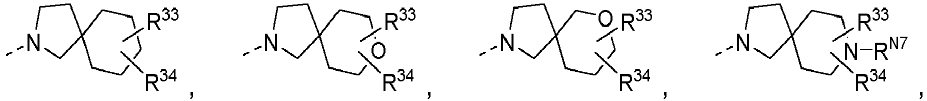 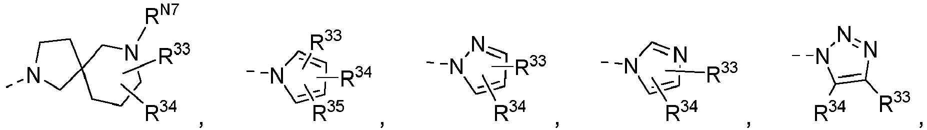 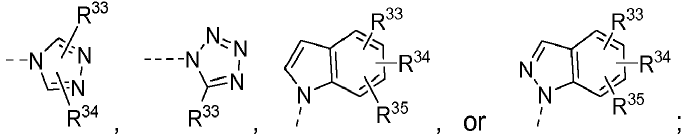
R represents -H, -CH₃, -CH₂OH, -CH₂SH, -CH₂CO₂H, -CH₂CONH₂, -CH(OH)CH₃, -CH(CH₃)₂, -CH₂CH(CH₃)₂, -CH(CH₃)CH₂CH₃, -CH₂CH₂SCH₃, -CH₂CH₂CO₂H, -CH₂CH₂CONH₂, -CH₂Ph, -CH₂CH₂CH₂NHC(=NH)NH₂, -CH₂CH₂CH₂CH₂NH₂, 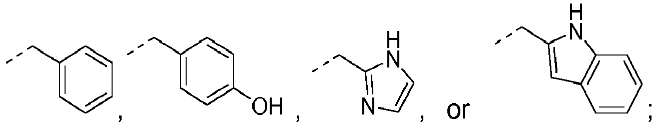
**L¹**, **L²**, **L³**, and **L⁴** represent independently of each other a covalent bond, C₁-C₆ alkyl group, linear C₂-C₆ alkenyl group, wherein one or more of the -CH₂-groups present in the C₁-C₆ alkyl or C₂-C₆ alkenyl group are optionally and independently of each other replaced by -O-, -S-, -N(R^{N9})-, -CO-, -COO-, -OOC-, -CON(R^{N9})-, -N(R^{N9})CO-, -NHCON(R^{N9})-, -N(R^{N9})CONH-, -SO-, -SO₂-, -SO₂N(R^{N9})-, or -N(R^{N9})SO₂-;
**R^{N1}**, **R^{N2}**, **R^{N3}**, **R^{N4}**, **R^{N5}**, **R^{N6}**, **R^{N7}**, **R^{N8}**, and **R^{N9}** represent independently of each other -H, -CH₃, -C₂H₅, -C₃H₇, -CH(CH₃)₂, -C₄H₉, -CH₂-CH(CH₃)₂, -CH(CH₃)-C₂H₅, -C(CH₃)₃, -cyclo-C₃H₅, -CH₂-cyclo-C₃H₅, -Ph, -CH₂Ph, -CH₂F, -CHF₂, -CF₃, -CH₂Cl, -CH₂Br, -CH₂I, -CH₂-CH₂F, -CH₂-CHF₂, -CH₂-CF₃, -CH₂-CH₂Cl, -CH₂-CH₂Br, -CH₂-CH₂I, -CH₂-CH=CH₂, -CH₂-C≡CH, -OH, -OCH₃, -CHO, -COCH₃, -COC₂H₅, -COC₃H₇, -COCH(CH₃)₂, -COC(CH₃)₃, -COOCH₃, -COOC₂H₅, -COOC₃H₇, -COOCH(CH₃)₂, -COOC(CH₃)₃, 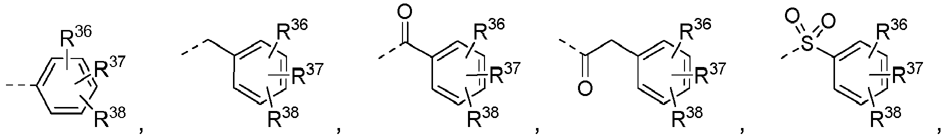 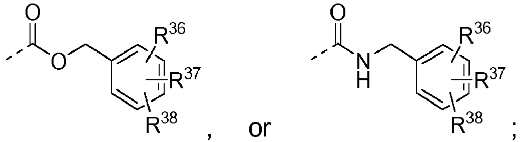
**R²⁰** represents -H, -CH₃, -C₂H₅, -C₃H₇, -CH(CH₃)₂, -C₄H₉, -CH₂-CH(CH₃)₂, -CH(CH₃)-C₂H₅, -C(CH₃)₃, -cyclo-C₃H₅, or -CH₂-cyclo-C₃H₅;
**R²¹**, **R²²**, **R²³**, **R²⁴**, R²⁵, **R**²⁶, **R²⁷**, **R²⁸**, **R²⁹**, **R³⁰**, **R³¹**, **R³²**, **R³³**, **R³⁴**, **R³⁵**, **R³⁶**, **R³⁷**, and **R³⁸** represent independently of each other -H, -OH, -OCH₃, -OC₂H₅, -OC₃H₇, -O-cyclo-C₃H₅, -OCH(CH₃)₂, -OC(CH₃)₃, -OC₄H₉, -OPh, -OCH₂-Ph, -OCPh₃, -CH₂-OCH₃, -C₂H₄-OCH₃, -C₃H₆-OCH₃, -CH₂-OC₂H₅, -C₂H₄-OC₂H₅, -C₃H₆-OC₂H₅, -CH₂-OC₃H₇, -C₂H₄-OC₃H₇, -C₃H₆-OC₃H₇, -CH₂-O-cyclo-C₃H₅, -C₂H₄-O-cyc!o-C₃H₅, -C₃H₆-O-cyclo-C₃H₅, -CH₂-OCH(CH₃)₂, -C₂H₄-OCH(CH₃)₂, -C₃H₆-OCH(CH₃)₂, -CH₂-OC(CH₃)₃, -C₂H₄-OC(CH₃)₃, -C₃H₆-OC(CH₃)₃ -CH₂-OC₄H₉, -C₂H₄-OC₄H₉, -C₃H₆-OC₄H₉, -CH₂-OPh, -C₂H₄-OPh, -C₃H₆-OPh, -CH₂-OCH₂-Ph, -C₂H₄-OCH₂-Ph, -C₃H₆-OCH₂-Ph, -SH, -SCH₃, -SC₂H₅, -SC₃H₇, -S-cyclo-C₃H₅, -SCH(CH₃)₂, -SC(CH₃)₃, -F, - Cl, -Br, -I, -CN, -COCH₃, -COC₂H₅, -COC₃H₇, -CO-cyclo-C₃H₅, -COCH(CH₃)₂, -COC(CH₃)₃, -COOH, -COOCH₃, -COOC₂H₅, -COOC₃H₇, -COO-cyclo-C₃H₅, -COOCH(CH₃)₂, -COOC(CH₃)₃, -OOC-CH₃, -OOC-C₂H₅, -OOC-C₃H₇, -OOC-cyclo-C₃H₅, -OOC-CH(CH₃)₂, -OOC-C(CH₃)₃, -CONH₂, -CONHCH₃, -CONHC₂H₅, -CONHC₃H₇, -CONH-cyclo-C₃H₅, -CONH[CH(CH₃)₂], -CONH[C(CH₃)₃], -CON(CH₃)₂, -CON(C₂H₅)₂, -CON(C₃H₇)₂, -CON(cyclo-C₃H₅)₂, -CON[CH(CH₃)₂]₂, -CON[C(CH₃)₃]₂, -NHCOCH₃, -NHCOC₂H₅, -NHCOC₃H₇, -NHCO-cyclo-C₃H₅, -NHCO-CH(CH₃)₂, -NHCO-C(CH₃)₃, -NHCO-OCH₃, -NHCO-OC₂H₅, -NHCO-OC₃H₇, -NHCO-O-cyclo-C₃H₅, -NHCO-OCH(CH₃)₂, -NHCO-OC(CH₃)₃, -NH₂, -NHCH₃, -NHC₂H₅, -NHC₃H₇, -NH-cyclo-C₃H₅, -NHCH(CH₃)₂, -NHC(CH₃)₃, -N(CH₃)₂, -N(C₂H₅)₂, -N(C₃H₇)₂, -N(cyclo-C₃H₅)₂, -N[CH(CH₃)₂]₂, -N[C(CH₃)₃]₂, -SOCH₃, -SOC₂H₅, -SOC₃H₇, -SO-cyclo-C₃H₅, -SOCH(CH₃)₂, -SOC(CH₃)₃, -SO₂CH₃, -SO₂C₂H₅, -SO₂C₃H₇, -SO₂-cyclo-C₃H₅, -SO₂CH(CH₃)₂, -SO₂C(CH₃)₃, -SO₃H, -SO₃CH₃, -SO₃C₂H₅, -SO₃C₃H₇, -SO₃-cyclo-C₃H₅, -SO₃CH(CH₃)₂, -SO₃C(CH₃)₃, -SO₂NH₂, -SO₂NHCH₃, -SO₂NHC₂H₅, -SO₂NHC₃H₇, -SO₂NH-cyclo-C₃H₅, -SO₂NHCH(CH₃)₂, -SO₂NHC(CH₃)₃, -SO₂N(CH₃)₂, -SO₂N(C₂H₅)₂, -SO₂N(C₃H₇)₂, -SO₂N(cyclo-C₃H₅)₂, -SO₂N[CH(CH₃)₂]₂, -SO₂N[C(CH₃)₃]₂, -O-S(=O)CH₃, -O-S(=O)C₂H₅, -O-S(=O)C₃H₇, -O-S(=O)-cyclo-C₃H₅, -O-S(=O)CH(CH₃)₂, -O-S(=O)C(CH₃)₃, -S(=O)(=NH)CH₃, -S(=O)(=NH)C₂H₅, -S(=O)(=NH)C₃H₇, -S(=O)(=NH)-cyclo-C₃H₅, -S(=O)(=NH)CH(CH₃)₂, -S(=O)(=NH)C(CH₃)₃, -NH-SO₂-CH₃, -NH-SO₂-C₂H₅, -NH-SO₂-C₃H₇, -NH-SO₂-cyclo-C₃H₅, -NH-SO₂-CH(CH₃)₂, -NH-SO₂-C(CH₃)₃, -O-SO₂-CH₃, -O-SO₂-C₂H₅, -O-SO₂-C₃H₇, -O-SO₂-cyclo-C₃H₅, -O-SO₂-CH(CH₃)₂, -O-SO₂-C(CH₃)₃, -OCH₂F, -OCHF₂ -OCF₃, -CH₂-OCF₃, -C₂H₄-OCF₃, -C₃H₆-OCF₃, -OC₂F₅, -CH₂-OC₂F₅, -C₂H₄-OC₂F₅, -C₃H₆-OC₂F₅, -O-COOCH₃, -O-COOC₂H₅, -O-COOC₃H₇, -O-COO-cyclo-C₃H₅, -O-COOCH(CH₃)₂, -O-COOC(CH₃)₃, -NH-CO-NH₂, -NH-CO-NHCH₃, -NH-CO-NHC₂H₅, -NH-CO-NHC₃H₇, -NH-C(=NH)-NH₂, -NH-CO-N(C₃H₇)₂, -NH-CO-NH[CH(CH₃)₂], -NH-CO-NH[C(CH₃)₃], -NH-CO-N(CH₃)₂, -NH-CO-N(C₂H₅)₂, -NH-CO-NH-cyclo-C₃H₅, -NH-CO-N(cyclo-C₃H₅)₂, -NH-CO-N[CH(CH₃)₂]₂, -NH-C(=NH)-NHCH₃, -NH-C(=NH)-NHC₂H₅, -NH-C(=NH)-NHC₃H₇, -O-CO-NH-cyclo-C₃H₅, -NH-C(=NH)-NH-cyclo-C₃H₅, -NH-C(=NH)-NH[CH(CH₃)₂], -O-CO-NH[CH(CH₃)₂], -NH-C(=NH)-NH[C(CH₃)₃], -NH-C(=NH)-N(CH₃)₂, -NH-C(=NH)-N(C₂H₅)₂, -NH-C(=NH)-N(C₃H₇)₂, -NH-C(=NH)-N(cyclo-C₃H₅)₂, -O-CO-NHC₃H₇, -NH-C(=NH)-N[CH(CH₃)₂]₂, -NH-C(=NH)-N[C(CH₃)₃]₂, -O-CO-NH₂, -O-CO-NHCH₃, -O-CO-NHC₂H₅, -O-CO-NH[C(CH₃)₃], -O-CO-N(CH₃)₂, -O-CO-N(C₂H₅)₂, -O-CO-N(C₃H₇)₂, -O-CO-N(cyclo-C₃H₅)₂, -O-CO-N[CH(CH₃)₂]₂, -O-CO-N[C(CH₃)₃]₂, -O-CO-OCH₃, -O-CO-OC₂H₅, -O-CO-OC₃H₇, -O-CO-O-cyclo-C₃H₅, -O-CO-OCH(CH₃)₂, - O-CO-OC(CH₃)₃, -CH₂F, -CHF₂, -CF₃, -CH₂-CH₂F, -CH₂-CHF₂, -CH₂-CF₃, cyclo-C₈H₁₅, -Ph, -CH₂-Ph, -CH₂-CH₂-Ph, -CH=CH-Ph, -CPh₃, -CH₃, -C₂H₅, -C₃H₇, -CH(CH₃)₂, -C₄H₉, -CH₂-CH(CH₃)₂, -CH(CH₃)-C₂H₅, -C(CH₃)₃, -C₅H₁₁, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, -C₂H₄-CH(CH₃)_{2J} -C₆H₁₃, -C₇H₁₅, -C₈H₁₇, -C₃H₆-CH(CH₃)₂, -C₂H₄-CH(CH₃)-C₂H₅, -CH(CH₃)-C₄H₉, -CH₂-CH(CH₃)-C₃H₇, -CH(CH₃)-CH₂-CH(CH₃)₂, -CH(CH₃)-CH(CH₃)-C₂H₅, -CH₂-CH(CH₃)-CH(CH₃)₂, -CH₂-C(CH₃)₂-C₂H₅, -C(CH₃)₂-C₃H₇, -C(CH₃)₂-CH(CH₃)₂, -C₂H₄-C(CH₃)₃, -CH(CH₃)-C(CH₃)₃, -CH=CH₂, -CH₂-CH=CH₂, -C(CH₃)=CH₂, -CH=CH-CH₃, -C₂H₄-CH=CH₂, -CH₂-CH=CH-CH₃, -CH=CH-C₂H₅, -CH₂-C(CH₃)=CH₂, -CH(CH₃)-CH=CH, -CH=C(CH₃)₂, -C(CH₃)=CH-CH₃, -CH=CH-CH=CH₂, -C₃H₆-CH=CH₂, -C₂H₄-CH=CH-CH₃, -CH₂-CH=CH-C₂H₅, -CH=CH-C₃H₇, -CH=CH-CH=CH-CH₃, -C₂H₄-C(CH₃)=CH₂, -CH₂-CH(CH₃)-CH=CH₂, -CH(CH₃)-CH₂-CH=CH₂, -CH₂-CH=C(CH₃)₂, -CH₂-C(CH₃)=CH-CH₃, -CH(CH₃)-CH=CH-CH₃, -CH=CH-CH(CH₃)₂, -CH=C(CH₃)-C₂H₅, -C(CH₃)=CH-C₂H₅, -C(CH₃)=C(CH₃)₂, -C(CH₃)₂-CH=CH₂, -CH(CH₃)-C(CH₃)=CH₂, -C₄H₃-CH=CH₂, -C₃H₆-CH=CH-CH₃, -C₂H₄-CH=CH-C₂H₅, -CH₂-CH=CH-C₃H₇, -CH=CH-C₄H₉, -C₃H₆-C(CH₃)=CH₂, -C₂H₄-CH(CH₃)-CH=CH₂, -CH₂-CH(CH₃)-CH₂-CH=CH₂, -C₂H₄-CH=C(CH₃)₂, -CH(CH₃)-C₂H₄-CH=CH₂, -C₂H₄-C(CH₃)=CH-CH₃, -CH₂-CH(CH₃)-CH=CH-CH₃, -CH(CH₃)-CH₂-CH=CH-CH₃, -CH₂-CH=CH-CH(CH₃)₂, -CH₂-CH=C(CH₃)-C₂H₅, -CH₂-C(CH₃)=CH-C₂H₅, -CH(CH₃)-CH=CH-C₂H₅, -CH=CH-CH₂-CH(CH₃)₂, -CH=CH-CH(CH₃)-C₂H₅, -CH=C(CH₃)-C₃H₇, -C(CH₃)=CH-C₃H₇, -CH₂-CH(CH₃)-C(CH₃)=CH₂, -C[C(CH₃)₃]=CH₂, -CH(CH₃)-CH₂-C(CH₃)=CH₂, -CH(CH₃)-CH(CH₃)-CH=CH₂, -CH=CH-C₂H₄-CH=CH₂, -C(CH₃)₂-CH₂-CH=CH₂, -CH₂-C(CH₃)=C(CH₃)₂, -CH(CH₃)-CH=C(CH₃)₂, -C(CH₃)₂-CH=CH-CH₃, -CH=CH-CH₂-CH=CH-CH₃, -CH(CH₃)-C(CH₃)=CH-CH₃, -CH=C(CH₃)-CH(CH₃)₂, -C(CH₃)=CH-CH(CH₃)₂, -C(CH₃)=C(CH₃)-C₂H₅, -CH=CH-C(CH₃)₃, -C(CH₃)₂-C(CH₃)=CH₂, -CH(C₂H₅)-C(CH₃)=CH₂, -C(CH₃)(C₂H₅)-CH=CH₂, -CH(CH₃)-C(C₂H₅)=CH₂, -CH₂-C(C₃H₇)=CH₂, -CH₂-C(C₂H₅)=CH-CH₃, -CH(C₂H₅)-CH=CH-CH₃, -C(C₄H₉)=CH₂, -C(C₃H₇)=CH-CH₃, -C(C₂H₅)=CH-C₂H₅, -C(C₂H₅)=C(CH₃)₂, -C[CH(CH₃)(C₂H₅)]=CH₂, -C[CH₂-CH(CH₃)₂]=CH₂, -C₂H₄-CH=CH-CH=CH₂, -CH₂-CH=CH-CH₂-CH=CH₂, -C₃H₆-C≡C-CH₃, -CH₂-CH=CH-CH=CH-CH₃, -CH=CH-CH=CH-C₂H₅, -CH(CH₃)-CH₂-C≡CH, -CH(CH₃)-C≡C-CH₃, -C₂H₄-CH(CH₃)-C≡CH, -CH=CH-CH=C(CH₃)₂, -CH₂-CH(CH₃)-CH₂-C≡CH, -CH=CH-C(CH₃)=CH-CH₃, -CH=C(CH₃)-CH=CH-CH₃, -CH₂-CH(CH₃)-C≡CH, -C(CH₃)=CH-CH=CH-CH₃, -C=CH, -C≡C-CH₃, -CH₂-C≡CH, -C₂H₄-C≡CH, -CH₂-C≡C-CH₃, -C≡C-C₂H₅, -C₃H₆-C≡CH, -C₂H₄-C≡C-CH₃, -CH₂-C≡C-C₂H₅, -C≡C-C₃H₇, -CH(CH₃)-C≡CH, -C₄H₈-C≡CH, -C₂H₄-C≡C-C₂H₅, -CH₂-C≡C-C₃H₇, -C≡C-C₄H₉, -C≡C-CH₂-CH(CH₃)₂, -CH(CH₃)-C₂H₄-C≡CH, -CH₂-CH(CH₃)-C≡C-CH₃, -C(CH₃)(C₂H₅)-C≡CH, -CH(CH₃)-CH₂-C≡C-CH₃, -CH(CH₃)-C≡C-C₂H₅, -CH₂-C≡C-CH(CH₃)₂, -C≡C-CH(CH₃)-C₂H₅, -CH₂-C≡C-C≡C-CH₃, -CH(C₂H₅)-C≡C-CH₃, -C(CH₃)₂-C≡C-CH₃, -CH(C₂H₅)-CH₂-C≡CH, -CH₂-CH(C₂H₅)-C≡CH, -C(CH₃)₂-CH₂-C≡CH, -CH₂-C(CH₃)₂-C≡CH, -CH(CH₃)-CH(CH₃)-C≡CH, -CH(C₃H₇)-C≡CH, -CH₂-CH(C≡CH)₂, -C≡C-C≡CH, -CH₂-C≡C-C≡CH, -C≡C-C≡C-CH₃, -CH(C≡CH)₂, -C₂H₄-C≡C-C≡CH, -CH₂-C≡C-CH₂-C≡CH, -C≡C-C₂H₄-C≡CH, -C≡C-C(CH₃)₃, -C≡C-CH₂-C≡C-CH₃, -C≡C-C≡C-C₂H₅, 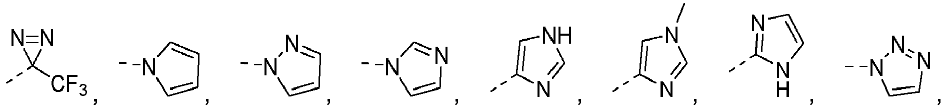 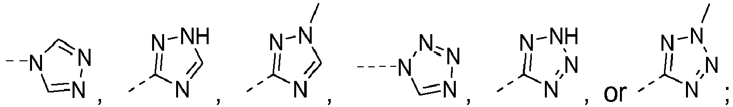
or tautomer, enantiomer, diastereomer, a mixture of tautomers, a mixture of enantiomers, a mixture of diastereomers, or a pharmaceutically acceptable salt thereof for use in the treatment or the prophylaxis of a disease or a disorder caused by or associated with the enzyme NAPE-PLD.

9. The compound for use according to Claim 8, wherein the disease or the disorder caused by or associated with the enzyme NAPE-PLD is selected from the group consisting of: neurodegenerative diseases, metabolic syndrome, insulin resistance, diabetes type II, obesity, liver steatosis, drug addiction, smoking cessation, mental health disorders, osteoporosis, traumatic brain injury, neuropathic and neuro-inflammatory pain, peripheral neuropathy in diabetes, endometriosis, eating disorders, and cancer.

10. The compound for use according to Claim 9, wherein the neurodegenerative disease is selected from the group consisting of:
Alzheimer's disease, Amyotrophic lateral sclerosis, Friedreich's ataxia, Huntington's disease, Lewy body disease, Parkinson's disease, Prion disease, Motor neuron diseases, Spinocerebellar ataxia, and Spinal muscular atrophy.

11. The compound for use according to Claim 9, wherein the cancer is selected from the group consisting of:
adenocarcinoma, choroidal melanoma, acute leukemia, acoustic neurinoma, ampullary carcinoma, anal carcinoma, astrocytoma, basal cell carcinoma, pancreatic cancer, desmoid tumor, bladder cancer, bronchial carcinoma, estrogen dependent and independent breast cancer, Burkitt's lymphoma, corpus cancer, CUP-syndrome (carcinoma of unknown primary), colorectal cancer, small intestine cancer, small intestinal tumors, ovarian cancer, endometrial carcinoma, ependymoma, epithelial cancer types, Ewing's tumors, gastrointestinal tumors, gastric cancer, gallbladder cancer, gall bladder carcinomas, uterine cancer, cervical cancer, glioblastomas, gynecologic tumors, ear, nose and throat tumors, hematologic neoplasias, hairy cell leukemia, urethral cancer, skin cancer, skin testis cancer, brain tumors (gliomas), brain metastases, testicle cancer, hypophysis tumor, carcinoids, Kaposi's sarcoma, laryngeal cancer, germ cell tumor, bone cancer, colorectal carcinoma, head and neck tumors (tumors of the ear, nose and throat area), colon carcinoma, craniopharyngiomas, oral cancer (cancer in the mouth area and on lips), cancer of the central nervous system, liver cancer, liver metastases, leukemia, eyelid tumor, lung cancer, lymph node cancer (Hodgkin's/Non-Hodgkin's lymphomas), lymphomas, stomach cancer, malignant melanoma, malignant neoplasia, malignant tumors gastrointestinal tract, breast carcinoma, rectal cancer, medulloblastomas, melanoma, meningiomas, Hodgkin's disease, mycosis fungoides, nasal cancer, neurinoma, neuroblastoma, kidney cancer, renal cell carcinomas, non-Hodgkin's lymphomas, oligodendroglioma, esophageal carcinoma, osteolytic carcinomas and osteoplastic carcinomas, osteosarcomas, ovarial carcinoma, pancreatic carcinoma, penile cancer, plasmocytoma, prostate cancer, pharyngeal cancer, rectal carcinoma, retinoblastoma, vaginal cancer, thyroid carcinoma, Schneeberger disease, esophageal cancer, spinalioms, T-cell lymphoma (mycosis fungoides), thymoma, tube carcinoma, eye tumors, urethral cancer, urologic tumors, urothelial carcinoma, vulva cancer, wart appearance, soft tissue tumors, soft tissue sarcoma, Wilm's tumor, cervical carcinoma, tongue cancer, invasive ductal carcinoma, invasive lobular carcinoma, ductal carcinoma in situ, lobular carcinoma in situ, small-cell lung carcinoma, non-small-cell lung carcinoma, bronchial adenoma, pleuropulmonary blastoma, mesothelioma, brain stem glioma, hypophtalmic glioma, cerebellar astrocytoma, cerebral astrocytoma, neuroectodermal tumours, pineal tumors, sarcoma of the uterus, salivary gland cancers, anal gland adenocarcinomas, mast cell tumors, pelvis tumours, ureter tumours, hereditary papillary renal cancers, sporadic papillary renal cancers, intraocular melanoma, hepatocellular carcinoma (liver cell carcinomas with or without fibrolamellar variant), cholangiocarcinoma (intrahepatic bile duct carcinoma), mixed hepatocellular cholangiocarcinoma, squamous cell carcinoma, malignant melanoma, Merkel cell skin cancer, non-melanoma skin cancer, hypopharyngeal cancer, nasopharyngeal cancer, oropharyngeal cancer, oral cavity cancer, squamous cell cancer, oral melanoma, AIDS-related lymphoma, cutaneous T-cell lymphoma, lymphoma of the central nervous system, malignant fibrous histiocytoma, lymphosarcoma, rhabdomyosarcoma, malignant histiocytosis, fibrosarcoma, hemangiosarcoma, hemangiopericytoma, leiomyosarcoma, canine mammary carcinoma, and feline mammary carcinoma.

12. A pharmaceutical composition comprising at least one compound of the general formula (I) defined in Claim 1 as an active ingredient, together with at least one pharmaceutically acceptable carrier, excipient and/or diluent.

13. A method for producing the compound of the formula (I) comprises:
**Step (0A):** providing a compound **1A** having an activated carboxylic group 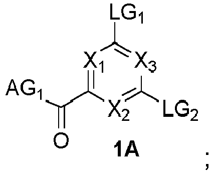
**Step 1A:** performing coupling reaction of the compound **1A** with an amine H₂N-R³ in the presence of a first base to obtain an intermediate compound **2A** 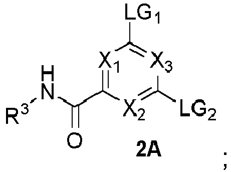
**Step 2A:** performing coupling reaction of the compound **2A** with H-R² in the presence of a second base to obtain an intermediate compound **3A** 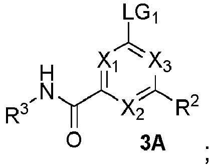
**Step 3A:** performing coupling reaction of the compound **3A** with H-R¹ in the presence of a third base and/or a catalyst to obtain a compound of the formula (I) 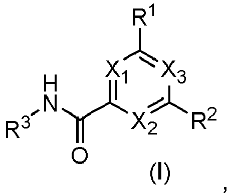 wherein LG₁ and LG₂ are leaving groups; AG₁ is an activating group; and X₁, X₂, X₃, R¹, R², R³ have the same meanings as defined in claim 1.

14. A method for producing the compound of the formula (I) comprises:
**Step (0B):** providing a compound **1B** having a protected carboxylic group 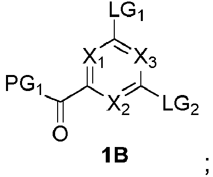
**Step 1B**: performing coupling reaction of the compound **1B** with H-R² in the presence of a first base to obtain an intermediate compound **2B** 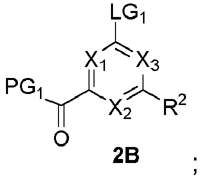
**Step 2B:**
i) deprotecting the carboxylic protecting group PG₁,
ii) performing coupling reaction of a resulting compound obtained by the step i) with an amine H₂N-R³ in the presence of a second base to obtain an intermediate compound **3B** 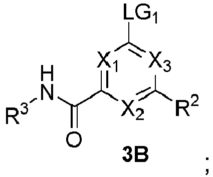
**Step 3C:** performing coupling reaction of the compound **3B** with H-R¹ in the presence of a third base and/or a catalyst to obtain a compound of the formula (I) 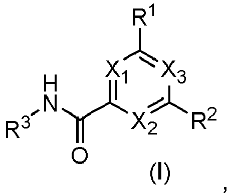 wherein LG₁ and LG₂ are leaving groups; PG₁ is a protectign group; and
X₁, X₂, X₃, R¹, R², R³ have the same meanings as defined in claim 1.

15. A method for producing the compound of the formula (I) comprises:
**Step (0B):** providing a compound **1B** having a protected carboxylic group 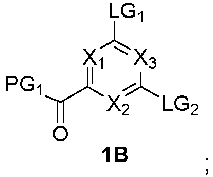
**Step 1C:** performing coupling reaction of the compound **1B** with H-R¹ in the presence of a first base and/or a catalyst to obtain an intermediate compound **2C** 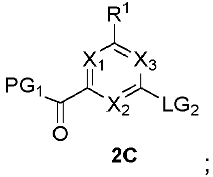
**Step 2C:**
i) deprotecting the carboxylic protecting group PG₁,
ii) performing coupling reaction of a resulting compound obtained by the step i) with an amine H₂N-R³ in the presence of a second base to obtain an intermediate compound **3C** 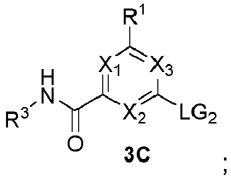
**Step 3C:** performing coupling reaction of the compound **3C** with H-R² in the presence of a third base to obtain a compound of the formula (I) 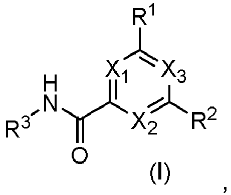 wherein LG₁ and LG₂ are leaving groups; PG₁ is a protectign group; and X₁, X₂, X₃, R¹, R², R³ have the same meanings as defined in claim 1.
